# EUROPEAN PATENT APPLICATION

(11) **EP 4 818 177 A1**
(43) Date of publication of application: **07.10.2026**
(21) Application number: 24896783.8
(22) Date of filing: 02.12.2024
(51) Int. Cl.: C07D 519/00, C07D 471/14, C07D 487/04, A61K 31/407, A61K 31/4353, A61P 35/00

(54) **TRICYCLIC DERIVATIVE INHIBITOR, AND PREPARATION METHOD AND USE THEREFOR**

(30) Priority: 01.12.2023 CN 202311642205; 12.01.2024 CN 202410052748; 06.02.2024 CN 202410172322; 23.02.2024 CN 202410205545; 29.02.2024 CN 202410232845; 03.04.2024 CN 202410405908; 15.04.2024 CN 202410453210; 31.05.2024 CN 202410705858; 20.06.2024 CN 202410806927; 27.06.2024 CN 202410855447; 03.07.2024 CN 202410890783; 26.07.2024 CN 202411022696; 16.08.2024 CN 202411138112; 27.09.2024 CN 202411369508
(71) Applicant: Shanghai Hansoh Biomedical Co., Ltd., Shanghai 201203 (CN); Jiangsu Hansoh Pharmaceutical Group Co., Ltd., Lianyungang, Jiangsu 222069 (CN)
(72) Inventor: WU, Jianrui, Shanghai 201203 (CN); GAO, Peng, Shanghai 201203 (CN); DONG, Jiaqiang, Shanghai 201203 (CN); ZHANG, Yang, Shanghai 201203 (CN); ZHENG, Lulu, Shanghai 201203 (CN)
(74) Representative: De Clercq & Partners
(86) International application number: PCT/CN2024/136098
(87) International publication number: WO 2025/113704

(57) **Abstract**

A tricyclic derivative inhibitor, and a preparation method and use therefor. In particular, the present invention relates to a tricyclic derivative compound, a preparation method therefor and a pharmaceutical composition containing the compound, and a use thereof in treating cancer.

## Description

### Technical Field

The present invention belongs to the field of drug synthesis and specifically relates to a tricyclic derivative inhibitor, and a preparation method therefor and the use thereof.

### Background Art

Protein arginine methyltransferases (PRMTs) are classified into three major types based on their catalytic activity and product types: Types I, II, and III. Type I mainly comprises PRMT1/2/3/4/6/8, which catalyse the formation of asymmetric dimethylarginine (ADMA) on substrates; Type II comprises PRMT5/9, which catalyse the formation of symmetric dimethylarginine (SDMA) on substrates; Type III comprises only PRMT7, which is responsible for catalysing the formation of monomethylarginine (MMA) on substrates. PRMT5 uses S-adenosyl-L-methionine (SAM) as a methyl donor to transfer methyl groups to substrates such as DNA, RNA and histones. Symmetric dimethylation of arginine residues on the substrates generates SDMA, thereby regulating multiple key cellular processes including transcription, translation and DNA repair to maintain cellular homeostasis. In addition, PRMT5 is also involved in regulating the growth and survival pathways of tumour cells and promoting tumourigenesis and progression. Elevated expression of PRMT5 has also been demonstrated to be associated with poor prognosis in a variety of cancers, making it a highly promising epigenetic target.

Methylthioadenosine phosphorylase (MTAP) catalyses the conversion of methylthioadenosine (MTA) to methionine, which is essential for maintaining normal cell functions. Deletion mutations in the MTAP gene lead to the accumulation of MTA in cells. MTA competes with PRMT5's substrate SAM, thereby reducing PRMT5 activity and resulting in abundant formation of PRMT5-MTA complexes. Deletion of the MTAP gene increases tumour dependency on PRMT5, and inhibition of PRMT5 in MTAP-deficient tumours can exert a "synthetic lethal" effect. PARP inhibitors based on the theory of "synthetic lethality" have achieved great success in the field of precision oncology. The MTAP gene is adjacent to CDKN2A, the most common tumour suppressor gene in human cancers, and is often co-deleted with CDKN2A. This co-deletion occurs in 10%-15% of all cancers, mainly in non-small cell lung cancer (12%-20%), glioma (53%), pancreatic cancer (30%), and DLBCL (20%), representing significant market potential.

Currently, no PRMT5 inhibitor has been approved for marketing. Early PRMT5 inhibitors were all non-selective, SAM-competitive inhibitors, which exhibited severe haematological toxicity and a narrow therapeutic window in clinical settings. The clinical progress of the first-generation PRMT5 inhibitors GSK-3326595, JNJ-64619178 and PF-06939999 has been suboptimal. The nextgeneration PRMT5 inhibitors targeting the PRMT5-MTA complex are effective specifically against MTAP-deficient, MTA-enriched tumours while exhibiting high selectivity for MTAP wild-type tumours. This mechanism reduces haematological toxicity, as has been validated in preclinical studies, and is expected to significantly widen the therapeutic window.

The present patent relates to a novel selective PRMT5-MTA inhibitor that exhibits activity specifically against MTAP-deficient cells while showing weak inhibition against MTAP wild-type cells, thereby avoiding the haematological toxicity and other side effects associated with the inhibition of MTAP wild-type cells by non-selective PRMT5 inhibitors in clinical settings. Highly selective PRMT5-MTA inhibitors, as novel PRMT5-MTA inhibitors, can be used for the treatment of various tumours, cancers and other diseases.

### Summary of the Invention

The objective of the present invention is to provide a compound represented by general formula (I), or a stereoisomer or a pharmaceutically acceptable salt thereof, wherein the compound represented by general formula (I) has the following structure: 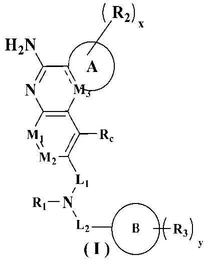
M₁ is selected from -N- or -CRₐ-;
M₂ is selected from -N- or -CR_{b}-;
M₃ is selected from N or C;
ring A is selected from C₃₋₈ cycloalkyl, 3- to 8-membered heterocyclyl, C₆₋₁₀ aryl or 5- to 10-membered heteroaryl; preferably C₃₋₆ cycloalkyl, 3- to 6-membered heterocyclyl, phenyl or 5- to 6-membered heteroaryl;
ring B is selected from C₃₋₁₄ cycloalkyl, 3- to 14-membered heterocyclyl, C₆₋₁₄ aryl or 5- to 14-membered heteroaryl; preferably C₃₋₁₂ cycloalkyl, 3- to 12-membered heterocyclyl, C₆₋₁₂ aryl or 5- to 12-membered heteroaryl; more preferably C₃₋₆ cycloalkyl, 3- to 6-membered heterocyclyl, C₆₋₁₀ fused cycloalkyl, 6- to 10-membered fused heterocyclyl, C₆₋₁₀ aryl or 5- to 10-membered heteroaryl;
L₁ is selected from a bond, -(CRₐₐR_{bb})ₘ₂-, -(CRₐₐR_{bb})ₘ₂C(O)-, - (CRₐₐR_{bb})ₘ₂C(S)-, -(CRₐₐR_{bb})ₘ₂C(NR_{cc})-, -(CRₐₐR_{bb})ₘ₂NR_{cc}C(O)-, - (CRₐₐR_{bb})ₘ₂S(O)ₘ₁-, -(CRₐₐR_{bb})ₘ₂NRₑₑ-, -(CR_{aaRbb})ₘ₂P(O)₂-, - (CRₐₐR_{bb})ₘ₂P(O)(OR_{cc})-, C₃₋₁₂ cycloalkylene, 3- to 12-membered heterocyclylene, C₆₋₁₂ arylene or 5- to 12-membered heteroarylene, wherein the C₃₋₁₂ cycloalkylene, 3- to 12-membered heterocyclylene, C₆₋₁₂ arylene and 5- to 12-membered heteroarylene are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl; preferably -CRₐₐR_{bb}-, - C(O)-, -S(O)ₘ₁- or NR_{cc};
L₂ is selected from a bond, -(CRₐₐR_{bb})ₘ₂-, -(CRₐₐR_{bb})ₘ₂C(O)-, - (CRₐₐR_{bb})ₘ₂NR_{cc}C(O), -(CRₐₐR_{db})ₘ₂S(O)ₘ₁- or -(CRₐₐR_{bb})ₘ₂NR_{cc}-; preferably - CRₐₐR_{bb}-, -C(O)-, -S(O)ₘ₁- or NR_{cc};
R₁ is selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, -(CR_{cc}R_{dd})ₙ₁-C₃₋₁₂ cycloalkyl, - (CR_{cc}R_{dd})ₙ₁-3- to 12-membered heterocyclyl, -(CR_{cc}R_{dd})ₙ₁-C₆₋₁₂ aryl, -(CR_{cc}R_{dd})ₙ₁-5- to 12-membered heteroaryl, -SF₅, -ORₑ, -NRₑR_{f}, -C(O)Rₑ, -C(O)ORₑ, - C(O)NRₑR_{f}, -N=S(O)RₑR_{f}, -S(O)Rₑ(=NR_{f}) or -P(O)RₑR_{f}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl and =CR_{gg}Rₕₕ; preferably hydrogen, deuterium, fluorine, chlorine, bromine, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ haloalkoxy, C₁₋₃ hydroxyalkyl, -(CR_{cc}R_{dd})ₙ₁-C₃₋₈ cycloalkyl, -(CR_{cc}R_{dd})ₙ₁-3- to 8-membered heterocyclyl, -(CR_{cc}R_{dd})ₙ₁-C₆₋₁₀ aryl, -(CR_{cc}R_{dd})ₙ₁-5- to 10-membered heteroaryl, -ORₑ, -NRₑR_{f}, -C(O)Rₑ, -C(O)NRₑR_{f} or -P(O)RₑR_{f}, wherein the amino, C₁₋₃ alkyl, C₂₋₆ alkenyl, C₂₋₆ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3-to 8-membered heterocyclyl, C₆₋₁₀ aryl and 5- to 10-membered heteroaryl are optionally substituted with one or more substituents selected from deuterium, halogen, amino, hydroxyl, cyano, nitro, C₁₋₃ alkyl, C₂₋₄ alkenyl, C₂₋₄ alkynyl, oxo, thio, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl;
R₂ is selected from hydrogen, deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl, -(CH₂)ₙ₂ORₑₑ, -(CH₂)ₙ₂NRₑₑR_{ff}, -(CH₂)ₙ₂C(O)Rₑₑ, -(CH₂)ₙ₂C(O)NRₑₑR_{ff}, -(CH₂)ₙ₂P(O)RₑₑR_{ff} or =CRₑₑR_{ff}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl; preferably hydrogen, deuterium, fluorine, chlorine, bromine, amino, hydroxyl, cyano, nitro, C₁₋₃ alkyl, C₂₋₄ alkenyl, C₂₋₄ alkynyl, oxo, thio, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl or 5- to 10-membered heteroaryl, wherein the amino, C₁₋₃ alkyl, C₂₋₆ alkenyl, C₂₋₆ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl are optionally substituted with one or more substituents selected from deuterium, halogen, amino, hydroxyl, cyano, nitro, C₁₋₃ alkyl, C₂₋₄ alkenyl, C₂₋₄ alkynyl, oxo, thio, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl;
R₃ is selected from hydrogen, deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, -Y₁-C₃₋₁₂ cycloalkyl, -Y₁-3- to 12-membered heterocyclyl, -Y₁-C₆₋₁₂ aryl, -Y₁-5- to 12-membered heteroaryl, -SF₅, -OR_{g}, -NR_{g}Rₕ, -C(O)R_{g}, -C(O)OR_{g}, -C(O)NR_{g}Rₕ, - N=S(O)R_{g}Rₕ, -S(O)R_{g}(=NRₕ), -P(O)R_{g}Rₕ, -C(=NRᵢ)NR_{g}Rₕ or =R_{g}Rₕ, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, substituted or unsubstituted C₃₋₁₂ cycloalkyl, substituted or unsubstituted 3- to 12-membered heterocyclyl, substituted or unsubstituted C₆₋₁₂ aryl, substituted or unsubstituted 5- to 12-membered heteroaryl and =CRₑR_{f}; preferably hydrogen, deuterium, fluorine, chlorine, bromine, amino, hydroxyl, cyano, nitro, C₁₋₃ alkyl, C₂₋₄ alkenyl, C₂₋₄ alkynyl, oxo, thio, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl or 5- to 10-membered heteroaryl, wherein the amino, C₁₋₃ alkyl, C₂₋₆ alkenyl, C₂₋₆ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl are optionally substituted with one or more substituents selected from deuterium, halogen, amino, hydroxyl, cyano, nitro, C₁₋₃ alkyl, C₂₋₄ alkenyl, C₂₋₄ alkynyl, oxo, thio, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl, 5- to 10-membered heteroaryl and =CRₑR_{f};
alternatively, any two R₃, together with adjacent atoms to which they are attached, form C₃₋₁₂ cycloalkyl, 3- to 12-membered heterocyclyl, C₆₋₁₂ aryl and 5- to 12-membered heteroaryl, which are optionally further substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl; preferably form C₃₋₈ cycloalkyl, 3- to 8-membered heterocyclyl, C₆₋₁₀ aryl and 5- to 10-membered heteroaryl, which are optionally further substituted with one or more substituents selected from deuterium, fluorine, chlorine, bromine, amino, hydroxyl, cyano, nitro, C₁₋₃ alkyl, C₂₋₄ alkenyl, C₂₋₄ alkynyl, oxo, thio, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl;
Rₐ, R_{b}, Rₑ, Rₑ and R_{f} are each independently selected from hydrogen, deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl, -(CH₂)ₙ₂ORₑₑ, - (CH₂)ₙ₂NRₑₑR_{ff}, -(CH₂)ₙ₂C(O)Rₑₑ, -(CH₂)ₙ₂C(O)ORₑₑ, -(CH₂)ₙ₂C(O)NRₑₑR_{ff}, - (CH₂)ₙ₂N=S(O)RₑₑR_{ff}, -(CH₂)ₙ₂S(O)Rₑₑ(=NR_{ff}) or -(CH₂)ₙ₂P(O)RₑₑR_{ff}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl and =CR_{gg}Rₕₕ; preferably hydrogen, deuterium, fluorine, chlorine, bromine, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, -C(O)-C₁₋₃ alkyl, C₃₋₈ cycloalkyl, 3- to 8-membered heterocyclyl, C₆₋₁₀ aryl or 5- to 10-membered heteroaryl, wherein the amino, C₁₋₃ alkyl, C₂₋₆ alkenyl, C₂₋₆ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl are optionally substituted with one or more substituents selected from deuterium, halogen, amino, hydroxyl, cyano, nitro, C₁₋₃ alkyl, C₂₋₄ alkenyl, C₂₋₄ alkynyl, oxo, thio, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl;
alternatively, Rₐ and R_{b} are connected to form C₃₋₁₂ cycloalkyl, 3- to 12-membered heterocyclyl, C₆₋₁₂ aryl and 5- to 12-membered heteroaryl, which are optionally further substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
Y₁ is selected from a bond, -O-, -S-, -C(O), -NRⱼ-, -C(O)NRⱼ-, -NRⱼC(O)-, - S(O)₂NRj-, -NRⱼS(O)₂-, C₁₋₆ alkylene, -O-C₁₋₆ alkylene-, -C₁₋₆ alkylene-O-, -NRⱼ-C₁₋₆ alkylene-, -C₁₋₆ alkylene-NRⱼ-, C₂₋₆ alkenylene or C₂₋₆ alkynylene, wherein the C₁₋₆ alkylene, C₂₋₆ alkenylene and C₂₋₆ alkynylene are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
R_{g}, Rₕ, Rᵢ and Rⱼ are each independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, -C(O)-C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl; preferably hydrogen, deuterium, fluorine, chlorine, bromine, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, -C(O)-C₁₋₃ alkyl, C₃₋₈ cycloalkyl, 3-to 8-membered heterocyclyl, C₆₋₁₀ aryl or 5- to 10-membered heteroaryl, wherein the amino, C₁₋₃ alkyl, C₂₋₆ alkenyl, C₂₋₆ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl are optionally substituted with one or more substituents selected from deuterium, halogen, amino, hydroxyl, cyano, nitro, C₁₋₃ alkyl, C₂₋₄ alkenyl, C₂₋₄ alkynyl, oxo, thio, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl;
Rₐₐ, R_{bb}, R_{cc} and R_{dd} are each independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, -C(O)-C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl; preferably hydrogen, deuterium, fluorine, chlorine, bromine, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, -C(O)-C₁₋₃ alkyl, C₃₋₈ cycloalkyl, 3-to 8-membered heterocyclyl, C₆₋₁₀ aryl or 5- to 10-membered heteroaryl, wherein the amino, C₁₋₃ alkyl, C₂₋₆ alkenyl, C₂₋₆ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl are optionally substituted with one or more substituents selected from deuterium, halogen, amino, hydroxyl, cyano, nitro, C₁₋₃ alkyl, C₂₋₄ alkenyl, C₂₋₄ alkynyl, oxo, thio, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl;
Rₑₑ and R_{ff} are each independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, -C(O)-C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl; preferably hydrogen, deuterium, fluorine, chlorine, bromine, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, -C(O)-C₁₋₃ alkyl, C₃₋₈ cycloalkyl, 3-to 8-membered heterocyclyl, C₆₋₁₀ aryl or 5- to 10-membered heteroaryl, wherein the amino, C₁₋₃ alkyl, C₂₋₆ alkenyl, C₂₋₆ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl are optionally substituted with one or more substituents selected from deuterium, halogen, amino, hydroxyl, cyano, nitro, C₁₋₃ alkyl, C₂₋₄ alkenyl, C₂₋₄ alkynyl, oxo, thio, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl;
R_{gg} and Rₕₕ are each independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, -C(O)-C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl; preferably hydrogen, deuterium, fluorine, chlorine, bromine, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, -C(O)-C₁₋₃ alkyl, C₃₋₈ cycloalkyl, 3-to 8-membered heterocyclyl, C₆₋₁₀ aryl or 5- to 10-membered heteroaryl, wherein the amino, C₁₋₃ alkyl, C₂₋₆ alkenyl, C₂₋₆ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl are optionally substituted with one or more substituents selected from deuterium, halogen, amino, hydroxyl, cyano, nitro, C₁₋₃ alkyl, C₂₋₄ alkenyl, C₂₋₄ alkynyl, oxo, thio, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl;
x is selected from 0, 1, 2, 3, 4, 5 or 6;
y is selected from 0, 1, 2, 3, 4, 5 or 6;
m1 is selected from 0, 1 or 2;
m2 is selected from 0, 1 or 2;
n1 is selected from 0, 1, 2, 3 or 4; and
n2 is selected from 0, 1, 2, 3 or 4.

In a preferred embodiment of the present invention, the compound, or the stereoisomer or the pharmaceutically acceptable salt thereof, is characterised in that the 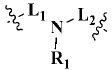 is selected from 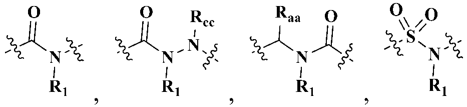 or

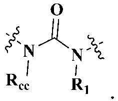

In a preferred embodiment of the present invention, the compound, or the stereoisomer or the pharmaceutically acceptable salt thereof, is characterised in that the compound is further as represented by general formula (II): 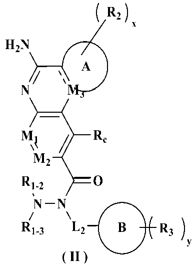
R₁₋₂ is selected from hydrogen, deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C6-12 aryl, 5- to 12-membered heteroaryl, -(CH₂)ₙ₂ORₑₑ, -(CH₂)ₙ₂NRₑₑR_{ff}, -(CH₂)ₙ₂C(O)Rₑₑ, -(CH₂)ₙ₂C(O)ORₑₑ, - (CH₂)ₙ₂C(O)NRₑₑR_{ff}, -(CH₂)ₙ₂N=S(O)RₑₑR_{ff}, -(CH₂)ₙ₂S(O)Rₑₑ(=NR_{ff}) or - (CH₂)ₙ₂P(O)RₑₑR_{ff}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl and =CR_{gg}Rₕₕ;
R₁₋₃ is selected from hydrogen, deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl, -(CH₂)ₙ₂ORₑₑ, -(CH₂)ₙ₂NRₑₑR_{ff}, -(CH₂)ₙ₂C(O)Rₑₑ, -(CH₂)ₙ₂C(O)ORₑₑ, - (CH₂)ₙ₂C(O)NRₑₑR_{ff}, -(CH₂)ₙ₂N=S(O)RₑₑR_{ff}, -(CH₂)ₙ₂S(O)Rₑₑ(=NR_{ff}) or - (CH₂)ₙ₂P(O)RₑₑR_{ff}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl and =CR_{gg}Rₕₕ;
alternatively, R₁₋₂ and R₁₋₃ are connected to form 3- to 10-membered heterocyclyl, wherein the 3- to 10-membered heterocyclyl is optionally substituted with one or more substitutents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl and =CR_{gg}Rₕₕ;
Rₑₑ and R_{ff} are each independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, -C(O)-C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
R_{gg} and Rₕₕ are each independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, -C(O)-C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
n2 is selected from 0, 1, 2, 3 or 4;
n3 is selected from 0, 1, 2, 3 or 4;
ring A, ring B, M₁, M₂, M₃, L₂, R₂, R₃, Rₑ, x and y are as defined in any of the preceding embodiments.

The present invention further provides a compound represented by general formula (III) or (III-1), or a stereoisomer or a pharmaceutically acceptable salt thereof, which has the following structure: 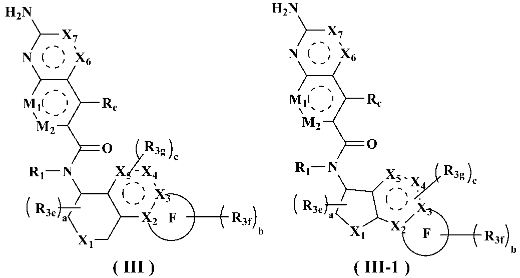
X₁ is selected from O or CH₂;
X₂ is selected from C or N;
X₃ is selected from C or N;
X₄ is selected from CH or N;
X₅ is selected from CH or N;
X₆ is selected from CR₄ₐ, NR₄ₐ or N; preferably CR₄ₐ or N;
X₇ is selected from CR_{4b}, NR_{4b} or N; preferably CR_{4b} or N;
alternatively, R₄ₐ and R_{4b} are connected to form ring A;
ring A is selected from C₃₋₈ cycloalkyl, 3- to 8-membered heterocyclyl, C₆₋₁₀ aryl or 5- to 10-membered heteroaryl, which is optionally substituted with 0, 1, 2, 3, 4, 5 or 6 R_{2c};
ring F is selected from C₃₋₁₀ cycloalkyl, 3- to 10-membered heterocyclyl, C₆₋₁₀ aryl or 5- to 10-membered heteroaryl;
R_{2c} is selected from hydrogen, deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl, -(CH₂)ₙ₂ORₑₑ, -(CH₂)ₙ₂NRₑₑR_{ff}, -(CH₂)ₙ₂C(O)Rₑₑ, -(CH₂)ₙ₂C(O)NRₑₑR_{ff}, -(CH₂)ₙ₂P(O)RₑₑR_{ff} or =CRₑₑR_{ff}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
R₃ₑ and R_{3g} are each independently selected from hydrogen, deuterium, halogen, amino, hydroxyl, cyano, nitro, C₁₋₃ alkyl, C₂₋₄ alkenyl, C₂₋₄ alkynyl, oxo, thio, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 10-membered heterocyclyl, C₆₋₁₀ aryl, 5- to 10-membered heteroaryl, -SF₅, -ORₐ₁, -NRₐ₁Rₐ₂, -C(O)Rₐ₁, - C(O)NRₐ₁Rₐ₂, -N=S(O)Rₐ₁Rₐ₂, -S(O)Rₐ₁(=NRₐ₂), -P(O)Rₐ₁Rₐ₂ or =Rₐ₁Rₐ₂, wherein the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 10-membered heterocyclyl, C₆₋₁₀ aryl and 5- to 10-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, substituted or unsubstituted C₃₋₁₂ cycloalkyl, substituted or unsubstituted 3- to 12-membered heterocyclyl, substituted or unsubstituted C₆₋₁₂ aryl, substituted or unsubstituted 5- to 12-membered heteroaryl and =CRₐ₃Rₐ₄;
alternatively, two R₃ₑ are connected to form C₃₋₁₀ cycloalkyl, 3- to 10-membered heterocyclyl, C₆₋₁₀ aryl or 5- to 10-membered heteroaryl, wherein the C₃₋₁₀ cycloalkyl, 3- to 10-membered heterocyclyl, C₆₋₁₀ aryl and 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -SF₅, =N-ORₐ₅, =CRₐ₅CRₐ₆, -S(O)Rₐ₅(=NRₐ₆) and -N=S(O)Rₐ₅Rₐ₆;
R_{3f} is selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, -(CR_{b1}R_{b2})ₙ₆-C₃₋₈ cycloalkyl, -(CR_{b1}R_{b2})ₙ₆-3- to 8-membered heterocyclyl, -(CR_{b1}R_{b2})ₙ₆-C₆₋₁₀ aryl, -(CR_{b1}R_{b2})ₙ₆-5- to 10-membered heteroaryl, -SF₅, -OR_{b1}, -NR_{b1}R_{b2}, -C(O)R_{b1}, - C(O)NR_{b1}R_{b2}, =N-OR_{b1}, =CR_{b1}CR_{b2}, -S(O)R_{b1}(=NR_{b2}) and -N=S(O)R_{b1}R_{b2}, wherein the hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl and =CR_{b3}CR_{b4}; preferably hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -SF₅, -OR_{b1}, -NR_{b1}R_{b2}, -C(O)R_{b1}, - C(O)NR_{b1}R_{b2}, =N-OR_{b1}, =CR_{b1}CR_{b2}, -S(O)R_{b1}(=NR_{b2}) and -N=S(O)R_{b1}R_{b2};
alternatively, two R_{3f} are connected to form C₃₋₁₀ cycloalkyl, 3- to 10-membered heterocyclyl, C₆₋₁₀ aryl or 5- to 10-membered heteroaryl, wherein the C₃₋₁₀ cycloalkyl, 3- to 10-membered heterocyclyl, C₆₋₁₀ aryl and 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R₄ₐ is selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl or 5- to 14-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R_{4b} is selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, 5- to 14-membered heteroaryl, -OR_{c1}, -C(O)R_{c1}, -C(O)OR_{c1}, -NR_{c1}R_{c2}, -P(O)R_{c1}R_{c2}, - NR_{c3}C(O)R_{c1}, -C(O)NR_{c1}R_{c2}, -S(O)₂R_{c1}, -S(O)₂NR_{c1}R_{c2}, -NR_{c3}S(O)₂R_{c1}, - S(O)R_{c1}(=NR_{c3}) or -N=S(O)R_{c1}R_{c2}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
Rₐ₁, Rₐ₂, Rₐ₃, Rₐ₄, Rₐ₅ and Rₐ₆ are each independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl or 5- to 14-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R_{b1}, R_{b2}, R_{b3} and R_{b4} are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl or 5- to 14-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R_{c1}, R_{c2} and R_{c3} are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl or 5- to 14-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
a is selected from 0, 1, 2, 3 or 4;
b is selected from 0, 1, 2, 3 or 4;
c is selected from 0, 1, 2, 3 or 4; and
n6 is selected from 0, 1, 2, 3 or 4;
M₁, M₂, R₁, R_{c}, Rₑₑ, R_{ff} and n2 are as defined in any of the preceding embodiments.

In a preferred embodiment of the present invention, the compound, or the stereoisomer or the pharmaceutically acceptable salt thereof, is characterised in that the compound is further as represented by general formula (IV) or (IV-1): 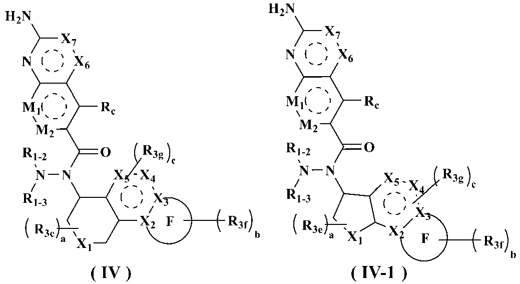
R₁₋₂ is selected from hydrogen, deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C6-12 aryl, 5- to 12-membered heteroaryl, -(CH₂)ₙ₂ORₑₑ, -(CH₂)ₙ₂NRₑₑR_{ff}, -(CH₂)ₙ₂C(O)Rₑₑ, -(CH₂)ₙ₂C(O)ORₑₑ, - (CH₂)ₙ₂C(O)NRₑₑR_{ff}, -(CH₂)ₙ₂N=S(O)RₑₑR_{ff}, -(CH₂)ₙ₂S(O)Rₑₑ(=NR_{ff}) or - (CH₂)ₙ₂P(O)RₑₑR_{ff}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl and =CR_{gg}Rₕₕ;
R₁₋₃ is selected from hydrogen, deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl, -(CH₂)ₙ₂ORₑₑ, -(CH₂)ₙ₂NRₑₑR_{ff}, -(CH₂)ₙ₂C(O)Rₑₑ, -(CH₂)ₙ₂C(O)ORₑₑ, - (CH₂)ₙ₂C(O)NRₑₑR_{ff}, -(CH₂)ₙ₂N=S(O)RₑₑR_{ff}, -(CH₂)ₙ₂S(O)Rₑₑ(=NR_{ff}) or - (CH₂)ₙ₂P(O)RₑₑR_{ff}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl and =CR_{gg}Rₕₕ;
alternatively, R₁₋₂ and R₁₋₃ are connected to form 3- to 10-membered heterocyclyl, wherein the 3- to 10-membered heterocyclyl is optionally substituted with one or more substitutents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl and =CR_{gg}Rₕₕ;
ring F, M₁, M₂, M₃, X₁, X₂, X₃, X₄, X₅, X₆, X₇, R₃ₑ, R_{3g}, R_{3f}, R₁₋₂, R₁₋₃, R_{c}, Rₑₑ, R_{ff}, R_{gg}, Rₕₕ, x, a, b, c and n2 are as defined in any of the preceding embodiments.

In a preferred embodiment of the present invention, the compound, or the stereoisomer or the pharmaceutically acceptable salt thereof, is characterised in that the compound is further as represented by general formula (V) or (V-1): 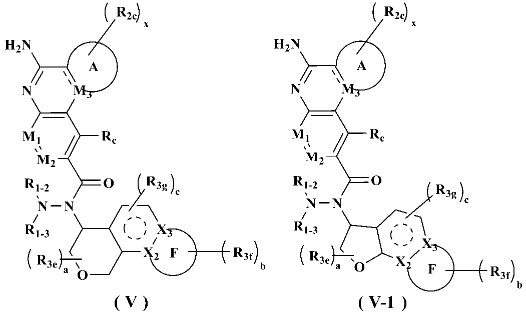 ring A, ring F, X₂, X₃, M₁, M₂, M₃, R_{2c}, R₃ₑ, R_{3g}, R_{3f}, R₁₋₂, R₁₋₃, R_{c}, x, a, b and c are as defined in any of the preceding embodiments.

In a preferred embodiment of the present invention, the compound, or the stereoisomer or the pharmaceutically acceptable salt thereof, is characterised in that ring A is selected from 3- to 12-membered heterocyclyl or 5- to 12-membered heteroaryl; preferably 5-membered heterocyclyl, 6-membered heterocyclyl, 5-membered heteroaryl or 6-membered heteroaryl; more preferably 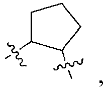 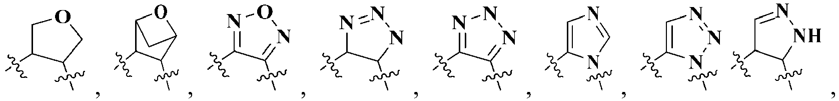 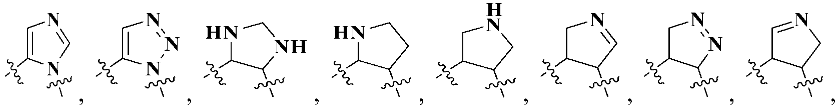 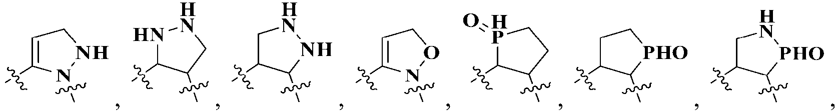 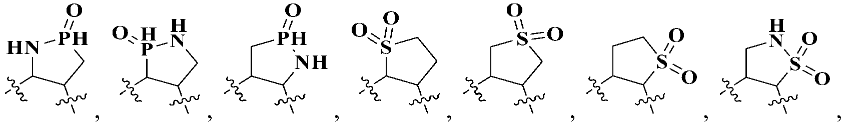 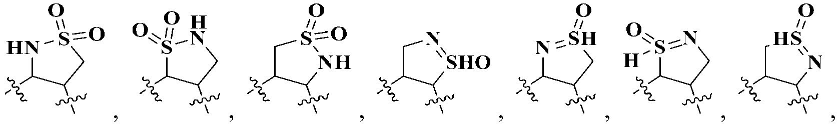 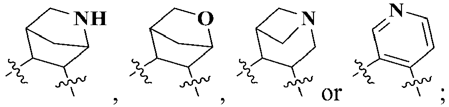 further preferably,

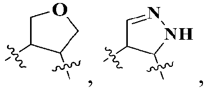

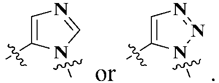

In a preferred embodiment of the present invention, the compound, or the stereoisomer or the pharmaceutically acceptable salt thereof, is characterised in that the compound is further as represented by general formula (V-2): 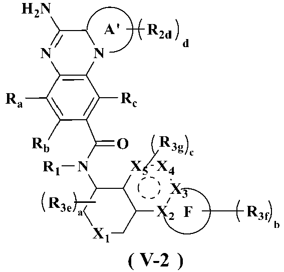
ring A' is selected from 3- to 8-membered heterocyclyl or 5- to 10-membered heteroaryl; preferably 5-membered heterocyclyl, 6-membered heterocyclyl, 5-membered heteroaryl or 6-membered heteroaryl;
R_{2d} is selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, 5- to 14-membered heteroaryl, -(CH₂)ₙ₂ORₑₑ, -(CH₂)ₙ₂NRₑₑR_{ff}, -(CH₂)ₙ₂C(O)Rₑₑ, - (CH₂)ₙ₂C(O)NRₑₑR_{ff}, -(CH₂)ₙ₂P(O)RₑₑR_{ff} or =CRₑₑR_{ff}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
Rₑₑ and R_{ff} are each independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, -C(O)-C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
   n2 is selected from 0, 1, or 2;
   d is selected from 0, 1, 2, 3 or 4;
   Rₐ, R_{b}, R_{c} and R₁, ring F, X₁, X₂, X₃, X₄, X₅, R₃ₑ, R_{3f}, R_{3g}, a, b and c are as defined in any of the preceding embodiments.

In a preferred embodiment of the present invention, the compound, or the stereoisomer or the pharmaceutically acceptable salt thereof, is characterised in that the compound is further as represented by general formula (VI):

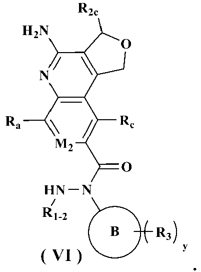

In a preferred embodiment of the present invention, the compound, or the stereoisomer or the pharmaceutically acceptable salt thereof, is characterised in that ring B is selected from 3- to 6-membered heterocyclyl fused phenyl or 3- to 6-membered heterocyclyl fused 5- to 6-membered heteroaryl; preferably

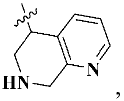

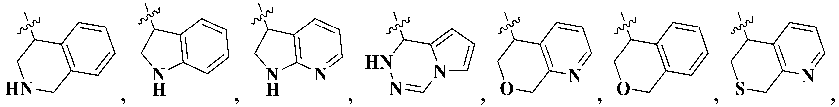

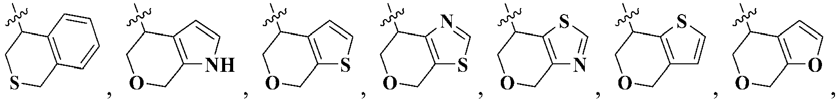

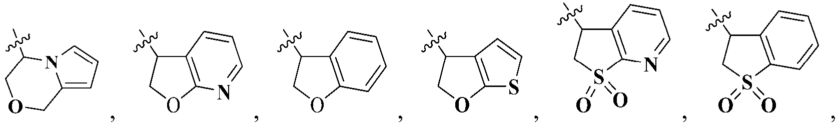

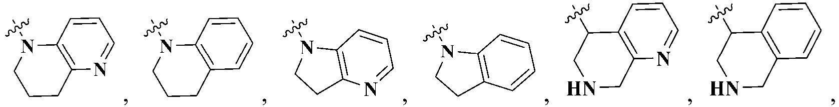

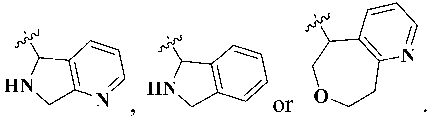

In a preferred embodiment of the present invention, the compound, or the stereoisomer or the pharmaceutically acceptable salt thereof, is characterised in that ring B is 6- to 14-membered tricyclic heterocyclyl; preferably 6- to 14-membered tricyclic spiro heterocyclyl or 6- to 14-membered tricyclic fused heterocyclyl; more preferably

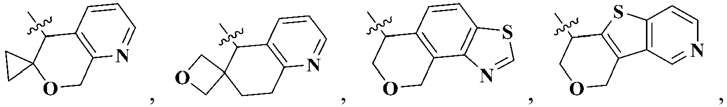

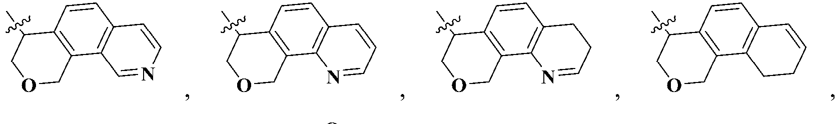

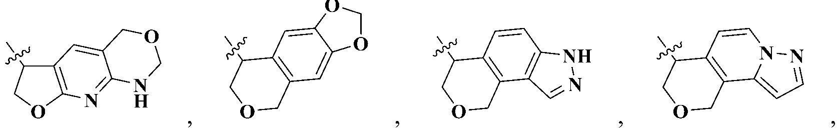

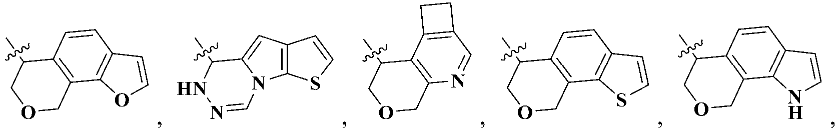

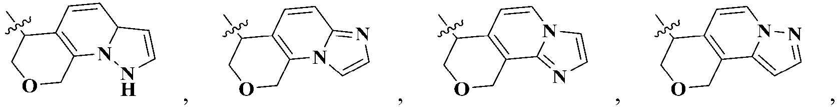

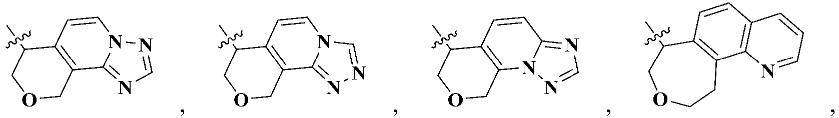

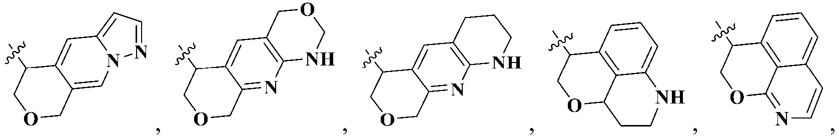

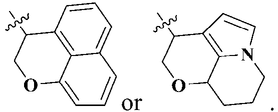

In a preferred embodiment of the present invention, the compound, or the stereoisomer or the pharmaceutically acceptable salt thereof, is characterised in that the compound is further as represented by general formula (VI-A) or (VI-B): 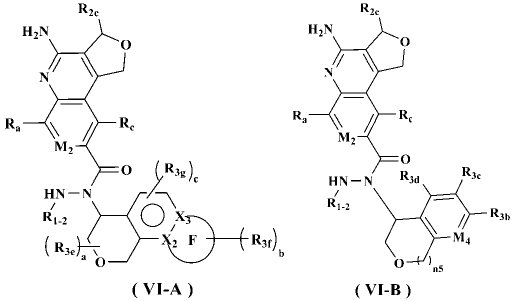
M₄ is selected from N or R₃ₐ;
X₂ and X₃ are selected from C or N;
R₃ₐ, R_{3c} and R_{3d} are each independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl,C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl and -SF₅, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy,C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, substituted or unsubstituted C₃₋₁₂ cycloalkyl, substituted or unsubstituted 3- to 12-membered heterocyclyl, substituted or unsubstituted C₆₋₁₂ aryl, substituted or unsubstituted 5- to 12-membered heteroaryl and =CRₑR_{f};
R_{3b} is selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl and -SF₅, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, substituted or unsubstituted C₃₋₁₂ cycloalkyl, substituted or unsubstituted 3- to 12-membered heterocyclyl, substituted or unsubstituted C₆₋₁₂ aryl, substituted or unsubstituted 5-to 12-membered heteroaryl and =CRₑR_{f};
n5 is selected from 0, 1 or 2.

In a preferred embodiment of the present invention, the compound, or the stereoisomer or the pharmaceutically acceptable salt thereof, is characterised in that each R₂ or R_{2c} is independently selected from hydrogen, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, cyano-substituted C₁₋₃ alkyl, C₃₋₈ cycloalkyl or -C(O)NRₑₑR_{ff}, wherein the amino, C₁₋₃ alkyl, C₂₋₄ alkenyl, C₂₋₄ alkynyl, C₁₋₃ deuteroalkyl, C₁₋₃ haloalkyl, cyano-substituted C₁₋₃ alkyl and C₃₋₈ cycloalkyl are optionally substituted with one or more substituents selected from deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, cyano-substituted C₁₋₃ alkyl and C₃₋₈ cycloalkyl;
Rₑₑ and R_{ff} are each independently selected from hydrogen, deuterium, C₁₋₃ alkyl, C₁₋₃ deuteroalkyl, C₁₋₃ haloalkyl, C₁₋₃ alkoxy, C₁₋₃ haloalkoxy, C₁₋₃ hydroxyalkyl or cyano-substituted C₁₋₃ alkyl;
preferably hydrogen, deuterium, fluorine, chlorine, methyl, deuterated methyl, difluoromethyl, trifluoromethyl,

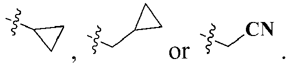

In a preferred embodiment of the present invention, the compound, or the stereoisomer or the pharmaceutically acceptable salt thereof, is characterised in that Rₐ is selected from hydrogen, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl or cyano-substituted C₁₋₃ alkyl; preferably hydrogen, deuterium, fluorine, chlorine, methyl, deuterated methyl, difluoromethyl or trifluoromethyl.

In a preferred embodiment of the present invention, the compound, or the stereoisomer or the pharmaceutically acceptable salt thereof, is characterised in that R_{b} is selected from hydrogen, deuterium, halogen, cyano, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₈ cycloalkyl, 3- to 8-membered heterocyclyl, --(CH₂)ₙ₂ORₑₑ, -(CH₂)ₙ₂NRₑₑR_{ff}, -(CH₂)ₙ₂C(O)Rₑₑ, -(CH₂)ₙ₂C(O)ORₑₑ, -(CH₂)ₙ₂C(O)NRₑₑR_{ff}, -(CH₂)ₙ₂N=S(O)RₑₑR_{ff}, -(CH₂)ₙ₂S(O)Rₑₑ(=NR_{ff}) or - (CH₂)ₙ₂P(O)RₑₑR_{ff}, wherein the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₈ cycloalkyl and 3- to 8-membered heterocyclyl are optionally substituted with one or more substituents selected from deuterium, halogen, amino, hydroxyl, cyano, nitro, C₁₋₃ alkyl, C₂₋₄ alkenyl, C₂₋₄ alkynyl, oxo, thio, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl and =CR_{gg}Rₕₕ; preferably hydrogen, deuterium, halogen, cyano, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₈ cycloalkyl, 3- to 8-membered heterocyclyl, -(CH₂)ₙ₂ORₑₑ, -(CH₂)ₙ₂NRₑₑR_{ff}, - (CH₂)ₙ₂C(O)Rₑₑ, -(CH₂)ₙ₂C(O)NRₑₑR_{ff} or -(CH₂)ₙ₂P(O)RₑₑR_{ff}, wherein the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₈ cycloalkyl and 3- to 8-membered heterocyclyl are optionally substituted with one or more substituents selected from deuterium, halogen, amino, hydroxyl, cyano, nitro, C₁₋₃ alkyl, C₂₋₄ alkenyl, C₂₋₄ alkynyl, oxo, thio, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl or 3- to 8-membered heterocyclyl;
Rₑₑ and R_{ff} are each independently selected from hydrogen, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, -C(O)-C₁₋₃ alkyl, C₃₋₈ cycloalkyl or 3- to 8-membered heterocyclyl, wherein the amino, C₁₋₃ alkyl, C₂₋₆ alkenyl, C₂₋₆ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl and 3- to 8-membered heterocyclyl are optionally substituted with one or more substituents selected from deuterium, halogen, amino, hydroxyl, cyano, nitro, C₁₋₃ alkyl, C₂₋₄ alkenyl, C₂₋₄ alkynyl, oxo, thio, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl;
n2 is selected from 0, 1 or 2;
preferably hydrogen, deuterium, fluorine, chlorine, cyano, methyl, ethyl, ethynyl, propynyl, deuterated methyl, deuterated ethyl, difluoromethyl, trifluoromethyl, trifluoroethyl, methoxy, ethoxy, deuterated methoxy, difluoromethoxy, trifluoromethoxy, 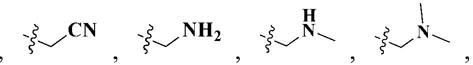 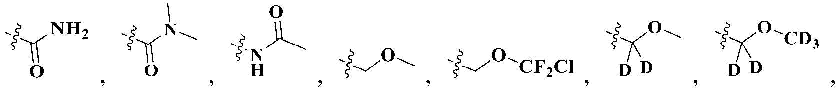 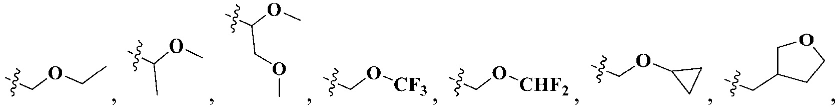 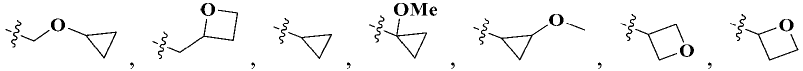 or

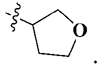

In a preferred embodiment of the present invention, the compound, or the stereoisomer or the pharmaceutically acceptable salt thereof, is characterised in that R₃ and R_{3f} are each independently selected from hydrogen, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₈ cycloalkyl, 3- to 8-membered heterocyclyl, C₆₋₁₀ aryl, 5-to 10-membered heteroaryl or -SF₅, wherein the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
R₃ₑ and R_{3g} are each independently selected from hydrogen, deuterium, halogen, amino, hydroxyl, cyano, nitro, C₁₋₃ alkyl, C₂₋₄ alkenyl,C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₈ cycloalkyl, 3- to 8-membered heterocyclyl, C₆₋₁₀ aryl or 5- to 10-membered heteroaryl, wherein the amino, C₁₋₃ alkyl,C₂₋₄ alkenyl,C₂₋₄ alkynyl,C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy,C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl.

The present invention further provides a compound represented by general formula (VII), or a stereoisomer or a pharmaceutically acceptable salt thereof, which has the following structure: 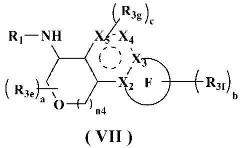 preferably, the general formula (VII) is further as represented by formula (VII-1): 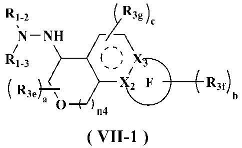
n4 is selected from 0 or 1;
X₂, X₃, R₁₋₂, R₁₋₃, R₃ₑ, R_{3f}, R_{3g}, a, b, c and n4 are as defined in any of the preceding embodiments.

The present invention further provides a method for preparing a compound represented by general formula (III), or a stereoisomer or a pharmaceutically acceptable salt thereof, wherein 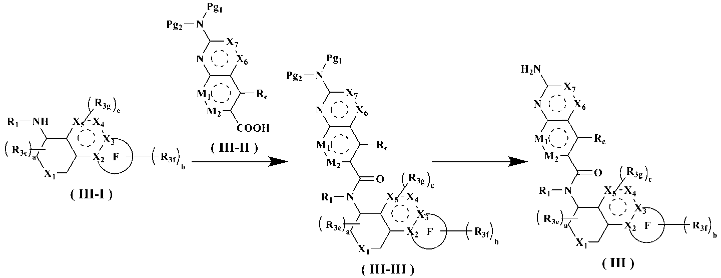
a compound represented by general formula (III-I) and a compound represented by general formula (III-II) are reacted in the presence of a condensing agent and a base to obtain a compound represented by general formula (III-III), which is further deprotected to obtain the compound represented by general formula (III);
preferably, the method is a method for preparing a compound represented by general formula (IV), or a stereoisomer or a pharmaceutically acceptable salt thereof: 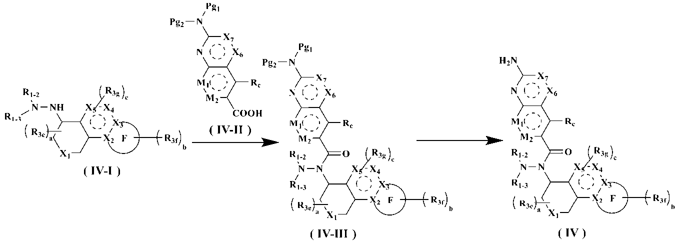
a compound represented by general formula (IV-I) and a compound represented by general formula (IV-II) are reacted in the presence of a condensing agent and a base to obtain a compound represented by general formula (IV-III), which is further deprotected to obtain the compound represented by general formula (IV);
more preferably, the method is a method for preparing a compound represented by general formula (V), or a stereoisomer or a pharmaceutically acceptable salt thereof: 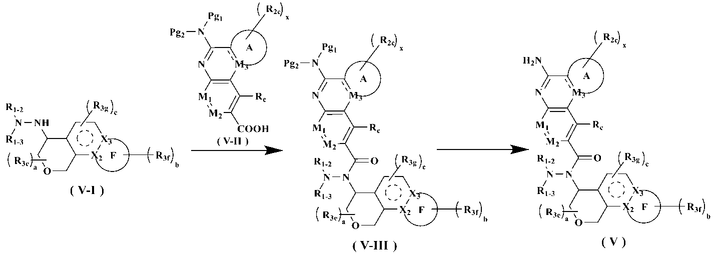
a compound represented by general formula (V-I) and a compound represented by general formula (V-II) are reacted in the presence of a condensing agent and a base to obtain a compound represented by general formula (V-III), which is further deprotected to obtain the compound represented by general formula (V);
Pg₁ is selected from hydrogen, allyloxycarbonyl, trifluoroacetyl, tert-butylsulphinyl, 2,4-dimethoxybenzyl, 3,5-dimethoxybenzyl, nitrobenzenesulphonyl, trityl, fluorenylmethoxycarbonyl, 9-fluorenylmethoxycarbonyl, benzyl, p-toluenesulphonyl, p-methoxybenzyl, formate, acetyl, benzyloxycarbonyl, phthaloyl, tert-butyloxycarbonyl, benzyl or p-methoxyphenyl; preferably benzyl, p-methoxybenzyl or tert-butyloxycarbonyl;
Pg₂ is selected from hydrogen, allyloxycarbonyl, trifluoroacetyl, tert-butylsulphinyl, 2,4-dimethoxybenzyl, 3,5-dimethoxybenzyl, nitrobenzenesulphonyl, trityl, fluorenylmethoxycarbonyl, 9-fluorenylmethoxycarbonyl, benzyl, p-toluenesulphonyl, p-methoxybenzyl, formate, acetyl, benzyloxycarbonyl, phthaloyl, tert-butyloxycarbonyl, benzyl or p-methoxyphenyl; preferably hydrogen, benzyl, p-methoxybenzyl or tert-butyloxycarbonyl;
X₂, X₃, R₁₋₂, R₁₋₃, R_{2c}, R₃ₑ, R_{3f}, R_{3g}, M₁, M₂, M₃, Rₐ, R_{b}, R_{c}, a, b, c, x and n4 are as defined in any of the preceding embodiments.

In a preferred embodiment of the present invention, the condensing agent in the preparation method is selected from thionyl chloride, phosphorus oxychloride, p-toluenesulphonyl chloride, methanesulphonyl chloride, dicyclohexylcarbodiimide, diisopropylcarbodiimide, 1-(3-dimethylaminopropyl)-3-ethylcarbodiimide, carbonyldiimidazole, ethyl chloroformate, 2-(7-azobenzotriazol-1-yl)-N,N,N',N'-tetramethyluronium hexafluorophosphate, O-(benzotriazol-1-yl)-tetramethyluronium hexafluorophosphate or tetramethylchloroformamidinium hexafluorophosphate; preferably phosphorus oxychloride, 2-(7-azobenzotriazol-1-yl)-N,N,N',N'-tetramethyluronium hexafluorophosphate, O-(benzotriazol-1-yl)-tetramethyluronium hexafluorophosphate or tetramethylchloroformamidinium hexafluorophosphate;
the base is selected from potassium carbonate, methylamine, triethylamine, diisopropylamine, pyridine, imidazole, N-methylimidazole or N-methylmorpholine.

The present invention further relates to a pharmaceutical composition comprising a therapeutically effective dose of the compound, or the stereoisomer or the pharmaceutically acceptable salt thereof as shown in any one of the above embodiments, and one or more pharmaceutically acceptable carriers, diluents, or excipients.

The present invention further relates to the use of the compound, or the stereoisomer or the pharmaceutically acceptable salt thereof as shown in any one of the above embodiments, or the pharmaceutical composition in the preparation of a PRMT5 inhibitor drug.

The present invention further relates to the use of the compound, or the stereoisomer or the pharmaceutically acceptable salt thereof as shown in any one of the above embodiments, or the pharmaceutical composition thereof in the preparation of a drug for the treatment of a cancer; preferably, the cancer is a cancer with MTAP gene deletion.

The present invention further relates to a method of using the compound, or the stereoisomer or the pharmaceutically acceptable salt thereof as shown in any one of the above embodiments, or the pharmaceutical composition thereof in the preparation of a drug for the treatment of a cancer; preferably, the cancer is a cancer with MTAP gene deletion.

In some embodiments, the cancer is selected from lung cancer, hepatocellular carcinoma, breast cancer, skin cancer, bladder cancer, liver cancer, pancreatic cancer, head and neck cancer, glioma, glioblastoma, oesophageal cancer, pancreatic cancer, mesothelioma, melanoma, astrocytoma, undifferentiated pleomorphic sarcoma, diffuse large B-cell lymphoma, leukaemia, gastric adenocarcinoma, myxofibrosarcoma, cholangiosarcoma, brain cancer, gastric cancer, renal cancer, endometrial cancer, ovarian tumour, prostate cancer, lymphoma, non-Hodgkin lymphoma, urinary tract cancer, soft tissue cancer, pleural cancer, large intestine cancer, colorectal cancer, biliary tract cancer or cholangiocarcinoma; the lung cancer is selected from non-small cell lung cancer, lung squamous cell carcinoma or lung adenocarcinoma; the oesophageal cancer is selected from oesophageal squamous cell carcinoma or oesophageal adenocarcinoma.

The present invention further relates to a method for preventing and/or treating a cancer, wherein the method comprises administering to a patient a therapeutically effective dose of the compound, or the stereoisomer or the pharmaceutically acceptable salt thereof as shown in any one of the above embodiments, or the pharmaceutical composition thereof.

The present invention further relates to a method for treating a cancer in a mammal, wherein the method comprises administering to the mammal a therapeutically effective amount of the compound of the present invention, or the pharmaceutically acceptable salt, the ester, the prodrug, the solvate, the hydrate or the derivative thereof.

In certain embodiments of the present invention, the weight percent of the compound, or the stereoisomer or the pharmaceutically acceptable salt thereof in the pharmaceutical composition is 0.1%-95%, preferably 90%, 85%, 80%, 75%, 70%, 60%, 50%, based on free base.

In certain embodiments of the present invention, the pharmaceutical composition is selected from a tablet, a capsule, a liquid preparation or an injection, preferably further comprises a filler, optionally further comprises a disintegrant, or further comprises one or more of a glidant or a lubricant.

In certain embodiments of the present invention, the pharmaceutical composition is an immediate-release preparation or a sustained-release preparation.

In certain embodiments of the present invention, the unit dose of the compound, or the stereoisomer or the pharmaceutically acceptable salt thereof in the pharmaceutical composition is 1-1000 mg, preferably 1-500 mg, or preferably 1 mg, 2 mg, 3 mg, 5 mg, 10 mg, 20 mg, 40 mg, 50 mg, 60 mg, 80 mg, 100 mg, 200 mg, 300 mg, 400 mg or 500 mg, based on free base.

In certain embodiments of the present invention, the compound, or the stereoisomer or the pharmaceutically acceptable salt thereof can be administered by any convenient method, for example, by oral, parenteral, buccal, sublingual, nasal, rectal, intrathecal or transdermal administration, and the pharmaceutical composition can be adjusted accordingly.

In certain embodiments of the present invention, the compound, or the stereoisomer or the pharmaceutically acceptable salt thereof can be formulated into a liquid or solid preparation, such as a syrup, a suspension, an emulsion, a tablet, a capsule, powder, a granule, or a lozenge.

In some embodiments, the method relates to the treatment of conditions such as lung cancer, hepatocellular carcinoma, breast cancer, skin cancer, bladder cancer, liver cancer, pancreatic cancer, head and neck cancer, glioma, glioblastoma, esophageal cancer, pancreatic cancer, mesothelioma, melanoma, astrocytoma, undifferentiated pleomorphic sarcoma, diffuse large B-cell lymphoma, leukaemia, gastric adenocarcinoma, myxofibrosarcoma, cholangiosarcoma, brain cancer, gastric cancer, renal cancer, endometrial cancer, ovarian tumour, prostate cancer, lymphoma, non-Hodgkin lymphoma, urinary tract cancer, soft tissue cancer, pleural cancer, large intestine cancer, colorectal cancer, biliary tract cancer or cholangiocarcinoma.

In some embodiments, the lung cancer is selected from non-small cell lung cancer, lung squamous cell carcinoma or lung adenocarcinoma; the oesophageal cancer is selected from oesophageal squamous cell carcinoma or oesophageal adenocarcinoma.

### Detailed Description of the Invention

Unless stated to the contrary, all technical and scientific terms used herein generally have the same meanings as commonly understood by one of ordinary skill in the art. Specifically, the terms used in the description and claims have the following meanings.

The term "alkyl" refers to a linear or branched saturated aliphatic hydrocarbon group which may be optionally substituted with one or more substituents. In particular embodiments, the alkyl refers to a linear saturated hydrocarbon group having 1 to 20 (C₁₋₂₀), 1 to 15 (C₁₋₁₅), 1 to 12 (C₁₋₁₂), 1 to 10 (C₁₋₁₀), 1 to 8 (C₁₋₈), 1 to 6 (C₁₋₆), or 1 to 3 (C₁₋₃) carbon atoms, or a branched saturated hydrocarbon group having 3 to 20 (C₃₋₂₀), 3 to 15 (C₃₋₁₅), 3 to 12 (C₃₋₁₂), 3 to 10 (C₃₋₁₀), 3 to 8 (C₃₋₈) or 3 to 6 (C₃₋₆) carbon atoms. As used herein, linear C₁₋₆ alkyl and branched C₃₋₆ alkyl groups are also referred to as "lower alkyl". For example, C₁₋₆ alkyl refers to a linear saturated monovalent hydrocarbon group having 1 to 6 carbon atoms or a branched saturated monovalent hydrocarbon group having 3 to 6 carbon atoms. In one embodiment, the C₁₋₆ alkyl contains 1 to 6 (e.g., 1, 2, 3, 4, 5 or 6) carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, various branched isomers thereof, etc. In one embodiment, the alkyl is optionally substituted alkyl as described elsewhere herein.

The term "alkylene" refers to alkyl in which one hydrogen atom is further substituted, wherein the "alkyl" is as defined above. Non-limiting examples of "alkylene" include: methylene (-CH₂-), ethylene (-(CH₂)₂-), propylene (-(CH₂)₃-) or butylene (-(CH₂)₄-). In one embodiment, the alkylene is optionally substituted alkyl as described elsewhere herein.

The term "alkenyl" refers to a linear or branched unsaturated aliphatic hydrocarbon group containing at least one carbon-carbon double bond which may be located at any position within the alkenyl, and the alkenyl may be optionally substituted with one or more substituents. In particular embodiments, the alkenyl is a linear unsaturated hydrocarbon group having 2 to 20 (C₂₋₂₀), 2 to 15 (C₂₋₁₅), 2 to 12 (C₂₋₁₂), 2 to 10 (C₂₋₁₀), 2 to 8 (C₂₋₈), 2 to 6 (C₂₋₆) or 2 to 4 (C₂₋₄) carbon atoms, or a branched unsaturated hydrocarbon group having 3 to 20 (C₃₋₂₀), 3 to 15 (C₃₋₁₅), 3 to 12 (C₃₋₁₂), 3 to 10 (C₃₋₁₀), 3 to 8 (C₃₋₈) or 3 to 6 (C₃₋₆) carbon atoms. Unless otherwise specified, the term "alkenyl" as used herein includes both linear and branched alkenyl. For example, C₂₋₆ alkenyl refers to a linear unsaturated hydrocarbon group having 2 to 6 carbon atoms or a branched unsaturated hydrocarbon group having 3 to 6 carbon atoms. In one embodiment, the C₂₋₆ alkenyl contains 2 to 6 (e.g., 2, 3, 4, 5 or 6) carbon atoms. Non-limiting examples of alkenyl include: 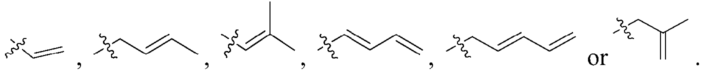 One of ordinary skill in the art can understand that the term "alkenyl" may also include groups having "cis" and "trans" configurations, or alternatively, groups having "E" and "Z" configurations. In one embodiment, the alkenyl is optionally substituted alkenyl as described elsewhere herein.

The term "alkenylene" refers to alkenyl in which one hydrogen atom is further substituted, wherein the "alkenyl" is as defined above. In one embodiment, the alkenylene is optionally substituted alkyl as described elsewhere herein.

The term "alkynyl" refers to a linear or branched unsaturated aliphatic hydrocarbon group containing at least one carbon-carbon triple bond which may be located at any position within the alkynyl, and the alkynyl may be optionally substituted with one or more substituents. In particular embodiments, the alkynyl is a linear unsaturated hydrocarbon group having 2 to 20 (C₂₋₂₀), 2 to 15 (C₂₋₁₅), 2 to 12 (C₂₋₁₂), 2 to 10 (C₂₋₁₀), 2 to 8 (C₂₋₈), 2 to 6 (C₂₋₆), or 2 to 4 (C₂₋₄) carbon atoms, or a branched unsaturated hydrocarbon group having 3 to 20 (C₃₋₂₀), 3 to 15 (C₃₋₁₅), 3 to 12 (C₃₋₁₂), 3 to 10 (C₃₋₁₀), 3 to 8 (C₃₋₈) or 3 to 6 (C₃₋₆) carbon atoms. Unless otherwise specified, the term "alkynyl" as used herein includes both linear and branched alkynyl. For example, C₂₋₆ alkynyl refers to a linear unsaturated hydrocarbon group having 2 to 6 carbon atoms or a branched unsaturated hydrocarbon group having 3 to 6 carbon atoms. In one embodiment, the C₂₋₆ alkynyl contains 2 to 6 (e.g., 2, 3, 4, 5 or 6) carbon atoms. Non-limiting examples of alkynyl include: 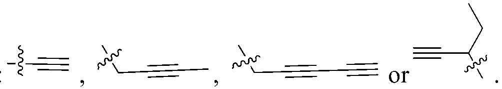 In one embodiment, the alkynyl is optionally substituted alkynyl as described elsewhere herein.

The term "alkynylene" refers to alkynyl in which one hydrogen atom is further substituted, wherein the "alkynyl" is as defined above. In one embodiment, the alkynylene is optionally substituted alkyl as described elsewhere herein.

The term "cycloalkyl" refers to a saturated or partially unsaturated aliphatic hydrocarbon cyclic group, which may be monocyclic or polycyclic (containing two or more rings), and which may be optionally substituted with one or more substituents. In particular embodiments, the cycloalkyl ring contains 3 to 20 (C₃₋₂₀), 3 to 14 (C₃₋₁₄), 3 to 12 (C₃₋₁₂), 3 to 8 (C₃₋₈) or 3 to 6 (C₃₋₆) carbon atoms. In one embodiment, the cycloalkyl ring contains 6 to 14 (C₆₋₁₄) or 7 to 10 (C₇₋₁₀) carbon atoms; it may contain one or more double bonds, but does not have a completely conjugated π electron system. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, etc.; and polycyclic cycloalkyl includes spirocycloalkyl, fused cycloalkyl and bridged cycloalkyl in one embodiment. In one embodiment, the cycloalkyl is optionally substituted cycloalkyl as described elsewhere herein or cycloalkyl that is optionally fused to heterocyclyl, aryl or heteroaryl, and non-limiting examples include indanyl, tetrahydronaphthyl, benzocycloheptyl, etc.

The term "spirocycloalkyl" refers to an aliphatic hydrocarbon polycyclic group with monocyclic rings sharing one carbon atom (called a spiro atom); and it may contain one or more double bonds, but no ring has a completely conjugated π electron system. In particular embodiments, the spirocycloalkyl contains 5 to 20 (C₅₋₂₀), 6 to 14 (C₆₋₁₄) or 7 to 10 (C₇₋₁₀) (e.g., 7, 8, 9 or 10) carbon atoms. According to the number of shared spiro atoms between the rings, the spirocycloalkyl is divided into monospirocycloalkyl, bispirocycloalkyl or polyspirocycloalkyl. In one embodiment, the spirocycloalkyl is monospirocycloalkyl and bispirocycloalkyl. In one embodiment, the spirocycloalkyl is 4-membered/4-membered, 3-membered/5-membered, 4-membered/5-membered, 4-membered/6-membered, 5-membered/5-membered or 5-membered/6-membered monospirocycloalkyl. In one embodiment, the spirocycloalkyl is optionally substituted spirocycloalkyl as described elsewhere herein. Non-limiting examples of spirocycloalkyl include:

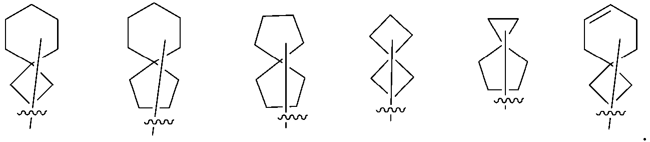

The term "fused cycloalkyl" refers to an all-carbon polycyclic group with each ring in the system sharing a pair of adjacent carbon atoms with another ring in the system, wherein one or more rings may contain one or more double bonds, but no ring has a completely conjugated π electron system. In particular embodiments, the fused cycloalkyl contains 5 to 20 (C₅₋₂₀), 6 to 14 (C₆₋₁₄) or 7 to 10 (C₇₋₁₀) (e.g., 7, 8, 9 or 10) carbon atoms. According to the number of constituent rings, the fused cycloalkyl can be divided into bicyclic, tricyclic, tetracyclic or polycyclic fused cycloalkyl. In one embodiment, the fused cycloalkyl is bicyclic or tricyclic, and in a further embodiment, the fused cycloalkyl is 3-membered/5-membered, 4-membered/5-membered, 5-membered/5-membered or 5-membered/6-membered bicyclic cycloalkyl. In one embodiment, the fused cycloalkyl is optionally substituted fused cycloalkyl as described elsewhere herein, or fused cycloalkyl that is optionally fused to heterocyclyl, aryl or heteroaryl. Non-limiting examples of fused cycloalkyl include: 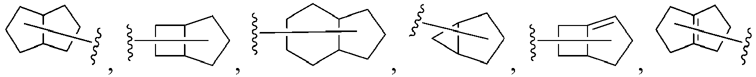 or

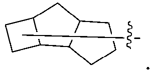

The term "bridged cycloalkyl" refers to an all-carbon polycyclic group in which any two rings share two carbon atoms that are not directly connected; and it may contain one or more double bonds, but no ring has a completely conjugated π electron system. In particular embodiments, the bridged cycloalkyl contains 5 to 20 (C₅₋₂₀), 6 to 14 (C₆₋₁₄) or 7 to 10 (C₇₋₁₀) (e.g., 7, 8, 9 or 10) carbon atoms. According to the number of constituent rings, the bridged cycloalkyl can be divided into bicyclic, tricyclic, tetracyclic or polycyclic bridged cycloalkyl, preferably bicyclic or tricyclic. In one embodiment, the bridged cycloalkyl is optionally substituted bridged cycloalkyl as described elsewhere herein. Non-limiting examples of bridged cycloalkyl include:

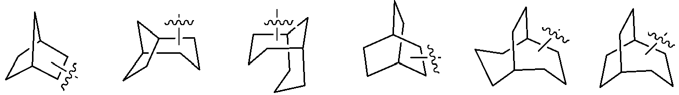

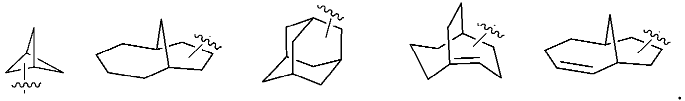

The term "cycloalkylene" refers to divalent cycloalkyl formed by further substituting one hydrogen atom on cycloalkyl, wherein the cycloalkylene is optionally substituted or unsubstituted, and the cycloalkyl is as defined above.

The term "heterocyclyl" refers to a saturated or partially unsaturated monocyclic or polycyclic cyclic hydrocarbon group, wherein one or more ring atoms are heteroatoms selected from nitrogen, oxygen, boron, phosphorus or sulphur, wherein the nitrogen, phosphorus or sulphur atom may be optionally oxidized, the nitrogen atom may be optionally quaternized, the ring carbon atoms may be optionally substituted with oxygen, but excluding ring moieties of -O-O- and -O-S-, and the remaining ring atoms are carbon atoms. The heterocyclyl may contain one or more double bonds but do not have a completely conjugated π electron system. In particular embodiments, the heterocyclyl contains 3 to 20, 3 to 14, 3 to 12, 3 to 8 or 3 to 6 ring atoms, of which 1 to 4 are heteroatoms. In one embodiment, the heterocyclyl contains 3 to 6, 4 to 6, 3 to 8, 3 to 10, 6 to 10 or 7 to 11 ring atoms. In one embodiment, the heterocyclyl contains 3 to 8 (e.g., 3, 4, 5, 6, 7 or 8) ring atoms. Non-limiting examples of monocyclic heterocyclyl include tetrahydropyrrolyl, azetidinyl, oxetanyl, oxanyl, tetrahydrofuryl, tetrahydropyranyl, tetrahydrothienyl, dihydroimidazolyl, dihydrofuryl, dihydropyrazolyl, dihydropyrrolyl, piperidinyl, piperazinyl, morpholinyl, thiomorpholinyl, homopiperazinyl, pyranyl, etc. Polycyclic heterocyclyl includes spiroheterocyclyl, fused heterocyclyl and bridged heterocyclyl. In one embodiment, the heterocyclyl is optionally substituted heterocyclyl as described elsewhere herein, or heterocyclyl that is further fused to other cycloalkyl, heterocyclyl, aryl and heteroaryl through any two or more atoms on the ring.

The term "spiroheterocyclyl" refers to a polycyclic heterocyclic group in which the rings share one atom (called a spiro atom), wherein one or more ring atoms are heteroatoms selected from nitrogen, oxygen, boron, phosphorus or sulphur, and the remaining ring atoms are carbon atoms. The spiroheterocyclyl may contain one or more double bonds, but no ring has a completely conjugated π electron system. In particular embodiments, the spiroheterocyclyl contains 5 to 20 or 6 to 14 ring atoms. In one embodiment, the spiroheterocyclyl contains 7 to 11 (e.g., 7, 8, 9, 10 or 11) ring atoms. According to the number of shared spiro atoms between the rings, the spiroheterocyclyl is divided into monospiroheterocyclyl, bispiroheterocyclyl or polyspiroheterocyclyl, preferably monospiroheterocyclyl and bispiroheterocyclyl. In one embodiment, the spiroheterocyclyl is 4-membered/4-membered, 4-membered/5-membered, 4-membered/6-membered, 5-membered/5-membered or 5-membered/6-membered monospiroheterocyclyl. In one embodiment, the spiroheterocyclyl is optionally substituted spiroheterocyclyl as described elsewhere herein. Non-limiting examples of spiroheterocyclyl include:

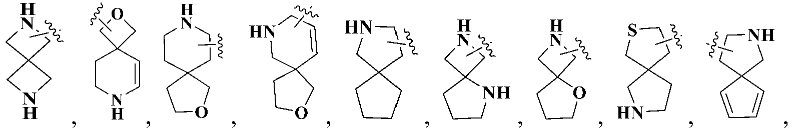

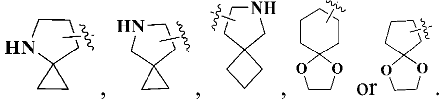

The term "fused heterocyclyl" refers to a polycyclic heterocyclic group with each ring in the system sharing a pair of adjacent atoms with another ring in the system, and one or more rings may contain one or more double bonds, but no ring has a completely conjugated π electron system, wherein one or more ring atoms are heteroatoms selected from nitrogen, oxygen, boron, phosphorus or sulphur, and the remaining ring atoms are carbon atoms. In particular embodiments, the fused heterocyclyl contains 5 to 20 or 6 to 14 ring atoms, and in one embodiment, the fused heterocyclyl contains 7 to 10 (e.g., 7, 8, 9 or 10) ring atoms. According to the number of constituent rings, the fused heterocyclyl can be divided into bicyclic, tricyclic, tetracyclic or polycyclic fused heterocyclyl, preferably bicyclic or tricyclic. In one embodiment, the fused heterocyclyl is 5-membered/5-membered or 5-membered/6-membered bicyclic fused heterocyclyl. In one embodiment, the fused heterocyclyl is optionally substituted fused heterocyclyl as described elsewhere herein, or fused heterocyclyl that can be fused to cycloalkyl, heterocyclyl, aryl or heteroaryl. Non-limiting examples of fused heterocyclyl include:

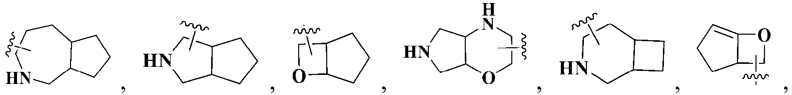

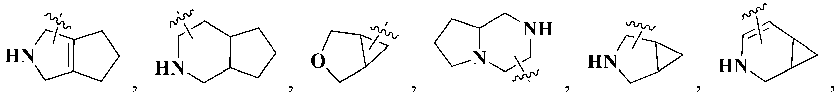

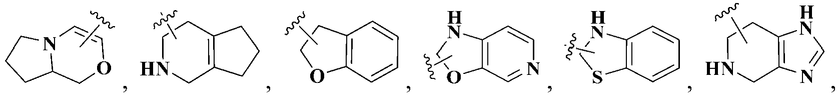

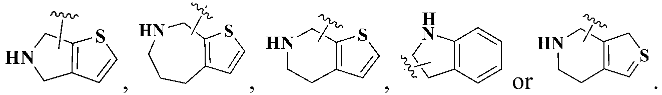

The term "bridged heterocyclyl" refers to a polycyclic heterocyclic group with any two rings sharing two atoms that are not directly connected. The bridged heterocyclyl may contain one or more double bonds, but no ring has a completely conjugated π electron system, wherein one or more ring atoms are heteroatoms selected from nitrogen, oxygen, boron, phosphorus or sulphur, and the remaining ring atoms are carbon atoms. In particular embodiments, the bridged heterocyclyl contains 5 to 20 or 6 to 14 ring atoms. In one embodiment, the bridged heterocyclyl contains 7 to 10 (e.g., 7, 8, 9 or 10) ring atoms. According to the number of constituent rings, the bridged heterocyclyl can be divided into bicyclic, tricyclic, tetracyclic or polycyclic bridged heterocyclyl, preferably bicyclic, tricyclic or tetracyclic. In one embodiment, the bridged heterocyclyl is bicyclic or tricyclic. In one embodiment, the bridged heterocyclyl is optionally substituted bridged heterocyclyl as described elsewhere herein. Non-limiting examples of bridged heterocyclyl include:

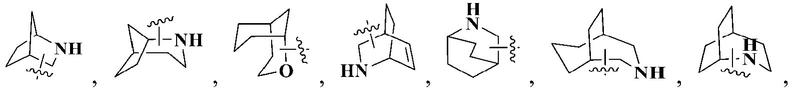

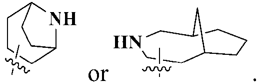

The term "tricyclic heterocyclyl" refers to a heterocyclyl group having three rings in the system, wherein the three rings may be a fused ring system, a spiro ring system, or a bridged ring system. In one embodiment, at least one of the three rings in the tricyclic heterocyclyl system is heterocyclyl, and the other two rings may be cycloalkyl, heterocyclyl, aryl or heteroaryl, wherein the cycloalkyl, heterocyclyl, aryl or heteroaryl is as defined above. Non-limiting examples thereof preferably include the following tricyclic heterocyclyl:

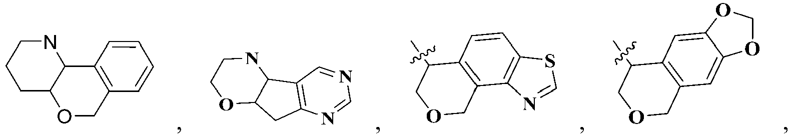

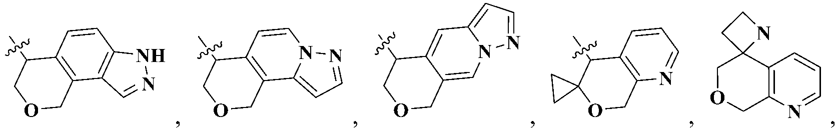

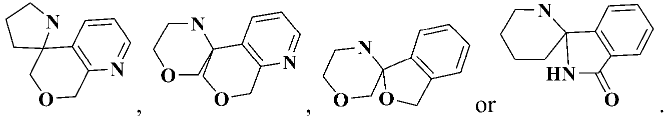

The term "heterocyclylene" refers to divalent heterocyclyl formed by further substituting one hydrogen atom on heterocyclyl, wherein the heterocyclylene is optionally substituted or unsubstituted, and the heterocyclyl is as defined above.

The term "aryl" refers to an all-carbon monocyclic or fused polycyclic (i.e., rings sharing a pair of adjacent carbon atoms) group containing at least one conjugated π electron system, which may be optionally substituted with one or more substituents. In particular embodiments, the aryl contains 6 to 20, 6 to 14, 6 to 12 or 6 to 10 ring atoms. In one embodiment, the aryl may further refer to a bicyclic, tricyclic or tetracyclic ring system, in which at least one ring is an aromatic ring and the other rings may be saturated or partially unsaturated carbocyclic rings or rings containing one or more heteroatoms independently selected from O, S and N. In one embodiment, the aryl is selected from benzo 5- to 10-membered heteroaryl, benzo 3- to 10-membered cycloalkyl or benzo 3- to 10-membered heterocyclyl. In one embodiment, the aryl is selected from benzo 5- or 6-membered heteroaryl, benzo 3-to 6-membered cycloalkyl or benzo 3- to 6-membered heterocyclyl, wherein the heterocyclyl is heterocyclyl containing 1 to 3 nitrogen atoms, oxygen atoms or sulphur atoms. Non-limiting examples thereof include phenyl, naphthyl, fluorenyl, azulenyl, anthryl, phenanthrenyl, pyrenyl, biphenyl, terphenyl, dihydronaphthyl, indenyl, tetrahydronaphthyl (tetralinyl), 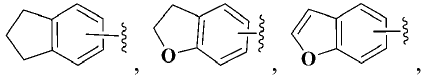 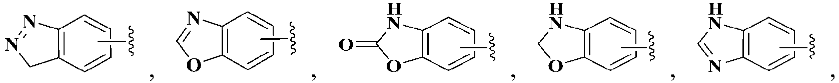 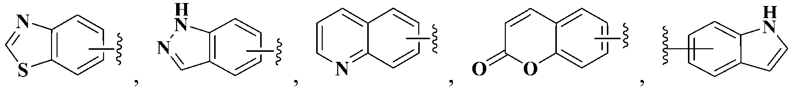 and

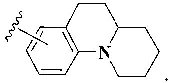

The term "arylene" refers to divalent aryl formed by further substituting one hydrogen atom on aryl, wherein the arylene is optionally substituted or unsubstituted, and the aryl is as defined above.

The term "heteroaryl" refers to an optionally substituted monocyclic or polycyclic group or ring system containing at least one aromatic ring, wherein the aromatic ring has one or more heteroatoms independently selected from O, S and N. In particular embodiments, the heteroaryl contains 5 to 20, 5 to 14, 5 to 12 or 5 to 10 ring atoms, of which 1 to 4 are heteroatoms. In one embodiment, the heteroaryl contains 5 or 6 ring atoms. In particular embodiments, the heteroaryl may further refer to a bicyclic, tricyclic or tetracyclic ring, in which at least one ring is an aromatic ring having one or more heteroatoms independently selected from O, S and N, and the other rings may be saturated or partially unsaturated carbocyclic rings or rings containing one or more heteroatoms independently selected from O, S and N. In one embodiment, the heteroaryl is selected from heteroaryl fused 6- to 10-membered aryl, heteroaryl fused 3- to 10-membered cycloalkyl, or heteroaryl fused 3- to 10-membered heterocyclyl. Further in one embodiment, the heteroaryl is selected from 5- or 6-membered heteroaryl fused 6- to 10-membered aryl, 5- or 6-membered heteroaryl fused 3- to 6-membered cycloalkyl, or 5- or 6-membered heteroaryl fused 3- to 6-membered heterocyclyl, wherein the heterocyclyl is heterocyclyl containing 1 to 3 nitrogen atoms, oxygen atoms or sulphur atoms. Non-limiting examples thereof include: furyl, imidazolyl, isothiazolyl, isoxazolyl, oxadiazolyl, oxazolyl, pyrazinyl, pyrazolyl, pyridazinyl, pyridyl, pyrimidyl, pyrrolyl, thiadiazolyl, thiazolyl, thienyl, tetrazolyl, triazinyl, triazolyl, benzofuryl, benzoimidazolyl, benzisoxazolyl, benzopyranyl, benzothiadiazolyl, benzothienyl, nenzophenylthio, benzothienyl, benzotriazolyl, imidazopyridyl, imidazothiazolyl, indolizinyl, indolyl, indazolyl, isobenzofuryl, isobenzothienyl, isoindolyl, isoquinolyl, naphthyridinyl, oxazolopyridyl, phthalazinyl, pteridyl, purinyl, pyridopyridyl, pyrrolopyridyl, quinolyl, quinoxalinyl, quinazolinyl, thiadiazolopyrimidyl, thienopyridyl, acridyl, benzoindolyl, carbazolyl, dibenzofuryl, phenanthrolinyl, phenanthridinyl, phenarsazinyl, phenazinyl, phenothiazinyl, phenoxazinyl, xanthenyl, 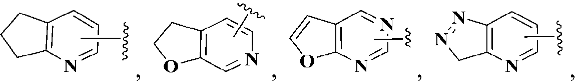 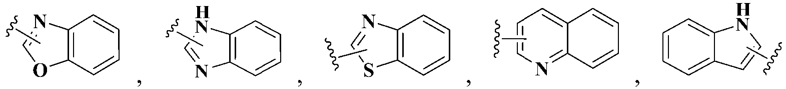 and

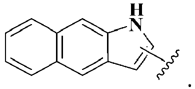

The term "heteroarylene" refers to divalent heteroaryl formed by further substituting one hydrogen atom on cycloalkyl, wherein the heteroarylene is optionally substituted or unsubstituted, and the heteroaryl is as defined above.

The term "heteroalkyl" refers to a stable linear or branched, or cyclic hydrocarbon group, or a combination thereof, and is composed of the indicated number of carbon atoms and one or more (in one embodiment, one to three) heteroatoms selected from O, N, Si and S, wherein the nitrogen and sulphur atoms are optionally oxidized and the nitrogen heteroatom may be optionally quaternized. In one embodiment, the heteroatoms O, N and S may be placed at any interior position of the heteroalkyl group. In one embodiment, the heteroatom Si may be placed at any position (e.g., interior or terminal position) of the heteroalkyl group, including the position where the alkyl group is connected to the rest of the molecule. Non-limiting examples thereof include: -CH₂-CH₂-O-CH₃, -CH₂-CH₂-NH-CH₃, - CH₂-CH₂-N (CH₃)-CH₃, -CH₂-S-CH₂-CH₃, -CH₂-CH₂-S(O)-CH₃, -CH₂-CH₂-S(O)₂-CH₃, -CH = CH-O-CH₃, -Si(CH₃)₃, -CH₂-CH = N-OCH₃ and-CH = CH-N (CH₃)-CH₃. Up to two heteroatoms can be consecutive, for example, -CH₂-NH-O-CH₃ and -CH₂-O-Si(CH₃)₃. In particular embodiments, the heteroalkyl is optionally substituted heteroalkyl as described elsewhere herein.

The term "alkoxy" refers to -O-(alkyl) and -O-(unsubstituted cycloalkyl), wherein the alkyl or cycloalkyl is as defined above. Non-limiting examples of alkoxy include: methoxy, ethoxy, propoxy, butoxy, cyclopropoxy, cyclobutoxy, cyclopentyloxy or cyclohexyloxy. In one embodiment, the alkoxy is optionally substituted alkoxy as described elsewhere herein.

The term "alkylacyl" refers to -C(O)-alkyl, wherein the alkyl is as defined above.

The term "haloalkyl" refers to alkyl substituted with one or more halogen, wherein the alkyl is as defined above. Non-limiting examples of haloalkyl include: trifluoromethyl, -CH₂CF₃,

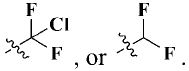

The term "haloalkoxy" refers to alkoxy substituted with one or more halogen, wherein the alkoxy is as defined above.

The term "hydroxyalkyl" refers to alkyl substituted with hydroxyl, wherein the alkyl is as defined above.

The term "alkylthio" refers to -S-(alkyl) and -S-(unsubstituted cycloalkyl), wherein the alkyl or cycloalkyl is as defined above. Non-limiting examples of alkylthio include: methylthio, ethylthio, propylthio, butylthio, cyclopropylthio, cyclobutylthio, cyclopentylthio or cyclohexylthio. In one embodiment, the alkylthio is optionally substituted alkylthio as described elsewhere herein.

The term "haloalkylthio" refers to alkylthio substituted with one or more halogen, wherein the alkylthio is as defined above.

The term "alkenylcarbonyl" refers to -C(O)-(alkenyl), wherein the alkenyl is as defined above. Non-limiting examples of alkenylcarbonyl include: ethenylcarbonyl, propenylcarbonyl, or butenylcarbonyl. In one embodiment, the alkenylcarbonyl is optionally substituted alkenylcarbonyl as described elsewhere herein.

The term "aminocarbonyl" refers to NH₂-C(O)-.

The term "alkylaminocarbonyl" refers to aminocarbonyl (NH₂-C(O)-) in which one or both of the hydrogen are substituted with alkyl, wherein the alkyl is as defined above.

The term "alkylamino" refers to amino in which one or both of the hydrogen are substituted with alkyl, wherein the alkyl is as defined above.

The term "carbonyl" refers to a -C(O)-, -(CO)- or -C(=O)- group. All the symbols are used interchangeably in the description.

The term "halogen" refers to fluorine, chlorine, bromine or iodine.

The term "oxo" or "side oxygen" refers to =O.

The term "C(X)" or "C(=X)" refers to 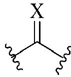 group. When X is O, it indicates that the group is carbonyl; when X is S, it indicates that the group is mercapto; when X is NR, it indicates that the group is 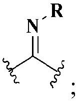 when X is CRR, it indicates that the group is

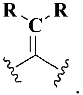

The term "hydrogen" includes proton (¹H), deuterium (²H), tritium (³H) and/or a mixture thereof. In particular embodiments, one or more hydrogen-occupied positions in the compound may be enriched with deuterium and/or tritium. Such isotopically enriched analogues may be prepared from suitable isotopically labelled starting materials obtained from commercial sources or by known literature procedures.

The alkyl, alkylene, alkenyl, alkynyl, cycloalkyl, heterocyclyl, aryl, arylene, heteroaryl, heteroarylene, heteroalkyl, alkoxy, alkylthio, hydroxyalkyl, alkenylcarbonyl, aminocarbonyl, alkylaminocarbonyl, alkylamino or alkylacyl may be substituted or unsubstituted. In one embodiment, the substituent is selected from one or more of the following groups: alkyl, alkenyl, alkynyl, alkoxy, alkylthio, alkylamino, alkylacyl, halogen, mercapto, hydroxyl, nitro, cyano, azidyl, oximido, phosphate group, oxo, thio, carboxyl, carboxylate group, cycloalkyl, heterocyclyl, aryl, heteroaryl, heterocycloalkoxy, cycloalkylthio or heterocycloalkylthio.

Different expressions such as "X is selected from A, B, or C", "X is selected from A, B and C", "X is A, B or C", and "X is A, B and C" all express the same meaning, that is, X can be any one or more of A, B, and C.

"Optional" or "optionally" means that the event or circumstance subsequently described may but not necessarily occur, and the description includes the occasion where the event or circumstance occurs or does not occur. For example, "heterocyclic group optionally substituted with alkyl" means the alkyl may but need not be present, the description includes the case where the heterocyclic group is substituted with alkyl and the case where the heterocyclic group is not substituted with alkyl.

In various parts of the present invention, linking substituents are described. When it is clear that a linking group is required for the structure, the markush variables listed for that group should be understood as referring to the linking group. For example, if a linking group is required for the structure and the markush group definition for that variable lists "alkyl" or "aryl," it should be understood that the "alkyl" or "aryl" respectively represents the attached alkylene group or arylene group.

The term "substituted" means that any one or more hydrogen atoms on the designated atom are substituted with a substituent, provided that the valence state of the designated atom is normal, and the substituted compound is stable in one embodiment. When the substituent is oxo (i.e., =O), it means that two hydrogen atoms are substituted. The term "optionally substituted" means that a group may or may not be substituted. Unless otherwise specified, the type and number of substituents may be arbitrary on the basis of chemical practicability. It goes without saying, the substituents may be only in possible chemical positions thereof, and those skilled in the art can determine the possible or impossible substitutions (by experiment or theory) without paying too much effort. For example, an amino or hydroxyl group with free hydrogen may be unstable when attached to a carbon atom with an unsaturated (e.g., olefinic) bond. The substituents can be selected from deuterium, halogen, amino, hydroxyl, cyano, nitro, alkyl, alkenyl, alkynyl, oxo, thio, deuteroalkyl, haloalkyl, alkoxy, haloalkoxy, hydroxyalkyl, cyano-substituted alkyl, cycloalkyl, heterocyclyl, aryl or heteroaryl.

The term "substituted or unsubstituted" means that a group may or may not be substituted. When a group may be substituted, the substituents are selected from hydrogen, deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, -SF₅, -C₃₋₁₂ cycloalkyl, 3- to 12-membered heterocyclyl, C₆₋₁₂ aryl, 5- to 12-membered heteroaryl, C(O)R, C(O)OR, C(O)NRR', N=S(O)RR', S(O)R(=NR'), P(O)RR' or =RR';
R and R' are each independently selected from hydrogen, deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, - -C₃₋₁₂ cycloalkyl, 3- to 12-membered heterocyclyl, C₆₋₁₂ aryl or 5- to 12-membered heteroaryl.

Unless stated to the contrary, the indefinite articles "a" and "an" and the definite article "the" in the present description and claims include plural as well as singular forms.

"Pharmaceutical composition" denotes a mixture containing one or more compounds described herein or physiologically/pharmaceutically acceptable salts or prodrugs thereof and other chemical components, as well as other components, such as physiologically/pharmaceutically acceptable carriers and excipients. The purpose of pharmaceutical compositions is to facilitate administration to living organisms and facilitate the absorption of active ingredients to exert biological activity.

The "pharmaceutically acceptable salt" refers to salts of the compound of the present invention, which are safe and effective when used in mammals, and have appropriate biological activity.

The "stereoisomer" comprises all enantiomerically/diastereomerically/stereoisomerically pure and enantiomerically/diastereomerically/stereoisomerically enriched compounds of the present invention.

"Stereoisomerically pure" means a composition comprising one stereoisomer of a compound and being substantially free of another stereoisomer of the compound. For example, a stereoisomerically pure composition of a compound having one chiral centre will be substantially free of the opposite enantiomer of the compound. A stereoisomerically pure composition of a compound having two chiral centres will be substantially free of other diastereomers of the compound. A typical stereoisomerically pure compound comprises greater than about 80% by weight of one stereoisomer of the compound and less than about 20% by weight of another stereoisomer of the compound, greater than about 90% by weight of one stereoisomer of the compound and less than about 10% by weight of another stereoisomer of the compound, greater than about 95% by weight of one stereoisomer of the compound and less than about 5% by weight of another stereoisomer of the compound, greater than about 97% by weight of one stereoisomer of the compound and less than about 3% by weight of another stereoisomer of the compound, or greater than about 99% by weight of one stereoisomer of the compound and less than about 1% by weight of another stereoisomer of the compound.

"Stereoisomerically enriched" means a composition comprising greater than about 55% by weight, greater than about 60% by weight, greater than about 70% by weight, or greater than about 80% by weight of one stereoisomer of a compound.

"Enantiomerically pure" means a stereoisomerically pure composition of a compound having one chiral centre. Similarly, the term "enantiomerically enriched" refers to a stereomerically enriched composition of a compound having one chiral centre.

"Optical activity" and "enantiomeric activity" mean a combination of molecules having an enantiomeric or diastereomeric excess of no less than about 50%, no less than about 70%, no less than about 80%, no less than about 90%, no less than about 91%, no less than about 92%, no less than about 93% , no less than about 94%, no less than about 95%, no less than about 96%, no less than about 97%, no less than about 98%, no less than about 99%, no less than about 99.5%, or no less than about 99.8%. In particular embodiments, the compound comprises about 95% or more of the desired enantiomer or diastereomer and about 5% or less of the less preferred enantiomer or diastereomer, based on the total weight of the racemate.

When describing optically active compounds, the prefixes R and S are used to indicate the absolute configuration of the molecule relative to its chiral centre. (+) and (-) are used to represent the optical rotation of the compound, that is, the direction of the plane of polarized light rotated by the optically active compound. The prefix (-) indicates that the compound is laevorotatory, that is, the compound rotates the plane of polarized light to the left or counterclockwise. The prefix (+) indicates that the compound is dextrorotatory, that is, the compound rotates the plane of polarized light to the right or clockwise. However, the signs of optical rotation (+) and (-) are independent of the absolute configurations R and S of the molecule.

### Detailed Description of Embodiments

The present invention will be further described below in conjunction with examples, but these examples are not meant to limit the scope of the present invention.

### Example

The structures of the compounds of the present invention are determined by nuclear magnetic resonance (NMR) or/and mass spectrometry (MS). The NMR shift (δ) is given in 10⁻⁶ (ppm). NMR is determined with a Bruker AVANCE-400 nuclear magnetic instrument. The solvents for determination are deuterated dimethyl sulphoxide (DMSO-*d₆*), deuterated chloroform (CDCl₃), and deuterated methanol (CD₃OD), and the internal standard is tetramethylsilane (TMS).

MS is determined with a FINNIGAN LCQAd (ESI) mass spectrometer (manufacturer: Thermo, model: Finnigan LCQ advantage MAX).

HPLC is determined with Agilent 1200DAD high-pressure liquid chromatograph instrument (Sunfire C₁₈ 150 × 4.6 mm chromatographic column) and Waters 2695-2996 high-pressure liquid chromatograph instrument (Gimini C₁₈ 150 × 4.6 mm chromatographic column).

The average kinase inhibition rate and IC₅₀ value are determined with NovoStar microplate reader (BMG Company, Germany).

For thin layer chromatography (TLC), silica gel plates from Yantai Huanghai HSGF254 or Qingdao GF254 are used. Silica gel plates with a thickness of 0.15 mm-0.2 mm are employed for TLC, while plates with a thickness of 0.4 mm-0.5 mm are used for TLC separation and purification.

For column chromatography, Yantai Huanghai silica gel of 200-300 mesh is typically used as a carrier.

The known starting materials of the present invention can be synthesized by methods known in the art, or can be purchased from ABCR GmbH & Co. KG, Acros Organics, Aldrich Chemical Company, Accela ChemBio Inc., Chembee Chemicals Inc. and other companies.

Unless otherwise specially specified in examples, reactions can all be carried out under argon atmosphere or nitrogen atmosphere. Argon atmosphere or nitrogen atmosphere means that a reaction flask is connected to an argon or nitrogen balloon with a volume of about 1 L. Hydrogen atmosphere means that a reaction flask is connected to a hydrogen balloon with a volume of about 1 L.

Pressurized hydrogenation reactions are performed using either Parr 3916EKX hydrogenation apparatus with Qinglan QL-500 hydrogen generator or HC2-SS hydrogenation apparatus.

Hydrogenation reactions are typically performed by evacuating the system and refilling it with hydrogen gas, repeating this operation three times.

Microwave reactions are performed using CEM Discover-S 908860 microwave reactor.

Unless otherwise specially specified in examples, a solution refers to an aqueous solution.

Unless otherwise specially specified in examples, the reaction temperature is room temperature, which is 20°C to 30°C.

The progress of a reaction in the examples is monitored by thin layer chromatography (TLC). Developing agent systems used in the reaction include: A: dichloromethane and methanol system, B: n-hexane and ethyl acetate system, C: petroleum ether and ethyl acetate system, D: acetone, the volume ratio of the solvents is adjusted according to the polarity of the compounds.

Eluent systems for column chromatography and developing agent systems for thin layer chromatography, which are used to purify compounds, include: A: n-hexane and ethyl acetate system, B: n-hexane and tetrahydrofuran system, the volume ratio of the solvents is adjusted according to the polarity of the compounds, and a small amount of basic or acidic reagents such as triethylamine and acetic acid can also be added for adjustment.

The conditions for chiral preparative HPLC resolution in the examples of the present invention are as follows, wherein t_{R} represents retention time:

### Condition 1:

Chiral preparative conditions

| | |
|---|---|
| Instrument | Water PLC 2545 |
| Column type | ChiralCel^{™} OD-H, 250 mm×30 I.D., 10 µm |
| Column pressure | 120 bar |
| Mobile phase | Hexane : EtOH : MeOH = 75 : 12.5 : 12.5 |
| Flow rate | 30 mL/min |
| Detection wavelength | UV 254 nm |
| Column temperature | 25°C |

Chiral analysis conditions:

| | |
|---|---|
| Instrument | UltiMate 3000 |
| Column type | Acclaim^{™} OD-H, 4.6×150 mm, 3 µm, 120Å |
| Column pressure | 150 bar |
| Mobile phase | H₂O (with 0.05% TFA) : ACN (with 0.05% TFA) = 70/30 |
| Flow rate | 1 mL/min |
| Detection wavelength | UV 254 nm |
| Column temperature | 35°C |

### Condition 2:

Chiral preparative conditions

| | |
|---|---|
| Instrument | WATERS 150 Mgm preparative SFC |
| Column type | ChiralCel AD, 250×30 mm I.D., 10 µm |
| Column pressure | 100 bar |
| Mobile phase | A for CO₂ and B for Ethanol, B 40% |
| Flow rate | 100 mL/min |
| Detection wavelength | UV 254 nm |
| Column temperature | 39°C |

Chiral analysis conditions

| | |
|---|---|
| Instrument | Waters Acquity UPC2 |
| Column type | Chiralcel AD-3 50*4.6 mm I.D., 3 um |
| Column pressure | 120 bar |
| Mobile phase | A for CO₂ and B for Ethanol (0.05% DEA), 40% |
| Flow rate | 2.5 mL/min |
| Detection wavelength | UV 254 nm |
| Column temperature | 25°C |

### Condition 3:

Chiral preparative conditions

| | |
|---|---|
| Instrument | WATERS 150 preparative SFC (SFC-26) |
| Column type | ChiralCel OJ, 250×30 mm I.D., 10 µm |
| Column pressure | 100 bar |
| Mobile phase | A for CO₂ and B for Ethanol, B 25% |
| Flow rate | 120 mL/min |
| Detection wavelength | UV 220 nm |
| Column temperature | 39°C |

Chiral analysis conditions

| | |
|---|---|
| Instrument | Waters UPC2 analytical SFC (SFC-H) |
| Column type | Chiralcel OJ-3 150*4.6 mm I.D., 3 um |
| Column pressure | 100 bar |
| Mobile phase | A for CO₂ and B for Ethanol (0.05% DEA) |
| Flow rate | 2.5 mL/min |
| Detection wavelength | UV 220 nm |
| Column temperature | 35°C |

The compounds in the embodiments of the present invention are prepared with reference to the preparation steps of the following examples:

### Example 1

### (4-Amino-1-methylimidazo[1,5-a]quinoxalin-8-yl)(3-(4-(trifluoromethyl)phenyl)morpholinyl)methanone

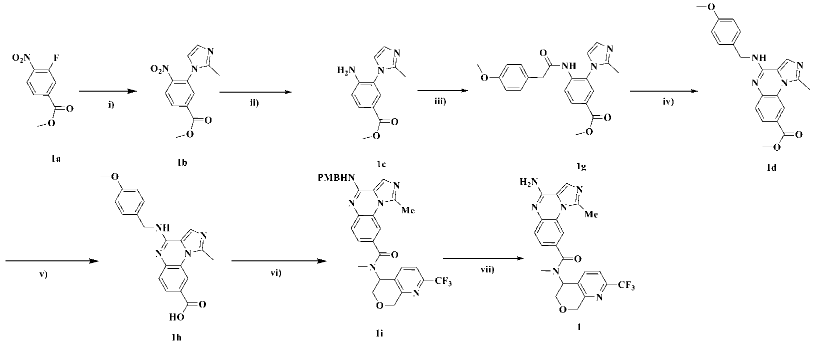

Step 1: 2-Methyl-1H-imidazole **1a** (5 g, 60.89 mmol) was dissolved in DMSO (50 mL), DBU (13.91 g, 91.35 mmol, 14 mL) and methyl 3-fluoro-4-nitrobenzoate (13.34 g, 66.99 mmol) were slowly added, and the mixture was stirred and reacted at 25°C for 2 hours; water (200 mL) was added to the reaction solution, and the mixture was extracted with ethyl acetate (200 mL × 3); the organic phases were combined, washed with a saturated sodium chloride aqueous solution (200 mL × 3), dried over anhydrous sodium sulphate, and filtered, the filtrate was concentrated under reduced pressure, and the resulting residue was purified by silica gel column chromatography using eluent system C to obtain **1b** (9 g, yield: 56.6%). MS m/z (ESI): 262 [M+1]⁺

Step 2: **1b** (2 g, 7.66 mmol) was dissolved in methanol (20 mL), Pd/C (816 mg, 766 µmol, 10% purity) was added, and the mixture was purged three times with hydrogen. Under hydrogen atmosphere, the mixture was stirred and reacted at 50°C for 12 hours; the reaction solution was filtered, the filtrate was concentrated under reduced pressure, and the filter cake was stirred with a mixed solvent of methanol : EA = 1: 10 (10 mL) for 15 minutes and filtered. The resulting filter cake was dried under reduced pressure to obtain **1c** (1.45 g, yield: 81.9%). MS m/z (ESI): 232 [M+1]⁺

Step 3: **1c** (1 g, 4.32 mmol) was dissolved in DCM (15 mL), DMAP (265 mg, 2.16 mmol) and 1-(isocyanatomethyl)-4-methoxybenzene (1.06 g, 6.49 mmol, 926 µL) were added, and the mixture was stirred and reacted at 80°C for 16 hours; the reaction solution was concentrated under reduced pressure, dichloromethane (10 mL) and methyl tert-butyl ether (10 mL) were added to the concentrated residue, and the mixture was stirred for 15 minutes and then filtered. The filter cake was collected and dried under reduced pressure to obtain **1g** (750 mg, yield: 45.8%). MS m/z (ESI): 380 [M+1]⁺

Step 4: **1g** (600 mg, 1.52 mmol) was dissolved in pyridine (2 mL), phosphorus oxychloride (280 mg, 1.83 mmol, 171 µL) was added, and the mixture was stirred and reacted at 120°C for 16 hours; the reaction solution was concentrated under reduced pressure, water (50 mL) was added to the concentrated residue, and the mixture was extracted with ethyl acetate (50 mL × 3); the organic phases were combined, washed with a saturated sodium chloride aqueous solution (50 mL × 3), dried over anhydrous sodium sulphate, and filtered, the filtrate was concentrated under reduced pressure, and the resulting residue was purified by silica gel column chromatography using eluent system C to obtain **1d** (250 mg, yield: 43.7%). MS m/z (ESI): 377 [M+1]⁺

Step 5: **1d** (250 mg, 664 µmol) was dissolved in water (2 mL)/methanol (2 mL)/tetrahydrofuran (6 mL), lithium hydroxide (32 mg, 1.33 mmol) was added, and the mixture was stirred and reacted at 80°C for 3 hours; the system was adjusted to pH 5 with 0.5 M hydrochloric acid and filtered, and the filter cake was stirred with ethanol (5 mL) for 0.5 hours and filtered. The resulting filter cake was dried under reduced pressure to obtain **1h** (110 mg, yield: 45.7%). MS m/z (ESI): 363 [M+1]⁺

Step 6: **1h** (50 mg, 138 µmol) and N-methyl-2-(trifluoromethyl)-6,8-dihydro-SH-pyran-[3,4-b]pyridin-5-amine (39 mg, 166 µmol, synthesized using the well-known method in "patent WO 2021163344") were dissolved in DMF (2 mL), N-methylimidazole (46 mg, 552 µmol, 44 µL) and TCFH (58 mg, 207 µmol) were added, and the mixture was stirred and reacted at 25°C for 1.5 hours; the reaction solution was concentrated under reduced pressure, water (15 mL) was added to the concentrated residue, and the mixture was extracted with ethyl acetate (20 mL × 3); the organic phases were combined, washed with a saturated sodium chloride aqueous solution (20 mL × 3), dried over anhydrous sodium sulphate, and filtered, the filtrate was concentrated under reduced pressure, and the concentrated residue was separated by preparative high-performance liquid chromatography to obtain **1i** (55 mg, yield: 68.9%). MS m/z (ESI): 576 [M+1]⁺

Step 7: **1i** (45 mg, 78 µmol) was dissolved in trifluoroacetic acid (3 mL), and the mixture was stirred and reacted at 90°C for 16 hours; the reaction solution was concentrated under reduced pressure, and the concentrated residue was separated by preparative high-performance liquid chromatography to obtain **Example** 1 (20 mg, yield: 56.2%). MS m/z (ESI): 458 [M+1]⁺

¹H NMR (400 MHz, DMSO-d₆) δ 8.23-8.11 (m, 2H), 7.86 (d, 2H), 7.60-7.38 (m, 4H), 5.80 (s, 1H), 4.81 (dd, 2H), 4.20 (d, 2H), 2.97 (s, 3H), 2.87-2.71 (m, 3H).

### Example 1 Preparation of chiral isomers

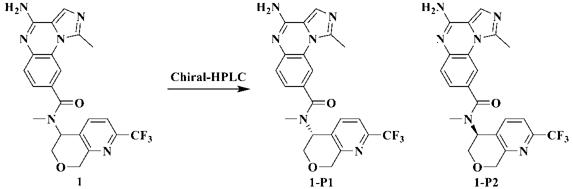

### Step 1 Preparation of chiral isomers

**Example 1** (20 mg, 0.044 mmol) was resolved by Chiral-HPLC to obtain **1-P1:** (R)-(4-amino-1-methylimidazo[1,5-a]quinoxalin-8-yl)(3-(4-(trifluoromethyl)phenyl)morpholinyl)methanone (9.5 mg, yield: 47.5%) and **1-P2:** (*S*)-(4-amino-1-methylimidazo[1,5-a]quinoxalin-8-yl)(3-(4-(trifluoromethyl)phenyl)morpholinyl)methanone (9.8 mg, yield: 49.0%), respectively.

The examples in the table below were prepared with reference to **Example 1:**

| **Example** | **Structure** | **MS m/z (ESI)** |
|---|---|---|
| | | **[M+1]⁺** |
| **2** | 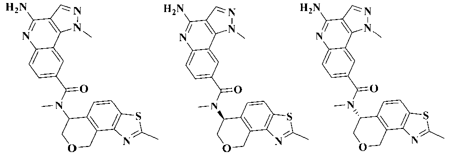 | 459 |
| **3** | 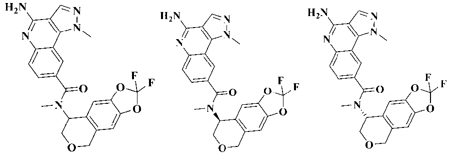 | 468 |
| **4** | 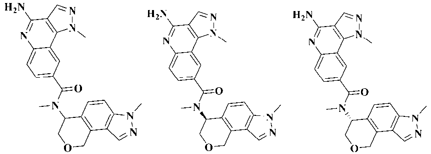 | 442 |
| **5** | 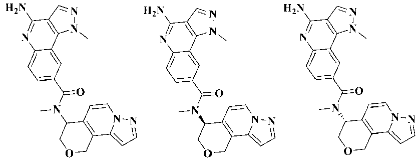 | 428 |
| **6** | 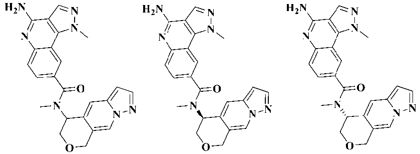 | 428 |
| **7** | 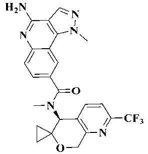 | 483 |

### Example 8

### 4-Amino-N-methyl-N-(2-(trifluoromethyl)-5,8-dihydro-6H-pyrano[3,4-b]pyridin-5-yl)-[1,2,3]triazolo[1,5-a]quinoxaline-8-carboxamide

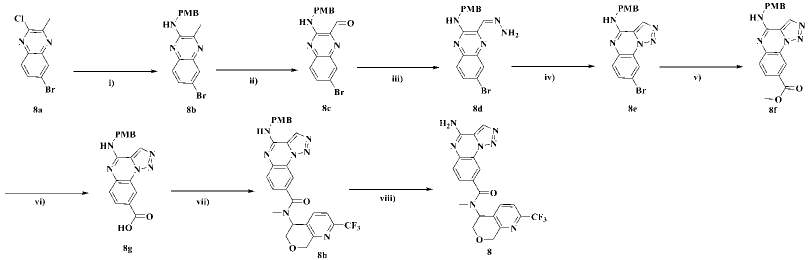

Step 1: 6-Bromo-2-chloro-3-methylquinoxaline **8a** (1.5 g, 5.82 mmol) and caesium carbonate (2.85 g, 8.47 mmol) were dispersed in dimethylformamide (15 mL), 4-methoxybenzylamine (799 mg, 5.82 mmol) was added, and the mixture was stirred and reacted at 100°C for 2 hours. The reaction solution was poured into ethyl acetate (50 ml), the mixture was then washed with a saturated sodium chloride solution (50 mL × 2), dried over anhydrous sodium sulphate, and filtered, and the filtrate was concentrated under reduced pressure. The resulting residue was purified by silica gel column chromatography using eluent system A to obtain **8b** (1.5 g, yield: 71.9%). MS m/z (ESI): 358 [M+1]⁺

Step 2: **8b** (1.5 g, 4.19 mmol) was dissolved in dioxane (10 mL) and water (1 mL), selenium dioxide (929 mg, 8.37 mmol) was added, and the mixture was stirred and reacted at 85°C for 2 hours. Water (100 mL) was added to the reaction solution, and the mixture was extracted with ethyl acetate (100 mL × 2). After extraction, the organic phase was separated, dried over anhydrous sodium sulphate, and filtered. The filtrate was concentrated under reduced pressure to obtain **8c** (1.43 g, yield: 91.7%). MS m/z (ESI): 372 [M+1]⁺

Step 3: **8c** (1.43 g, 3.84 mmol) and 4-methylbenzenesulphonyl hydrazide (1.07 g, 5.76 mmol) were dissolved in methanol (10 mL), and the mixture was stirred and reacted at 60°C for 3 hours. Water (50 mL) was added to the reaction solution, and the mixture was extracted with ethyl acetate (50 mL × 2). After extraction, the organic phase was separated, dried over anhydrous sodium sulphate, and filtered. The filtrate was concentrated under reduced pressure to obtain 8d (1.43 g, yield: 96.2%). MS m/z (ESI):386 [M+1] ⁺

Step 4: **8d** (1.43 g, 3.70 mmol) and sodium ethoxide (1.01 g, 14.80 mmol) were dissolved in dioxane (10 mL) and cyclohexane (10 mL), and the mixture was stirred and reacted at 60°C for 3 hours. The reaction solution was concentrated under reduced pressure, and the resulting residue was purified by silica gel column chromatography using eluent system A to obtain **8e** (560 mg, yield: 41.2%). MS m/z (ESI): 384 [M+1]⁺

Step 5: **8e** (250 mg, 0.65 mmol), triethylamine (131 mg, 1.30 mmol) and 1,1-bis(diphenylphosphino)ferrocene palladium(II) dichloride (48 mg, 0.07 mmol) were dissolved in ethanol (10 mL). Under carbon monoxide atmosphere, the mixture was stirred and reacted at 80°C for 72 hours. Water (50 mL) was added to the reaction solution, and the mixture was extracted with ethyl acetate (50 mL x 2). After extraction, the organic phase was separated, dried over anhydrous sodium sulphate, and filtered. The filtrate was concentrated under reduced pressure, and the resulting residue was purified by silica gel column chromatography using eluent system A to obtain **8f** (91 mg, yield: 37.1%). MS m/z (ESI): 363 [M+1]⁺

Referring to the synthesis method described in Step 5 to Step 7 of **Example 1, Example 8** (6 mg, yield: 5.6%) was obtained from **8f** (91 mg). MS m/z (ESI): 444 [M+1]⁺

¹H NMR (400 MHz, DMSO-d₆) δ 8.60 (s, 1H), 8.44 (d, 1H), 8.17 (d, 1H), 7.86 (d, 3H), 7.75 (s, 1H), 7.55 (d, 1H), 5.84 (s, 1H), 5.02-4.71 (m, 2H), 4.18 (d, 2H), 2.76 (d, 3H).

### Example 9

### 4-Amino-N-(2-(3,3-difluoroazetidin-1-yl)-5,8-dihydro-6H-pyrano[3,4-b]pyridin-5-yl)-N,1-dimethyl-1H-pyrazolo[4,3-c]quinoline-8-carboxamide

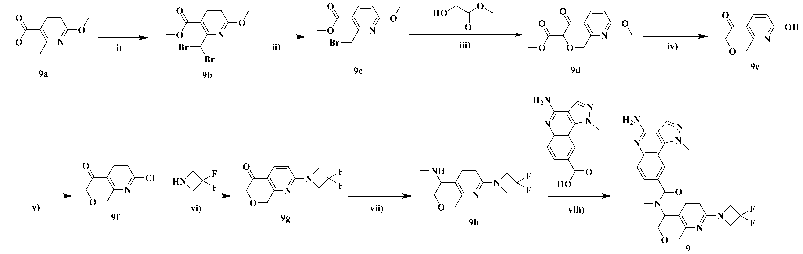

Step 1: Methyl 6-methoxy-2-methylnicotinate **9a** (15.00 g, 82.87 mmol), *N-*bromosuccinimide (29.00 g, 162.92 mmol) and azobisisobutyronitrile (2.60 g, 15.85 mmol) were dispersed in carbon tetrachloride (150 mL), and the mixture was stirred and reacted at 80°C for 16 hours. The reaction solution was filtered to remove insoluble materials. The filtrate was concentrated under reduced pressure to obtain **9b** (30.50 g, crude). MS m/z (ESI): 338 [M+1]⁺

Step 2: **9b** (30.50 g, crude), diethyl phosphite (14.00 g, 101.45 mmol) and *N,N-*diisopropylethylamine (26.00 g, 201.55 mmol) were dispersed in dichloromethane (400 mL), and the mixture was stirred and reacted at 25°C for 2 hours. The reaction solution was concentrated, and the resulting residue was purified by silica gel column chromatography using eluent system B to obtain **9c** (12.00 g, yield: 55.9%). MS m/z (ESI): 260 [M+1]⁺

Step 3: Methyl 2-hydroxyacetate (8.50 g, 94.44 mmol) was dispersed in *N,N-*dimethylformamide (200 mL). At 0°C, sodium hydride (5.70 g, 142.48 mmol, 60% dispersion in mineral oil) was added, and the mixture was stirred and reacted for 1 hour. **9c** (12.00 g, 46.33 mmol) was added, and the mixture was stirred and reacted at 25°C for 16 hours. The reaction solution was poured into water (2000 mL), and the mixture was extracted with ethyl acetate (300 mL×3); the organic phases were combined, washed with a saturated sodium chloride aqueous solution (200 mL × 3), dried over anhydrous sodium sulphate, and filtered, and the filtrate was concentrated under reduced pressure to obtain **9d** (22.00 g, crude). MS m/z (ESI): 238 [M+1]⁺

Step 4: **9d** (22.00 g, crude) was dispersed in ethanol (100 mL) and concentrated hydrochloric acid (100 mL), and the mixture was stirred and reacted at 100°C for 2 hours. The reaction solution was concentrated under reduced pressure, and the resulting residue was purified by silica gel column chromatography using eluent system A to obtain **9e** (4.00 g, yield: 52.3%). MS m/z (ESI): 166 [M+1]⁺

Step 5: 9e (4.00 g, 24.24 mmol) was dispersed in phosphorus oxychloride (30 mL), and the mixture was stirred and reacted at 90°C for 2 hours. The mixture was then concentrated under reduced pressure, the residue was dispersed in dichloromethane (300 mL), then sequentially washed with a saturated sodium bicarbonate solution (200 mL × 2) and a sodium chloride solution (200 mL × 2), dried over anhydrous sodium sulphate, and filtered, and the filtrate was concentrated under reduced pressure to obtain **9f** (4.30 g, yield: 96.9%). MS m/z (ESI): 184 [M+1]⁺

Step 6: **9f** (300 mg, 1.64 mmol), 3,3-difluoroazetidine hydrochloride (260 mg, 2.02 mmol) and *N,N**-***diisopropylethylamine (800 mg, 6.20 mmol) were dispersed in acetonitrile (5 mL), and the mixture was stirred and reacted at 80°C for 16 hours. The reaction solution was concentrated under reduced pressure, and the resulting residue was purified by silica gel column chromatography using eluent system B to obtain **9g** (240 mg, yield: 61.0%). MS m/z (ESI): 241 [M+1]⁺

Step 7: **9g** (240 mg, 1.00 mmol) and methylamine (310 mg, 3.00 mmol, 30% methanol solution) were dispersed in trifluoroethanol (3 mL), and the mixture was stirred and reacted at 25°C for 16 hours. Sodium cyanoborohydride (130 mg, 2.06 mmol) was added, and the mixture was stirred and reacted at 25°C for 1 hour. The reaction solution was concentrated under reduced pressure, and the resulting residue was purified by silica gel column chromatography using eluent system A to obtain **9h** (150 mg, yield: 58.8%). MS m/z (ESI): 256 [M+1]⁺

Referring to the synthesis method described in Step 7 of **Example 1, Example 9** (9 mg, yield: 9.3%) was obtained from **9h** (50 mg, 0.20 mmol). MS m/z (ESI): 480 [M+1]⁺

### Example 10

### 4-Amino-7-(difluoromethoxy)-N,1-dimethyl-N-(2-(trifluoromethyl)-5,8-dihydro-6H-pyrano[3,4-b]pyridin-5-yl)-1H-pyrazolo[4,3-c]quinoline-8-carboxamide

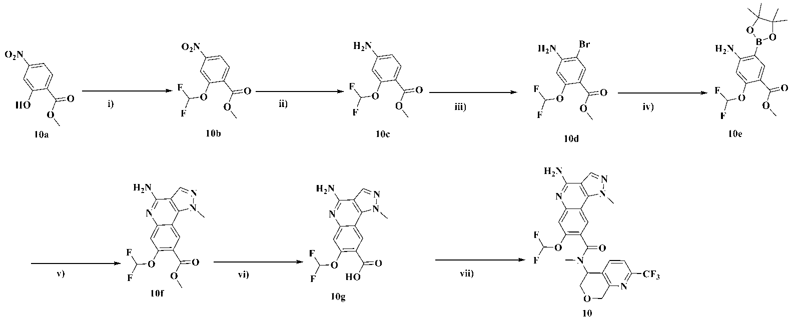

Step 1: Methyl 2-hydroxyl-4-nitrobenzoate **10a** (1.1 g, 5.58 mmol), sodium chlorodifluoroacetate (1.02 g, 6.70 mmol), and sodium carbonate (768 mg, 7.25 mmol) were dispersed in dimethylformamide (15 mL), and under nitrogen atmosphere, the mixture was stirred and reacted at 100°C for 16 hours. The reaction solution was poured into water (100 ml), and the mixture was extracted with ethyl acetate (50 mL × 2). The organic phases were combined, washed with a saturated sodium chloride solution (50 mL × 2), dried over anhydrous sodium sulphate, and filtered, the filtrate was concentrated under reduced pressure, and the resulting residue was purified by silica gel column chromatography using eluent system A to obtain **10b** (330 mg, yield: 23.9%). MS m/z (ESI): 248 [M+1]⁺

Step 2: **10b** (330 mg, 1.34 mmol) was dissolved in methanol (8 mL), palladium on carbon (284 mg, 5% purity, 0.13 mmol) was added, and under hydrogen atmosphere, the mixture was stirred and reacted at room temperature for 2 hours. The reaction solution was filtered through Celite to remove palladium on carbon, and the reaction solution was concentrated under reduced pressure to obtain **10c** (250 mg, yield: 86.2%). MS m/z (ESI): 218[M+1]⁺

Step 3: **10c** (250 mg, 1.15 mmol) and N-bromosuccinimide (204 mg, 1.15 mmol) were dissolved in dichloromethane (6 mL), and the mixture was stirred and reacted at room temperature for 30 minutes. The reaction solution was concentrated under reduced pressure, and the resulting residue was purified by silica gel column chromatography using eluent system A to obtain **10d** (160 mg, yield: 47.0%). MS m/z (ESI): 296[M+1]⁺

Step 4: **10d** (160 mg, 0.54 mmol), 1,1-bis(diphenylphosphino)ferrocene palladium(II) dichloride (40 mg, 0.05 mmol), potassium acetate (106 mg, 1.08 mmol) and bis(pinacolato)diboron (292 mg, 1.15 mmol) were dissolved in dioxane (5 mL), and the mixture was stirred and reacted at 90°C for 16 hours. The reaction solution was filtered, the filtrate was poured into water (100 ml), and the mixture was extracted with ethyl acetate (50 mL × 2). The organic phases were combined, washed with a saturated sodium chloride solution (50 mL × 2), dried over anhydrous sodium sulphate, and filtered. The filtrate was concentrated under reduced pressure to obtain **10e** (71 mg, yield: 38.3%). MS m/z (ESI): 344[M+1]⁺

Step 5: **10e** (71 mg, 0.21 mmol), 5-bromo-1-methylpyrazole-4-carbonitrile (38 mg, 0.21 mmol), tetrakis(triphenylphosphine)palladium (24 mg, 0.02 mmol) and sodium carbonate (44 mg, 0.41 mmol) were dissolved in dioxane (5 mL) and water (1 mL), and the mixture was stirred and reacted at 90°C for 16 hours. The reaction solution was filtered, the filtrate was poured into water (100 ml), and the mixture was extracted with ethyl acetate (50 mL × 2). The organic phases were combined, washed with a saturated sodium chloride solution (50 mL × 2), dried over anhydrous sodium sulphate, and filtered, and the filtrate was concentrated under reduced pressure to obtain **10f** (37 mg, yield: 55.5%). MS m/z (ESI): 323[M+1]⁺

Referring to the synthesis method described in Step 5 to Step 6 of **Example 1, Example 10** (7.1 mg, yield: 11.8%) was obtained from **10f** (37 mg, 0.11 mmol). MS m/z (ESI): 523 [M+1]⁺

### Example 11 was prepared with reference to Example 10:

| **Example** | **Structure** | **MS m/z (ESI) [M+1]⁺** |
|---|---|---|
| **11** | 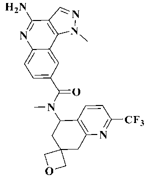 | 497 |

### Example 12

### 4-Amino-N-methyl-N-[2-(trifluoromethyl)-6,8-dihydro-5H-pyrano[3,4-b]pyridin-5-yl]imidazo[1,5-a]quinoxaline-8-carboxamide

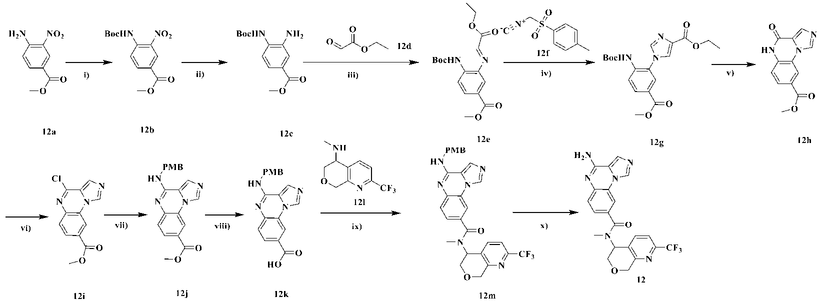

Step 1: Methyl 4-amino-3-nitro-benzoate **12a** (5 g, 25.49 mmol) was dissolved in dichloromethane (50 mL), and DIEA (3.95 g, 30.59 mmol, 5.5 mL), DMAP (155.70 mg, 1.27 mmol) and di-tert-butyl dicarbonate (6.68 g, 30.59 mmol) were sequentially added. The mixture was stirred and reacted at 25°C for 2 hours; water (100 mL) was added to the reaction solution, and the mixture was extracted with dichloromethane (100 mL × 3); the organic phases were combined, washed with a saturated sodium chloride aqueous solution (100 mL × 3), dried over anhydrous sodium sulphate, and filtered, the filtrate was concentrated under reduced pressure, and the concentrated residue was purified using eluent system C to obtain **12b** (6.5 g, yield: 86.0%).

Step 2: **12b** (5 g, 16.88 mmol) was dissolved in methanol (60 mL), Pd/C (2.05 g, 1.69 mmol, 10% purity) was added, and the mixture was stirred and reacted at 50°C for 16 hours; a layer of Celite was spread evenly inside a suction filter funnel, the reaction solution was filtered, and the filtrate was collected and concentrated under reduced pressure to obtain **12c** (4 g, yield: 89.0%). MS m/z (ESI): 267 [M+1]⁺

Step 3: **12c** (1 g, 3.76 mmol) was dissolved in N,N-dimethylformamide (12 mL), ethyl 2-oxoacetate **12d** (575.05 mg, 5.63 mmol, 559 µL) was added, and the mixture was stirred and reacted under microwave irradiation at 100°C for 10 minutes. Upon completion of the reaction, the reaction product was used directly in the next step.

Step 4: Methyl 4-(tert-butyloxycarbonylamino)-3-[(E)-(2-methoxy-2-oxo-ethylidene)amino]benzoate **12e** (600 mg, 1.78 mmol) was dissolved in N,N-dimethylformamide (7 mL), and tosylmethyl isocyanide **12f** (417.95 mg, 2.14 mmol) and potassium carbonate (493.11 mg, 3.57 mmol) were added. The mixture was stirred and reacted under microwave irradiation at 80°C for 10 minutes; water (60 mL) was added to the reaction solution, and the mixture was extracted with ethyl acetate (60 mL × 3); the organic phases were combined, washed with a saturated sodium chloride aqueous solution (60 mL × 3), dried over anhydrous sodium sulphate, and filtered, and the filtrate was concentrated under reduced pressure to obtain **12g** (700 mg), which was used directly in the next step.

Step 5: **12g** (600 mg, 1.54 mmol) was dissolved in 1,2-dichloroethane (6 mL)/trifluoroacetic acid (0.6 mL), and the mixture was stirred and reacted under microwave irradiation at 80°C for 10 minutes; the reaction solution was concentrated under reduced pressure, and the concentrated residue was stirred with dichloromethane (10 mL) for 15 minutes and filtered. The filter cake was dried under reduced pressure to obtain **12h** (200 mg, yield: 53.4%). MS m/z (ESI): 244 [M+1]⁺

Step 6: **12h** (200 mg, 822 µmol) was dissolved in phosphorus oxychloride (3 mL), and the mixture was stirred and reacted at 180°C for 8 hours; the reaction solution was concentrated under reduced pressure, water (15 mL) was added to the concentrated residue, and the organic phase was separated; the aqueous layer was extracted with ethyl acetate (15 mL × 3); the organic phases were combined, washed with a saturated sodium chloride aqueous solution (15 mL × 3), dried over anhydrous sodium sulphate, and filtered, and the filtrate was concentrated under reduced pressure to obtain **12i** (100 mg), which was used directly in the next step. MS m/z (ESI): 262 [M+1]⁺

Step 7: **12i** (100 mg, 383 µmol) was dissolved in N,N-dimethylformamide (2 mL), and 4-methoxybenzylamine (99.52 mg, 726 µmol) and potassium carbonate (150.40 mg, 1.09 mmol) were sequentially added. The mixture was stirred and reacted at 120°C for 2 hours; water (20 mL) was added to the reaction solution, and the organic phase was separated; the aqueous layer was extracted with ethyl acetate (20 mL × 3); the organic phases were combined, washed with a saturated sodium chloride aqueous solution (20 mL × 3), dried over anhydrous sodium sulphate, and filtered, and the filtrate was concentrated under reduced pressure to obtain **12j** (200 mg), which was used directly in the next step. MS m/z (ESI): 363 [M+1]⁺

Step 8: **12j** (100 mg, 276µmol) was dissolved in water (1 mL)/methanol (1 mL)/tetrahydrofuran (3 mL), lithium hydroxide (25.45 mg, 1.06 mmol) was added, and the mixture was stirred and reacted at 80°C for 3 hours, then adjusted to pH 5 with 0.5 M hydrochloric acid, and filtered. The filter cake was rinsed with water (5 mL) and then dried under reduced pressure to obtain **12k** (60 mg, yield: 62.4%). MS m/z (ESI): 349 [M+1]⁺

Step 9: **12k** (50 mg, 144 µmol) and N-(cyclopropylmethyl)-2-(trifluoromethyl)-6,8-dihydro-5H-pyrano[3,4-b]pyridin-5-amine **12l** (40 mg, 172 µmol) were dissolved in N,N-dimethylformamide, N-methylpyrazole (45.31 mg, 552 µmol, 46 µL) and TCFH (58.07 mg, 207 µmol) were added, and the mixture was stirred and reacted at 25°C for 16 hours; the reaction solution was directly separated and purified by Pre-HPLC to obtain **12m** (18 mg, yield: 22.3%). MS m/z (ESI): 563 [M+1]⁺

Step 10: **12m** (18 mg, 32 µmol) was dissolved in trifluoroacetic acid (2 mL), and the mixture was stirred and reacted at 90°C for 3 hours; the reaction solution was concentrated under reduced pressure, and the concentrated residue was separated by Pre-HPLC (acidic) to obtain **Example 12** (6 mg, yield: 42.4%). MS m/z (ESI): 443[M+1]⁺

¹H NMR (400 MHz, DMSO-d₆) δ 9.21 (s, 2H), 8.38 (s, 1H), 8.12 (d, 1H), 7.94 (s, 1H), 7.87 (d, 1H), 7.62 - 7.44 (m, 3H), 5.83 (s, 1H), 4.93 - 4.61 (m, 2H), 4.21 (s, 2H), 2.81 (s, 3H).

### Example 13

### 4-Amino-N,1-dimethyl-N-(2-(trifluoromethyl)-4,7-dihydro-5H-thieno[2,3-c]pyran-4-yl)-1H-pyrazolo[4,3-c]quinoline-8-carboxamide

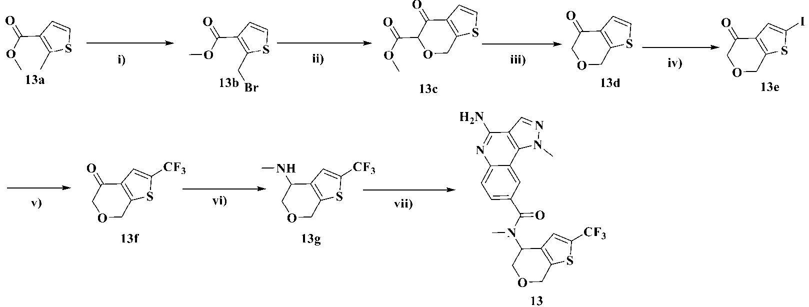

Step 1: Methyl 2-methyl-thiophene-3-carboxylate 13a (8.00 g, 51.22 mmol) was dissolved in carbon tetrachloride (100 mL), NBS (10.03 g, 56.34 mmol) and AIBN (841 mg, 5.12 mmol) were added, and the mixture was stirred and reacted at 80°C for 16 hours; water (100 mL) was added to the reaction solution, and the mixture was extracted with dichloromethane (200 mL × 3); the organic phases were combined, washed with a saturated sodium chloride aqueous solution (200 mL × 3), dried over anhydrous sodium sulphate, and filtered, the filtrate was concentrated under reduced pressure, and the concentrated residue was purified using eluent system C to obtain **13b** (8.60 g, yield: 71.4%). MS m/z (ESI): 235 [M+1]⁺.

Step 2: At 0°C, sodium hydride (510 mg, 12.76 mmol, 60% purity) was added to a solution of methyl 2-hydroxyacetate (766 mg, 8.51 mmol) in DMF (3 mL). After the mixture was stirred at 0°C for 20 minutes, **13b** (1.00 g, 4.25 mmol) was added to the reaction solution, and the resulting mixture was stirred at room temperature for 16 hours. The reaction solution was diluted with ethyl acetate and washed with water and a sodium chloride aqueous solution. The organic phase was concentrated, and the residue was purified using eluent system C to obtain **13c** (430 mg, yield: 47.6%). MS m/z (ESI): 213 [M+1]⁺.

Step 3: **13c** (3.10 g, 14.61 mmol) was dissolved in dioxane (15 mL) and concentrated hydrochloric acid (15 mL, 12 M). The reaction solution was heated to 100°C and stirred for 2 hours. The reaction solution was concentrated, and the residue was extracted with dichloromethane (80 mL × 3). The organic phases were combined, washed with a saturated sodium chloride aqueous solution (80 mL × 2), dried over anhydrous sodium sulphate, and filtered, the filtrate was concentrated under reduced pressure, and the concentrated residue was purified using eluent system C to obtain **13d** (1.80 g, yield: 79.9%). MS m/z (ESI): 155 [M+1]⁺.

Step 4: **13d** (800 mg, 5.19 mmol) and NIS (1.20 g, 6.75 mmol) were dissolved in acetonitrile (20 mL) and acetic acid (5 mL). The reaction solution was stirred at 30°C for 6 hours. The reaction solution was diluted with ethyl acetate and washed with water and a sodium chloride aqueous solution. The organic phase was concentrated. The concentrated residue was purified using eluent system C to obtain **13e** (680 mg, yield: 54.6%). MS m/z (ESI): 281 [M+1]⁺.

Step 5: **13e** (850 mg, 3.03 mmol), methyl 2,2-difluoro-2-(fluorosulphonyl)acetate (2.33 g, 12.14 mmol) and cuprous iodide (1.73 g, 9.10 mmol) were dispersed in DMF (10 mL), and under nitrogen atmosphere, the mixture was stirred at 90°C for 16 hours. The reaction solution was diluted with ethyl acetate and filtered to remove solid matters, and the organic phase was washed with water and a sodium chloride aqueous solution and concentrated. The concentrated residue was purified using eluent system C to obtain **13f** (520 mg, yield: 77.1%).

Step 6: **13f** (100 mg, 0.43 mmol) and methylamine (237 mg, 2.15 mmol, 28% methanol solution) were dissolved in trifluoroethanol (3 mL). The reaction solution was stirred at room temperature for 3 hours. Sodium borohydride (16 mg, 0.49 mmol) was added to the reaction solution, and the mixture was stirred at room temperature for 1 hour. The reaction solution was diluted with ethyl acetate and washed with water and a sodium chloride aqueous solution. The organic phase was concentrated. The concentrated residue was purified using eluent system C to obtain **13g** (58 mg, yield: 46.9%). MS m/z (ESI): 238 [M+1]⁺.

Step 7: **13g** (20 mg, 0.08 mmol) and 4-amino-1-methyl-1H-pyrazolo[4,3-c]quinoline-8-carboxylic acid (39 mg, 0.16 mmol) were dissolved in N,N-dimethylformamide (2 mL), N-methylpyrazole (20 mg, 0.24 mmol) and TCFH (45 mg, 0.16 mmol) were added, and the mixture was stirred and reacted at 25°C for 16 hours; the reaction solution was directly separated and purified by Pre-HPLC to obtain Example 13 (16 mg, yield: 42.0%). MS m/z (ESI): 462 [M+1]⁺

¹H NMR (400 MHz, CD₃OD) δ 8.54 (d, 1H), 8.49 (s, 1H), 7.93 (d, 1H), 7.81 (d, 1H), 7.61 (d, 1H), 5.76 (s, 1H), 4.99 (s, 1H), 4.79 (d, 1H), 4.54 (s, 3H), 4.38 (d, 1H), 4.10 (d, 1H), 2.91 (s, 3H).

### Example 14

### 4-Amino-N-(cyclopropylmethyl)-3-methyl-N-[2-(trifluoromethyl)-6,8-dihydro-5-pyrano[3,4-b]]pyridin-5-yl]imidazo[1,5-a]quinoxaline-8-carboxamide

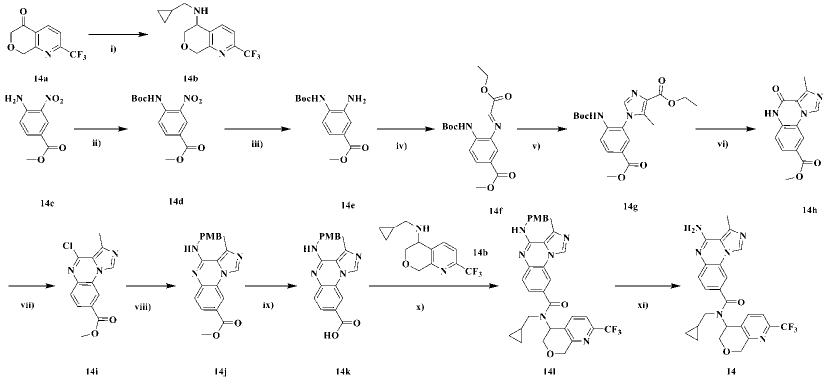

Step 1: 2-(Trifluoromethyl)-8H-pyrano[3,4-b]pyridin-5-one 14a (500 mg, 2.30 mmol) and cyclopropylmethanamine (1.31 g, 18.42 mmol, 1.6 mL) were dissolved in methanol (15 mL), tetraisopropyl titanate (1.96 g, 6.91 mmol, 2 mL) was added, and the mixture was stirred and reacted at 25°C for 16 hours. Sodium borohydride acetate (732.02 mg, 3.45 mmol) was added, and the mixture was stirred at 25°C for 30 minutes; a saturated sodium bicarbonate aqueous solution (8 mL) was added to the reaction solution, and the mixture was filtered. The filtrate was collected and concentrated under reduced pressure, and the concentrated residue was separated by Pre-TLC to obtain **14b** (52 mg, yield: 8.3%). MS m/z (ESI): 273 [M+1]⁺

Step 2: Methyl 4-amino-3-nitro-benzoate **14e** (5 g, 25.49 mmol) was dissolved in dichloromethane (50 mL), and DIEA (3.95 g, 30.59 mmol, 5.5 mL), DMAP (155.70 mg, 1.27 mmol) and di-tert-butyl dicarbonate (6.68 g, 30.59 mmol) were sequentially added. The mixture was stirred and reacted at 25°C for 2 hours; water (100 mL) was added to the reaction solution, and the mixture was extracted with dichloromethane (100 mL × 3); the organic phases were combined, washed with a saturated sodium chloride aqueous solution (100 mL × 3), dried over anhydrous sodium sulphate, and filtered, the filtrate was concentrated under reduced pressure, and the concentrated residue was purified using eluent system C to obtain **14d** (6.5 g, yield: 86.0%).

Step 3: **14d** (5 g, 16.88 mmol) was dissolved in methanol (60 mL), Pd/C (2.05 g, 1.69 mmol, 10%wt) was added, and the mixture was stirred and reacted at 50°C for 16 hours; a layer of Celite was spread evenly inside a suction filter funnel, the reaction solution was filtered, and the filtrate was collected and concentrated under reduced pressure to obtain **14e** (4 g, yield: 89.0%). MS m/z (ESI): 267 [M+1]⁺

Step 4: **14e** (1 g, 3.76 mmol) was dissolved in N,N-dimethylformamide (12 mL), ethyl 2-oxoacetate (575.05 mg, 5.63 mmol, 559 µL) was added, and the mixture was stirred and reacted under microwave irradiation at 100°C for 10 minutes. Upon completion of the reaction, the reaction product was used directly in the next step.

Step 5: Methyl 4-(tert-butyloxycarbonylamino)-3-[(E)-(2-methoxy-2-oxo-ethylidene)amino]benzoate **14f** (500 mg, 1.49 mmol) was dissolved in N,N-dimethylformamide (7 mL), and 1-methyl-1-tosylmethyl isocyanide (373.30 mg, 1.78 mmol) and potassium carbonate (410.92 mg, 2.97 mmol) were added. The mixture was stirred and reacted under microwave irradiation at 80°C for 10 minutes; water (60 mL) was added to the reaction solution, and the mixture was extracted with ethyl acetate (60 mL × 3); the organic phases were combined, washed with a saturated sodium chloride aqueous solution (60 mL × 3), dried over anhydrous sodium sulphate, and filtered, and the filtrate was concentrated under reduced pressure to obtain **14g,** which was used directly in the next step.

Step 6: **14g** (600 mg, 1.49 mmol) was dissolved in 1,2-dichloroethane (6 mL)/trifluoroacetic acid (0.6 mL), and the mixture was stirred and reacted under microwave irradiation at 80°C for 10 minutes; the reaction solution was concentrated under reduced pressure, and the concentrated residue was stirred with dichloromethane (10 mL) for 15 minutes and filtered. The filter cake was dried under reduced pressure to obtain **14h** (200 mg, yield: 52.3%). MS m/z (ESI): 258 [M+1]⁺

Step 7: **14h** (200 mg, 778 µmol) was dissolved in phosphorus oxychloride (3 mL), and the mixture was stirred and reacted at 180°C for 8 hours; the reaction solution was concentrated under reduced pressure, water (15 mL) was added to the concentrated residue, and the organic phase was separated; the aqueous layer was extracted with ethyl acetate (15 mL × 3); the organic phases were combined, washed with a saturated sodium chloride aqueous solution (15 mL × 3), dried over anhydrous sodium sulphate, and filtered, and the filtrate was concentrated under reduced pressure to obtain **14i** (100 mg), which was used directly in the next step. MS m/z (ESI): 276 [M+1]⁺

Step 8: **14i** (100 mg, 363 µmol) was dissolved in N,N-dimethylformamide (2 mL), and 4-methoxybenzylamine (99.52 mg, 726 µmol) and potassium carbonate (150.40 mg, 1.09 mmol) were sequentially added. The mixture was stirred and reacted at 120°C for 2 hours; water (20 mL) was added to the reaction solution, and the organic phase was separated; the aqueous layer was extracted with ethyl acetate (20 mL × 3); the organic phases were combined, washed with a saturated sodium chloride aqueous solution (20 mL × 3), dried over anhydrous sodium sulphate, and filtered, and the filtrate was concentrated under reduced pressure to obtain **14j** (200 mg), which was used directly in the next step. MS m/z (ESI): 377 [M+1]⁺

Step 9: **14j** (100 mg, 266 µmol) was dissolved in water (1 mL)/methanol (1 mL)/tetrahydrofuran (3 mL), lithium hydroxide (25.45 mg, 1.06 mmol) was added, and the mixture was stirred and reacted at 80°C for 3 hours, then adjusted to pH 5 with 0.5 M hydrochloric acid, and filtered. The filter cake was rinsed with water (5 mL) and then dried under reduced pressure to obtain **14k** (60 mg, yield: 62.3%). MS m/z (ESI): 363 [M+1]⁺

Step 10: **14k** (50 mg, 138 µmol) and **14b** (45.08 mg, 166 µmol) were dissolved in N,N-dimethylformamide, N-methylpyrazole (45.31 mg, 552 µmol, 46 µL) and TCFH (58.07 mg, 207 µmol) were added, and the mixture was stirred and reacted at 25°C for 16 hours; the reaction solution was directly separated and purified by Pre-HPLC to obtain **14l** (18 mg, yield: 21.2%). MS m/z (ESI): 617 [M+1]⁺

Step 11: **141** (18 mg, 29 µmol) was dissolved in trifluoroacetic acid (2 mL), and the mixture was stirred and reacted at 90°C for 3 hours; the reaction solution was concentrated under reduced pressure, and the concentrated residue was separated by Pre-HPLC (acidic) to obtain **Example 14** (6 mg, yield: 41.4%). MS m/z (ESI): 497 [M+1]⁺

¹H NMR (400 MHz, DMSO-d₆) δ 9.07 (s, 1H), 8.25 (d, 2H), 7.81 (d, 1H), 7.41 (s, 2H), 6.88 (s, 2H), 5.32 (t, 1H), 4.94 - 4.63 (m, 2H), 4.28 (s, 2H), 2.63 (s, 3H), 2.00 (q, 1H), 1.26 - 1.21 (m, 2H), 1.09 - 0.73 (m, 2H), 0.32 (d, 2H).

The examples in the table below were prepared with reference to **Example 1:**

| **Example** | **Structure** | **MS m/z (ESI)** |
|---|---|---|
| | | **[M+1]⁺** |
| **15** | 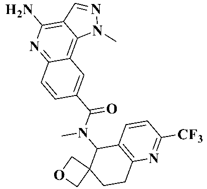 | 497 |
| **16** | 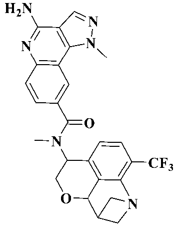 | 509 |
| **17** | 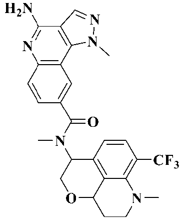 | 511 |
| **18** | 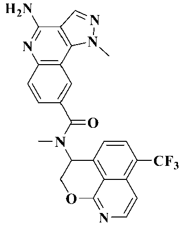 | 493 |
| **19** | 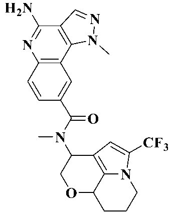 | 485 |
| **20** | 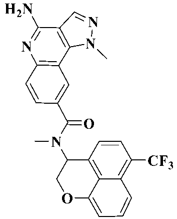 | 492 |
| **21** | 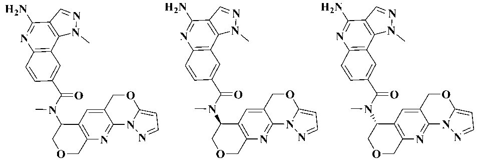 | 483 |
| **22** | 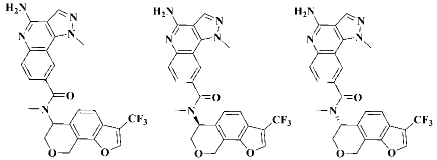 | 496 |
| **23** | 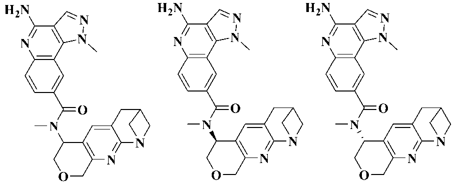 | 456 |
| **24** | 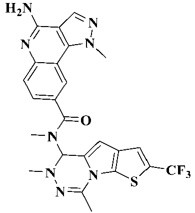 | 527 |
| **25** | 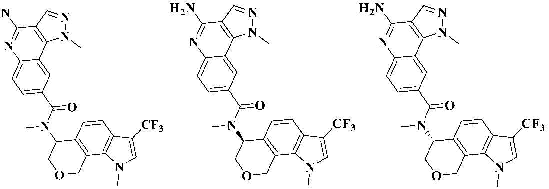 | 509 |
| **26** | 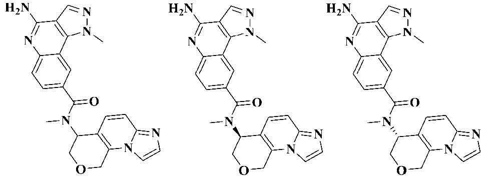 | 428 |
| **27** | 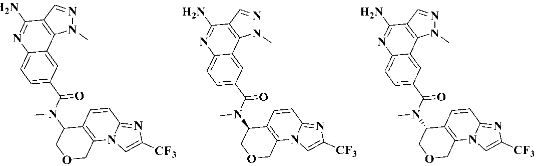 | 496 |

### Example 28

### 4-Amino-7-(methoxymethyl)-N-methyl-N-[2-(trifluoromethyl)-6,8-dihydro-5H-pyrano[3,4-b]pyridin-5-yl]imidazo[1,5-a]quinoxaline-8-carboxamide

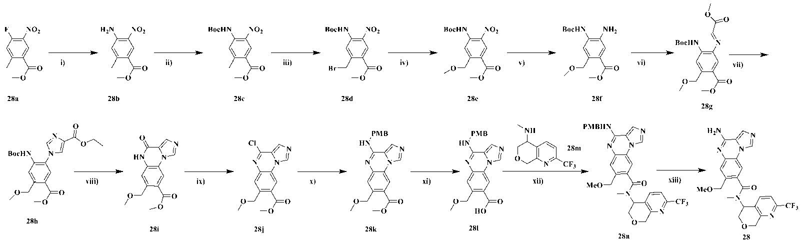

Step 1: Methyl 4-fluoro-2-methyl-5-nitro-benzoate **28a** (20 g, 93.83 mmol) was dissolved in 1'4-dioxane, aqueous ammonia (2 M, 93.8 mL) was added, and the mixture was stirred and reacted at 25°C for 16 hours; the reaction solution was concentrated under reduced pressure to obtain a crude product; the crude product was stirred with dichloromethane (100 mL) for 15 minutes and filtered, and the filter cake was dried under reduced pressure to obtain **28b** (22 g, crude). MS m/z (ESI): 211 [M+1]⁺

Step 2: **28b** (22 g, 104.67 mmol) was dissolved in DMF (150 mL), and the reaction system was cooled to 0°C. Boc₂O (34.27 g, 157.00 mmol, 36.0 mL), DIEA (16.23 g, 125.60 mmol, 21.9 mL) and DMAP (1.28 g, 10.47 mmol) were sequentially added, and the mixture was stirred and reacted at 25°C for 2 hours; water (200 mL) was added to the reaction solution, and the mixture was extracted with ethyl acetate (200 mL × 3); the organic phases were combined, washed with a saturated sodium chloride aqueous solution (200 mL × 3), dried over anhydrous sodium sulphate, and filtered, the filtrate was concentrated under reduced pressure, and the concentrated residue was purified using eluent system C to obtain **28c** (18 g, yield: 55.4%).

¹H NMR (400 MHz, DMSO-d₆) δ 9.82 (s, 1H), 8.42 (s, 1H), 7.80 (s, 1H), 3.85 (s, 3H), 2.58 (s, 3H), 1.48 (s, 9H).

Step 3: **28c** (10 g, 32.23 mmol) was dissolved in carbon tetrachloride (100 mL), NBS (17.21 g, 96.68 mmol) and BPO (1.12 g, 3.22 mmol, 70% purity) were added, and the mixture was stirred and reacted at 80°C for 16 hours; water (100 mL) was added to the reaction solution, and the mixture was extracted with dichloromethane (200 mL × 3); the organic phases were combined, washed with a saturated sodium chloride aqueous solution (200 mL × 3), dried over anhydrous sodium sulphate, and filtered, and the filtrate was concentrated under reduced pressure; tetrahydrofuran (120 mL) was added to the residue, diethyl phosphite (7.03 g, 51.27 mmol, 6.6 mL) and DIEA (6.63 g, 51.27 mmol, 9.0 mL) were added, and the mixture was stirred at 25°C for 1 hour; water (100 mL) was added, and the mixture was extracted with ethyl acetate (120 mL × 3); the organic phases were combined, washed with a saturated sodium chloride aqueous solution (120 mL × 3), dried over anhydrous sodium sulphate, and filtered, the filtrate was concentrated under reduced pressure, and the concentrated residue was purified using eluent system C to obtain **28d** (9 g, yield: 90.2%).

1H NMR (400 MHz, DMSO-d₆) δ 9.93 (s, 1H), 8.44 (s, 1H), 8.03 (s, 1H), 5.05 (s, 2H), 3.89 (s, 3H), 1.47 (s, 9H).

Step 4: **28d** (4.8 g, 12.33 mmol) was dissolved in DMF (50 mL), sodium methoxide (5 M, 9.9 mL, 30% purity)/DMF (20 mL) were added dropwise, and the mixture was stirred and reacted at 25°C for 2 hours; water (150 mL) was added to the reaction solution, and the mixture was extracted with ethyl acetate (150 mL × 3); the organic phases were combined, washed with a saturated sodium chloride aqueous solution (150 mL × 3), dried over anhydrous sodium sulphate, and filtered, the filtrate was concentrated under reduced pressure, and the concentrated residue was purified using eluent system C to obtain **28e** (1.3 g, yield: 31.0%).

Referring to the synthesis method described in Step 3 to Step 11 of **Example 14, Example 28** (5.5 mg, yield: 1%) was obtained from **28e** (500 mg). MS m/z (ESI): 487 [M+1]⁺

¹H NMR (400 MHz, DMSO-d₆) δ 9.07 (s, 1H), 8.15 (s, 2H), 7.96 - 7.78 (m, 2H), 7.48 (s, 1H), 7.16 (s, 2H), 5.92 (s, 1H), 4.83 (d, 2H), 4.52 (d, 2H), 4.22 (s, 2H), 3.33 (s, 3H), 2.86 - 2.60 (m, 3H).

The examples in the table below were prepared with reference to **Example 13:**

| **Example** | **Structure** | **MS m/z (ESI)** |
|---|---|---|
| | | **[M+1]⁺** |
| **29** | 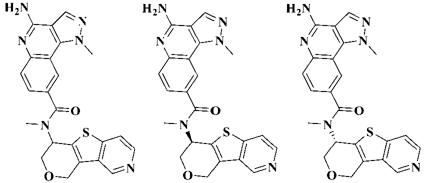 | 445 |
| **30** | 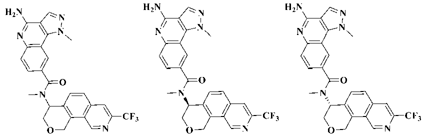 | 507 |
| **31** | 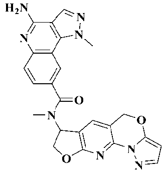 | 469 |

### Example 32

### 4-Amino-7-fluoro-N-methyl-N-[2-(trifluoromethyl)-6,8-dihydro-5H-pyrano[3,4-b]pyridin-5-yl]imidazo[1,5-a]quinoxaline-8-carboxamide

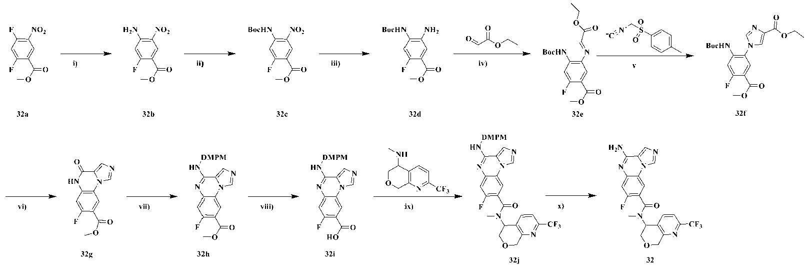

Step 1: Methyl 2,4-difluoro-5-nitro-benzoate 32a (12 g, 55.27 mmol) was dissolved in tetrahydrofuran (50 mL), ammonia (2 M, 10.4 mL) was added in an ice bath, and the mixture was stirred and reacted at 0°C for 0.5 hours. Tetrahydrofuran (50 mL) was then added, and the resulting mixture was stirred and reacted at 25°C for 16 hours; the reaction solution was concentrated under reduced pressure, water (100 mL) was added to the concentrated residue, and the mixture was stirred for 15 minutes and filtered. The filter cake was dried under reduced pressure to obtain **32b** (12 g, crude). MS m/z (ESI): 214 [M+1]⁺

Referring to the synthesis method described in Step 1 to Step 10 of **Example 12, Example 32** (10 mg, yield: 0.9%) was obtained from **32b** (500 mg, 2.33 mmol). MS m/z (ESI): 461 [M+1]⁺

¹H NMR (400 MHz, DMSO-d₆) δ 9.06 (s, 1H), 8.32 (s, 1H), 8.19 - 7.68 (m, 3H), 7.38 (s, 1H), 7.24 (d, 1H), 5.89 (s, 1H), 4.83 (d, 2H), 4.32 - 3.99 (m, 2H), 2.78 (s, 3H).

### Example 33

### 4-Amino-N,7-dimethyl-N-[2-(trifluoromethyl)-6,8-dihydro-5H-pyrano[3,4-b]pyridin-5-yl]imidazo[1,5-a]quinoxaline-8-carboxamide

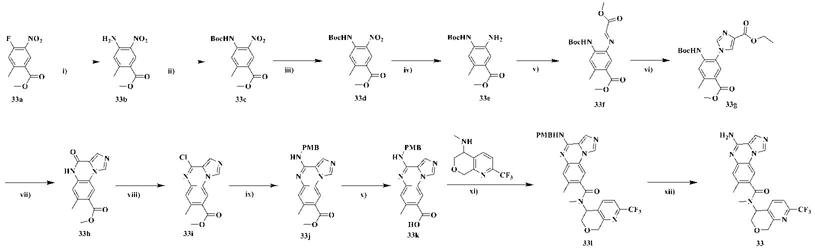

Step 1: Methyl 4-fluoro-2-methyl-5-nitro-benzoate **33a** (10 g, 46.92 mmol) was dissolved in 1'4-dioxane, aqueous ammonia (2 M, 46.9 mL) was added, and the mixture was stirred and reacted at 25°C for 16 hours; the reaction solution was concentrated under reduced pressure to obtain a crude product; the crude product was stirred with dichloromethane (50 mL) for 15 minutes and filtered, and the filter cake was dried under reduced pressure to obtain **33b** (11 g, crude). MS m/z (ESI): 211 [M+1]⁺

Referring to the synthesis method described in Step 1 to Step 10 of **Example 12, Example 33** (9.8 mg, yield: 1.2%) was obtained from **33b** (500 mg, 1.78 mmol). MS m/z (ESI): 458 [M+1]⁺

¹H NMR (400 MHz, DMSO-d₆) δ 9.02 (s, 1H), 8.25 - 7.95 (m, 2H), 7.88 (s, 2H), 7.40 (d, 1H), 7.10 (s, 2H), 5.94 (s, 1H), 4.92 - 4.66 (m, 2H), 4.22 (s, 2H), 2.74 (d, 3H), 2.39 (d, 3H).

### Example 34

### 4-Amino-N, 7-dimethyl-N-(8-methyl-2-(trifluoromethyl)-5,8-dihydro-6H-pyrano[3,4-b]pyridin-5-yl)imidazo[1,5-a]quinoxaline-8-carboxamide

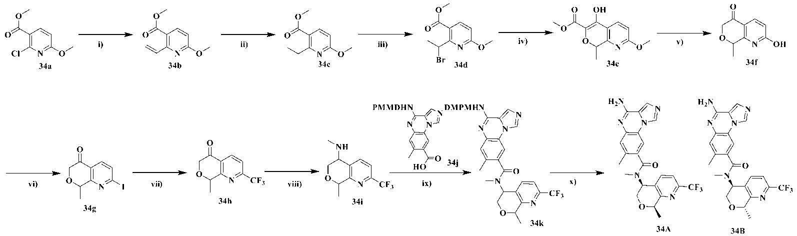

Step 1: Methyl 2-chloro-6-methoxy-pyridine-3-carboxylate **34a** (9 g, 44.64 mmol), potassium vinylfluoroborate (11.96 g, 89.28 mmol) and potassium carbonate (12.34 g, 89.28 mmol) were dissolved in water (20 mL) and 1'4-dioxane (100 mL), and the mixture was purged three times with nitrogen. 1,1'-Bis(diphenylphosphino)ferrocene palladium(II) dichloride (3.27 g, 4.46 mmol) was added, and the reaction system was stirred at 90°C for 15 hours. The reaction system was cooled to room temperature, and water was added. The mixture was extracted with ethyl acetate (500 mL × 3), and the organic phases were combined, washed with a saturated sodium chloride aqueous solution (200 mL), dried over anhydrous sodium sulphate, and filtered. The filtrate was concentrated under reduced pressure, and the residue was purified by silica gel column chromatography using eluent system B to obtain **34b** (6 g, yield: 69.6%). MS m/z (ESI): 194 [M+1]⁺

Step 2: **34b** (6 g, 31.06 mmol) was dissolved in methanol (100 mL), and the mixture was purged three times with nitrogen. Palladium on carbon (377 mg, 0.31 mmol, 10% purity) was added, and the reaction system was stirred at 25°C under a hydrogen balloon for 2 hours. The reaction system was filtered and concentrated to obtain **34c** (5.90 g, crude). MS m/z (ESI): 196 [M+1]⁺

Step 3: **34c** (5.9 g, 30.22 mmol), azobisisobutyronitrile (496 mg, 3.02 mmol) and *N*-bromosuccinimide (8.61 g, 48.36 mmol) were dissolved in carbon tetrachloride (60 mL), and the mixture was purged three times with nitrogen. The system was heated to 90°C and reacted. The reaction system was cooled to room temperature and concentrated. Water was added, and the mixture was extracted with ethyl acetate (100 mL × 3), washed with a sodium chloride aqueous solution (100 mL), dried over anhydrous sodium sulphate, filtered, and concentrated. The residue was purified by silica gel column chromatography using eluent system B to obtain **34d** (7.50 g, yield: 90.5%). MS m/z (ESI): 275 [M+1]⁺

Referring to the synthesis method described in Step 3 to Step 4 of **Example 9, 34f** (2.60 g, yield: 51.0%) was obtained from **34d** (7.80 g, 28.46 mmol). MS m/z (ESI): 180 [M+1]⁺

Step 6: **34f** (0.5 g, 2.79 mmol) was dissolved in dichloromethane (40 mL), and the mixture was purged three times with nitrogen. The reaction system was cooled to 0°C, triethylamine (339 mg, 3.35 mmol, 0.47 mL) and trifluoromethanesulphonic anhydride (866 mg, 3.07 mmol) were added, and the system was reacted at 25°C for 2 hours. Sodium iodide (2.09 g, 13.95 mmol) and concentrated hydrochloric acid (12 M, 0.28 mL) were then added to the system. The system was reacted at 20°C for 15 hours. The system was filtered and concentrated. The residue was purified by silica gel column chromatography using eluent system B to obtain **34g** (0.35 g, yield: 43.4%). MS m/z (ESI): 290 [M+1]⁺

Step 7: **34g** (0.32 g, 1.11 mmol) and cuprous iodide (527 mg, 2.77 mmol) were dissolved in N,N-dimethylformamide (5 mL), methyl 2,2-difluoro-2-(fluorosulphonyl)acetate (532 mg, 2.77 mmol) was added, and the mixture was purged three times with nitrogen, then heated to 100°C and reacted for 2 hours. The reaction system was cooled to room temperature and concentrated. Water was added, and the mixture was extracted with ethyl acetate (5 mL × 3), washed with a sodium chloride aqueous solution (3 mL), dried over anhydrous sodium sulphate, filtered, and concentrated. The residue was purified by silica gel column chromatography using eluent system B to obtain **34h** (0.14 g, yield: 54.7%). MS m/z (ESI): 232 [M+1]⁺

Step 8: **34h** (0.14 g, 0.61 mmol) and a methylamine alcohol solution (314 mg, 3.03 mmol, 30% wt) were dissolved in trifluoroethanol (10 mL), and the mixture was purged three times with nitrogen and then reacted at 25°C for 2 hours. Sodium cyanoborohydride (76 mg, 1.21 mmol) was added to the system. After the addition, the system was reacted 25°C for 1 hour. A saturated sodium bicarbonate solution (10 mL) was added to the reaction, and the system was stirred at room temperature for one hour, extracted with dichloromethane/methanol = 10/1 (50 mL × 3), washed with a saturated sodium chloride aqueous solution (20 mL), dried over anhydrous sodium sulphate, filtered, and concentrated. The residue was purified by silica gel column chromatography using eluent system A to obtain **34i** (80 mg, yield: 53.6%). MS m/z (ESI): 247 [M+1]⁺

Referring to the synthesis method described in Step 9 to Step 10 of **Example 12,** 4-amino-*N*, 7-dimethyl-*N*-(8-methyl-2-(trifluoromethyl)-5,8-dihydro-6H-pyrano[3,4-b]pyridin-5-yl)imidazo[1,5-a]quinoxaline-8-carboxamide **34A** (23 mg), MS m/z (ESI): 471 [M+1]⁺ and 4-amino-N,7-dimethyl-N-(8-methyl-2-(trifluoromethyl)-5,8-dihydro-6H-pyrano[3,4-b]pyridin-5-yl)imidazo[1,5-a]quinoxaline-8-carboxamide **34B** (10 mg, yield: 9.5%) were obtained from 4-[(3,5-dimethoxyphenyl)methylamino]-7-methyl-imidazo[1,5-a]quinoxaline-8-carboxylic acid **34j** (160 mg, 0.41mol) and **34i** (100 mg, 0.41 mmol). MS m/z (ESI): 471 [M+1]⁺.

**34A:** ¹H NMR (400 MHz, DMSO-d₆) δ 9.05 (s, 1H), 8.12 (s, 2H), 7.89 (s, 2H), 7.36 (s, 1H), 7.12 (s, 2H), 5.96 (s, 1H), 4.96 (s, 1H), 4.31 (s, 1H), 4.08 (s, 1H), 2.73 (d, 3H), 2.38 (s, 3H), 1.50 (d, 3H).

**34B:** ¹H NMR (400 MHz, DMSO-d₆) δ 9.05 (s, 1H), 8.11 (d, 2H), 7.98-7.78 (m, 2H), 7.36 (s, 1H), 7.21 (s, 2H), 5.93 (s, 1H), 4.85 (s, 1H), 4.26 (d, 2H), 2.68 (s, 3H), 2.36 (s, 3H), 1.59 (d, 3H).

The examples in the table below were prepared with reference to **Example 12:**

| **Example** | **Structure** | **MS m/z (ESI)** |
|---|---|---|
| | | **[M+1]⁺** |
| **35** | 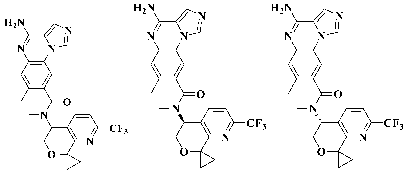 | 483 |
| **36** | 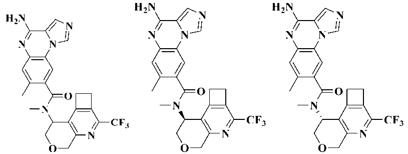 | 483 |

### Example 37

### 4-Amino-N-(2-bromo-7,10-dihydro-8H-imidazo[1,2-a]pyrano[3,4-c]pyridin-7-yl)-N,1-dimethyl-1H-pyrazolo [4,3-c] quinoline-8-carboxamide

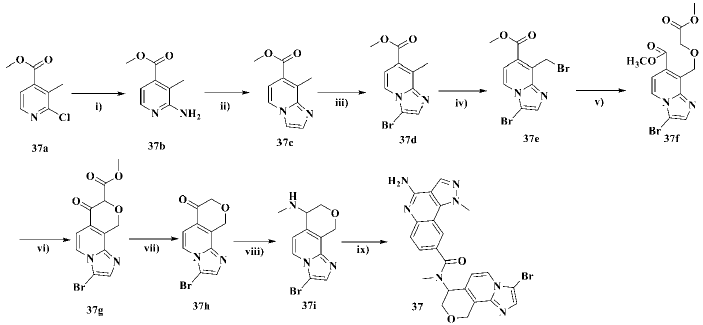

Step 1: Methyl 2-chloro-3-methyl-pyridine-4-carboxylate **37a** (5 g, 26.94 mmol), diphenylmethanimine (5.37 g, 29.63 mmol), tris(dibenzylideneacetone)dipalladium (2.47 g, 2.69 mmol), 4,5-bis(diphenylphosphino)-9,9-dimethylxanthene (3.12 g, 5.39 mmol), caesium carbonate (13.17 g, 40.41 mmol) and dioxane (25 mL) were reacted at 100°C for 16 hours. The reaction solution was yellow. The reaction solution was first quenched with H₂O, water (50 mL) and ethyl acetate (300 mL) were then added to the mixed solution, and the organic phase was washed with a saturated sodium chloride aqueous solution (30 mL × 3), then dried and subjected to rotary evaporation to dryness. The resulting residue was purified by silica gel column chromatography using eluent system A to obtain **37b** (1.9 g, yield: 42.2%). MS m/z (ESI): 167 [M+1]⁺

Step 2: **37b** (1.4 g, 8.42 mmol), 2-bromo-1,1-dimethoxy-ethane (1.42 g, 8.42 mmol), p-toluenesulphonic acid (725 mg, 4.21 mmol) and N,N-dimethylformamide (15 mL) were reacted at 100°C for 16 hours, and the reaction solution was yellow. The reaction solution was quenched with a saturated ammonium chloride solution (10 mL), and extracted with ethyl acetate (100 mL × 3). The organic phases were combined, washed with a saturated sodium chloride solution (30 mL × 2), and subjected to phase separation to obtain an organic phase, which was then dried over anhydrous sodium sulphate and filtered. The filtrate was concentrated under reduced pressure, and the resulting residue was purified by silica gel column chromatography using eluent system A to obtain **37c** (0.64 g, yield: 39.8%). MS m/z (ESI): 191 [M+1]⁺

Step 3: **37c** (60 mg, 0.315 mmol), N-bromosuccinimide (56 mg, 0.31 mmol), azobisisobutyronitrile (15 mg, 0.094 mmol) and carbon tetrachloride (4 mL) were reacted at 80°C for 16 hours, and the reaction solution was yellow. The reaction solution was cooled, concentrated, quenched with water (10 mL), and extracted with ethyl acetate (50 mL×3). The organic phases were combined, dried over anhydrous sodium sulphate, and filtered, the filtrate was concentrated under reduced pressure, and the resulting residue was purified by silica gel column chromatography using eluent system A to obtain **37d** (0.06 g, yield: 70.8%). MS m/z (ESI):269 [M+1]⁺

Step 4: **37d** (30 mg, 0.11 mmol), N-bromosuccinimide (20 mg, 0.11 mmol), azobisisobutyronitrile (6 mg, 0.034 mmol) and carbon tetrachloride (3 mL) were reacted at 80°C for 16 hours, and the reaction solution was yellow. The reaction solution was stirred and reacted at 90°C for 16 hours. The reaction solution was cooled, filtered, and subjected to rotary evaporation to dryness to obtain 37e (25 mg, yield: 65.1%). MS m/z (ESI): 349 [M+1]⁺

Step 5: **37e** (380 mg, 1.09 mmol), methyl hydroxyacetate (216 mg, 2.40 mmol), sodium hydride (65 mg, 2.73 mmol) and N,N-dimethylformamide (8 mL) were reacted at 20°C for 16 hours. The mixture was quenched with water (10 mL), and extracted with ethyl acetate (100 mL × 3). The organic phases were combined, dried over anhydrous sodium sulphate, and filtered, the filtrate was concentrated under reduced pressure, and the resulting residue was purified by silica gel column chromatography using eluent system A to obtain **37f** (180 mg, yield: 46.3%). MS m/z (ESI): 357 [M+1]⁺

Step 6: **37f** (50 mg, 0.14 mmol), lithium bis(trimethylsilyl)amide (46 mg, 0.28 mmol) and tetrahydrofuran (2 mL) were reacted at -55°C for 1 hour, and the reaction solution was yellow. The resulting residue as a crude product was purified by C₁₈ column chromatography using eluent system A to obtain **37g** (35 mg, yield: 76.9%). MS m/z (ESI): 325 [M+1]⁺

Step 7: **37g** (26 mg, 0.08 mmol), hydrochloric acid (2 mL) and ethanol (2 mL) were reacted at 80°C for 16 hours, and the reaction solution was yellow. The reaction solution was cooled, concentrated, and subjected to rotary evaporation to dryness. The resulting residue as a crude was purified by C₁₈ column chromatography using eluent system A to obtain **37h** (8 mg, yield: 37.5%). MS m/z (ESI): 267 [M+1]⁺

Step 8: **37h** (30 mg, 0.11 mmol), methylamine (7 mg, 0.225 µmol) and trifluoroethanol (2 mL) were reacted at 60°C for 2 hours, and the reaction solution was yellow. The reaction solution was cooled, concentrated, and subjected to rotary evaporation to dryness. The resulting residue as a crude was purified by C₁₈ column chromatography using eluent system A to obtain **37i** (15 mg, yield: 48.3%). MS m/z (ESI): 282 [M+1]⁺

Step 9: **37i** (24 mg, 0.086 mmol), N,N,N',N'-tetramethylchloroformamidinium hexafluorophosphate (36 mg, 0.13 mmol), N-methylimidazole (21 mg, 0.26 mmol) and N,N-dimethylformamide (4 mL) were reacted at 40°C for 1 hour, and the reaction solution was yellow. The reaction solution was quenched with water (10 mL) and extracted with ethyl acetate (20 mL × 3). The organic phases were combined, washed with water (10 mL) and a saturated sodium chloride solution (10 mL × 2), dried over anhydrous sodium sulphate, and filtered, the filtrate was concentrated under reduced pressure, and the resulting residue was purified by silica gel column chromatography using eluent system A to obtain **Example 37** (16.8 mg, yield: 38.6%). MS m/z (ESI): 506 [M+1]⁺

¹HNMR (400 MHz, DMSO-d₆) δ 8.35 (d, 1H), 8.27 (d, 2H), 7.72-7.61 (m, 3H), 7.15 (s, 3H), 4.94 (d, 3H), 4.42 (s, 3H), 4.30 (dd, 1H), 4.16 (s, 1H), 2.84 (s, 3H).

### Example 37 Preparation of chiral isomers

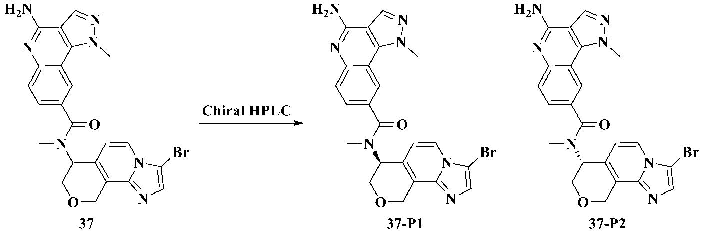

**Example 37** (16 mg, 0.032 mmol) was resolved by Chiral-HPLC (condition 1) to obtain **37-P1:** (S)-4-amino-N-(2-bromo-7,10-dihydro-8H-imidazo[1,2-a]pyrano[3,4-c]pyridin-7-yl)-N,1-dimethyl-1H-pyrazolo[4,3-c]quinoline-8-carboxamide (7.3 mg, yield: 45.6%) and **37-P2:** (R)-4-amino-N-(2-bromo-7,10-dihydro-8H-imidazo[1,2-a]pyrano[3,4-c]pyridin-7-yl)-N,1-dimethyl-1H-pyrazolo[4,3-c]quinoline-8-carboxamide (7.6 mg, yield: 47.5%), respectively.

**37-P1 (t_{R}: 6.687 min):** ¹HNMR (400 MHz, DMSO-d₆) δ 8.35 (d, 1H), 8.27 (d, 2H), 7.72-7.61 (m, 3H), 7.15 (s, 3H), 4.94 (d, 3H), 4.42 (s, 3H), 4.30 (dd, 1H), 4.16 (s, 1H), 2.84 (s, 3H).

**37-P2 (t_{R}: 9.125 min):** ¹HNMR (400 MHz, DMSO-d₆) δ 8.35 (d, 1H), 8.27 (d, 2H), 7.72-7.61 (m, 3H), 7.15 (s, 3H), 4.94 (d, 3H), 4.42 (s, 3H), 4.30 (dd, 1H), 4.16 (s, 1H), 2.84 (s, 3H).

The examples in the table below were prepared with reference to **Example 37:**

| **Example** | **Structure** | **MS m/z (ESI) [M+1]⁺** |
|---|---|---|
| **38** | 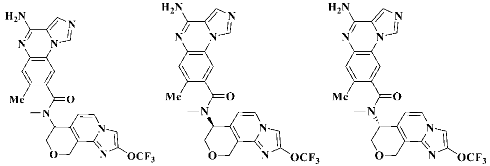 | 512 |
| **39** | 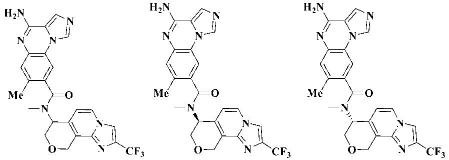 | 496 |
| **40** | 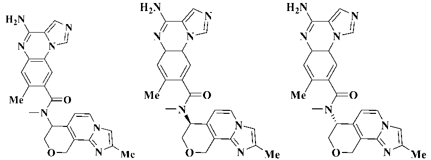 | 442 |
| **41** | 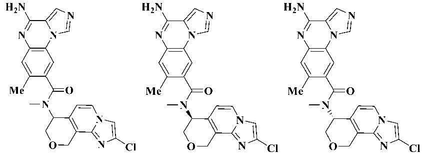 | 462 |
| **42** | 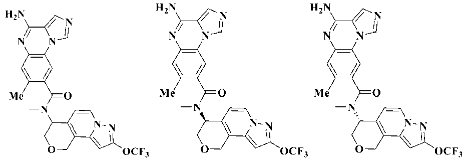 | 512 |
| **43** | 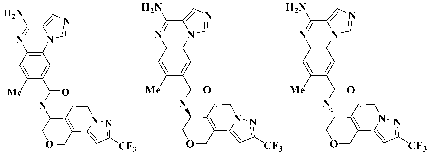 | 496 |
| **44** | 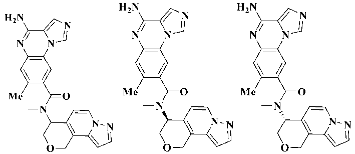 | 428 |
| **45** | 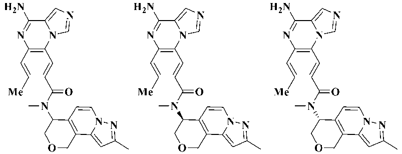 | 442 |
| **46** | 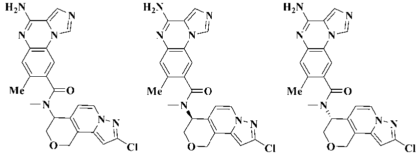 | 462 |
| **47** | 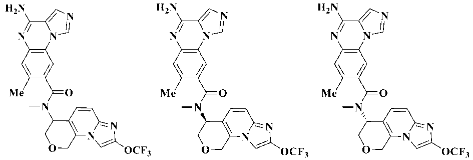 | 512 |
| **48** | 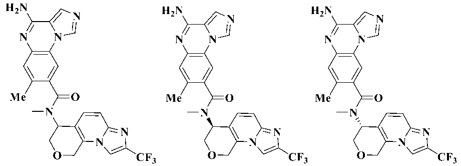 | 496 |
| **49** | 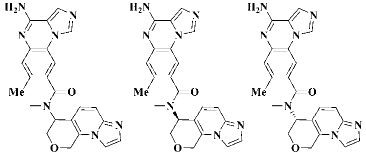 | 428 |
| **50** | 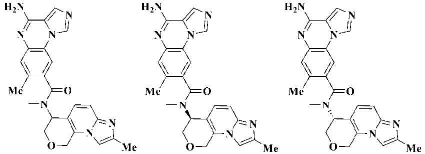 | 442 |
| **51** | 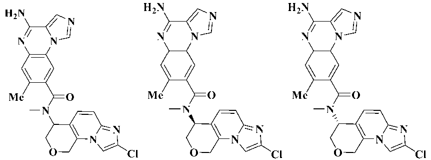 | 462 |
| **52** | 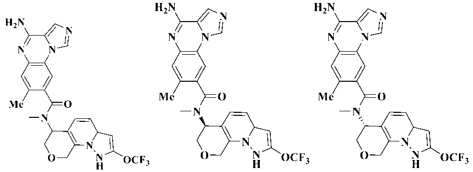 | 514 |
| **53** | 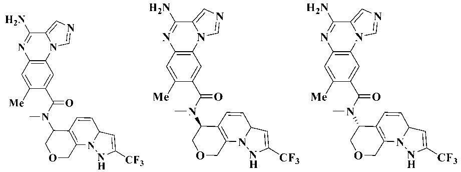 | 498 |
| **54** | 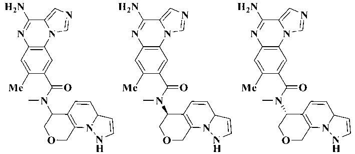 | 430 |
| **55** | 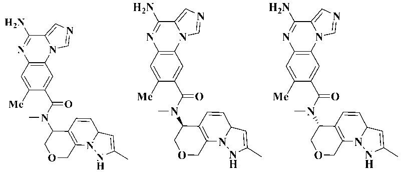 | 444 |
| **56** | 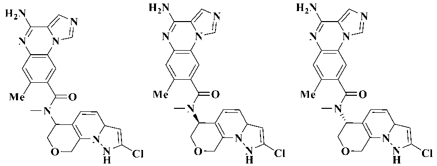 | 464 |
| **57** | 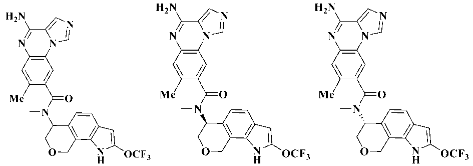 | 511 |
| **58** | 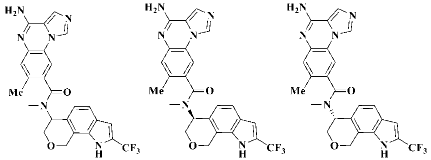 | 495 |
| **59** | 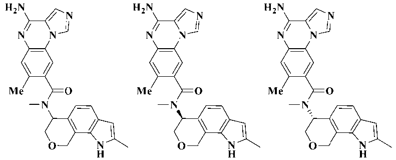 | 411 |
| **60** | 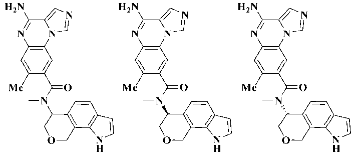 | 427 |
| **61** | 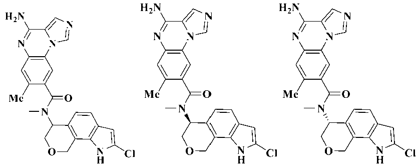 | 461 |
| **62** | 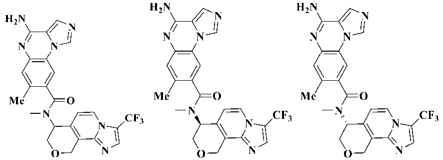 | 496 |
| **63** | 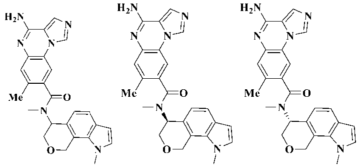 | 441 |
| **64** | 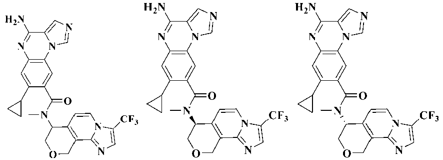 | 522 |
| **65** | 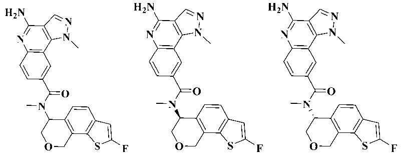 | 462 |
| **66** | 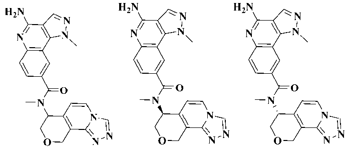 | 429 |
| **67** | 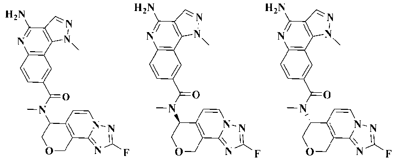 | 447 |
| **68** | 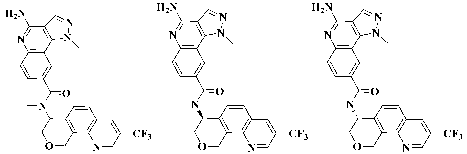 | 507 |
| **69** | 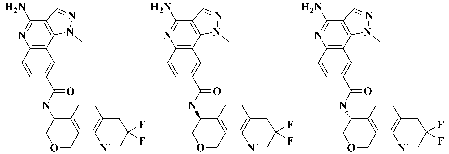 | 477 |
| **70** | 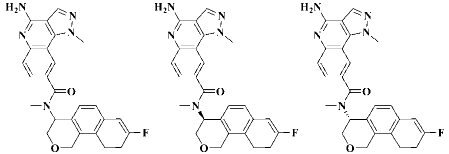 | 458 |
| **71** | 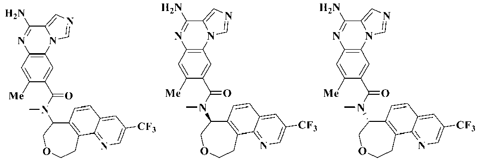 | 521 |
| **72** | 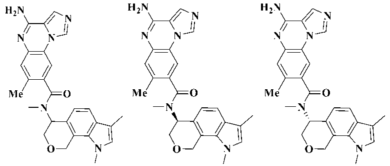 | 455 |
| **73** | 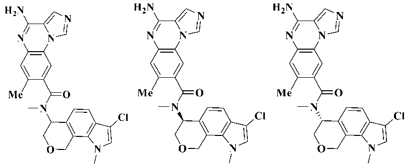 | 475 |
| **74** | 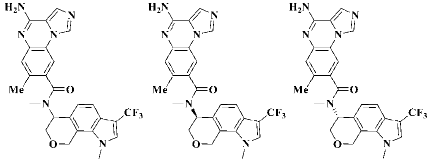 | 509 |
| **75** | 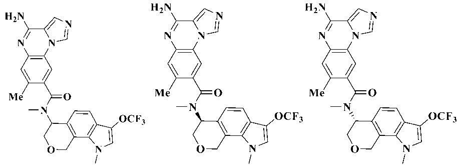 | 525 |
| **76** | 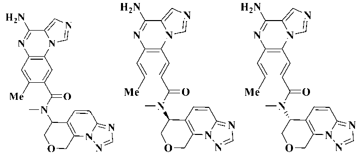 | 429 |
| **77** | 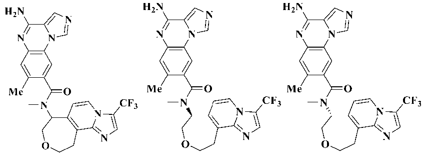 | 510 |
| **78** | 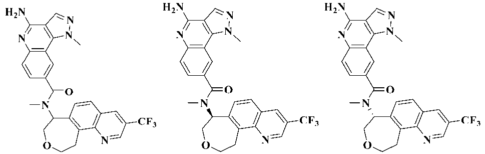 | 521 |

Alternatively, **Examples 39, 48, 62 and 68 were** prepared using the following method:

### Example 39

### 4-Amino-N,7-dimethyl-N-(2-(trifluoromethyl)-7,10-dihydro-8H-imidazo[1,2-a]pyrano[3,4-c]pyridin-7-yl)imidazo[1,5-a]quinoxaline-8-carboxamide

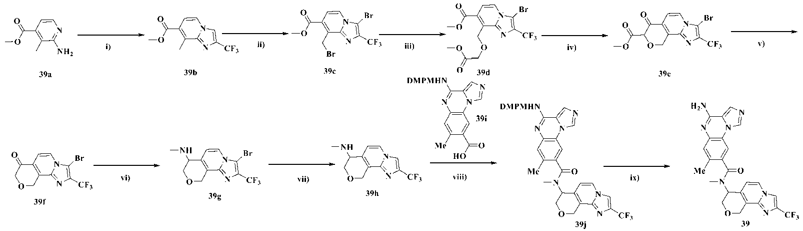

Step 1: Methyl 2-amino-3-methylisonicotinate **39a** (3 g, 18.07 mmol) and 3-bromo-1,1,1-trifluoroacetone (3.43 g, 18.07 mmol) were dispersed in acetonitrile (50 mL), and the mixture was stirred and reacted at 80°C for 16 hours. The reaction solution was concentrated under reduced pressure, and the resulting residue was purified by silica gel column chromatography using eluent system A to obtain **39b** (2.71 g, yield: 57.9%). MS m/z (ESI): 259 [M+1]⁺

Referring to the synthesis method described in Step 3 to Step 8 of **Example 34, 39g** (100 mg, yield: 5.1%) was obtained from **39b** (1.45 g, 5.62 mmol). MS m/z (ESI): 350 [M+1]⁺

Step 7: **39g** (100 mg, 0.29 mmol) and palladium on carbon (100 mg, 5%wt) were dispersed in methanol (10 mL), and the mixture was stirred and reacted under hydrogen atmosphere for 1 hour. The reaction solution was filtered to remove insoluble materials. The filtrate was concentrated under reduced pressure to obtain **39h** (70 mg, yield: 90.1%). MS m/z (ESI): 272 [M+1]

Referring to the synthesis method described in Step 9 to Step 10 of **Example 12,** Example **39** (5 mg, yield: 14.4%) was obtained from **39h** (20 mg, 0.07 mmol). MS m/z (ESI): 496 [M+1]⁺

¹H NMR (400 MHz, DMSO-d₆) δ9.24 (s, 1H), 8.40 (d, 1H), 8.16 (d, 1H), 7.98 (broad s, 2H), 7.74 (m, 2H), 7.44 (d, 1H), 7.37 (d, 1H), 5.82 (s, 1H), 5.01 (d, 2H), 4.24 (s, 2H), 2.69 (s, 3H), 2.43 - 2.30 (m, 3H).

### Example 39 Preparation of chiral isomers

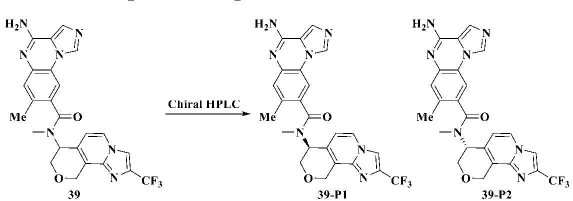

**Example 39** (21 mg, 0.042 mmol) was resolved by Chiral-HPLC (condition 2) to obtain **39-P1:** (*S)*-4-amino-*N*,7-dimethyl-*N*-(2-( trifluoromethyl)-7,10-dihydro-8*H*-imidazo[1,2-*a*]pyrano[3,4-*c*]pyridin-7-yl)imidazo[1,5-a]quinoxaline-8-carboxamide (10.1 mg, yield: 48.1%) and **39-P2:** (*R*)-4-amino-*N*,7-dimethyl-N-(2-(trifluoromethyl)-7,10-dihydro-8*H*-imidazo[1,2-*a*] pyrano[3,4-c]pyridin-7-yl)imidazo[1,5-a]quinoxaline-8-carboxamide (10.2 mg, yield: 48.2%), respectively.

**39-P1 (t_{R}: 1.562 min):** ¹H NMR (400 MHz, DMSO-d₆) δ9.24 (s, 1H), 8.40 (d, 1H), 8.16 (d, 1H), 7.98 (broad s, 2H), 7.74 (m, 2H), 7.44 (d, 1H), 7.37 (d, 1H), 5.82 (s, 1H), 5.01 (d, 2H), 4.24 (s, 2H), 2.69 (s, 3H), 2.43 - 2.30 (m, 3H).

**39-P2 (t_{R}: 1.943 min):** ¹H NMR (400 MHz, DMSO-d₆) δ9.24 (s, 1H), 8.40 (d, 1H), 8.16 (d, 1H), 7.98 (broad s, 2H), 7.74 (m, 2H), 7.44 (d, 1H), 7.37 (d, 1H), 5.82 (s, 1H), 5.01 (d, 2H), 4.24 (s, 2H), 2.69 (s, 3H), 2.43 - 2.30 (m, 3H).

### Example 48

### 4-Amino-N,7-dimethyl-N-(8-(trifluoromethyl)-3,4-dihydro-1H-imidazo[1,2-a]pyrano[4,3-e]pyridin-4-yl)imidazo[1,5-a]quinoxaline-8-carboxamide

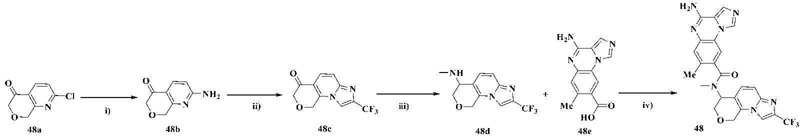

Step 1: 2-Chloro-7,8-dihydro-6H-quinolin-5-one **48a** (1.7 g, 9.36 mmol, synthesized using the well-known method in "patent WO 2021163344"), diphenylmethanimine (1.70 g, 9.36 mmol), Pd₂(dba)₃ (0.86 g, 0.94 mmol), 4,5-bis(diphenylphosphino)-9,9-dimethylxanthene (1.08 g, 1.87 mmol), caesium carbonate (6.10 g, 18.72 mmol) and dioxane (20 mL) were reacted at 100°C for 16 hours. The reaction solution was yellow. Upon completion of the reaction, the reaction solution was filtered. A hydrogen chloride methanol solution was added to the filtrate, and the mixture was reacted at 20°C for 3 hours. The reaction solution was yellow. The reaction solution was quenched with a NaHCO₃ solution and then concentrated under reduced pressure. The resulting residue was purified by silica gel column chromatography using eluent system A to obtain **48b** (1.1 g, yield: 71.2%). MS m/z (ESI): 165 [M+1]⁺

Step 2: **48b** (400 mg, 2.44 mmol), 3-bromo-1,1,1-trifluoro-propan-2-one (465 mg, 2.44 mmol) and acetonitrile (6 mL) were reacted at 80°C for 3 hours, and the reaction solution was yellow. The reaction solution was quenched with NaHCO₃ solution and then quenched with water (10 mL), and extracted with ethyl acetate (40 mL × 3). The organic phases were combined, washed with water (10 mL) and a saturated sodium chloride solution (10 mL × 2), dried over anhydrous sodium sulphate, and filtered, the filtrate was concentrated under reduced pressure, and the resulting residue was purified by silica gel column chromatography using eluent system A to obtain **48c** (30 mg, yield: 4.8%). MS m/z (ESI): 257 [M+1]⁺

Step 3: **48c** (70 mg, 0.273 mmol), methylamine (8.5 mg, 0.273 mmol), sodium cyanoborohydride (51 mg, 0.819 mmol), acetic acid (3.3 mg, 0.054 mmol) and methanol (3 mL) were reacted at 60°C for 4 hours, and the reaction solution was yellow. The reaction solution was quenched with NaHCO₃ solution and then quenched with water (2 mL), and extracted with ethyl acetate (40 mL × 3). The organic phases were combined, dried over anhydrous sodium sulphate, and filtered, and the filtrate was concentrated under reduced pressure to obtain **48d** (28 mg, yield: 37.7%). MS m/z (ESI): 272 [M+1]⁺

Step 4: **48d** (17 mg, 0.062 mmol), 4-amino-7-methyl-imidazo[1,5-a]quinoxaline-8-carboxylic acid **48e** (15 mg, 0.062 mmol, referring to Step 1 to Step 11 of **Example 28),** N,N,N',N'-tetramethylchloroformamidinium hexafluorophosphate (26 mg, 0.093 mmol), N-methylimidazole (15 mg, 0.186 mmol) and N,N-dimethylformamide (2 mL) were reacted at 20°C for 2 hours, and the reaction solution was yellow. The reaction solution was quenched with H₂O and then quenched with water (10 mL), and extracted with ethyl acetate (40 mL × 3). The organic phases were combined, washed with water (10 mL) and a saturated sodium chloride solution (10 mL × 2), dried over anhydrous sodium sulphate, and filtered, the filtrate was concentrated under reduced pressure, and the resulting residue was purified by silica gel column chromatography using eluent system A to obtain **Example 417** (10 mg, yield: 32.5%). MS m/z (ESI): 496 [M+1]⁺

¹HNMR (400 MHz, DMSO-d₆) δ 9.15 (s, 1H), 8.48 (d, 1H), 8.16 (d, 1H), 7.98 (d, 1H), 7.80 - 7.68 (m, 2H), 7.44 (d, 1H), 7.37 (s, 1H), 5.86 (s, 1H), 5.13 (d, 1H), 4.87 (d, 1H), 4.18 (m, 1H), 3.49 (t, 1H), 3.42 (t, 1H), 2.88 - 2.84 (m, 1H), 2.64 (s, 2H), 2.34 (s, 3H).

### Example 62

### 4-Amino-N,7-dimethyl-N-(3-(trifluoromethyl)-7,10-dihydro-8H-imidazo[1,2-a]pyrano[3,4-c]pyridin-7-yl)imidazo[1,5-a]quinoxaline-8-carboxamide

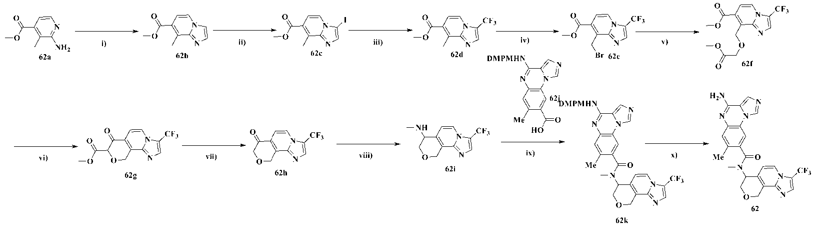

Step 1: Methyl 2-amino-3-methylisonicotinate **62a** (5 g, 30.12 mmol) and bromoacetaldehyde dimethyl acetal (10.12 g, 60.24 mmol) were dispersed in ethanol (50 mL) and hydrochloric acid (10 mL, 2 M/L), and the mixture was stirred and reacted at 80°C for 16 hours. The reaction solution was concentrated under reduced pressure, and the resulting residue was purified by silica gel column chromatography using eluent system A to obtain **62b** (3.2 g, yield: 55.9%). MS m/z (ESI): 191 [M+1]⁺

Step 2: **62b** (3.2 g, 16.84 mmol) was dispersed in acetonitrile (50 mL), and N-iodosuccinimide (4.5 g, 20 mmol) was added. The mixture was stirred and reacted for 1 hour. The reaction solution was concentrated under reduced pressure, and the resulting residue was purified by silica gel column chromatography using eluent system A to obtain **62c** (2.80 g, yield: 52.6%). MS m/z (ESI): 317 [M+1]⁺

Step 3: **62c** (2.80 g, 8.86 mmol), cuprous iodide (8.42 g, 44.30 mmol) and potassium fluoride (1.54 g, 26.58 mmol) were dispersed in N,Ndimethylformamide (30 mL), and (trifluoromethyl)trimethylsilane (3.77 g, 26.58 mmol) was added. The reaction solution was stirred and reacted at 90°C for 16 hours. The reaction solution was filtered, the filtrate was concentrated under reduced pressure, and the resulting residue was purified by silica gel column chromatography using eluent system A to obtain **62d** (830 mg, yield: 36.3%). MS m/z (ESI): 259 [M+1]⁺

Referring to the synthesis method described in Step 3 to Step 8 of **Example 34** and Step 9 to Step 10 of **Example 12, Example 62** (19 mg, yield: 2.5%) was obtained from **62d** (400 mg, 1.55 mmol). MS m/z (ESI): 496 [M+1]⁺

¹H NMR (400 MHz, DMSO-d₆) δ9.29 (s, 1H), 8.87 (s, 2H), 8.31 (dd, 4H), 7.50 (s, 1H), 7.16 (s, 1H), 5.93 - 4.56 (m, 3H), 4.27 (s, 2H), 2.79 (d, 3H), 2.42 (s, 3H).

### Example 62 Preparation of chiral isomers

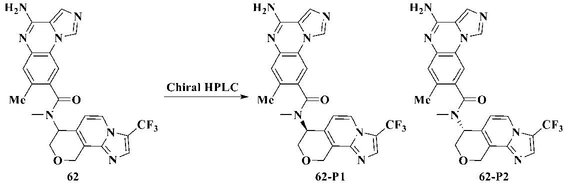

**Example 62** (19 mg, 0.042 mmol) was resolved by Chiral-HPLC (condition 2) to obtain **62-P1:** (*S*)-4-amino-*N*,7-dimethyl-N-(3-(trifluoromethyl)-7,10-dihydro-8*H*-imidazo[1,2-*a*]pyrano[3,4-*c*]pyridin-7-yl)imidazo[1,5-*a*]quinoxaline-8-carboxamide (9.1 mg, yield: 47.8%) and **62-P2:** (*R*)-4-amino-N,7-dimethyl-N-(3-(trifluoromethyl)-7,10-dihydro-8*H*-imidazo[1,2*-a*]pyrano[3,4-*c*]pyridin-7-yl)imidazo[1,5-*a*]quinoxaline-8-carboxamide (8.8 mg, yield: 46.3%), respectively.

**62-P1 (t_{R}: 1.982 min):** ¹H NMR (400 MHz, DMSO-d₆) δ9.29 (s, 1H), 8.87 (s, 2H), 8.31 (dd, 4H), 7.50 (s, 1H), 7.16 (s, 1H), 5.93 - 4.56 (m, 3H), 4.27 (s, 2H), 2.79 (d, 3H), 2.42 (s, 3H).

**62-P2 (t_{R}: 1.436 min):** ¹H NMR (400 MHz, DMSO-d₆) δ9.29 (s, 1H), 8.87 (s, 2H), 8.31 (dd, 4H), 7.50 (s, 1H), 7.16 (s, 1H), 5.93 - 4.56 (m, 3H), 4.27 (s, 2H), 2.79 (d, 3H), 2.42 (s, 3H).

### Example 68

### 4-Amino-N,1-dimethyl-N-(3-(trifluoromethyl)-7,10-dihydro-8H-pyrano[4,3-h]quinolin-7-yl)-1H-pyrazolo[4,3-c]quinoline-8-carboxamide

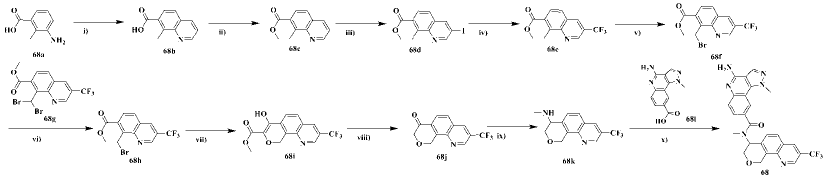

Step 1: 3-Amino-2-methyl-benzoic acid **68a** (20 g, 132.31 mmol), sodium iodide (500 mg, 3.33 mmol) and glycerol (12.18 g, 132.31 mmol, 20 mL) were dissolved in water (30 mL) and concentrated sulphuric acid (40 mL), and the reaction system was stirred at 150°C for 1 hour. The reaction system was cooled to room temperature, adjusted to pH 3 with 12 M sodium hydroxide aqueous solution, extracted with ethyl acetate (500 mL × 3), washed with a saturated sodium chloride aqueous solution, dried over anhydrous sodium sulphate, filtered, and concentrated to obtain **68b** (15 g, crude). MS m/z (ESI): 188 [M+1]⁺

Step 2: **68b** (0.7 g, 3.74 mmol) was dissolved in methanol (10 mL), thionyl chloride (1.33 g, 11.22 mmol, 0.81 mL) was added, and the reaction system was stirred at 70°C for 15 hours. The resulting reaction system was cooled to room temperature, quenched with water, extracted with ethyl acetate (80 mL × 3), washed with a saturated sodium chloride aqueous solution, dried over anhydrous sodium sulphate, filtered, and concentrated. The residue was purified by silica gel column chromatography using eluent system B to obtain **68c** (350 mg, yield: 46.5%). MS m/z (ESI): 202 [M+1]⁺

Step 3: **68c** (2.9 g, 14.41 mmol) and N-iodosuccinimide (3.89 g, 17.29 mmol) were dissolved in acetic acid (10 mL), the mixture was purged three times with nitrogen, and the reaction system was stirred at 100°C for 18 hours. The resulting reaction system was concentrated, quenched with water, extracted with ethyl acetate (100 mL × 3), washed with a saturated sodium chloride aqueous solution, dried over anhydrous sodium sulphate, filtered, and concentrated. The residue was purified by silica gel column chromatography using eluent system B to obtain **68d** (3.0 g, yield: 63.6%). MS m/z (ESI): 328 [M+1]⁺

Step 4: **68d** (2.9 g, 8.87 mmol) and cuprous iodide (4.22 g, 22.16 mmol) were dissolved in N,N-dimethylformamide (20 mL), methyl fluorosulphonyldifluoroacetate (4.26 g, 22.16 mmol, 2.82 mL) was added, and the mixture was purged three times with nitrogen, and then heated and reacted at 100°C for 2 hours. The reaction system was cooled to room temperature, filtered, concentrated, and subjected to rotary evaporation to dryness. Water was added, the mixture was extracted with ethyl acetate (100 mL × 3), and the organic phases were combined, washed with a saturated sodium chloride aqueous solution, dried over anhydrous sodium sulphate, filtered, and concentrated. The residue was purified by silica gel column chromatography using eluent system B to obtain **68e** (2.1 g, yield: 88.0%). MS m/z (ESI): 270 [M+1]⁺

Referring to the synthesis method described in Step 1 to Step 4 of **Example 9, 68j** (520 mg, yield: 21.0%) was obtained from **68e** (2.5 g, 9.29 mmol). MS m/z (ESI): 268 [M+1]⁺

Referring to the synthesis method described in Step 7 of **Example 9, 68k** (320 mg, yield: 58.3%) was obtained from **68j** (520 mg, 1.95 mmol). MS m/z (ESI): 283 [M+1]⁺

Referring to the synthesis method described in Step 7 of **Example 1, Example 68** (44 mg, yield: 42.1%) was obtained from **68k** (58 mg, 0.20 mmol). MS m/z (ESI): 507 [M+1]⁺

¹H NMR (400 MHz, DMSO-d₆) δ 13.21 (s, 1H), 9.63 (s, 1H), 9.23 (d, 1H), 9.00 (d, 1H), 8.71-8.43 (m, 3H), 8.18 (t, 1H), 8.00 (dd, 1H), 7.87 (q, 2H), 5.92 (s, 1H), 5.45 (dd, 1H), 5.15 (t, 1H), 4.51 (d, 3H), 4.39-4.10 (m, 2H), 2.79 (d, 3H).

### Example 79

### 4-Amino-N-(7,10-dihydro-8H-imidazo[1,2-a]pyrano[3,4-c]pyridin-7-yl)-N,7-dimethylimidazo[1,5-a]quinoxaline-8-carboxamide

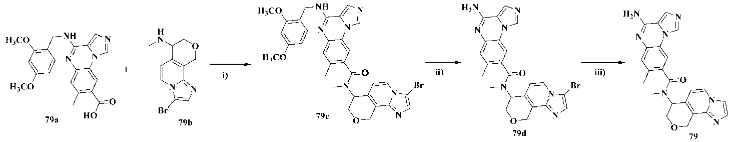

Referring to Step 9 to Step 10 of **Example 12, 79d** (47 mg, yield: 40.5%) was obtained from 4-((2,4-dimethoxybenzyl)amino)-7-methylimidazo[1,5-a]quinoxaline-8-carboxylic acid **79a** (90 mg, referring to Step 1 to Step 11 of **Example 28)** and 3-bromo-N-methyl-7,10-dihydro-8H-imidazo[1,2-a]pyrano[3,4-c]pyridin-7-amine **79b** (40 mg, referring to Step 1 to Step 8 of **Example 37).**

Step 3: **79d** (15 mg, 0.029 mmol), Pd/C (3 mg, 20% weight) and methanol (2 mL) were reacted at 20°C under H₂ atmosphere for 4 hours. The reaction solution was yellow. The reaction solution was filtered, the filtrate was concentrated under reduced pressure, and the resulting residue was purified by silica gel column chromatography using eluent system A to obtain **Example 79** (2.1 mg, yield: 16%). MS m/z (ESI): 428 [M+1]⁺

¹HNMR (400 MHz, DMSO-d₆) δ 9.12 (d, 1H), 8.53 (d, 1H), 8.13 (s, 1H), 7.99 (d, 1H), 7.88 (s, 1H), 7.56 - 7.51 (m, 1H), 7.32 (s, 2H), 5.77 (s, 1H), 5.10 (d, 1H), 4.83 (d, 1H), 4.17 (s, 2H), 2.85 (s, 1H), 2.68 - 2.62 (m, 3H), 2.33 (d, 3H).

### Example 80

### 4-Amino-N,1-dimethyl-N-(8-(trifluoromethyl)-3,4-dihydro-1H-imidazo[1,2-a]pyrano[4,3-e]pyridin-4-yl)-1H-pyrazolo[4,3-c]quinoline-8-carboxamide

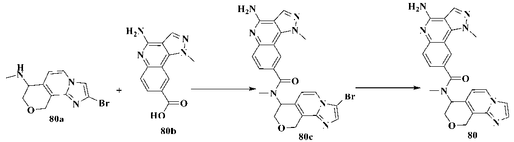

Referring to Step 1 and Step 3 of **Example 79, Example 80** (2.2 mg, yield: 5.6%) was obtained from 2-bromo-N-methyl-7,10-dihydro-8H-imidazo[1,2-a]pyrano[3,4-c]pyridin-7-amine **80a** (20 mg, referring to Step 1 to Step 3 of **Example 48)** and 4-amino-1-methyl-1H-pyrazolo[4,3-c]quinoline-8-carboxylic acid **80b** (20 mg, synthesized using the well-known method in "patent WO 2022169948"). MS m/z (ESI): 428 [M+1]⁺

¹HNMR (400 MHz, DMSO-d₆) δ 8.50 (s, 1H), 8.35 (d, 1H), 8.25 (s, 1H), 7.97 (s, 1H), 7.66 (s, 1H), 7.63 (s, 1H), 7.54 (s, 1H), 7.22 (s, 1H), 7.16 (s, 2H), 6.68 (s, 1H), 5.32 (t, 2H), 4.42 (s, 3H), 4.28 (dd, 2H), 2.83 (s, 3H).

### Example 81

### 4-Amino-N,1-dimethyl-N-(8-(trifluoromethyl)-3,4-dihydro-1H-imidazo[1,2-a]pyrano[4,3-e]pyridin-4-yl)-1H-pyrazolo[4,3-c]quinoline-8-carboxamide

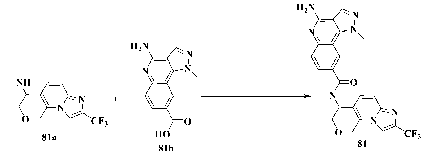

N-Methyl-4-(trifluoromethyl)-12-oxa-2,5-diazatricyclo[7.4.0.02,6]trideca-1(9),3,5,7-tetraen-10-amine **81a** (11 mg, 0.041 mmol, synthesized referring to Step 1 to Step 3 of **Example 48),** 4-amino-1-methyl-1H-pyrazolo[4,3-c]quinoline-8-carboxylic acid **81b** (10 mg, 0.041 mmol, synthesized using the well-known method in "patent WO 2022169948"), O-(7-azabenzotriazol-1-yl)-N,N,N',N'-tetramethyluronium hexafluorophosphate (2 mg, 0.066 mmol) and N-methylimidazole (10 mg, 0.124 mmol) were dissolved in N,N-dimethylformamide (2 mL), and the mixture was stirred and reacted at 20°C for 2 hours. The reaction solution was quenched with water (10 mL) and extracted with ethyl acetate (20 mL × 3). The organic phases were combined, washed with water (10 mL) and a saturated sodium chloride solution (10 mL × 2), dried over anhydrous sodium sulphate, and filtered, the filtrate was concentrated under reduced pressure, and the resulting residue was purified by silica gel column chromatography using eluent system A to obtain **Example 81** (2 mg, yield: 9.8%). MS m/z (ESI): 496 [M+1]⁺

¹HNMR (400 MHz, DMSO-d₆) δ 8.41 (d, 2H), 8.21 (d, 1H), 7.73 - 7.60 (m, 2H), 7.50 (s, 1H), 7.13 (s, 1H), 5.08 (s, 1H), 4.83 (s, 1H), 4.56 (d, 1H), 4.42 (s, 2H), 4.37 - 4.30 (m, 1H), 4.15 (s, 1H), 3.96 (d, 1H), 3.64 - 3.41 (m, 2H), 2.75 (d, 3H).

### Example 82

### 4-Amino-N-(2-chloro-3-(trifluoromethyl)-7,10-dihydro-8H-imidazo[1,2-a]pyrano[3,4-c]pyridin-7-yl)-N,7-dimethylimidazo[1,5-a]quinoxaline-8-carboxamide

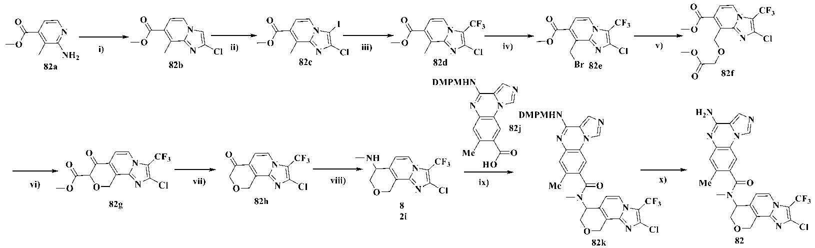

Step 1: Methyl 2-amino-3-methylisonicotinate **82a** (5 g, 30.12 mmol) and ethyl bromoacetate (5.03 g, 30.12 mmol) were dispersed in tetrahydrofuran (50 mL), and the mixture was stirred and reacted at 25°C for 16 hours. The reaction solution was concentrated under reduced pressure to dryness, phosphorus oxychloride (20 mL) was added, and the mixture was stirred and reacted at 90°C for 1 hour. The reaction solution was concentrated under reduced pressure, and the resulting residue was purified by silica gel column chromatography using eluent system A to obtain **82b** (3.31 g, yield: 49.1%). MS m/z (ESI): 225 [M+1]⁺

Referring to the synthesis method described in Step 2 to Step 3 of **Example 62,** Step 3 to Step 8 of **Example 34** and Step 9 to Step 10 of **Example 12, Example 82** (20 mg, yield: 0.7%) was obtained from **82b** (1.21 g, 5.40 mmol). MS m/z (ESI): 530 [M+1]⁺

### Example 83

### 4-Amino-N,1-dimethyl-N-(3-(trifluoromethyl)-7,10-dihydro-8H-imidazo[1,2-a]pyrano[3,4-c]pyridin-7-yl)-1H-pyrazolo[4,3-c]quinoline-8-carboxamide

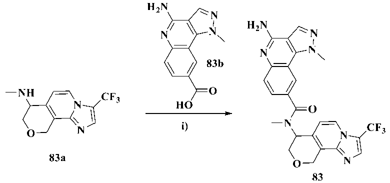

Referring to the synthesis method described in Step 7 of **Example 1, Example 83** (10 mg, yield: 18.4%) was obtained from N-methyl-3-(trifluoromethyl)-7,10-dihydro-8*H*-imidazo[1,2-*a*]pyrano[3,4-*c*]pyridin-7-amine **83a** (30 mg, 0.11 mmol). MS m/z (ESI): 496 [M+1]⁺

¹H NMR (400 MHz, DMSO-d₆) δ8.35 (dd, 6H), 7.89 - 7.75 (m, 2H), 7.23 (s, 1H), 5.01 (d, 3H), 4.48 (d, 3H), 4.26 (d, 2H), 2.86 (d, 3H).

### Example 84

### 4-Amino-N,1-dimethyl-N-(2-(trifluoromethyl)-7,10-dihydro-8H-imidazo[1,2-a]pyrano[3,4-c]pyridin-7-yl)-1H-pyrazolo[4,3-c]quinoline-8-carboxamide

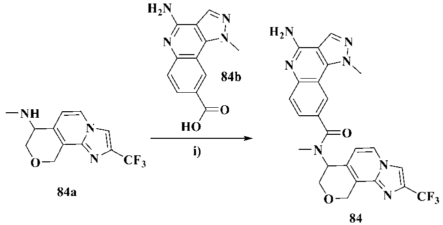

Referring to the synthesis method described in Step 7 of **Example 1, Example 84** (10 mg, yield: 18.4%) was obtained from *N*-methyl-2-(trifluoromethyl)-7,10-dihydro-8*H*-imidazo[1,2-*a*]pyrano[3,4-*c*]pyridin-7-amine **84a** (40 mg, 0.11 mmol). MS m/z (ESI): 496 [M+1]⁺

¹H NMR (400 MHz, DMSO-d₆) δ8.36 (dd, 6H), 7.83 (d, 2H), 7.11 (s, 1H), 4.96 (dd, 3H), 4.48 (d, 3H), 4.23 (d, 2H), 2.85 (d, 3H).

The examples in the table below were prepared with reference to **Example 37:**

| **Example** | **Structure** | **MS m/z (ESI)** | **Example** | **Structure** | **MS m/z (ESI)** |
|---|---|---|---|---|---|
| | | **[M+1]⁺** | | | **[M+1]⁺** |
| **85** | 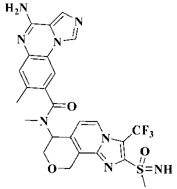 | 573 | **94** | 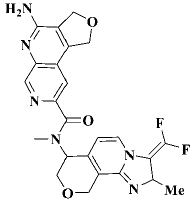 | 481 |
| **86** | 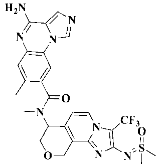 | 587 | **95** | 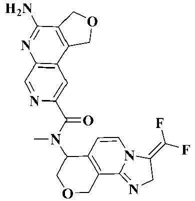 | 467 |
| **87** | 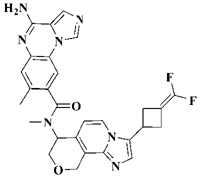 | 530 | **96** | 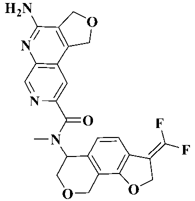 | 467 |
| **88** | 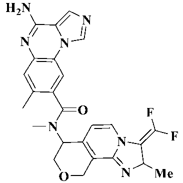 | 492 | **97** | 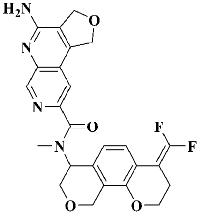 | 481 |
| **89** | 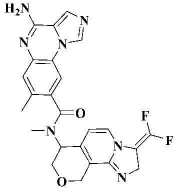 | 478 | **98** | 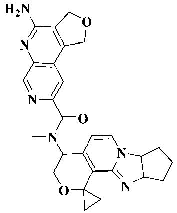 | 485 |
| **90** | 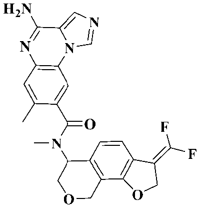 | 478 | **99** | 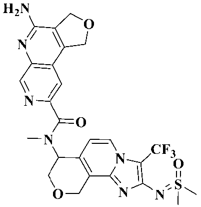 | 576 |
| **91** | 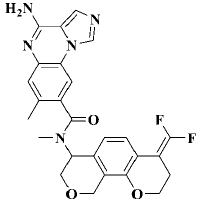 | 492 | **100** | 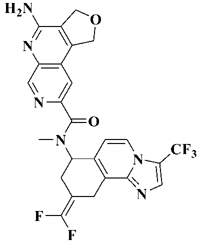 | 531 |
| **92** | 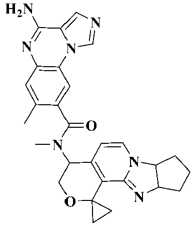 | 496 | **101** | 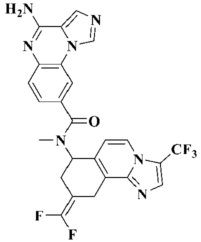 | 528 |
| **93** | 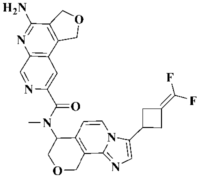 | 519 | **102** | 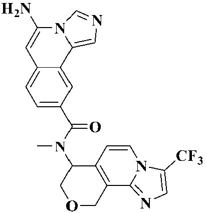 | 481 |

The examples in the table below were prepared with reference to Example 62:

| **Example** | **Structure** | **MS m/z (ESI)** | **Example** | **Structure** | **MS m/z (ESI)** |
|---|---|---|---|---|---|
| | | **[M+1]⁺** | | | **[M+1]⁺** |
| **103** | 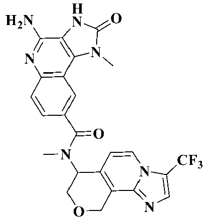 | 512 | **145** | 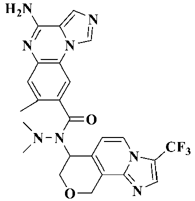 | 525 |
| **104** | 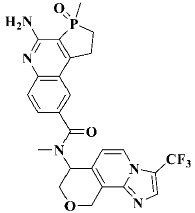 | 530 | **146** | 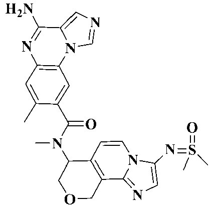 | 519 |
| **105** | 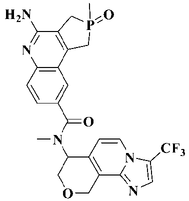 | 530 | **147** | 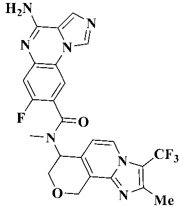 | 514 |
| **106** | 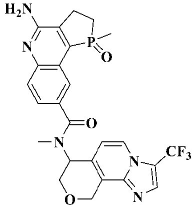 | 530 | **148** | 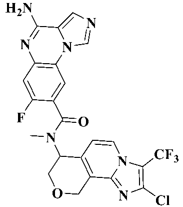 | 534 |
| **107** | 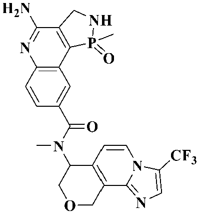 | 531 | **149** | 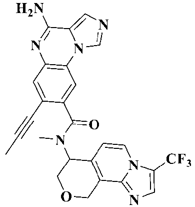 | 520 |
| **108** | 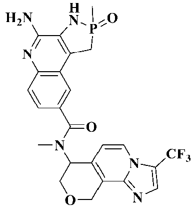 | 531 | **150** | 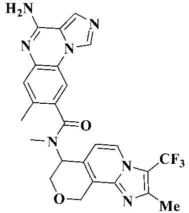 | 510 |
| **109** | 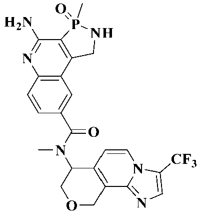 | 531 | **151** | 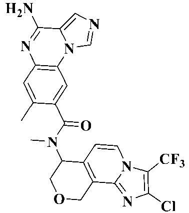 | 530 |
| **110** | 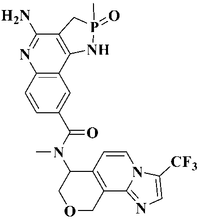 | 531 | **152** | 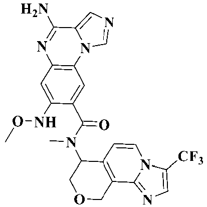 | 527 |
| **111** | 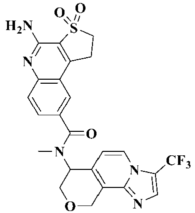 | 532 | **153** | 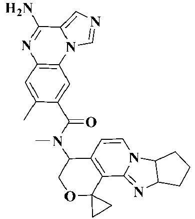 | 496 |
| **112** | 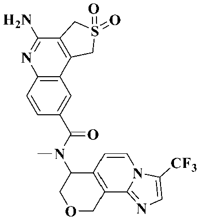 | 532 | **154** | 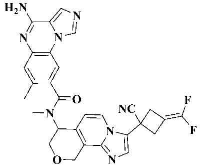 | 555 |
| **113** | 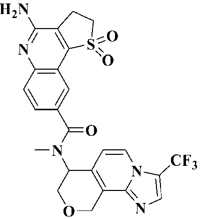 | 532 | **155** | 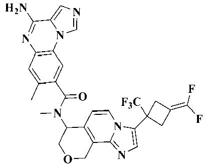 | 598 |
| **114** | 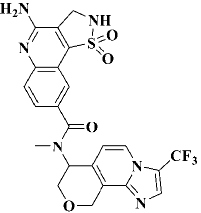 | 533 | **156** | 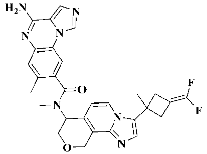 | 544 |
| **115** | 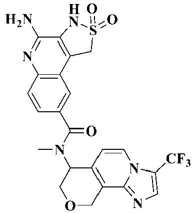 | 533 | **157** | 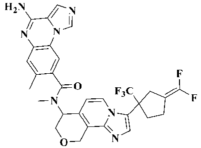 | 612 |
| **116** | 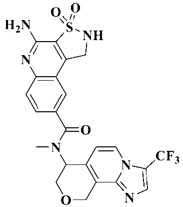 | 533 | **158** | 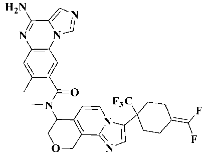 | 626 |
| **117** | 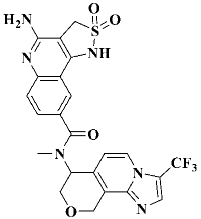 | 533 | **159** | 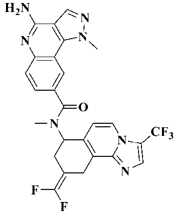 | 542 |
| **118** | 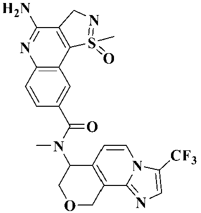 | 531 | **160** | 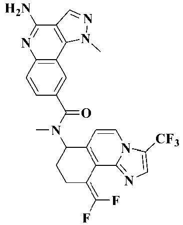 | 542 |
| **119** | 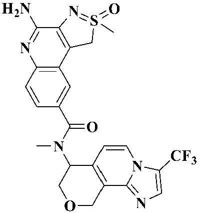 | 531 | **161** | 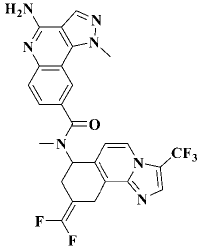 | 542 |
| **120** | 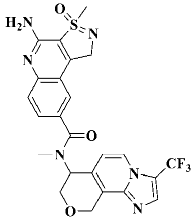 | 531 | **162** | 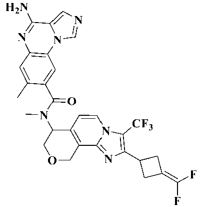 | 598 |
| **121** | 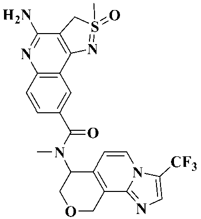 | 531 | **163** | 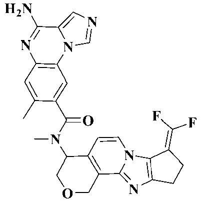 | 516 |
| **122** | 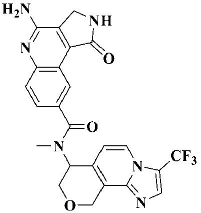 | 497 | **164** | 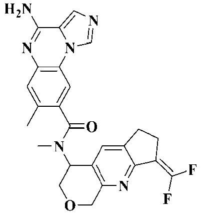 | 477 |
| **123** | 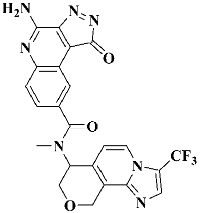 | 496 | **165** | 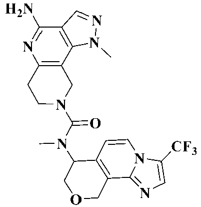 | 501 |
| **124** | 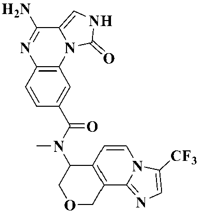 | 498 | **166** | 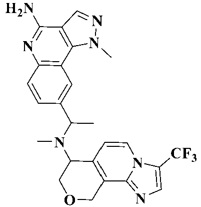 | 496 |
| **125** | 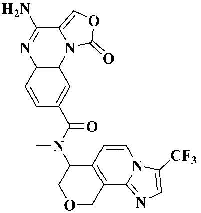 | 499 | **167** | 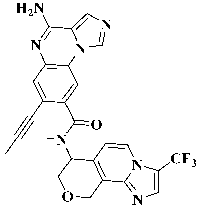 | 520 |
| **126** | 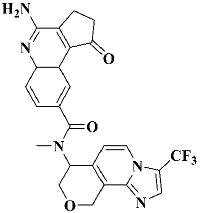 | 496 | **168** | 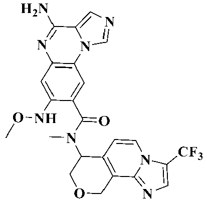 | 527 |
| **127** | 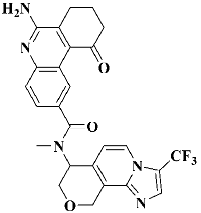 | 510 | **169** | 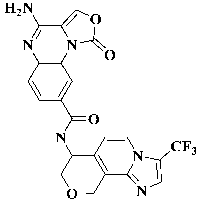 | 499 |
| **128** | 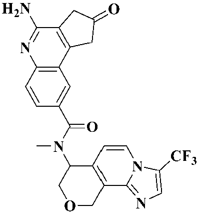 | 496 | **170** | 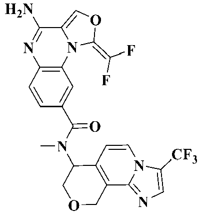 | 533 |
| **129** | 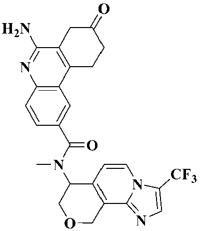 | 510 | **171** | 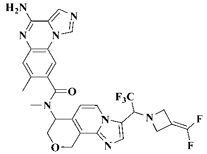 | 613 |
| **130** | 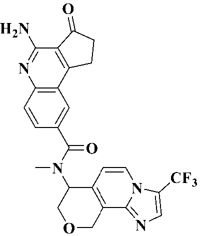 | 496 | **172** | 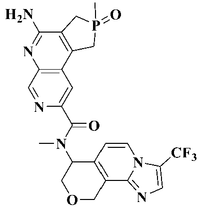 | 531 |
| **131** | 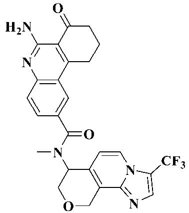 | 510 | **173** | 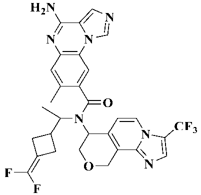 | 612 |
| **132** | 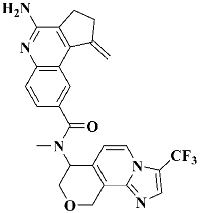 | 494 | **174** | 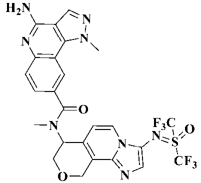 | 627 |
| **133** | 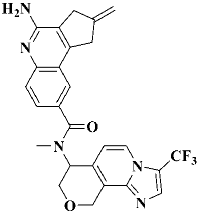 | 494 | **175** | 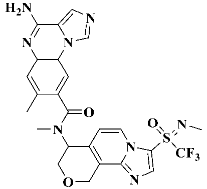 | 573 |
| **134** | 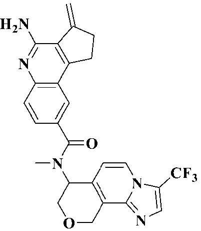 | 494 | **176** | 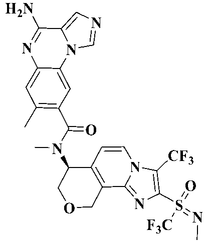 | 641 |
| **135** | 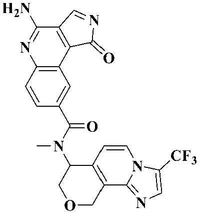 | 495 | **177** | 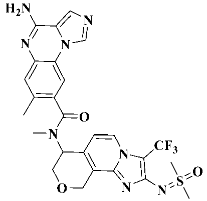 | 587 |
| **136** | 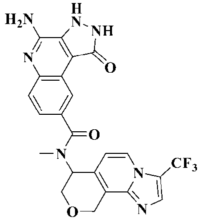 | 498 | **178** | 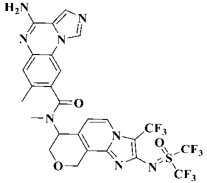 | 695 |
| **137** | 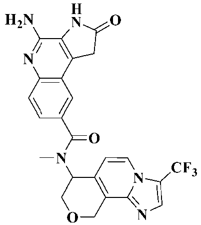 | 497 | **179** | 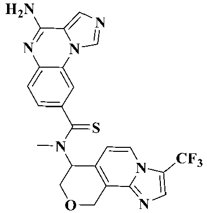 | 498 |
| **138** | 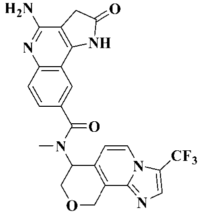 | 497 | **180** | 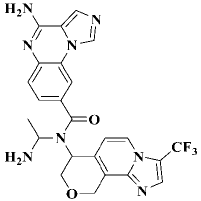 | 511 |
| **139** | 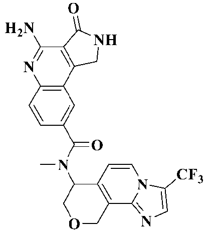 | 497 | **181** | 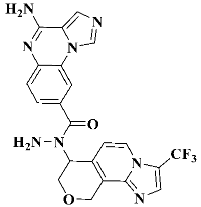 | 483 |
| **140** | 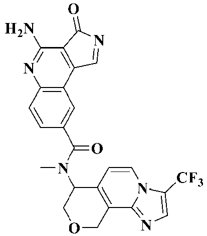 | 495 | **182** | 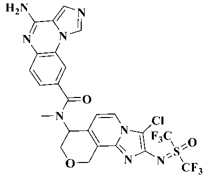 | 647 |
| **141** | 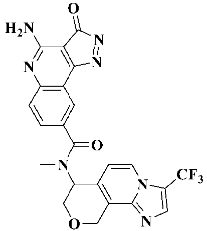 | 496 | **183** | 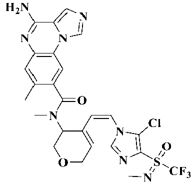 | 607 |
| **142** | 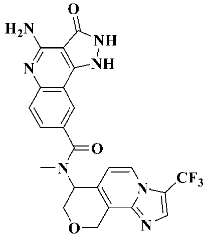 | 498 | **184** | 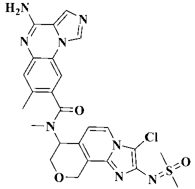 | 553 |
| **143** | 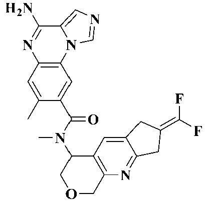 | 477 | **185** | 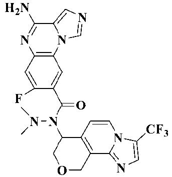 | 529 |
| **144** | 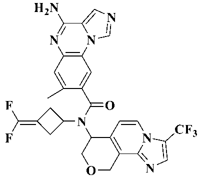 | 584 | | | |

The examples in the table below were prepared with reference to **Example 82:**

| **Example** | **Structure** | **MS m/z (ESI)** | **Example** | **Structure** | **MS m/z (ESI)** |
|---|---|---|---|---|---|
| | | **[M+1]⁺** | | | **[M+1]⁺** |
| **186** | 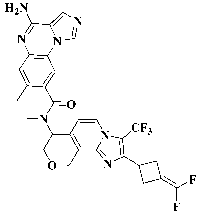 | 598 | **189** | 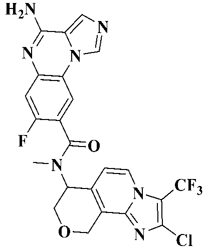 | 534 |
| **187** | 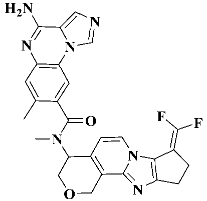 | 516 | **190** | 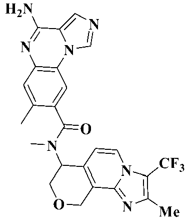 | 510 |
| **188** | 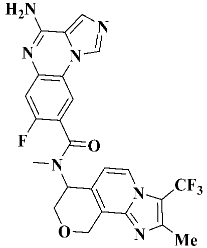 | 514 | **191** | 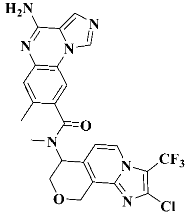 | 530 |

**Examples 192, 193** and **194** were prepared with reference to **Example 33;** and **Examples 195** and **196** were prepared with reference to **Example 14:**

| **Example** | **Structure** | **MS m/z (ESI)** | **Example** | **Structure** | **MS m/z (ESI)** |
|---|---|---|---|---|---|
| | | **[M+1]⁺** | | | **[M+1]⁺** |
| **192** | 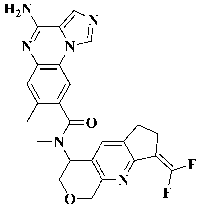 | 477 | **195** | 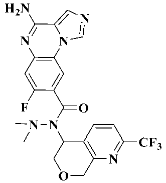 | 490 |
| **193** | 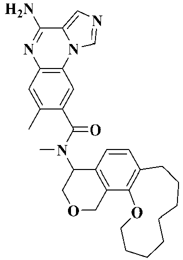 | 514 | **196** | 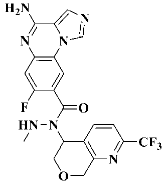 | 476 |
| **194** | 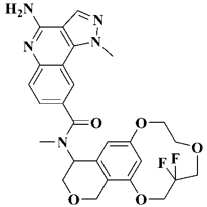 | 540 | | | |

Alternatively, **Examples 144** and **146** were prepared using the following method:

### Example 144

### 4-Amino-N-[3-(difluoromethylene)cyclobutyl]-7-methyl-N-[5-(trifluoromethyl)-12-oxa-3,6-diazatricyclo[7.4.0.02,6]trideca-1(9),2,4,7-tetraen-10-yl]imidazo[1,5-a]quinoxaline-8-carboxamide

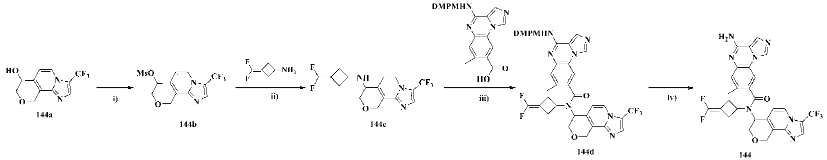

Step 1: 3-Trifluoromethyl-7,10-dihydro-8H-imidazo[1,2-a]pyrano[3,4-c]pyridin-7-ol **144a** (200 mg, 774.60 µmol) and triethylamine (391.91 mg, 3.87 mmol, 540.19 µL) were dissolved in dichloromethane (4 mL), the mixture was cooled to 0°C, and methanesulphonic anhydride (404.80 mg, 2.32 mmol) was added. The mixture was stirred at room temperature for 2 h. The solvent was removed under reduced pressure, and the residue was purified and separated by column chromatography using eluents: petroleum ether/ethyl acetate (1:1) to obtain **144b** (100 mg, 297.37 µmol, yield: 38.4%). MS m/z (ESI): 337 [M+1]⁺

Step 2: **144b** (100 mg, 297.37 µmol), 3-(difluoromethylene)cyclobutanaminium (70.84 mg, 594.73 µmol), caesium carbonate (290.66 mg, 892.10 µmol) and sodium iodide (44.57 mg, 297.37 µmol) were added to acetonitrile (1.5 mL), and the mixture was stirred and reacted at 60°C for 16 h and cooled to room temperature. The solvent was removed under reduced pressure, and the residue was purified and separated by column chromatography using eluents: petroleum ether/ethyl acetate (1:2) to obtain **144c** (50 mg, 139.16 µmol, yield: 46.7%). MS m/z (ESI): 360 [M+1]⁺

Step 3: **144c** (55.17 mg, 153.08 µmol) was dissolved in dichloromethane (1.5 mL), and at 0°C, oxalyl chloride (97.15 mg, 765.39 µmol) and N,N-dimethylformamide (0.01 mL) were added. The mixture was stirred and reacted at 0°C for 3 h. The reaction solution was subjected to rotary evaporation to dryness. N-[3-(difluoromethylene)cyclobutyl]-5-(trifluoromethyl)-12-oxa-3,6-diazatricyclo[7.4.0.02,6]trideca-1(9),2,4,7-tetraen-10-amine (55 mg, 153.08 µmol) was dissolved in DCM (1.5 mL), and at 0°C, acyl chloride was added, followed by triethylamine (31 mg, 306.16 µmol). The mixture was stirred and reacted at room temperature for 16 h. The solvent was removed under reduced pressure, and the residue was purified and separated by column chromatography using eluents: petroleum ether/ethyl acetate (10:1) to obtain **144d** (25 mg, 35.63 µmol, yield: 22.2%). MS m/z (ESI): 734 [M+1]⁺

Step 4: **144d** (20 mg, 28.50 µmol) was added to trifluoroacetic acid (200 µL), and the mixture was stirred and reacted at 90°C for 2 h. The resulting reaction system was cooled to room temperature. The solvent was removed under reduced pressure, and the residue was purified and separated by HPLC to obtain **Example 144** (4.2 mg, 7.20 µmol, yield: 25.2%). MS m/z (ESI): 584 [M+1]⁺

¹H NMR (400 MHz, DMSO-d₆) δ 9.08 (s, 1H), 8.41 (d, 1H), 8.11 (s, 2H), 7.90 (d, 1H), 7.37 (s, 1H), 7.25-7.03 (m, 3H), 5.10- 4.77 (m, 3H), 4.42- 4.02 (m, 3H), 3.67-3.27 (m, 2H), 2.96 - 2.62 (m, 2H), 2.41 (s, 3H).

### Example 146

### 4-Amino-N-(3-((dimethyl(carbonyl)-16-sulphanylidene)amino)-7,10-dihydro-8H-imidazo[1,2-a]pyrano[3,4-c]pyridin-7-yl)-N,7-dimethylimidazo[1,5-a]quinoxaline-8-carboxamide

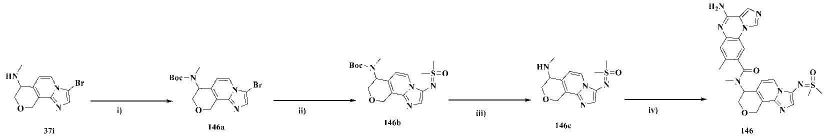

Step 1: 3-Bromo-N-methyl-7,10-dihydro-8H-imidazo[1,2-a]pyrano[3,4-c]pyridin-7-amine 37i (220 mg, 0.78 mmol), di-tert-butyl dicarbonate (218 mg, 1.17 mmol) and diisopropylethylamine (302 mg, 2.34 mmol) were dissolved in dichloromethane, and the mixture was stirred and reacted at room temperature for 1 hour. The reaction solution was concentrated under reduced pressure, and the resulting residue was purified by silica gel column chromatography using eluent system A to obtain **146a** (240 mg, yield: 80.5%). MS m/z (ESI): 382 [M+1]⁺

Step 2: **146a** (100 mg, 0.26 mmol), imino-dimethyl-carbonyl-sulphane (37 mg, 0.39 mmol), caesium carbonate (127 mg, 0.39 mmol), 4,5-bis(diphenylphosphino)-9,9-dimethylxanthene (28 mg, 0.5 mmol) and tris(dibenzylideneacetone)palladium (24 mg, 0.03 mmol) were dissolved in dioxane (5 mL), and under nitrogen atmosphere, the mixture was stirred and reacted at 80°C for 2 hours. The reaction solution was extracted with ethyl acetate (50 mL) and washed with a saturated sodium chloride aqueous solution (50 mL × 2). After extraction, the organic phase was separated, dried over anhydrous sodium sulphate, and filtered, and the filtrate was concentrated under reduced pressure to obtain **146b** (57 mg, yield: 55.1%). MS m/z (ESI): 395 [M+1]⁺

Step 3: **146b** (57 mg, 0.13 mmol) was dissolved in 2M hydrogen chloride dioxane (5 mL), and the mixture was stirred and reacted at room temperature for 2 hours. The reaction solution was concentrated under reduced pressure to obtain **146c** (33 mg, yield: 85.7%). MS m/z (ESI): 295 [M+1]⁺

Referring to Step 9 of **Example 37, Example 146** (4.2 mg, yield: 7.4%) was obtained from **146c** (33 mg, 0.11 mmol). MS m/z (ESI): 519 [M+1]⁺

¹H NMR (400 MHz, DMSO-d₆) δ 9.08 (s, 1H), 8.13 - 8.06 (m, 2H), 7.88 (s, 1H), 7.34 (d, 2H), 7.32 (s, 1H), 7.00 (d, 1H), 6.78 (d, 1H), 5.74 (s, 1H), 5.07 (d, 1H), 4.78 (d, 1H), 4.23 (d, 1H), 4.15 (dd, 1H), 3.30 (d, 6H), 2.63 (s, 3H), 2.33 (s, 3H).

### Example 197

### 4-Amino-7-[3-(difluoromethylene)cyclobutyl]-N-methyl-N-[2-(trifluoromethyl)-6,8-dihydro-5H-pyrano[3,4-b]pyridin-5-yl]imidazo[1,5-a]quinoxaline-8-carboxamide

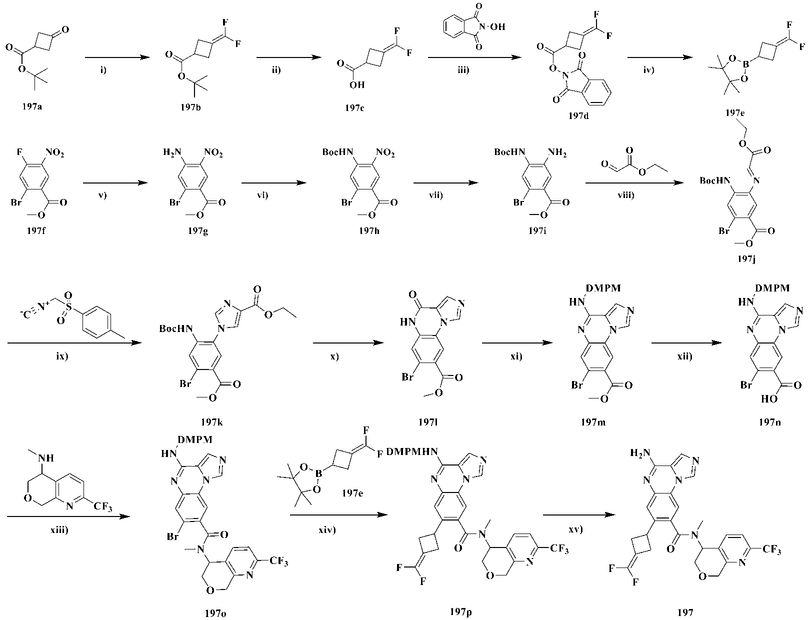

Step 1: THF (70 mL) was added to a three-necked flask, and the system was purged three times with nitrogen and cooled to 0°C. Dibromo(difluoro)methane (20.72 g, 98.74 mmol, 9.02 mL) was immediately added. The mixture was stirred in an ice bath for 10 minutes. HMPT (16.12 g, 98.74 mmol, 18 mL) was slowly added dropwise. Formation of a white solid was observed; upon completion of the dropwise addition, the mixture was stirred at 0°C for another 1 h; tert-butyl 3-oxocyclobutane carboxylate **197a** (3.86 g, 22.7 mmol)/THF (10 mL) were slowly added dropwise, and the mixture was heated to 25°C and stirred for 1 hour. Zinc powder (3.86 g, 90.8 mmol) was added, followed by HMPT (0.3 mL). The mixture was transferred to 75°C and stirred for 5 hours; water (100 mL) was added to the reaction solution, and the organic phase was separated; the aqueous layer was extracted with ethyl acetate (100 mL × 3); the organic phases were combined, washed with a saturated sodium chloride aqueous solution (100 mL × 3), dried over anhydrous sodium sulphate, and filtered, the filtrate was concentrated under reduced pressure, and the concentrated residue was purified using purification system C to obtain **197b** (2 g, yield: 43.1%).

Step 2: **197b** (1.5 g, 7.35 mmol) was dissolved in DCM (15 mL) and TFA (10 mL), and the mixture was stirred and reacted at 25°C for 1 hour; the reaction solution was directly concentrated under reduced pressure to obtain **197c** (1 g, crude).

Step 3: **197c** (1 g, crude) and 2-hydroxyisoindole-1,3-dione (1.21 g, 7.43 mmol) were dissolved in DCM (10 mL), and DMAP (82.49 mg, 675.19 µmol) was added. N,N-Diisopropylcarbodiimide (937.29 mg, 7.43 mmol, 1.2 mL) was added dropwise, and the mixture was purged three times with nitrogen, and stirred and reacted at 25°C under nitrogen atmosphere for 16 hours; water (20 mL) was added to the reaction solution, and the mixture was extracted with dichloromethane (20 mL × 3); the organic phases were combined, washed with a saturated sodium chloride aqueous solution (20 mL × 3), dried over anhydrous sodium sulphate, and filtered, the filtrate was concentrated under reduced pressure, and the concentrated residue was separated and purified using purification system C to obtain **197d** (1.92 g, yield: 96.9%). MS m/z (ESI): 294 [M+1]⁺

Step 4: **197d** (1.92 g, 6.55 mmol) and 2,2-bi-1,3,2-benzodioxaborolane (1.95 g, 8.18 mmol) were dissolved in DMA (120 mL), and the mixture was purged three times with nitrogen. Under nitrogen atmosphere, the mixture was stirred under irradiation from a blue light reactor (18 w, 456 nm) for 15 hours. Then Pinacol (3.10 g, 26.19 mmol) and TEA (4.18 g, 41.32 mmol, 5.8 mL) were sequentially added, and the mixture was purged three times with nitrogen, and stirred and reacted at 25°C under nitrogen atmosphere for 1 hour; water (200 mL) was added to the reaction solution, and the mixture was extracted with ethyl acetate (200 mL × 3); the organic phases were combined, washed with a saturated sodium chloride aqueous solution (200 mL × 3), dried over anhydrous sodium sulphate, and filtered, the filtrate was concentrated under reduced pressure, and the concentrated residue was separated and purified using purification system C to obtain **197e** (540 mg, yield: 35.9%).

Referring to the synthesis method described in Step 1 to Step 10 of **Example 32, 197o** (600 mg, yield: 0.1%) was obtained from methyl 2-bromo-4-fluoro-5-nitrobenzoate **197f** (2 g, 3.60 mmol). MS m/z (ESI): 671 [M+1]⁺

Step 14: **197o** (100 mg, 149 µmol) and **197e** (137.05 mg, 596 µmol) were dissolved in DMF (5 mL). Ni(dtbppy)Cl₂ (29.64 mg, 75 µmol), [Ir(dtbbpy)[dF(CF₃)ppy]₂]PF₆ (7.52 mg, 8 µmol) and morpholine (19.46 mg, 224 µmol) were sequentially added, and the mixture was purged three times with nitrogen, and stirred and reacted at 25°C under nitrogen atmosphere for 16 hours; water (10 mL) was added to the reaction solution, and the mixture was extracted with ethyl acetate (10 mL × 3); the organic phases were combined, washed with a saturated sodium chloride aqueous solution (10 mL × 3), dried over anhydrous sodium sulphate, and filtered, and the filtrate was concentrated under reduced pressure to obtain **197p** (100 mg, crude). MS m/z (ESI): 695 [M+1]⁺

Step 15: **197p** (100 mg, crude) was dissolved in TFA (1 mL), and the mixture was stirred and reacted at 90°C for 3 hours; The reaction solution was concentrated under reduced pressure, and the concentrated residue was separated and purified by Pre-HPLC to obtain **Example 197** (7.5 mg). MS m/z (ESI): 545 [M+1]⁺

¹H NMR (400 MHz, DMSO-d₆) δ 9.07 (s, 1H), 8.32 - 8.03 (m, 2H), 8.02 - 7.79 (m, 2H), 7.54 (s, 1H), 7.23 (s, 2H), 5.90 (s, 1H), 5.00 - 4.70 (m, 2H), 4.24 (s, 2H), 3.98 - 3.69 (m, 3H), 2.92 - 2.73 (m, 3H), 2.66 (s, 2H).

### Example 198

### 4-Amino-N-[2-[4-(difluoromethylene)-1-piperidyl]-6,8-dihydro-5H-pyrano[3,4-b]pyridin-5-yl]-N,7-dimethyl-imidazo[1,5-a]quinoxaline-8-carboxamide

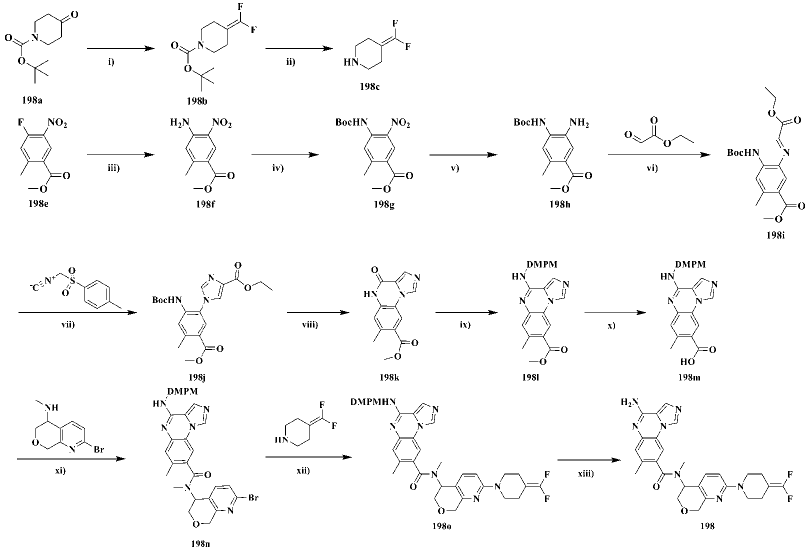

Referring to the synthesis method described in Step 1 to Step 2 of **Example 28, 198c** (1.5 g, yield: 44.9%) was obtained from tert-butyl 4-oxopiperidine-1-carboxylate **198a** (5 g, 25.09 mmol).

Referring to the synthesis method described in Step 1 to Step 10 of **Example 32, 198n** (600 mg, yield: 12%) was obtained from methyl 4-fluoro-2-methyl-5-nitrobenzoate **198e** (2 g, 9.38 mmol). MS m/z (ESI): 617 [M+1]⁺

Step 12: **198n** (120 mg, 195 µmol) and **198c** (51.75 mg, 389 µmol) were dissolved in DMF (3 mL). Xphos-Pd-G2 (15.27 mg, 20 µmol) and t-BuoNa (112.05 mg, 1.17 mmol) were sequentially added, and the mixture was purged three times with nitrogen, and stirred and reacted under microwave irradiation at 110°C under nitrogen atmosphere for 3 hours; water (10 mL) was added to the reaction solution, and the mixture was extracted with ethyl acetate (10 mL × 3); the organic phases were combined, washed with a saturated sodium chloride aqueous solution (10 mL × 3), dried over anhydrous sodium sulphate, and filtered, and the filtrate was concentrated under reduced pressure to obtain **198o** (100 mg, crude). MS m/z (ESI): 670 [M+1]⁺

Step 13: **198o** (100 mg, 150 µmol) was dissolved in TFA (3 mL), and the mixture was purged three times with nitrogen, and stirred and reacted at 90°C under nitrogen atmosphere for 3 hours; the reaction solution was concentrated under reduced pressure, and the concentrated residue was separated and purified by Pre-HPLC to obtain **Example 198** (18 mg). MS m/z (ESI): 520 [M+1]⁺

¹H NMR (400 MHz, DMSO-d₆) δ 9.03 (s, 1H), 8.08 (d, 1H), 7.89 (s, 1H), 7.50 (s, 1H), 7.33 (s, 1H), 7.11 (s, 2H), 6.85 (d, 1H), 5.71 (s, 1H), 4.54 (q, 2H), 4.09 (d, 2H), 3.62 (s, 4H), 2.60 (s, 3H), 2.32 (s, 3H), 2.22 (s, 4H).

### Example 199

### 4-Amino-N-methoxy-7-methyl-N-(2-(trifluoromethyl)-5,8-dihydro-6H-pyrano[3,4-b]pyridin-5-yl)imidazo[1,5-a]quinoxaline-8-carboxamide

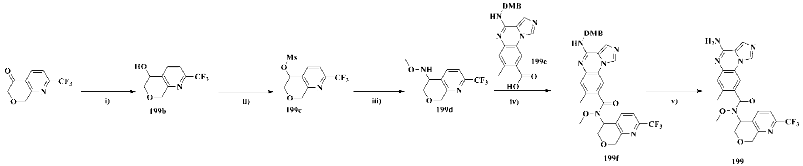

Step 1: 2-(Trifluoromethyl)-6H-pyrano[3,4-b]pyridin-5(8H)-one (500 mg, 2.30 mmol, synthesized using the well-known method in "patent WO 2022169948") was dissolved in methanol, sodium borohydride (435 mg, 11.51 mmol) was added portionwise in an ice bath, and the mixture was stirred and reacted at 0°C for 1 hour. The reaction was quenched by adding a saturated ammonium chloride solution (30 mL) to the reaction solution, and the mixture was extracted with ethyl acetate (30 mL×2). After extraction, the organic phase was separated, dried over anhydrous sodium sulphate, and filtered. The filtrate was concentrated under reduced pressure, and the resulting residue was purified by silica gel column chromatography using eluent system B to obtain **199b** (470 mg, yield: 93.1%). MS m/z (ESI): 220 [M+1]⁺

Step 2: **199b** (470 mg, 2.14 mmol) and triethylamine (651 mg, 6.43 mmol) were dissolved in dichloromethane (10 mL), methanesulphonic anhydride (560 mg, 3.22 mmol) was added, and the mixture was stirred and reacted at room temperature for 1 hour. The reaction was quenched by adding a saturated ammonium chloride solution (30 mL) to the reaction solution, and the mixture was extracted with ethyl acetate (30 mL × 2). After extraction, the organic phase was separated, dried over anhydrous sodium sulphate, and filtered. The filtrate was concentrated under reduced pressure to obtain **199c** (430 mg, yield: 66.8%). MS m/z (ESI): 298 [M+1]⁺

Step 3: **199c** (220 mg, 0.74 mmol) was dissolved in acetonitrile (10 mL), potassium carbonate (511 mg, 3.7 mmol) was added, and the mixture was stirred and reacted at 80°C in a sealed tube for 16 hours. The reaction solution was cooled to room temperature and filtered, the filtrate was concentrated under reduced pressure, and the resulting residue was purified by silica gel column chromatography using eluent system A to obtain **199d** (139 mg, yield: 75.7%). MS m/z (ESI): 249 [M+1]⁺

Step 4: **199d** (139 mg, 0.56 mmol) and 4-((3,4-dimethylbenzyl)amino)-7-methylimidazo[1,5-a]quinoxaline-8-carboxylic acid **199e** (241 mg, 0.62 mmol) were dissolved in dichloromethane (5 mL), phosphorus oxychloride (257 mg, 1.68 mmol) and pyridine (265 mg, 3.36 mmol) were sequentially added, and the mixture was stirred and reacted at room temperature for 2 hours. The reaction was quenched by adding 2M hydrochloric acid (30 mL) to the reaction solution, and the mixture was extracted with dichloromethane (30 mL × 2). After extraction, the organic phase was separated, dried over anhydrous sodium sulphate, and filtered. The filtrate was concentrated under reduced pressure, and the resulting residue was purified by silica gel column chromatography using eluent system B to obtain 199f (67 mg, yield: 19.2%). MS m/z (ESI): 623 [M+1]⁺

Referring to Step 7 of **Example 1, Example 199** (20.2 mg, yield: 38.9%) was obtained from **199f** (67 mg, 0.11 mmol). MS m/z (ESI): 473 [M+1]⁺

¹H NMR (400 MHz, DMSO-d₆) δ 9.24 (s, 1H), 8.32 (s, 1H), 8.20 (s, 1H), 8.13 (d, 1H), 7.90 (d, 1H), 7.38 (s, 1H), 5.70 (s, 1H), 4.90 (d, 1H), 4.78 (d, 1H), 4.37 - 4.15 (m, 2H), 3.43(s, 3H), 2.38 (s, 3H).

### Example 200

### 4-Amino-N-methyl-N-(3-(trifluoromethyl)-7,10-dihydro-8H-imidazo[1,2-a]pyrano[3,4-c]pyridin-7-yl)benzofuro[3,2-c]pyridine-8-carboxamide

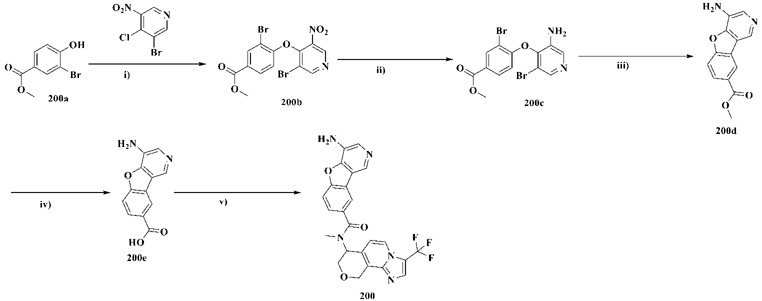

Step 1: A reaction solution of methyl 3-bromo-4-hydroxybenzoate **200a** (5.00 g, 21.64 mmol), 3-bromo-4-chloro-5-nitropyridine (5.65 g, 23.81 mmol) and caesium carbonate (10.55 g, 32.46 mmol) in N,N-dimethylformamide (50 mL) was stirred at 35°C for 16 hours. The reaction solution was filtered to remove solids and then concentrated. The organic phase was concentrated, and the residue was purified by silica gel column chromatography to obtain **200b** (7.60 g, yield: 81.3%). MS m/z (ESI): 433 [M+1]⁺

Step 2: A reaction solution of **200b** (5.00 g, 11.55 mmol) and iron powder (6.48 g, 115.51 mmol) in methanol (100 mL) and a saturated ammonium chloride aqueous solution (50 mL) was stirred at 50°C for 16 hours. Upon completion of the reaction, the reaction solution was filtered to remove solids. The filtrate was concentrated, and the residue was purified by silica gel column chromatography to obtain **200c** (3.92 g, yield: 84.2%). MS m/z (ESI): 403 [M+1]⁺

Step 3: A reaction solution of **200c** (20 mg, 0.050 mmol), bis(pinacolato)diboron (18.95 mg, 0.074 mmol), potassium phosphate (15.82 mg, 0.075 mmol) and 1,1'-bis(diphenylphosphino)ferrocene palladium(II) dichloride (3.64 mg, 0.005 mmol) in 1,4-dioxane (3 mL) and water (0.3 mL) was purged with nitrogen, sealed in a microwave vial, and stirred at 90°C for 16 hours. The reaction solution was filtered to remove solids and then concentrated. The organic phase was concentrated, and the residue was purified by silica gel column chromatography to obtain **200d** (8 mg, yield: 66.4%). MS m/z (ESI): 243 [M+1]⁺

Step 4: A reaction solution of **200d** (50 mg, 0.21 mmol) and lithium hydroxide (17 mg, 0.42 mmol) in methanol (2 mL), tetrahydrofuran (2 mL) and water (1 mL) was stirred at room temperature for 16 hours. The reaction solution was concentrated. The organic phase was concentrated, and the residue was purified by reversed-phase column chromatography to obtain **200e** (22 mg, yield: 46.7%). MS m/z (ESI): 229 [M+1]⁺

Step 5: A reaction solution of **200e** (15 mg, 0.066 mmol), N-methyl-3-(trifluoromethyl)-7,10-dihydro-8H-imidazo[1,2-a]pyrano[3,4-c]pyridin-7-amine (27 mg, 0.099 mmol), N-methylimidazole (16 mg, 0.197 mmol) and N,N,N',N'-tetramethylchloroformamidinium hexafluorophosphate (28 mg, 0.099 mmol) in N,N-dimethylformamide (2 mL) was stirred at room temperature for 3 hours. The reaction solution was concentrated. The organic phase was concentrated, and the residue was purified by prep-HPLC column chromatography to obtain **Example 200** (5 mg, yield: 15.8%). MS m/z (ESI): 482 [M+1]⁺

¹H NMR (400 MHz, CD₃OD) *δ* 8.83 (s, 1H), 8.42 (m, 2H), 8.10 (d, 2H), 7.90 (m, 2H), 7.28 (d, 1H), 5.88 (s, 1H), 5.23 (d, 1H), 4.30 (m, 3H), 2.94 (s, 3H).

### Example 201

### 4-Amino-7-fluoro-N-methoxy-N-(2-(trifluoromethyl)-5,8-dihydro-6H-pyrano[3,4-b]pyridin-5-yl)imidazo[1,5-a]quinoxaline-8-carboxamide

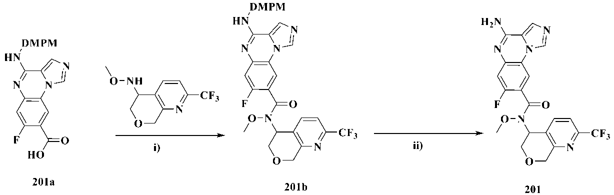

Referring to Step 4 of **Example 199** and Step 7 of **Example 1, Example 201** (20 mg, yield: 17.4%) was obtained from 4-((3,4-dimethoxybenzyl)amino)-7-fluoroimidazo[1,5-a]quinoxaline-8-carboxylic acid **201a** (88 mg, 0.24 mmol). MS m/z (ESI): 477 [M+1]⁺

¹H NMR (400 MHz, DMSO-d₆) δ 9.14 (s, 1H), 8.44 (d, 1H), 8.07 (d, 1H), 7.95 - 7.89 (m, 2H), 7.65 (s, 2H), 7.23 (d, 1H), 5.68 (s, 1H), 4.89 (d, 1H), 4.78 (d, 1H), 4.25 (qd, 2H), 3.47 (s, 3H).

### Example 202

### 4-Amino-N-(2-(cyclohexylidenefluoromethyl)-5,8-dihydro-6H-pyrano[3,4-b]pyridin-5-yl)-7-fluoro-N-methylimidazo[1,5-a]quinoxaline-8-carboxamide

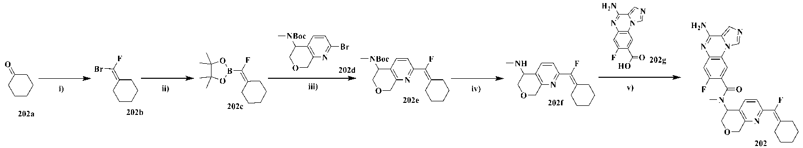

Step 1: Cyclohexanone **202a** (2 g, 20.38 mmol, 2.11 mL), tribromo(fluoro)methane (6.62 g, 24.45 mmol, 2.39 mL) and triphenylphosphine (6.41 g, 24.45 mmol) were dissolved in tetrahydrofuran (60 mL), and the mixture was purged three times with nitrogen. Diethylzinc (3.02 g, 24.45 mmol, 24.5 mL, 1 M) was added dropwise to the system at 25°C. After the addition, the system was reacted at 25°C for 1 hour. TLC showed completion of the reaction. Then the mixture was quenched with methanol (50 ml), concentrated, and filtered. The residue was purified by silica gel column chromatography using eluent system B to obtain **202b** (1.20 g, yield: 30.5%).

¹H NMR (400 MHz, CDCl₃) δ 2.27 (td, 2H), 2.15-2.10 (m, 2H), 1.59-1.49 (m, 6H).

Step 2: **202b** (2 g, 10.36 mmol), bis(pinacolato)diboron (5.26 g, 20.72 mmol) and potassium acetate (3.05 g, 31.08 mmol) were dissolved in 1'4-dioxane (20 mL), and the mixture was purged three times with nitrogen. 1,1'-Bis(diphenylphosphino)ferrocene palladium(II) dichloride (758 mg, 1.04 mmol) was added to the system, and the reaction system was reacted at 90°C for 5 hours. The resulting reaction system was cooled to room temperature, quenched with water, extracted with ethyl acetate (80 mL × 3), washed with a saturated sodium chloride aqueous solution, dried over anhydrous sodium sulphate, filtered, and concentrated. The residue was purified by silica gel column chromatography using eluent system B to obtain **202c** (0.55 g, yield: 22.1%).

¹H NMR (400 MHz, CDCl₃) δ 2.39 (d, 2H), 2.31 (d, 2H), 1.56 (d, 6H), 1.30 (s, 12H).

Step 3: tert-Butyl N-(2-bromo-6,8-dihydro-5H-pyrano[3,4-b]pyridin-5-yl)-N-methylcarbamate **202d** (0.13 g, 0.38 mmol), **202c** (455 mg, 1.89 mmol) and sodium carbonate (200 mg, 1.89 mmol) were dissolved in 1'4-dioxane (8 mL) and water (1.5 mL), and the mixture was purged three times with nitrogen. Tetrakis(triphenylphosphine)palladium (44 mg, 38 µmol) was added to the system, and the reaction system was reacted at 100°C for 15 hours. The resulting reaction system was cooled to room temperature, concentrated, extracted with ethyl acetate (50 mL × 3), washed with a saturated sodium chloride aqueous solution, dried over anhydrous sodium sulphate, filtered, and concentrated. The residue was purified by silica gel column chromatography using eluent system B to obtain **202e** (130 mg, yield: 91.2%). MS m/z (ESI): 377 [M+1]⁺

Step 4: **202e** (130 mg, 0.35 mmol) was dissolved in 1'4-dioxane (4 mL), and the mixture was purged three times with nitrogen. Hydrogen chloride/1'4-dioxane (4 M, 4 mL) was added to the system, and the reaction system was reacted at 25°C for 1 hour and then concentrated to obtain **202f** (0.10 g, crude). MS m/z (ESI): 277 [M+1]⁺

Step 5: 4-Amino-7-fluoro-imidazo[1,5-a]quinoxaline-8-carboxylic acid **202g** (40 mg, 0.16 mmol), **202f** (54 mg, 0.19 mmol) and N,N,N',N'-tetramethylchloroformamidinium hexafluorophosphate (55 mg, 0.19 mmol) were dissolved in N,N-dimethylformamide (5 mL), N-methylimidazole (45 mg, 0.49 mmol, 39 µL) was added, and the reaction mixture was purged three times with nitrogen and stirred at 25°C for 1 hour. The resulting reaction system was quenched with water, extracted with ethyl acetate (50 mL × 3), washed with a saturated sodium chloride aqueous solution, dried over anhydrous sodium sulphate, filtered, and concentrated. The residue was purified by silica gel column chromatography using eluent system A to obtain **Example 202** (36 mg, yield: 43.9%). MS m/z (ESI): 505 [M+1]⁺

¹H NMR (400 MHz, DMSO-d₆) δ 9.14 (d, 1H), 8.37 (d, 1H), 7.95 (d, 1H), 7.83-7.63 (m, 3H), 7.51 (d, 1H), 7.27 (t, 1H), 5.83 (t, 1H), 4.86-4.75 (m, 1H), 4.62 (dd, 1H), 4.19-3.99 (m, 2H), 2.76 (d, 3H), 2.65 (dt, 2H), 2.42-2.32 (m, 2H), 1.64-1.46 (m, 6H).

The examples in the table below were prepared with reference to Example 34:

| **Example** | **Structure** | **MS m/z (ESI)** | **Example** | **Structure** | **MS m/z (ESI)** |
|---|---|---|---|---|---|
| | | **[M+1]⁺** | | | **[M+1]⁺** |
| **203** | 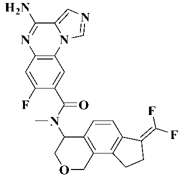 | 480 | **209** | 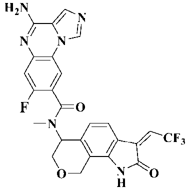 | 527 |
| **206** | 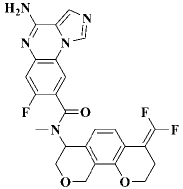 | 496 | **210** | 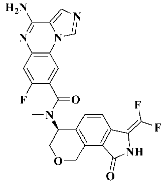 | 495 |
| **207** | 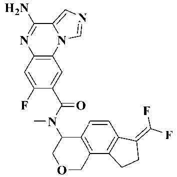 | 480 | **211** | 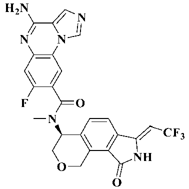 | 527 |
| **208** | 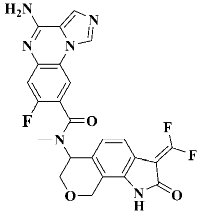 | 495 | | | |

The examples in the table below were prepared with reference to Example 146:

| **Example** | **Structure** | **MS m/z (ESI)** | **Example** | **Structure** | **MS m/z (ESI)** |
|---|---|---|---|---|---|
| | | **[M+1]⁺** | | | **[M+1]⁺** |
| **212** | 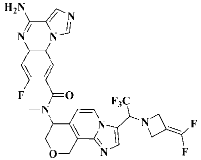 | 617 | **239** | 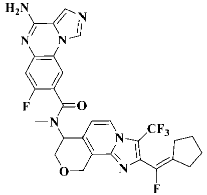 | 598 |
| **213** | 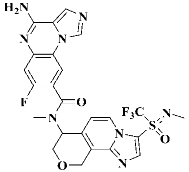 | 577 | **241** | 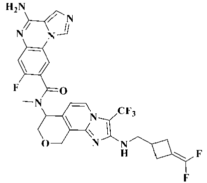 | 631 |
| **214** | 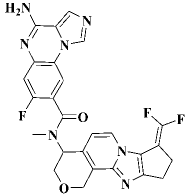 | 520 | **242** | 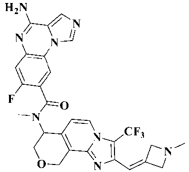 | 581 |
| **215** | 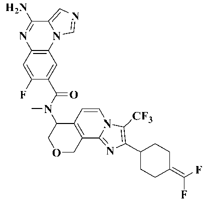 | 630 | **243** | 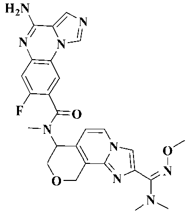 | 532 |
| **216** | 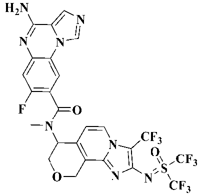 | 699 | **244** | 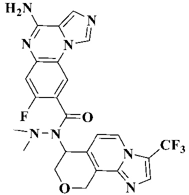 | 529 |
| **217** | 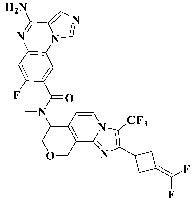 | 602 | **245** | 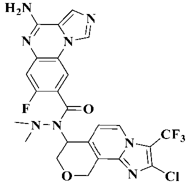 | 563 |
| **218** | 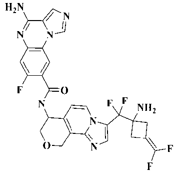 | 599 | **246** | 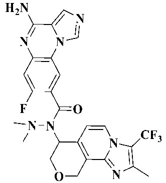 | 543 |
| **219** | 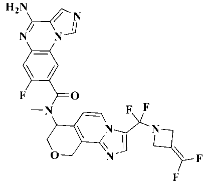 | 585 | **247** | 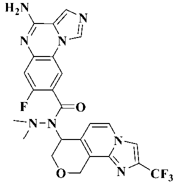 | 529 |
| **220** | 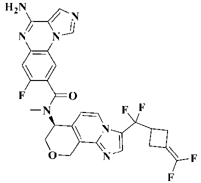 | 584 | **248** | 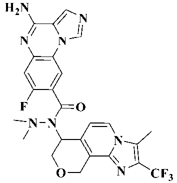 | 543 |
| **221** | 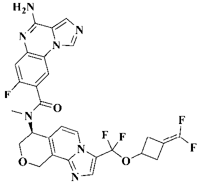 | 600 | **249** | 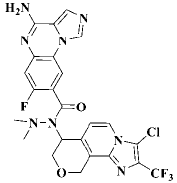 | 563 |
| **222** | 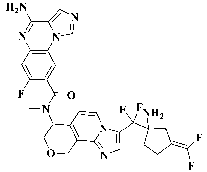 | 613 | **250** | 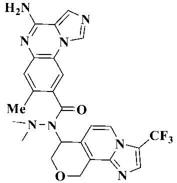 | 525 |
| **223** | 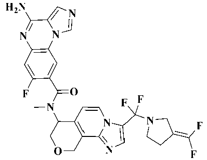 | 599 | **251** | 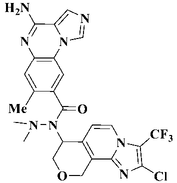 | 559 |
| **224** | 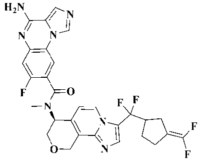 | 598 | **252** | 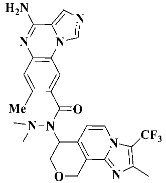 | 539 |
| **225** | 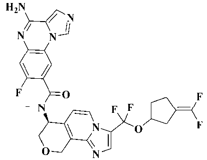 | 614 | **253** | 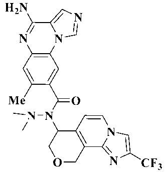 | 525 |
| **226** | 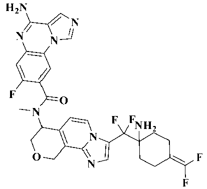 | 627 | **254** | 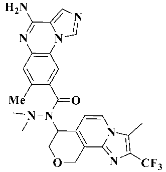 | 539 |
| **227** | 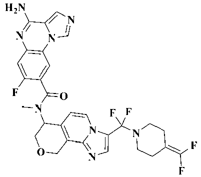 | 613 | **255** | 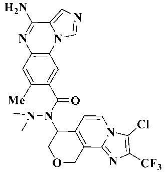 | 559 |
| **228** | 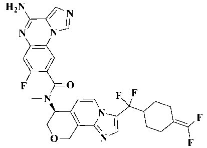 | 612 | **256** | 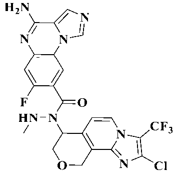 | 549 |
| **229** | 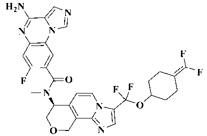 | 628 | **257** | 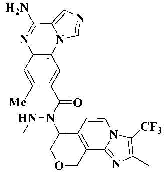 | 525 |
| **230** | 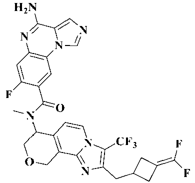 | 616 | **258** | 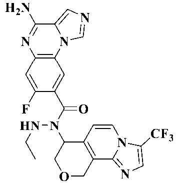 | 529 |
| **231** | 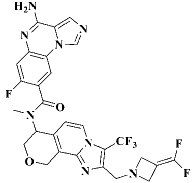 | 617 | **259** | 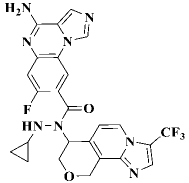 | 541 |
| **232** | 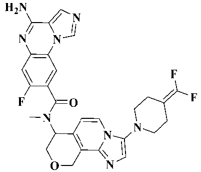 | 563 | **260** | 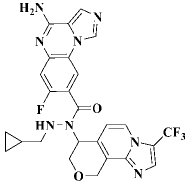 | 555 |
| **233** | 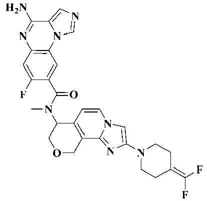 | 563 | **261** | 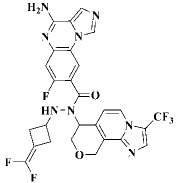 | 603 |
| **234** | 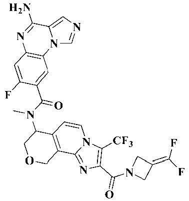 | 631 | **262** | 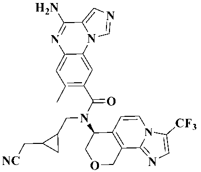 | 575 |
| **235** | 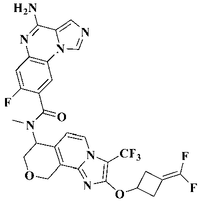 | 618 | **263** | 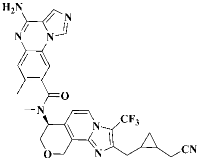 | 589 |
| **236** | 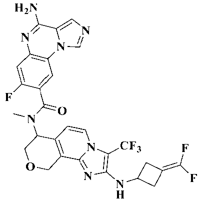 | 617 | **264** | 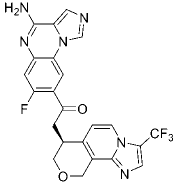 | 485 |
| **238** | 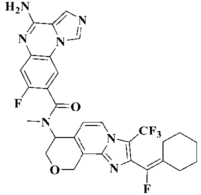 | 612 | | | |

The examples in the table below were prepared with reference to Example 201:

| **Example** | **Structure** | **MS m/z (ESI)** | **Example** | **Structure** | **MS m/z (ESI)** |
|---|---|---|---|---|---|
| | | **[M+1]⁺** | | | **[M+1]⁺** |
| **265** | 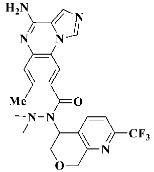 | 486 | **271** | 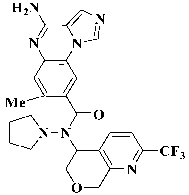 | 512 |
| **266** | 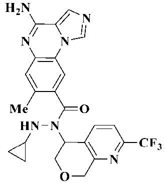 | 498 | **272** | 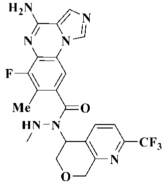 | 490 |
| **267** | 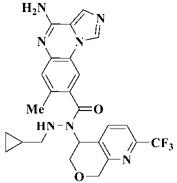 | 512 | **273** | 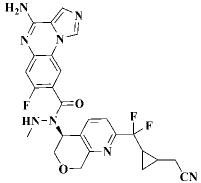 | 537 |
| **268** | 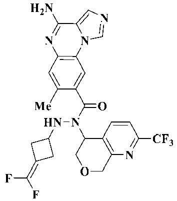 | 560 | **274** | 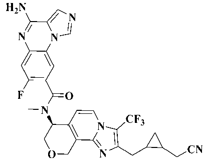 | 593 |
| **269** | 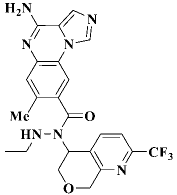 | 486 | **275** | 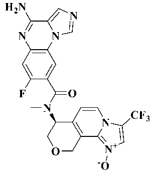 | 516 |
| **270** | 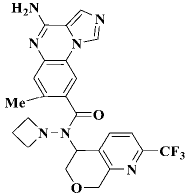 | 498 | | | |

### Example 204

### 4-Amino-N-(2-(4-(difluoromethylene)piperidin-1-yl)-3-(trifluoromethyl)-7,10-dihydro-8H-imidazo[1,2-a]pyrano[3,4-c]pyridin-7-yl)-7-fluoro-N-methylimidazo [1,5-a]quinoxaline-8-carboxamide

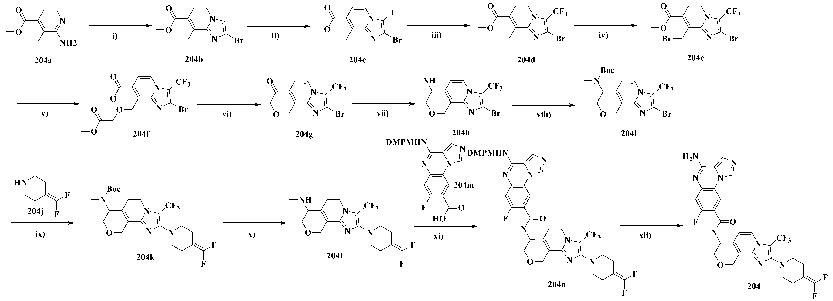

Step 1: Methyl 2-amino-3-methylisonicotinate **204a** (15 g, 90.36 mmol) and ethyl bromoacetate (15.09 g, 90.36 mmol) were dispersed in tetrahydrofuran (70 mL), and the mixture was stirred and reacted at 25°C for 16 hours. The reaction solution was concentrated under reduced pressure to dryness, and the residue was dispersed in acetonitrile (800 mL). Phosphorus oxybromide (64.16 g, 225.90 mmol) was added, and the mixture was stirred and reacted at 90°C for 16 hours. The reaction solution was concentrated under reduced pressure, and the resulting residue was purified by silica gel column chromatography using eluent system A to obtain **204b** (5.41 g, yield: 22.3%). MS m/z (ESI): 269 [M+1]⁺

Referring to the synthesis method described in Step 2 to Step 8 of **Example 62, 204h** (490 mg, yield: 6.9%) was obtained from **204b** (5.41 g, 20.19 mmol). MS m/z (ESI): 350 [M+1]⁺

Step 8: **204h** (490 mg, 1.40 mmol) and triethylamine (202 mg, 2.00 mmol) were dispersed in dichloromethane (10 mL), and di-tert-butyl dicarbonate (436 mg, 2.00 mmol) was added. The reaction solution was stirred and reacted at 25°C for 1 hour. The reaction solution was concentrated under reduced pressure, and the resulting residue was purified by silica gel column chromatography using eluent system A to obtain **204i** (610 mg, yield: 96.8%). MS m/z (ESI): 450 [M+1]⁺

Referring to the synthesis method described in Step 12 of **Example 198, 204k** (62 mg, yield: 55.5%) was obtained from **204i** (100 mg, 0.22 mmol). MS m/z (ESI): 503 [M+1]⁺

Step 10: **204k** (62 mg, 0.12 mmol) was dispersed in trifluoroacetic acid (2 mL). The reaction solution was stirred and reacted at 25°C for 1 hour. The reaction solution was concentrated under reduced pressure to obtain **204l** (49 mg, yield: 98.7%). MS m/z (ESI): 403 [M+1]⁺

Referring to the synthesis method described in Step 9 and Step 10 of **Example 12, Example 204** (18 mg, yield: 23.4%) was obtained from **204l** (49 mg, 0.12 mmol). MS m/z (ESI): 631 [M+1]⁺

### Example 205

### 4-Amino-N-(2-((dimethyl(carbonyl)-16-sulphanylidene)amino)-3-(trifluoromethyl)-7,10-dihydro-8H-imidazo[1,2-a]pyrano[3,4-c]pyridin-7-yl)-7-fluoro-N-methylimidazo[1,5-a]quinoxaline-8-carboxamide

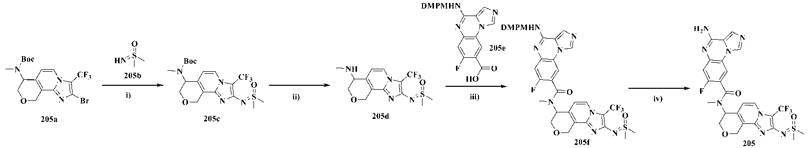

Referring to the synthesis method described in Step 12 of **Example 198,** Step 10 of **Example 204,** and Step 9 and Step 10 of **Example 12, Example 205** (18 mg, yield: 13.7%) was obtained from tert-butyl (2-bromo-3-(trifluoromethyl)-7,10-dihydro-8*H*-imidazo[1,2-*a*]pyrano[3,4-*c*]pyridin-7-yl)(methyl)carbamate **205a** (100 mg, 0.22 mmol). MS m/z (ESI): 591 [M+1]⁺

Alternatively, **Examples 206** and **234** were prepared using the following method:

### Example 206

### 4-Amino-N-(4-(difluoromethylene)-3,4,7,10-tetrahydro-2H,8H-pyrano[4,3-h]chroman-7-yl)-7-fluoro-N-methylimidazo[1,5-a]quinoline-8-carboxamide

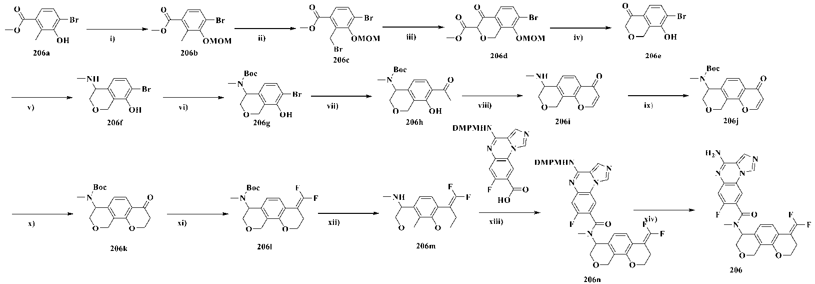

Step 1: **206a** (8.00 g, 32.79 mmol), triethylamine (5.05 g, 50.00 mmol) and bromomethyl methyl ether (6.25 g, 50.00 mmol) were dispersed in dichloromethane (100 mL), and the mixture was stirred and reacted at 25°C for 16 hours. The reaction solution was concentrated under reduced pressure to dryness, and the resulting residue was purified by silica gel column chromatography using eluent system A to obtain **206b** (9.00 g, yield: 95.3%). MS m/z (ESI): 289 [M+1]⁺

Referring sequentially to the method described in Step 1 to Step 4 and Step 7 of **Example 9, 206f** (4.50 g, yield: 56.0%) was obtained from **206b** (9.00 g, 31.25 mmol). MS m/z (ESI):258 [M+1]⁺

Step 6: **206f** (1.00 g, 3.89 mmol) and di-tert-butyl dicarbonate (1.09 g, 5.00 mmol) were dispersed in dichloromethane (10 mL), and the mixture was stirred and reacted at 25°C for 16 hours. The reaction solution was concentrated under reduced pressure to dryness, and the resulting residue was purified by silica gel column chromatography using eluent system A to obtain **206g** (1.51 g, crude). MS m/z (ESI): 358 [M+1]⁺

Step 7: **206g** (1.51 g, crude), tributyl(1-ethoxyvinyl)stannane (1.44 g, 4.00 mmol) and bis(triphenylphosphine)palladium(II) chloride (280 mg, 0.40 mmol) were dispersed in 1,4-dioxane (20 mL), and the mixture was stirred and reacted at 100°C for 16 hours. The reaction solution was concentrated under reduced pressure to dryness, and the resulting residue was purified by silica gel column chromatography using eluent system A to obtain **206h** (1.21 g, crude). MS m/z (ESI): 322 [M+1]⁺

Step 8: **206h** (1.21 g, crude) and *N,N-*dimethylformamide dimethyl acetal (952 mg, 8.00 mmol) were dispersed in toluene (10 mL), and the mixture was stirred and reacted at 80°C for 2 hours. Concentrated hydrochloric acid (5 mL) was added, and the resulting mixture was stirred and reacted at 50°C for 1 hour. The reaction solution was concentrated under reduced pressure to dryness to obtain **206i** (2.21 g, crude). MS m/z (ESI): 232 [M+1]⁺

Step 9: **206i** (2.21 g, crude) and di-tert-butyl dicarbonate (3.27 g, 15.00 mmol) were dispersed in dichloromethane (20 mL), and the mixture was stirred and reacted at 25°C for 2 hours. The reaction solution was concentrated under reduced pressure to dryness, and the resulting residue was purified by silica gel column chromatography using eluent system A to obtain **206j** (270 mg, yield: 21.0%). MS m/z (ESI): 332 [M+1]⁺

Step 10: **206j** (270 mg, 0.82 mmol), tris(triphenylphosphine)ruthenium(II) dichloride (77 mg, 0.08 mmol), potassium carbonate (113 mg, 0.82 mmol) and paraformaldehyde (123 mg, 4.10 mmol) were dispersed in toluene (5 mL) and water (0.5 mL), and the mixture was stirred and reacted at 110°C for 16 hours. The reaction solution was filtered to remove insoluble materials, the filtrate was concentrated under reduced pressure to dryness, and the resulting residue was purified by silica gel column chromatography using eluent system A to obtain **206k** (160 mg, yield: 58.6%). MS m/z (ESI): 334 [M+1]⁺

Step 11: **206k** (160 mg, 0.48 mmol) and difluoromethyl(2-pyridyl)sulphone (139 mg, 0.72 mmol) were dispersed in N,N-dimethylformamide (10 mL). Potassium tert-butoxide (108 mg, 0.96 mmol) was added, and the mixture was stirred and reacted at -40°C for 1 hour. The reaction solution was filtered to remove insoluble materials, the filtrate was concentrated under reduced pressure to dryness, and the resulting residue was purified by silica gel column chromatography using eluent system A to obtain **206l** (80 mg, yield: 45.4%). MS m/z (ESI): 368 [M+1]⁺

Step 12: **206l** (80 mg, 0.22 mmol) was dispersed in trifluoroacetic acid (2 mL) and dichloromethane (2 mL). The reaction solution was stirred and reacted at 25°C for 1 hour. The reaction solution was concentrated under reduced pressure to dryness to obtain **206m** (100 mg, crude). MS m/z (ESI): 268 [M+1]⁺

Referring to the synthesis method described in Step 9 to Step 10 of **Example 12, Example 206** (36 mg, yield: 33.1%) was obtained from **206m** (100 mg, crude). MS m/z (ESI): 496 [M+1]⁺

¹H NMR (400 MHz, DMSO-d₆) δ 9.18 (s, 1H), 8.36 (d, 1H), 7.97 (s, 1H), 7.77 (s, 2H), 7.46 (d, 1H), 7.28 (t, 1H), 6.93 (d, 1H), 5.70 (s, 1H), 4.83 - 4.61 (m, 1H), 4.49 (dd, 1H), 4.24 (dd, 2H), 4.06 - 3.86 (ddd, 2H), 2.79 - 2.61 (m, 5H).

### Example 234

### 4-Amino-N-(2-(3-(difluoromethylene)azetidine-1-carbonyl)-3-(trifluoromethyl)-7,10-dihydro-8H-imidazo[1,2-a]pyrano[3,4-c]pyridin-7-yl)-7-fluoro-N-methylimidazo[1,5-a]quinoxaline-8-carboxamide

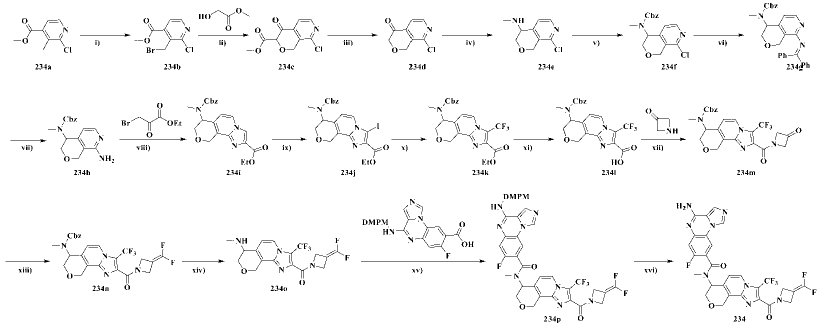

Step 1: Methyl 2-chloro-3-methylisonicotinate **234a** (10 g, 53.88 mmol) was weighed and added to a reaction flask, followed by carbon tetrachloride (200 mL). The mixture was stirred well at room temperature; N-bromosuccinimide (19.18 g, 107.75 mmol) and azobisisobutyronitrile (1.77 g, 10.78 mmol) were weighed and added to the above-mentioned reaction flask. The reaction system was purged three times with nitrogen and stirred at 77°C for 16 hours. The reaction solution was cooled to room temperature and filtered by suction through Celite, the filtrate was concentrated under reduced pressure, and the residue was purified by silica gel column chromatography using eluent system B to obtain **234b** (14 g, 52.93 mmol, yield: 98.2%). MS m/z (ESI): 265 [M+1]⁺

Step 2: Methyl glycolate (10.01 g, 111.15 mmol) was weighed and added to a reaction flask, followed by super-dry tetrahydrofuran (150 mL). The mixture was well mixed and then cooled to 0°C. Sodium hydride (6.35 g, 158.79 mmol, 60% purity) was added portionwise. After the addition, the resulting mixture was transferred to room temperature and reacted for 1 hour. The reaction solution was cooled to 0°C. **234b** (14 g, 52.93 mmol) was weighed and dissolved in super-dry tetrahydrofuran (30 mL), and the resulting mixture was slowly added dropwise to the above-mentioned reaction system. The reaction was kept at 0°C for 30 minutes. The reaction system was then stirred at room temperature for 16 hours. The reaction system was quenched with glacial acetic acid (4 mL) and water (100 mL), extracted with ethyl acetate (100 mL × 3), washed with a saturated sodium chloride aqueous solution, dried over anhydrous sodium sulphate, filtered, and concentrated. The residue was purified by silica gel column chromatography using eluent system B to obtain **234c** (9.45 g, 38.89 mmol, yield: 73.5%). MS m/z (ESI): 243 [M+1]⁺

Step 3: **234c** (12 g, 49.66 mmol) was weighed and dissolved in 1,4-dioxane (50 mL) and concentrated hydrochloric acid (50 mL) to form a clear solution, and the reaction system was stirred at 100°C for 2 hours. The reaction solution was concentrated to remove 1,4-dioxane. Water (100 mL) was added to the residual solution. The resulting mixture was adjusted to pH 7 with a saturated sodium bicarbonate solution, and extracted with ethyl acetate (100 mL × 3). The organic phase was washed with a saturated sodium chloride aqueous solution, dried over anhydrous sodium sulphate, filtered, and concentrated. The residue was purified by silica gel column chromatography using eluent system B to obtain **234d** (5.08 g, 27.67 mmol, yield: 55.7%). MS m/z (ESI): 184 [M+1]⁺

Step 4: **234d** (8.6 g, 46.84 mmol) was weighed and dissolved in trifluoroethanol (60 mL) to form a clear solution. A methylamine methanol solution (23.28 g, 187.37 mmol) was added, and the reaction system was reacted at room temperature for 16 hours. Sodium borohydride (3.54 g, 93.69 mmol) was added portionwise in an ice bath, and the reaction system was reacted at room temperature for 2 hours. The reaction system was quenched with an ammonium chloride aqueous solution (5 mL) and concentrated. Water (50 mL) was added to the residual solution. The resulting mixture was extracted with dichloromethane (50 mL × 3), and the organic phase was washed with a saturated sodium chloride aqueous solution, dried over anhydrous sodium sulphate, filtered, and concentrated. The residue was purified by silica gel column chromatography using eluent system A to obtain **234e** (4.04 g, 20.34 mmol, yield: 43.4%). MS m/z (ESI): 199 [M+1]⁺

Step 5: **234e** (4.04 g, 20.34 mmol) was weighed and dissolved in dichloromethane (40 mL), and triethylamine (6.17 g, 61.01 mmol, 8.51 mL) was added. Benzyl chloroformate (5.20 g, 30.51 mmol) was added dropwise in an ice bath, and the reaction system was reacted at room temperature for 1 hour. The reaction solution was directly concentrated, and the residue was purified by silica gel column chromatography using eluent system A to obtain 234f (5.6 g, 16.83 mmol, yield: 82.7%). MS m/z (ESI): 334 [M+1]⁺

Step 6: **234f** (5.6 g, 16.83 mmol) was weighed and dissolved in 1,4-dioxane (120 mL), tris(dibenzylideneacetone)dipalladium (1.54 g, 1.68 mmol), 4,5-bis(diphenylphosphino)-9,9-dimethylxanthene (1.95 g, 3.37 mmol), caesium carbonate (8.22 g, 25.24 mmol) and benzophenone imine (3.05 g, 16.83 mmol) were added, and the reaction system was purged three times with nitrogen and reacted at 100°C for 16 hours. The reaction solution was cooled to room temperature and filtered by suction through Celite. The resulting solid was washed with 1,4-dioxane, and the filtrate was concentrated and then used directly in the next step. MS m/z (ESI): 478 [M+1]⁺

Step 7: Methanol (40 mL) and hydrochloric acid (25 mL, 4 M in 1,4-dioxane) were directly added to the crude from the previous step, and the reaction system was reacted at room temperature for 6 hours. The reaction solution was concentrated, adjusted to weakly alkaline pH with a sodium bicarbonate aqueous solution, and extracted with dichloromethane (20% methanol). The organic phase was washed with a saturated sodium chloride aqueous solution, dried over anhydrous sodium sulphate, filtered, and concentrated. The residue was purified by silica gel column chromatography using eluent system A to obtain **234h** (4.12 g, 13.15 mmol, yield: 78.1%). MS m/z (ESI): 314 [M+1]⁺

Step 8: **234h** (4 g, 12.77 mmol) was dissolved in ethanol (40 mL), ethyl 3-bromo-2-oxopropanoate (3.73 g, 19.15 mmol) and sodium bicarbonate (2.14 g, 25.53 mmol) were added, and the reaction system was reacted at 70°C for 18 hours. The reaction solution was directly concentrated, and the residue was purified by silica gel column chromatography using eluent system B to obtain **234i** (3.88 g, 9.48 mmol, yield: 74.2%). MS m/z (ESI): 410 [M+1]⁺

Step 9: **234i** (3.88 g, 9.48 mmol) was dissolved in acetonitrile (40 mL), N-iodosuccinimide (2.56 g, 11.37 mmol) was added, and the reaction system was reacted at room temperature for 1 hour. The reaction solution was directly concentrated, and the residue was purified by silica gel column chromatography using eluent system B to obtain **234j** (3.94 g, 7.36 mmol, yield: 77.7%). MS m/z (ESI): 536 [M+1]⁺

Step 10: **234j** (3.84 g, 7.17 mmol) was dissolved in N,N-dimethylformamide (40 mL), methyl 2,2-difluoro-2-(fluorosulphonyl)acetate (4.13 g, 21.52 mmol) and cuprous iodide (4.10 g, 21.52 mmol) were added, and the reaction system was reacted at 100°C for 2 hours. The reaction solution was quenched with water and extracted with ethyl acetate (100 mL × 3), and the organic phase was washed with a saturated sodium chloride aqueous solution, dried over anhydrous sodium sulphate, filtered, and concentrated. The residue was purified by silica gel column chromatography using eluent system B to obtain **234k** (1.42 g, 2.97 mmol, yield: 41.5%). MS m/z (ESI): 478 [M+1]⁺

Step 11: **234k** (1.42 g, 2.97 mmol) was dissolved in tetrahydrofuran (20 mL) and water (20 mL), lithium hydroxide (213.70 mg, 8.92 mmol) was added, and the reaction system was reacted at room temperature for 2 hours. The reaction solution was distilled under reduced pressure. The residue was adjusted to acidic pH with 3 N hydrochloric acid. The aqueous phase was extracted with dichloromethane (plus 10% methanol), and the organic phase was dried over anhydrous sodium sulphate, filtered, and concentrated to obtain the crude product **234l** (1.15 g, 2.56 mmol, yield 86.0%). MS m/z (ESI): 450 [M+1]⁺

Step 12: **234l** (500 mg, 1.11 mmol) was dissolved in acetonitrile (5 mL), 3-oxoazetidine (143.58 mg, 1.34 mmol, CL), N,N-diisopropylethylamine (359.50 mg, 2.78 mmol, 484.50 µL) and N,N,N',N'-tetramethyl-O-(7-azabenzotriazol-1-yl)uronium hexafluorophosphate (629.65 mg, 1.67 mmol) were added, and the reaction system was reacted at room temperature for 2 hours. The reaction solution was quenched with water and extracted with ethyl acetate (100 mL × 3), and the organic phase was washed with a saturated sodium chloride aqueous solution, dried over anhydrous sodium sulphate, filtered, and concentrated. The residue was purified by silica gel column chromatography using eluent system B to obtain **234m** (213 mg, 423.93 µmol, yield: 38.1%). MS m/z (ESI): 503 [M+1]⁺

Step 13: **234m** (200 mg, 398.06 µmol) was dissolved in 1,4-dioxane (10 mL), 2,2-difluoro-2-(triphenylphosphonio)acetate (283.66 mg, 796.11 µmol) was added, and the reaction system was purged three times with nitrogen and reacted at 80°C for 1 hour. The reaction solution was quenched with water and extracted with ethyl acetate (100 mL × 3), and the organic phase was washed with a saturated sodium chloride aqueous solution, dried over anhydrous sodium sulphate, filtered, and concentrated. The residue was purified by silica gel column chromatography using eluent system B to obtain **234n** (106 mg, 197.60 µmol, yield: 49.6%). MS m/z (ESI): 537 [M+1]⁺

Step 14: **234n** (100 mg, 186.41 µmol) was added to trifluoroacetic acid (5 mL), and the reaction system was reacted at 80°C for 2 hours. The reaction solution was directly concentrated and adjusted to alkaline pH with a sodium bicarbonate aqueous solution, the aqueous phase was extracted with dichloromethane (plus 10% methanol), and the organic phase was dried over anhydrous sodium sulphate, filtered, and concentrated to obtain the crude product **234o** (55 mg, 136.71 µmol, yield: 73.3%). MS m/z (ESI): 403 [M+1]⁺

Step 15: **234o** (55 mg, 136.71 µmol) was dissolved in acetonitrile (5.02 mL), 4-((2,4-dimethoxybenzyl)amino)-7-fluoroimidazo[1,5-a]quinoxaline-8-carboxylic acid (65.02 mg, 164.05 µmol), N,N,N',N'-tetramethyl-O-(7-azabenzotriazol-1-yl)uronium hexafluorophosphate (77.36 mg, 205.06 µmol) and N,N-diisopropylethylamine (44.17 mg, 341.77 µmol, 59.53 µL) were added, and the reaction system was reacted at room temperature for 2 hours. The reaction solution was quenched with water and extracted with ethyl acetate (100 mL × 3), and the organic phase was washed with a saturated sodium chloride aqueous solution, dried over anhydrous sodium sulphate, filtered, and concentrated. The residue was purified by silica gel column chromatography using eluent system B to obtain **234p** (62 mg, 79.42 µmol, yield: 58.1%). MS m/z (ESI): 781 [M+1]⁺

Step 16: **234p** (62 mg, 79.42 µmol) was added to trifluoroacetic acid (5 mL), and the reaction system was reacted at 90°C for 2 hours. The reaction solution was directly concentrated and adjusted to alkaline pH with a sodium bicarbonate aqueous solution, the aqueous phase was extracted with dichloromethane (plus 10% methanol), and the organic phase was dried over anhydrous sodium sulphate, filtered, and concentrated. The residue was subjected to preparative chromatography to obtain **Example 234** (39 mg, 61.86 µmol, yield: 77.9%). MS m/z (ESI): 631 [M+1]⁺

¹H NMR (400 MHz, DMSO-d₆) δ 9.13 (d, 1H), 8.62 (d, 1H), 8.37 (d, 1H), 7.93 (d, 1H), 7.63 (s, 2H), 7.33 - 7.00 (m, 2H), 5.79 (s, 1H), 5.22 - 4.99 (m, 3H), 4.94 - 4.72 (m, 3H), 4.28 - 4.02 (m, 2H), 2.82 (s, 3H).

### Example 237

### 4-Amino-N-(2-(4-(difluoromethylene)azetidin-1-yl)-3-(trifluoromethyl)-7,10-dihydro-8H-imidazo[1,2-a]pyrano[3,4-c]pyridin-7-yl)-7-fluoro-N-methylimidazo[1,5-a]quinoxaline-8-carboxamide

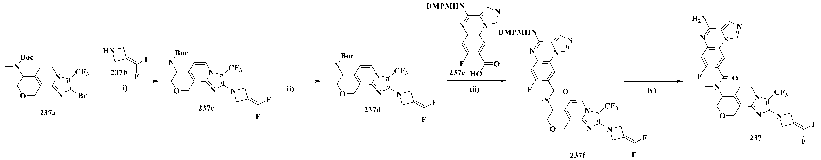

Referring to the synthesis method described in Step 12 of **Example 198,** Step 10 of **Example 204,** and Step 9 and Step 10 of **Example 12, Example 237** (8 mg, yield: 6.0%) was obtained from tert-butyl (2-bromo-3-(trifluoromethyl)-7,10-dihydro-8H-imidazo[1,2-a]pyrano[3,4-c]pyridin-7-yl)(methyl)carbamate 237a (100 mg, 0.22 mmol). MS m/z (ESI): 603 [M+1]⁺

¹H NMR (400 MHz, DMSO-d₆) δ9.07 (s, 1H), 8.29 (s, 2H), 7.94 (s, 1H), 7.40 (s, 2H), 7.24 (d, 1H), 6.96 (s, 1H), 5.77 (s, 1H), 5.13 - 4.64 (m, 6H), 4.19 (s, 2H), 2.76 (t, 3H).

### Example 240

### 4-Amino-N-(3-(cyclohexylidenefluoromethyl)-7,10-dihydro-8H-imidazo[1,2-a]pyrano[3,4-c]pyridin-7-yl)-7-fluoro-N-methylimidazo[1,5-a]quinoxaline-8-carboxamide

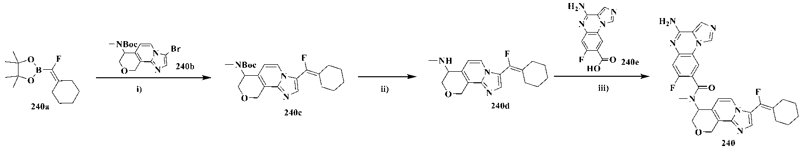

Referring to the synthesis method described in Step 3 to Step 5 of **Example 202, Example 240** (30 mg, yield: 27.8%) was obtained from 2-(cyclohexylidenefluoromethyl)-4,4,5,5-tetramethyl-1,3,2-dioxaborolane **240a** (943 mg, 3.92 mmol) and tert-butyl (3-bromo-7,10-dihydro-8H-imidazo[1,2-a]pyrano[3,4-c]pyridin-7-yl)(methyl)carbamate **240b** (300 mg, 0.78 mmol). MS m/z (ESI): 544 [M+1]⁺

¹H NMR (400 MHz, DMSO-d₆) δ 9.14 (d, 1H), 8.33 (dd, 2H), 7.93 (s, 1H), 7.72 (dd, 1H), 7.62 (s, 2H), 7.27 (dd, 1H), 6.91 (d, 1H), 5.76 (s, 1H), 5.10 (dd, 1H), 4.88-4.70 (m, 1H), 4.27-4.00 (m, 2H), 2.86 (s, 1H), 2.76 (s, 2H), 2.44 (d, 2H), 2.06 (d, 2H), 1.59 (d, 6H).

### Example 276

### 4-Amino-N',N',7-trimethyl-N-(2-(trifluoromethyl)-5,8-dihydro-6H-pyrano[3,4-b]pyridin-5-yl)imidazo[1,5-a]quinoxaline-8-carbohydrazide

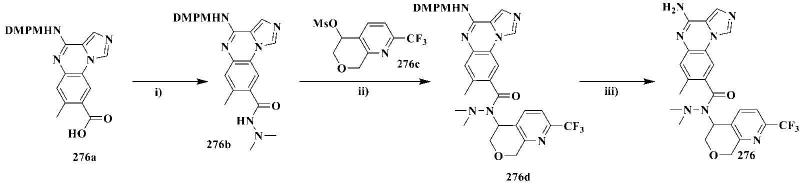

Step 1: 4-((3,5-Dimethoxybenzyl)amino)-7-methylimidazo[1,5-a]quinoxaline-8-carboxylic acid **276a** (0.5 g, 1.27 mmol), 1,1-dimethylhydrazine (115 mg, 1.91 mmol) and 2-(7-azobenzotriazol-1-e)-N,N,N',N'-tetramethyluronium hexafluorophosphate (721 mg, 1.91 mmol) were dissolved in N,N-dimethylformamide (10 mL), N,N-diisopropylethylamine (494 mg, 3.82 mmol, 0.66 mL) was added, and the reaction system was purged three times with nitrogen and stirred at 25°C for 1 hour. The resulting reaction system was quenched with water, extracted with ethyl acetate (50 mL × 3), washed with a saturated sodium chloride aqueous solution, dried over anhydrous sodium sulphate, filtered, and concentrated. The residue was purified by silica gel column chromatography using eluent system B to obtain **276b** (0.51 g, yield: 92.1%). MS m/z (ESI): 435 [M+1]⁺

Step 2: **276b** (0.40 g, 0.92 mmol), [2-(trifluoromethyl)-6,8-dihydro-5H-pyrano[3,4-b]pyridin-5-yl]methanesulphonate 276c (410 mg, 1.38 mmol) and caesium carbonate (900 mg, 2.76 mmol) were dissolved in N,N-dimethylformamide (10 mL), and the reaction system was purged three times with nitrogen and stirred at 60°C for 15 hours. The reaction system was cooled to room temperature, quenched with water, extracted with ethyl acetate (80 mL × 3), washed with a saturated sodium chloride aqueous solution, dried over anhydrous sodium sulphate, filtered, and concentrated. The residue was purified by silica gel column chromatography using eluent system B to obtain **276d** (30 mg, yield: 5.1%). MS m/z (ESI): 636 [M+1]⁺

Step 3: **276d** (24 mg, 38 µmol) was dissolved in TFA (3 mL), and the reaction system was purged three times with nitrogen and stirred at 90°C for 3 hours. The resulting reaction system was cooled to room temperature and concentrated. The residue was purified by silica gel column chromatography using eluent system A to obtain **Example 276** (5 mg, yield: 27.3%). MS m/z (ESI): 486 [M+1]⁺

¹H NMR (400 MHz, CD₃OD) δ 8.99 (s, 1H), 8.31 (d, 1H), 8.04 (s, 1H), 7.94 (s, 1H), 7.76 (d, 1H), 7.34 (s, 1H), 6.25 (d, 1H), 4.61 (s, 2H), 4.41 (dd, 1H), 4.22 (d, 1H), 3.60 (q, 1H), 3.35 (s, 1H), 2.43 (s, 6H), 2.35 (s, 3H)

### Example 277

### 4-Amino-N'-ethyl-7-methyl-N-(2-(trifluoromethyl)-5,8-dihydro-6H-pyrano[3,4-b]pyridin-5-yl)imidazo[1,5-a]quinoxaline-8-carbohydrazide

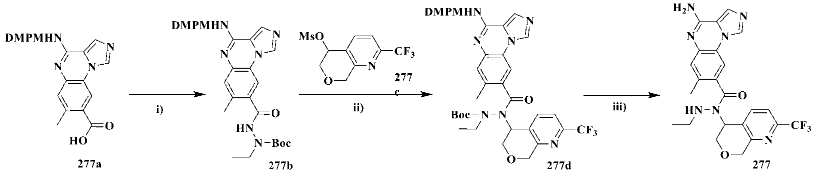

Referring to the synthesis method described in Step 1 to Step 3 of **Example 276, Example 277** (35 mg, yield: 14.1%) was obtained from 4-((3,5-dimethoxybenzyl)amino)-7-methylimidazo[1,5-a]quinoxaline-8-carboxylic acid **277a** (200 mg, 0.51 mmol). MS m/z (ESI): 486 [M+1]⁺

¹H NMR (400 MHz, DMSO-d₆) δ 9.05 (d, 1H), 8.12 (d, 2H), 7.92 (s, 1H), 7.81 (d, 1H), 7.28 (d, 3H), 5.04 (s, 1H), 4.89-4.61 (m, 2H), 4.36-4.08 (m, 2H), 2.39 (s, 3H), 0.67 (s, **3H).**

### Example 278

### 4-Amino-N',7-dimethyl-N-(2-(trifluoromethyl)-5,8-dihydro-6H-pyrano[3,4-b]pyridin-5-yl)imidazo[1,5-a]quinoxaline-8-carbohydrazide

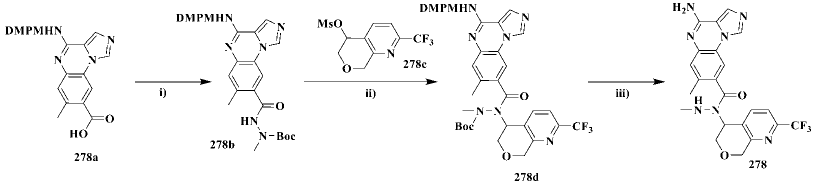

Referring to the synthesis method described in Step 1 to Step 3 of **Example 276, Example 278** (26 mg, yield: 10.9%) was obtained from 4-((3,5-dimethoxybenzyl)amino)-7-methylimidazo[1,5-a]quinoxaline-8-carboxylic acid **278a** (200 mg, 0.51 mmol). MS m/z (ESI): 472 [M+1]⁺

¹H NMR (400 MHz, DMSO-d₆) δ 9.17 (s, 1H), 8.41-8.16 (m, 1H), 8.12 (d, 1H), 8.00 (d, , 1H), 7.87 (d, 1H), 7.82 (d, 1H), 7.34 (d, 1H), 5.53-5.41 (m, 1H), 5.29 (d, 1H), 4.93-4.82 (m, 1H), 4.77-4.59 (m, 1H), 4.29 (qd, 1H), 4.03 (s, 1H), 2.57 (s, 1H), 2.44 (s, 2H), 2.29 (d, 3H).

### Example 278 Preparation of chiral isomers

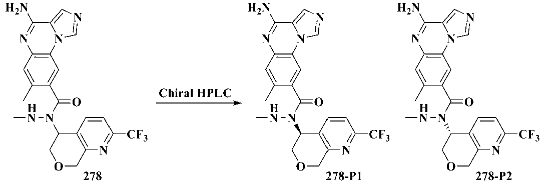

**Example 278** (26 mg, 0.055 mmol) was resolved by Chiral-HPLC (condition 1) to obtain **278-P1:** (*S*)-4-amino-N',7-dimethyl-N-(2-(trifluoromethyl)-5,8-dihydro-6H-pyrano[3,4-b]pyridin-5-yl)imidazo[1,5-a]quinoxaline-8-carbohydrazide (12.6 mg, yield: 48.5%) and **278-P2:** (*R*)-4-amino-N',7-dimethyl-N-(2-(trifluoromethyl)-5,8-dihydro-6H-pyrano[3,4-b]pyridin-5-yl)imidazo[1,5-a]quinoxaline-8-carbohydrazide (13.0 mg, yield: 50.0%), respectively.

**278-P1 (t_{R}: 5.897 min):** ¹H NMR (400 MHz, DMSO-d₆) δ 9.17 (s, 1H), 8.41-8.16 (m, 1H), 8.12 (d, 1H), 8.00 (d, 1H), 7.87 (d, 1H), 7.82 (d, 1H), 7.34 (d, 1H), 5.53-5.41 (m, 1H), 5.29 (d, 1H), 4.93-4.82 (m, 1H), 4.77-4.59 (m, 1H), 4.29 (qd, 1H), 4.03 (s, 1H), 2.57 (s, 1H), 2.44 (s, 2H), 2.29 (d, 3H).

**278-P2 (t_{R}: 5.240 min):** ¹H NMR (400 MHz, DMSO-d₆) δ 9.17 (s, 1H), 8.41-8.16 (m, 1H), 8.12 (d, 1H), 8.00 (d, 1H), 7.87 (d, 1H), 7.82 (d, 1H), 7.34 (d, 1H), 5.53-5.41 (m, 1H), 5.29 (d, 1H), 4.93-4.82 (m, 1H), 4.77-4.59 (m, 1H), 4.29 (qd, 1H), 4.03 (s, 1H), 2.57 (s, 1H), 2.44 (s, 2H), 2.29 (d, 3H).

### Example 279

### 4-Amino-7-fluoro-N'-methyl-N-[2-(trifluoromethyl)-6,8-dihydro-5H-pyrano[3,4-b]pyridin-5-yl]imidazo[1,5-a]quinoxaline-8-hydrazide

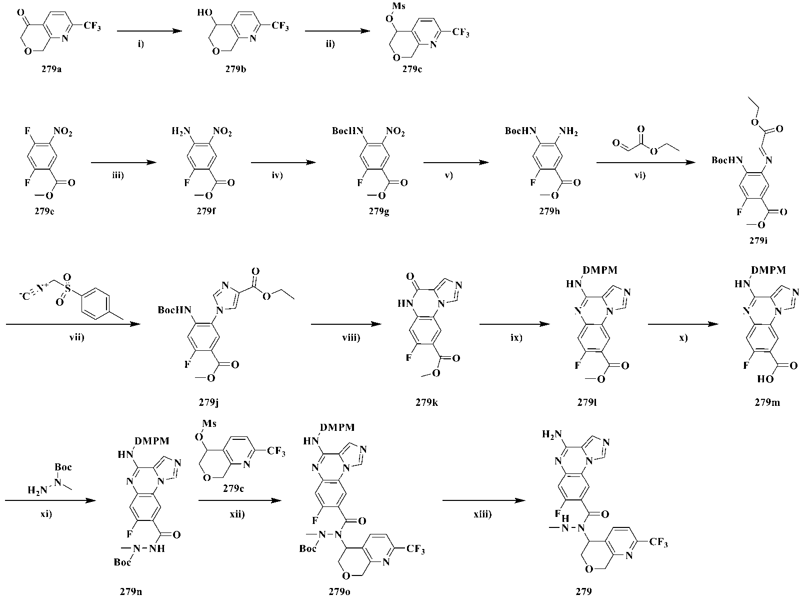

Referring to the synthesis method described in Step 1 to Step 3 **of Example 278, Example 279** (11 mg, yield: 0.2%) was obtained from compound methyl 2,4-difluoro-5-nitrobenzoate 279e (500 mg, 2.35 mmol). MS m/z (ESI): 476 [M+1]⁺

¹H NMR (400 MHz, DMSO-d₆) δ 9.07 (s, 1H), 8.26 (s, 1H), 8.04 (s, 1H), 7.92 (s, 1H), 7.79 (dd, 1H), 7.34 (s, 2H), 7.19 (d, 1H), 5.21 (s, 1H), 4.91 - 4.68 (m, 2H), 4.25 (s, 2H), 2.42 (s, 3H).

### Example 280

### 4-Amino-7-fluoro-N-(2-(trifluoromethyl)-5,8-dihydro-6H-pyrano[3,4-b]pyridin-5-yl)imidazo[1,5-a]quinoxaline-8-carbohydrazide

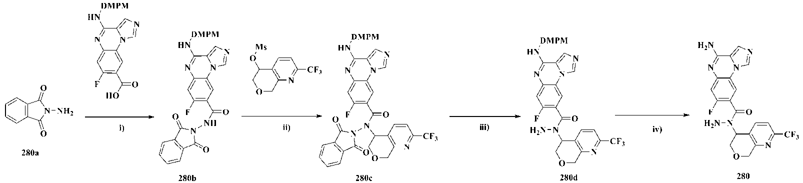

Step 1: 2-Aminoisoindoline-1,3-dione **280a** (184 mg, 1.14 mmol) and 4-((3,4-dimethylbenzyl)amino)-7-fluoroimidazo[1,5-a]quinoxaline-8-carboxylic acid (300 mg, 0.76 mmol) were dissolved in dichloromethane (10 mL), pyridine (239 mg, 3.03 mmol) and phosphorus oxychloride (348 mg, 2.27 mmol) were sequentially added, and the mixture was stirred and reacted at room temperature for 1 hour. The reaction solution was concentrated under reduced pressure, and the resulting residue was purified by silica gel column chromatography using eluent system A to obtain **280b** (162 mg, yield: 39.6%). MS m/z (ESI): 541 [M+1]⁺

Step 2: **280b** (162 mg, 0.30 mmol), sodium iodide (89 mg, 0.60 mmol), caesium carbonate (292 mg, 0.90 mmol) and 2-(trifluoromethyl)-5,8-dihydro-6H-pyrano[3,4-b]pyridin-5-ylmethanesulphonate (133 mg, 0.45 mmol) were dissolved in acetonitrile (10 mL), and the mixture was reacted under microwave irradiation at 100°C for 16 hours. The reaction solution was filtered, and ethyl acetate (100 mL) was added to the filtrate. The mixture was washed with a saturated sodium chloride aqueous solution (100 mL × 2), and the organic phase was dried over anhydrous sodium sulphate and filtered. The filtrate was concentrated under reduced pressure, and the resulting residue was purified by silica gel column chromatography using eluent system A to obtain **280c** (66 mg, yield: 29.7%). MS m/z (ESI): 742 [M+1]⁺

Step 3: **280c** (66 mg, 0.09 mmol) was dissolved in tetrahydrofuran (2 mL) and ethanol (2 mL), hydrazine hydrate (16 mg, 0.27 mmol, 85% purity) was added, and the mixture was reacted at 80°C in a sealed tube for 16 hours. The reaction solution was concentrated under reduced pressure, and the resulting residue was purified by silica gel column chromatography using eluent system A to obtain **280d** (16 mg, yield: 29.4%). MS m/z (ESI): 612 [M+1]⁺

Step 4: **280d** (16 mg, 0.02 mmol) was dissolved in dichloromethane (2 mL) and water (1 mL), 2,3-dichloro-5,6-dicyanobenzoquinone (10.91 mg, 0.05 mmol, 60% purity) was added, and the mixture was stirred and reacted at room temperature for 1 hour. The reaction solution was concentrated under reduced pressure, and the resulting residue was purified by HPLC (BASE) to obtain Example **280** (2 mg, yield: 16.1%). MS m/z (ESI): 462 [M+1]⁺

¹H NMR (400 MHz, DMSO-d₆) δ 9.12 (s, 1H), 8.32 (d, 1H), 7.97 (d, 1H), 7.91 (d, 2H), 7.55 (s, 2H), 7.16(d, 1H), 5.88 (d, 1H), 4.85 - 4.79 (m, 2H), 4.62 (s, 2H), 4.20 (d, 2H).

### Example 281

### 4-Amino-N',7-dimethyl-N-(3-(trifluoromethyl)-7,10-dihydro-8H-imidazo[1,2-a]pyrano[3,4-c]pyridin-7-yl)imidazo[1,5-a]quinoxaline-8-carbohydrazide

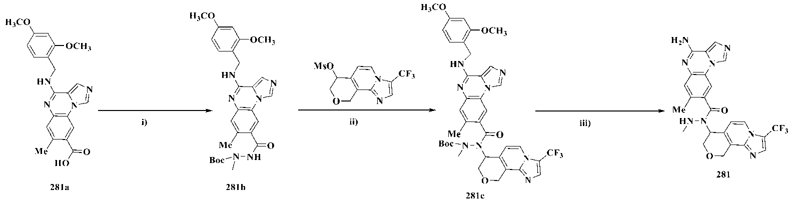

Referring to Step 1 to Step 2 of **Example 276, 281c** (60 mg, yield: 20%) was obtained from tert-butyl 2-(4-((2,4-dimethoxybenzyl)amino)-7-methylimidazo[1,5-a]quinoxaline-8-carbonyl)-1-methylhydrazine-1-carboxylate **281b** (200 mg, 0.38 mmol) and 3-(trifluoromethyl)-7,10-dihydro-8H-imidazo[1,2-a]pyrano[3,4-c]pyridin-7-ylmethanesulphonate (260 mg, 0.76 mmol). MS m/z (ESI): 761 [M+1]⁺

Step 3: **281c** (20 mg, 0.026 mmol) was dissolved in trifluoroacetic acid (2 mL), and the mixture was stirred and reacted at 90°C for 1 hour. The mixture was concentrated under reduced pressure, and the resulting residue was purified by silica gel column chromatography using eluent system A to obtain **Example 281** (6.7 mg, yield: 49%). MS m/z (ESI): 511 [M+1]⁺

¹HNMR (400 MHz, DMSO-d₆) δ 9.08 (s, 1H), 8.50 (d, 1H), 8.19 (s, 1H), 8.10 (d, 1H), 7.88 (s, 1H), 7.38 (d, 1H), 7.32 - 7.23 (m, 2H), 5.46 (d, 1H), 5.14 - 5.04 (m, 1H), 4.99 (d, 1H), 4.88 (q, 1H), 4.72 (d, 1H), 4.30 (dq, 1H), 4.09 (s, 1H), 2.74 - 2.52 (m, 2H), 2.45 - 2.16 (m, 4H).

### Example 282

### 4-Amino-7-fluoro-N'-methyl-N-(3-(trifluoromethyl)-7,10-dihydro-8H-imidazo[1,2-a]pyrano[3,4-c]pyridin-7-yl)imidazo[1,5-a]quinoxaline-8-carbohydrazide

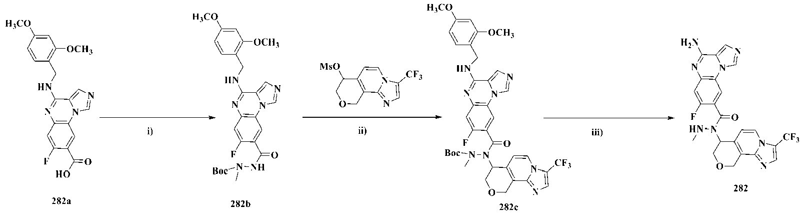

Referring to Step 1 to Step 2 of **Example 279, 282c** (200 mg, yield: 68%) was obtained from tert-butyl 2-(4-((2,4-dimethoxybenzyl)amino)-7-methylimidazo[1,5-a]quinoxaline-8-carbonyl)-1-methylhydrazine-1-carboxylate **282b** (200 mg, 0.38 mmol) and 3-(trifluoromethyl)-7,10-dihydro-8H-imidazo[1,2-a]pyrano[3,4-c]pyridin-7-ylmethanesulphonate (260 mg, 0.76 mmol). MS m/z (ESI): 765 [M+1]⁺

Step 3: **282c** (50 mg, 0.065 mmol) was dissolved in trifluoroacetic acid (2 mL), and the mixture was stirred and reacted at 90°C for 1 hour. The mixture was concentrated under reduced pressure, and the resulting residue was purified by silica gel column chromatography using eluent system A to obtain **Example 282** (15.3 mg, yield: 45%). MS m/z (ESI): 515 [M+1]⁺

¹HNMR (400 MHz, DMSO-d₆) δ 9.16 (d, 1H), 8.48 (dd, 1H), 8.27 (d, J = 6.5 Hz, 1H), 8.16 (d, 1H), 7.93 (d, 1H), 7.60 (d, 2H), 7.33 - 7.12 (m, 2H), 5.50 - 5.21 (m, 1H), 5.04 (q, 1H), 4.91 (td, 2H), 4.29 (dt, 1H), 4.21 - 3.94 (m, 1H), 2.58 (d, 1H), 2.35 (d, 2H).

### Example 283

### 4-Amino-N-[2-[(1R,4S)-5-(difluoromethylene)-2-azabicyclo[2.2.2]octan-2-yl]-6,8-dihydro-5H-pyrano[3,4-b]pyridin-5-yl]-7-fluoro-N-methyl-imidazo[1,5-a]quinoxaline-8-carboxamide

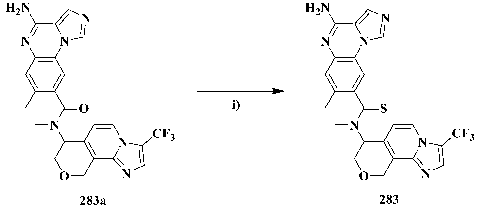

Step 1: 4-Amino-N,7-dimethyl-N-[5-(trifluoromethyl)-12-oxa-3,6-diazatricyclo[7.4.0.02,6]trideca-1(9),2,4,7-tetraen-10yl]imidazo[1,5-a]quinoxaline-8-carboxamide **283a** (50 mg, 101 µmol, **Example 62)** was dissolved in 1'4-dioxane (3 mL), Lawesson's Reagent (122.45 mg, 303 µmol) was added, and the mixture was purged three times with nitrogen, and stirred and reacted at 120°C under nitrogen atmosphere for 16 hours; the reaction solution was concentrated under reduced pressure, and the concentrated residue was separated and purified by Pre-HPLC to obtain **Example 283** (14 mg, yield: 27.1%). MS m/z (ESI): 512 [M+1]⁺

¹H NMR (400 MHz, DMSO-d₆) δ 9.18 - 8.98 (m, 1H), 8.57 - 8.44 (m, 1H), 8.23 - 8.08 (m, 1H), 8.03 - 7.83 (m, 2H), 7.33 (dt, 3H), 7.18 - 7.00 (m, 1H), 5.30 - 4.70 (m, 3H), 4.51 - 4.17 (m, 2H), 2.87 (d, 3H), 2.42 - 2.16 (m, 3H).

### Example 284

### 4-Amino-6-fluoro-N',7-dimethyl-N-(2-(trifluoromethyl)-5,8-dihydro-6H-pyrano[3,4-b]pyridin-5-yl)imidazo[1,5-a]quinoxaline-8-carbohydrazide

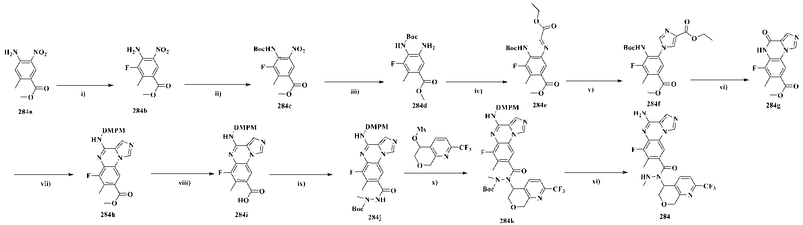

Step 1: Methyl 4-amino-2-methyl-5-nitrobenzoate **284a** (3 g, 14.27 mmol) was dissolved in acetonitrile (50 mL), 1-chloromethyl-4-fluoro-1,4-diazidobicyclo[2.2.2]octane bis(tetrafluoroboronate) (6.07 g, 17.13 mmol) was added, and the mixture was stirred and reacted at 50°C for 3 hours. The reaction solution was concentrated under reduced pressure, and the resulting residue was purified by silica gel column chromatography using eluent system C to obtain **284b** (1.03 g, yield: 31.6%). MS m/z (ESI): 229 [M+1]⁺

Referring sequentially to the method described in Step 1 to Step 9 of **Example 12,** Step 2 of **Example 280** and Step 10 of **Example 12, Example 284** (29 mg, yield: 1.3%) was obtained from **284b** (1.03 g, 4.51 mmol). MS m/z (ESI): 490 [M+1]⁺

The examples in the table below were prepared with reference to **Example 284:**

| **Example** | **Structure** | **MS m/z (ESI)** | **Example** | **Structure** | **MS m/z (ESI)** |
|---|---|---|---|---|---|
| | | **[M+1]⁺** | | | **[M+1]⁺** |
| **285** | 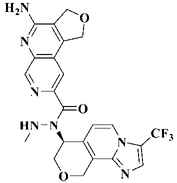 | 500 | **286** | 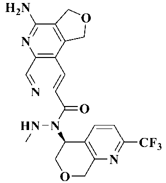 | 461 |

Alternatively, **Examples 285** and **286** were prepared using the following method:

### Example 285

### 4-Amino-N'-methyl-N-(3-(trifluoromethyl)-7,10-dihydro-8H-imidazo[1,2-a]pyrano[3,4-c]pyridin-7-yl)-1,3-dihydrofuro[3,4-c][1,7]diazanaphthalene-8-carbohydrazide

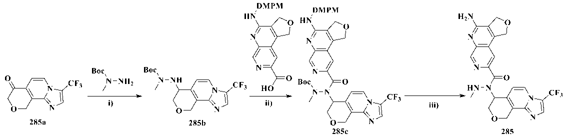

Step 1: 3-(Trifluoromethyl)-8H-imidazo[1,2-a]pyrano[3,4-c]pyridin-7(10H)-one **285a** (1.3 g, 5.07 mmol) and tert-butyl *N*-amino-N-methylcarbamate (1.48 g, 10.15 mmol) were dissolved in methanol (10 mL) and tetrahydrofuran (10 mL), acetic acid (305 mg, 5.07 mmol) and sodium cyanoborohydride (1.59 g, 25.37 mmol) were added, and the mixture was stirred and reacted at 20°C for 48 hours. The reaction was quenched by adding a saturated sodium bicarbonate solution (100 ml) to the reaction solution, and the mixture was extracted with dichloromethane (100 mL × 2). The organic phase was dried over anhydrous sodium sulphate, the system was concentrated under reduced pressure, and the resulting residue was purified by silica gel column chromatography using eluent system B to obtain **285b** (1.6 g, yield: 81.6%). MS m/z (ESI): 387 [M+1]⁺

Step 2: **285b** (400 mg, 1.04 mmol), 4-(2,4-dimethoxybenzylamino)-1,3-dihydrofuro[3,4-c][1,7]naphthyridine-8-carboxylic acid (395 mg, 1.04 mmol), POCl₃ (456 mg, 2.97 mmol) and pyridine (392 mg, 4.95 mmol) were dissolved in N,N-dimethylacetamide (20 mL), and the mixture was stirred and reacted at 20°C for 2 hours. The reaction solution was concentrated, and ethyl acetate (50 ml) was added. The mixture was washed with 2 M hydrochloric acid to remove pyridine, and the organic phase was dried over anhydrous sodium sulphate and filtered. The filtrate was concentrated under reduced pressure, and the resulting residue was purified by silica gel column chromatography using eluent system B to obtain **285c** (220 mg, yield: 28.3%). MS m/z (ESI): 750 [M+1]⁺

Referring to the synthesis method described in Step 11 of **Example 14, Example 285** (90 mg, yield: 61.7%) was obtained from **285c** (220 mg, 0.29 mmol). MS m/z (ESI): 499 [M+1]⁺

### Example 285 Preparation of chiral isomers

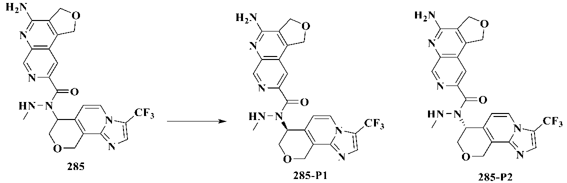

**Example 285** (90 mg, 0.18 mmol) was separated by chiral preparative HPLC (condition 2) to obtain (S)-4-amino-N'-methyl-N-(3-(trifluoromethyl)-7,10-dihydro-8H-imidazo[1,2-a]pyrano[3,4-c]pyridin-7-yl)-1,3-dihydrofuro[3,4-c][1,7]diazanaphthalene-8-carbohydrazide **285-P1** (38 mg, yield: 42.2%) and (R)-4-amino-N'-methyl-N-(3-(trifluoromethyl)-7,10-dihydro-8H-imidazo[1,2-a]pyrano[3,4-c]pyridin-7-yl)-1,3-dihydrofuro[3,4-c][1,7]diazanaphthalene-8-carbohydrazide **285-P2** (42 mg, yield: 46.7%).

**285-P1 (t_{R}: 0.765 min):** ¹H NMR (400 MHz, DMSO-d₆) 8.87 (d, 1H), 8.47 (dd, 1H), 8.16 (d, 3H), 7.96 (d, 1H), 7.22 - 7.04 (m, 3H), 6.18 (d, 1H), 5.57 (d, 1H), 5.41 (d, 2H), 5.32 (d, 1H), 5.05 (t, 2H), 5.01 (d, 1H), 4.93 (s, **1H),** 4.31 - 4.03 (m, 2H).

**285-P2 (t_{R}: 0.974 min):** ¹H NMR (400 MHz, DMSO-d₆) δ 8.86 (d, 1H), 8.47 (dd, 1H), 8.16 (d, 3H), 7.93 (s, 1H), 7.23-7.03 (m, 3H), 6.17 (d, 1H), 5.56 (d, 1H), 5.41 (s, 2H), 5.32 (d, 1H), 5.05 (t, 2H), 5.01 (d, 1H), 4.93 (s, 1H), 4.31 - 4.04 (m, 2H).

### Example 286

### 4-Amino-N'-methyl-N-(2-trifluoromethyl-5,8-dihydro-6H-pyrano[3,4-b]pyridin-5-yl)-1,3-dihydrofuro[3,4-c] [1,7]naphthyridine-8-carbohydrazide

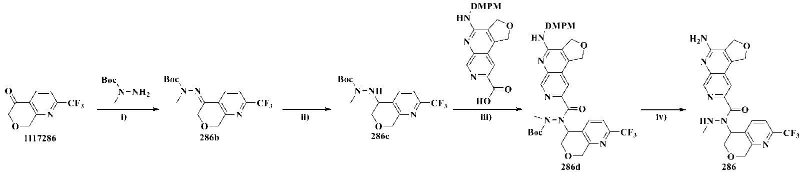

Step 1: 2-Trifluoromethyl-6H-pyrano[3,4-b]pyridin-5(8*H*)-one **286a** (640 mg, 2.95 mmol) and tert-butyl *N*-amino-*N*-methyl carbamate (430.86 mg, 2.95 mmol) were dissolved in 1,1,1-trifluoroethanol (5 mL), and the mixture was stirred and reacted at 20°C for 3 hours. The system was concentrated under reduced pressure, and the resulting residue was purified by silica gel column chromatography using eluent system A to obtain **286b** (1 g, yield: 98.3%). MS m/z (ESI): 346 [M+1]⁺

Step 2: **286b** (900 mg, 2.61 mmol) was dissolved in methanol (20 mL), and the mixture was purged with hydrogen, and stirred and reacted at 20°C for 3 hours. The system was filtered, and the filtrate was concentrated under reduced pressure to obtain **286c** (900 mg, yield: 99.4%). MS m/z (ESI): 348 [M+1]⁺

Step 3: **286c** (227 mg, 0.59 mmol), 4-(2,4-dimethoxybenzylamino)-1,3-dihydrofuro[3,4-c][1,7]naphthyridine-8-carboxylic acid (172 mg, 0.50 mmol), POCl₃ (456 mg, 2.97 mmol) and pyridine (392 mg, 4.95 mmol) were dissolved in 1,2-dichloroethane (20 mL), and the mixture was stirred and reacted at 20°C for 6 hours. The reaction was quenched with triethylamine (1 mL), and the system was concentrated to dryness. Ethyl acetate (20 mL) was then added to fully dissolve the residue, and the mixture was filtered. The filtrate was concentrated under reduced pressure, and the resulting residue was purified by silica gel column chromatography using eluent system A to obtain **286d** (150 mg, yield: 42.6%). MS m/z (ESI): 711 [M+1]⁺

Step 4: **286d** (150 mg) was dissolved in TFA (20 mL), and the mixture was reacted at 90°C for 1 h. The reaction system was cooled and quenched. The system was concentrated to dryness, and the resulting residue was purified by reversed-phase chromatography using eluent system D to obtain example **286** (92 mg, yield: 94.7%). MS m/z (ESI): 461 [M+1]⁺

¹H NMR (400 MHz, DMSO-d₆) δ 8.85 (s, 1H), 8.09-8.00 (m, 1H), 7.99-7.91 (m, 1H), 7.89-7.79 (m, 1H), 7.14 (s, 2H), 6.18 (s, 0.5H), 5.65 (m, 1H), 5.40 (s, 2H), 5.37-5.30 (m, 0.5H), 5.05 (t, 2H), 4.79 (m, 2H), 4.26 (td, 1H), 4.09 (dt, 1H), 2.47 (d, 2H), 2.17 (d, 1H).

### Example 286 Preparation of chiral isomers

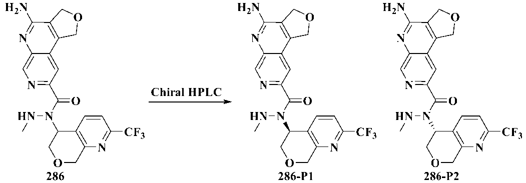

**Example 286** (50 mg, 0.108 mmol) was resolved by Chiral-HPLC (condition 2) to obtain **286-P1:** (*S*)-4-amino-*N'*-methyl-N-(2-trifluoromethyl-5,8-dihydro-6H-pyrano[3,4-b]pyridin-5-yl)-1,3-dihydrofuro[3,4-c][1,7]naphthyridine-8-carbohydrazide (23.8 mg, yield: 47.6%) and **286-P2:** (*R*)-4-amino-*N*'-methyl-N-(2-trifluoromethyl-5,8-dihydro-6H-pyrano[3,4-b]pyridin-5-yl)-1,3-dihydrofuro[3,4-c][1,7]naphthyridine-8-carbohydrazide (24.0 mg, yield: 48.0%), respectively.

**286-P1 (t_{R}: 2.521 min):** ¹H NMR (400 MHz, DMSO-d₆) δ 8.85 (s, 1H), 8.09-8.00 (m, 1H), 7.99-7.91 (m, 1H), 7.89-7.79 (m, 1H), 7.14 (s, 2H), 6.18 (s, 0.5H), 5.65 (m, 1H), 5.40 (s, 2H), 5.37-5.30 (m, 0.5H), 5.05 (t, 2H), 4.79 (m, 2H), 4.26 (td, 1H), 4.09 (dt, 1H), 2.47 (d, 2H), 2.17 (d, 1H).

**286-P2 (t_{R}: 2.818 min):** ¹H NMR (400 MHz, DMSO-d₆) δ 8.85 (s, 1H), 8.09-8.00 (m, 1H), 7.99-7.91 (m, 1H), 7.89-7.79 (m, 1H), 7.14 (s, 2H), 6.18 (s, 0.5H), 5.65 (m, 1H), 5.40 (s, 2H), 5.37-5.30 (m, 0.5H), 5.05 (t, 2H), 4.79 (m, 2H), 4.26 (td, 1H), 4.09 (dt, 1H), 2.47 (d, 2H), 2.17 (d, 1H).

The examples in the table below were prepared with reference to Example 277:

| **Example** | **Structure** | **MS m/z (ESI)** | **Example** | **Structure** | **MS m/z (ESI)** |
|---|---|---|---|---|---|
| | | **[M+1]⁺** | | | **[M+1]⁺** |
| **287** | 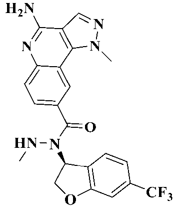 | 457 | **317** | 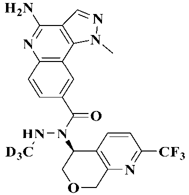 | 475 |
| **288** | 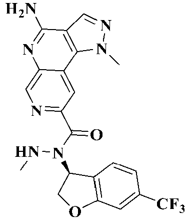 | 458 | **318** | 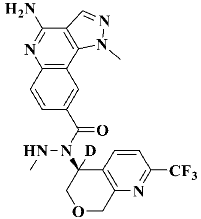 | 473 |
| **289** | 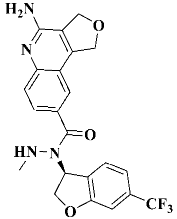 | 445 | **319** | 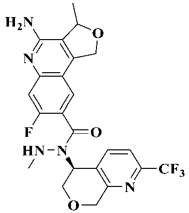 | 492 |
| **290** | 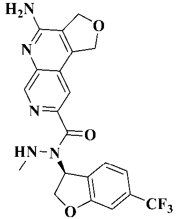 | 446 | **320** | 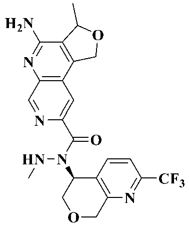 | 475 |
| **291** | 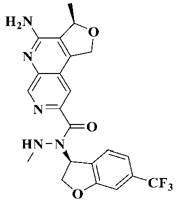 | 460 | **321** | 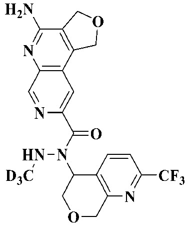 | 464 |
| **292** | 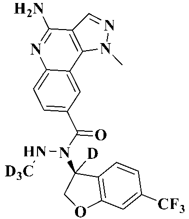 | 461 | **321-P1** | 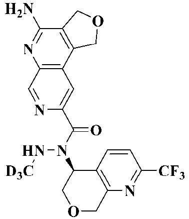 | 464 |
| **293** | 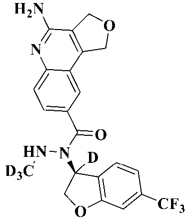 | 449 | **321-P2** | 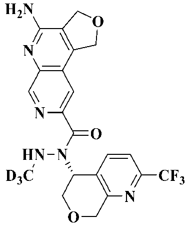 | 464 |
| **294** | 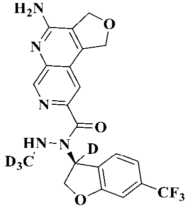 | 450 | **322** | 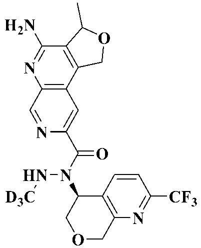 | 478 |
| **295** | 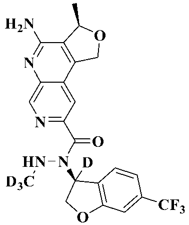 | 464 | **323** | 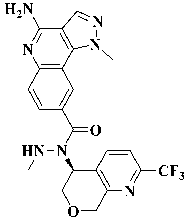 | 472 |
| **296** | 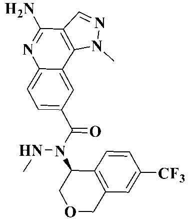 | 471 | **324** | 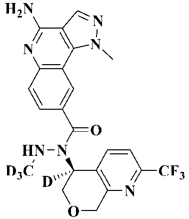 | 476 |
| **297** | 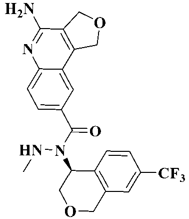 | 459 | **325** | 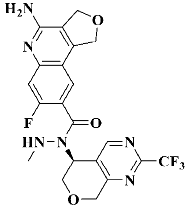 | 479 |
| **298** | 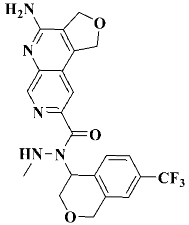 | 460 | **326** | 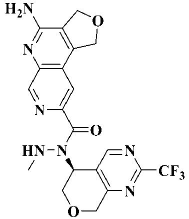 | 462 |
| **298-P1** | 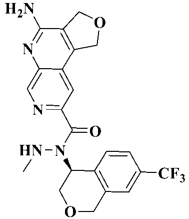 | 460 | **327** | 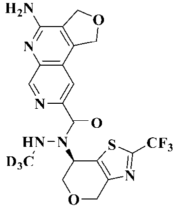 | 470 |
| **298-P2** | 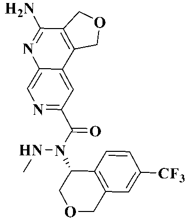 | 460 | **328** | 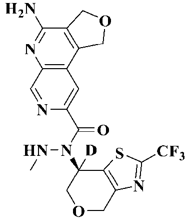 | 468 |
| **299** | 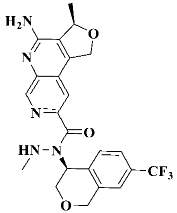 | 474 | **329** | 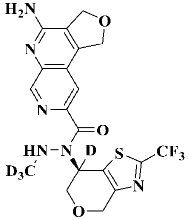 | 471 |
| **300** | 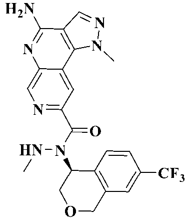 | 472 | **330** | 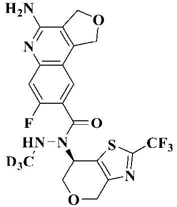 | 487 |
| **301** | 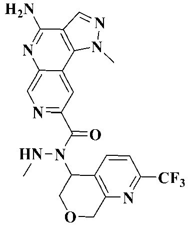 | 473 | **331** | 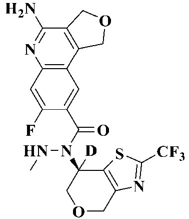 | 485 |
| **301-P1** | 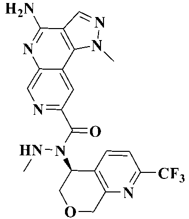 | 473 | **332** | 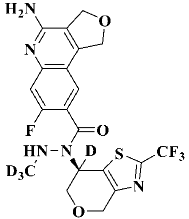 | 488 |
| **301-P2** | 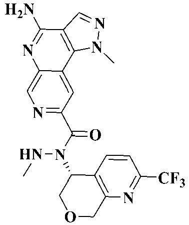 | 473 | **333** | 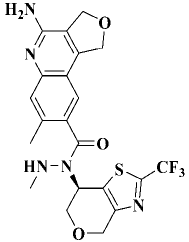 | 480 |
| **302** | 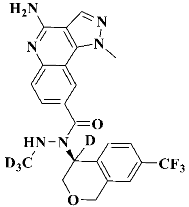 | 475 | **334** | 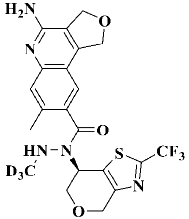 | 483 |
| **303** | 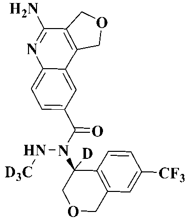 | 463 | **335** | 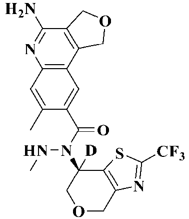 | 481 |
| **304** | 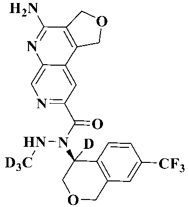 | 464 | **336** | 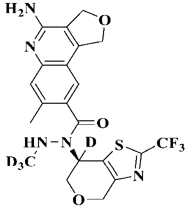 | 484 |
| **305** | 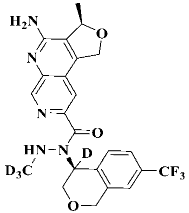 | 478 | **337** | 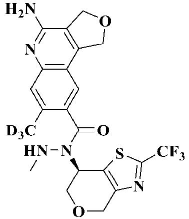 | 483 |
| **306** | 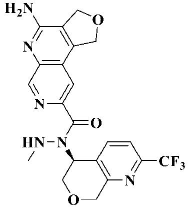 | 461 | **338** | 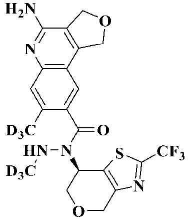 | 486 |
| **306** | 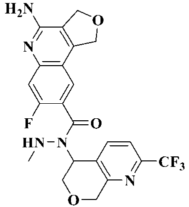 | 478 | **339** | 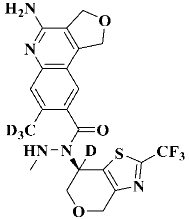 | 484 |
| **306-P1** | 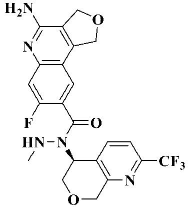 | 478 | **340** | 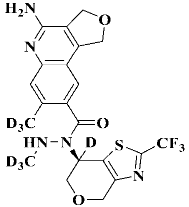 | 487 |
| **306-P2** | 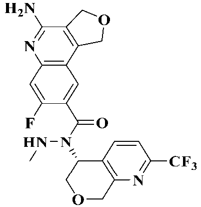 | 478 | **341** | 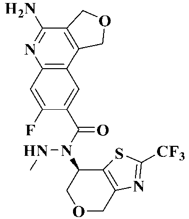 | 484 |
| **307** | 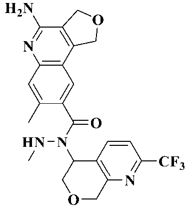 | 474 | **342** | 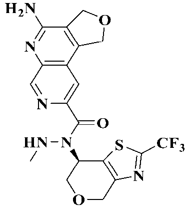 | 467 |
| **307-P1** | 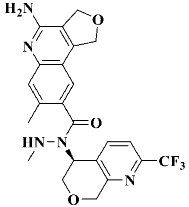 | 474 | **343** | 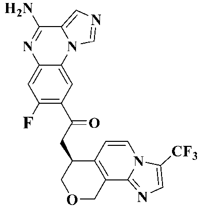 | 485 |
| **307-P2** | 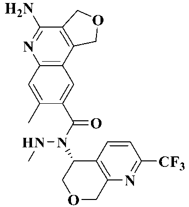 | 474 | **344** | 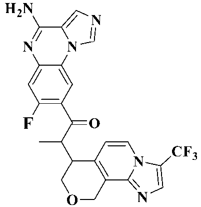 | 499 |
| **308** | 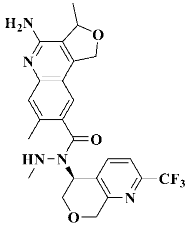 | 488 | **345** | 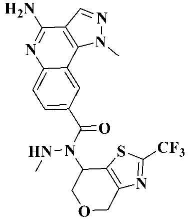 | 478 |
| **309** | 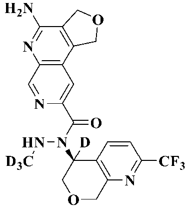 | 465 | **346** | 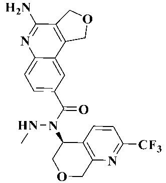 | 460 |
| **310** | 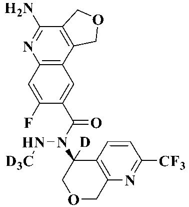 | 482 | **347** | 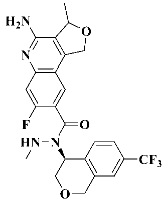 | 491 |
| **311** | 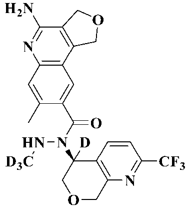 | 478 | **348** | 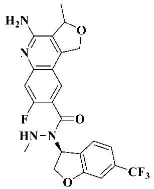 | 477 |
| **312** | 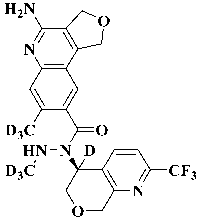 | 481 | **349** | 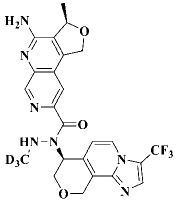 | 517 |
| **313** | 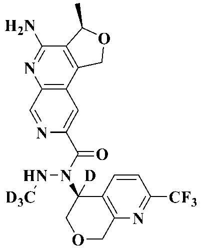 | 479 | **350** | 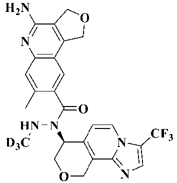 | 516 |
| **314** | 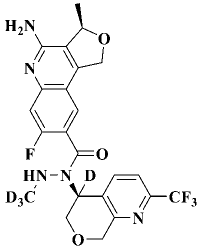 | 496 | **351** | 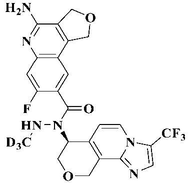 | 520 |
| **315** | 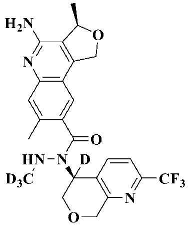 | 492 | **352** | 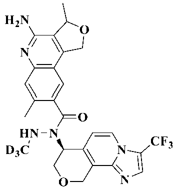 | 530 |
| **316** | 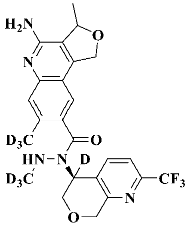 | 495 | | | |

The examples in the table below were prepared with reference to **Example 281:**

| **Example** | **Structure** | **MS m/z (ESI)** | **Example** | **Structure** | **MS m/z (ESI)** |
|---|---|---|---|---|---|
| | | **[M+1]⁺** | | | **[M+1]⁺** |
| **353** | 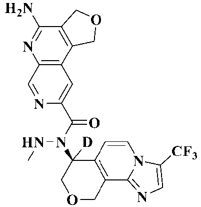 | 501 | **364** | 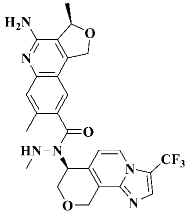 | 527 |
| **354-P1** | 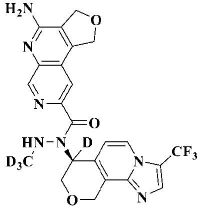 | 504 | **365** | 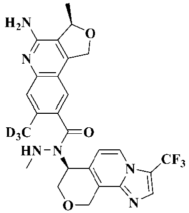 | 530 |
| **355** | 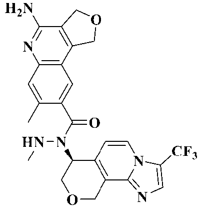 | 513 | **366** | 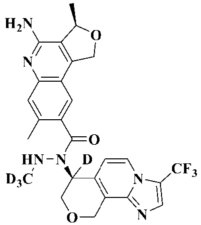 | 531 |
| **356** | 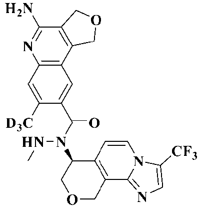 | 516 | **367** | 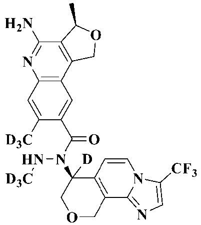 | 534 |
| **357** | 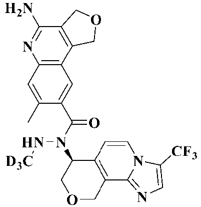 | 516 | **368** | 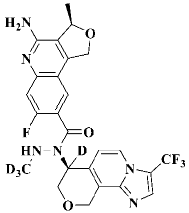 | 535 |
| **358** | 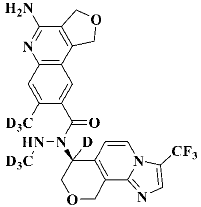 | 520 | **369** | 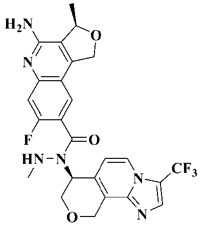 | 531 |
| **359** | 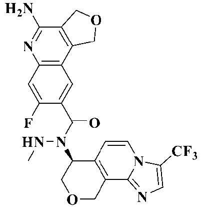 | 517 | **370** | 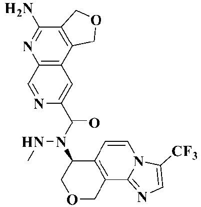 | 500 |
| **360** | 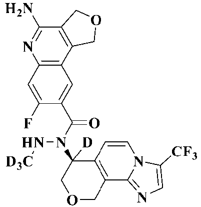 | 521 | **371** | 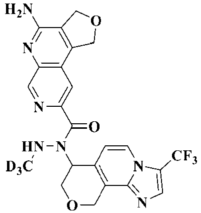 | 503 |
| **361** | 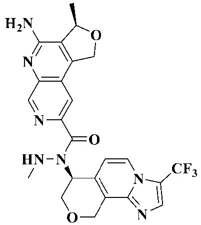 | 514 | **371-P1** | 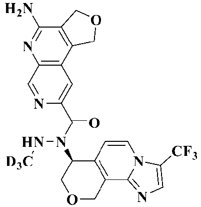 | 503 |
| **362-P1** | 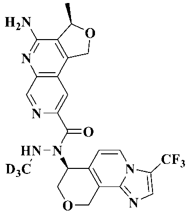 | 517 | **371-P2** | 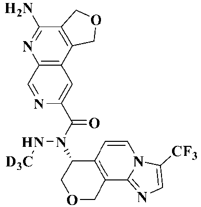 | 503 |
| **363** | 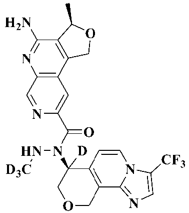 | 518 | | | |

The examples in the table below were prepared with reference to **Example 276:**

| **Example** | **Structure** | **MS m/z (ESI)** | **Example** | **Structure** | **MS m/z (ESI)** |
|---|---|---|---|---|---|
| | | **[M+1]⁺** | | | **[M+1]⁺** |
| **372** | 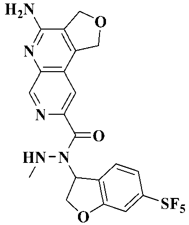 | 504 | **380** | 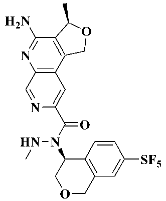 | 532 |
| **372-P1** | 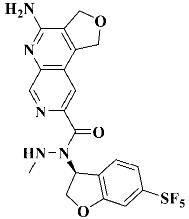 | 504 | **381** | 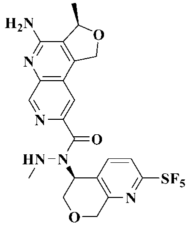 | 533 |
| **372-P2** | 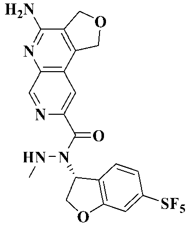 | 504 | **382** | 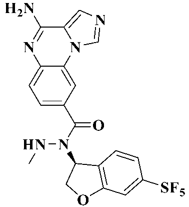 | 501 |
| **373** | 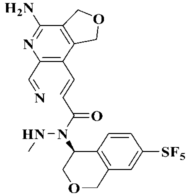 | 518 | **383** | 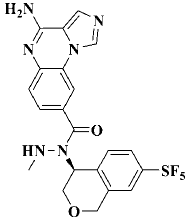 | 515 |
| **374** | 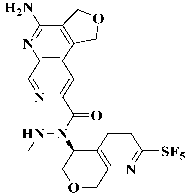 | 519 | **384** | 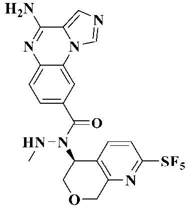 | 516 |
| **375-P1** | 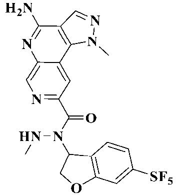 | 516 | **385-P1** | 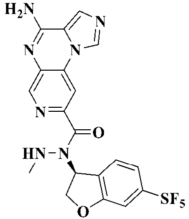 | 502 |
| **375-P1** | 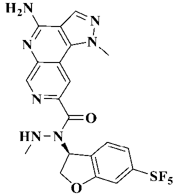 | 516 | **386** | 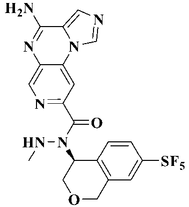 | 516 |
| **375-P2** | 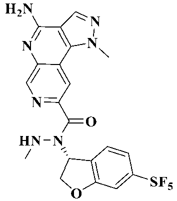 | 516 | **387-P1** | 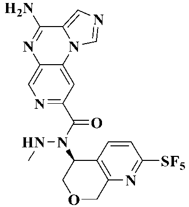 | 517 |
| **376** | 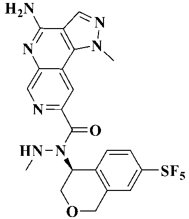 | 530 | **388** | 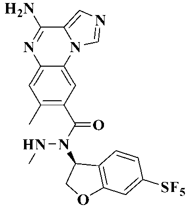 | 515 |
| **377** | 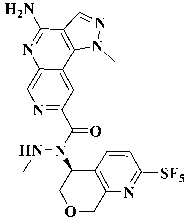 | 531 | **389** | 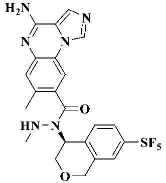 | 529 |
| **378** | 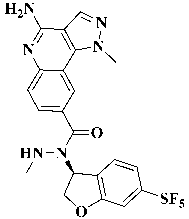 | 515 | **390** | 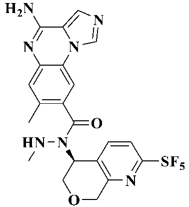 | 530 |
| **379** | 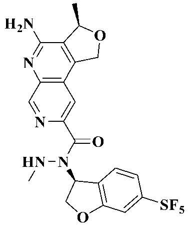 | 518 | **391-P1** | 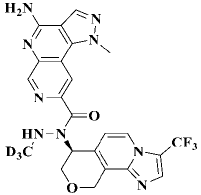 | 515 |

Alternatively, the following examples were prepared using the following method:

### Example 298

### 4-Amino-N'-methyl-N-(7-(trifluoromethyl)isochroman-4-yl)-1,3-dihydrofuro[3,4-c][1,7]diazanaphthalene-8-carbohydrazide

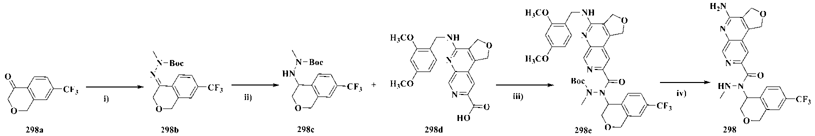

Referring to the synthesis method described in Step 1 to Step 4 of **Example 277,** Example **298** (12.2 mg, yield: 5.7%) was obtained from 7-(trifluoromethyl)isochroman-4-one **298a** (100 mg, 0.46 mmol). MS m/z (ESI): 460 [M+1]⁺

¹HNMR (400 MHz, DMSO-d₆) δ 8.85 (s, 1H), 7.94 (d, 1H), 7.62 (dd, 1H), 7.55 - 7.46 (m, 2H), 7.13 (s, 1H), 5.58 (dt, 1H), 5.39 (d, 2H), 5.24 (d, 1H), 5.04 (t, 2H), 4.85 - 4.73 (m, 2H), 4.21 (ddd, 1H), 4.10 - 3.97 (m, 1H), 2.29 (dd, 4.7 Hz, 3H).

### Example 301

### 4-Amino-N',1-dimethyl-N-[2-(trifluoromethyl)-6,8-dihydro-5H-pyrano[3,4-b]pyridin-5-yl]pyrazolo[4,3-c]quinoline-8-hydrazide

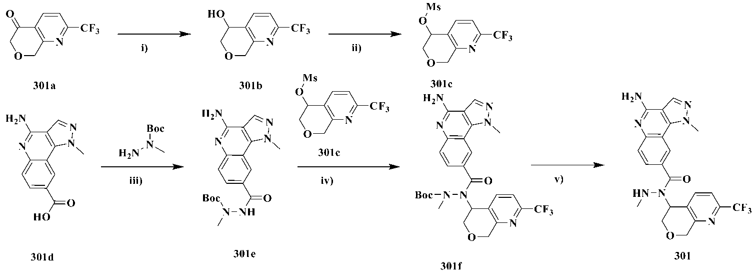

Referring to the synthesis method described in Step 1 to Step 3 of **Example 276, Example 301** (42 mg, yield: 4.3%) was obtained from 4-amino-1-methylpyrazolo[4,3-c]quinoline-8-carboxylic acid **301d** (500 mg, 2.05 mmol). MS m/z (ESI): 472 [M+1]⁺

¹H NMR (400 MHz, DMSO-d₆) δ 8.48 (s, 1H), 8.25 (s, 1H), 8.12 (d, 1H), 7.87 - 7.67 (m, 2H), 7.59 (d , 1H), 7.18 (s, 2H), 6.65 (broad s, 1H), 5.32 (s, 3H), 4.80 (t, 2H), 4.40 (s, 2H), 4.30 (m, 1H), 4.19 (s, 2H).

**Example 301** was resolved by Chiral-HPLC to obtain **301-P1** and **301-P2.**

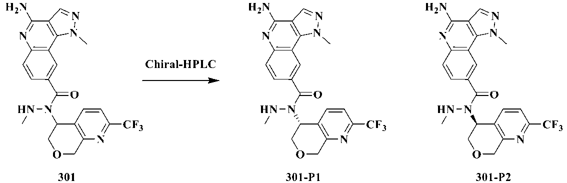

### Example 306

### 4-Amino-7-fluoro-N'-methyl-N-(2-trifluoromethyl-5,8-dihydro-6H-pyrano[3,4-b]pyridin-5-yl)-1,3-dihydrofuro[3,4-c]quinoline-8-carbohydrazide

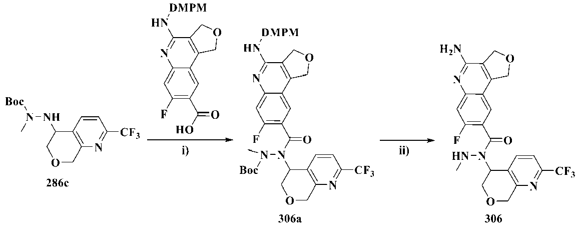

Referring to the synthesis method described in Step 3 to Step 4 in **Example 286, Example 306** (6 mg, yield: 2.1%) was obtained from tert-butyl 1-methyl-2-(2-trifluoromethyl-6*H-*pyrano[3,4-b]pyridin-5-yl)hydrazino-1-carboxylate **286c** (208 mg, 0.60 mmol). MS m/z (ESI): 478 [M+1]⁺

¹H NMR (400 MHz, DMSO-*d*₆) δ 8.06 (d, 1H), 7.87 (d, 1H), 7.53 (d, 1H), 7.23 (d, 1H), 6.89 (s, 1H), 6.75 (s, 2H), 5.42 (s, 1H), 5.33 (d, 3H), 4.98 (d, 2H), 4.77 (m, 2H), 4.25 (m, 2H), 2.31 (d, 2H).

### Example 307

### 4-Amino-7-methyl-N'-methyl-N-(2-trifluoromethyl-5,8-dihydro-6H-pyrano[3,4-b]pyridin-5-yl)-1,3-dihydrofuro[3,4-c]quinoline-8-carbohydrazide

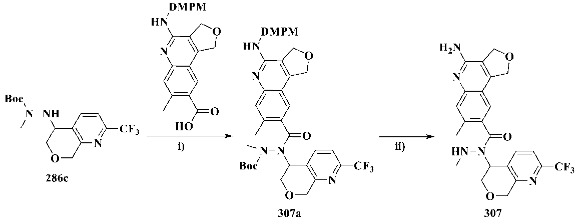

Referring to the synthesis method described in Step 3 to Step 4 of **Example 286, Example 307** (4 mg, yield: 8.4%) was obtained from tert-butyl 1-methyl-2-(2-trifluoromethyl-6*H*-pyrano[3,4-b]pyridin-5-yl)hydrazino-1-carboxylate **286c** (35 mg, 0.10 mmol). MS m/z (ESI): 474 [M+1]⁺

¹H NMR (400 MHz, DMSO-*d*₆) δ 8.15 (m, 1H), 7.87 (d, 1H), 7.82 (d, 1H), 7.50 (s, 1H), 7.45 (s, 1H), 5.38 (m, 4H), 5.03 (s, 2H), 4.76 (m, 2H), 4.27 (m, 2H), 2.37 (s, 3H), 2.22 (d, 2H).

### Example 324

### 4-Amino-N-[5-deuterio-2-(trifluoromethyl)-6,8-dihydropyrano[3,4-b]pyridin-5-yl]-1-methyl-N'-(trideuteriomethyl)pyrazolo[4,3-c]quinoline-8-hydrazide

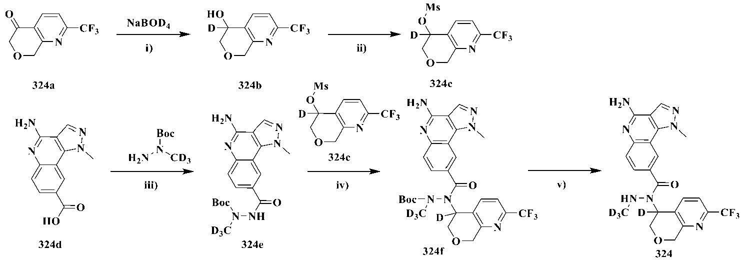

Referring to the synthesis method described in Step 1 to Step 3 of **Example 276, Example 324** (20 mg, yield: 5.0%) was obtained from 4-amino-1-methylpyrazolo[4,3-c]quinoline-8-carboxylic acid **324d** (200 mg, 826 µmol). MS m/z (ESI): 476 [M+1]⁺

¹H NMR (400 MHz, DMSO-d₆) δ 8.63 (s, 1H), 8.34 (s, 1H), 8.03 (d, 2H), 7.74 (d, 2H), 4.80 (s, 3H), 4.53 - 4.46 (m, 2H), 4.44 - 4.21 (m, 2H).

### Example 342

### 4-Amino-N'-methyl-N-(2-(trifluoromethyl)-6,7-dihydro-4H-pyrano[3,4-d]thiazol-7-yl)-1,3-dihydrofuro[3,4-c][1,7]naphthyridine-8-carbohydrazide

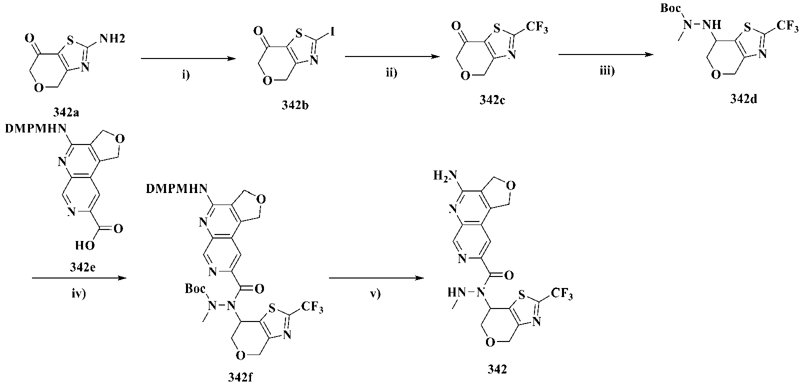

Step 1: 2-Amino-4*H*-pyrano[3,4-d]thiazol-7(6*H*)-one **342a** (4.00 g, 23.53 mmol), diiodomethane (26.80 g, 100.00 mmol) and tert-butyl nitrite (2.58 g, 25.00 mmol) were dispersed in tetrahydrofuran (40 mL), and the mixture was stirred and reacted at 65°C for 4 hours. The reaction solution was concentrated under reduced pressure to dryness, and the resulting residue was purified by silica gel column chromatography using eluent system A to obtain **342b** (3.41 g, yield: 51.4%). MS m/z (ESI): 282 [M+1]⁺

Step 2: **342b** (3.41 g, 12.14 mmol), methyl fluorosulphonyldifluoroacetate (4.61 g, 24.00 mmol) and cuprous iodide (4.56 g, 24.00 mmol) were dispersed in *N,N*-dimethylformamide (30 mL), and the mixture was stirred and reacted at 100°C for 4 hours. The reaction solution was filtered to remove insoluble materials, the filtrate was concentrated under reduced pressure to dryness, and the resulting residue was purified by silica gel column chromatography using eluent system A to obtain **342c** (1.81 g, yield: 66.9%). MS m/z (ESI): 224 [M+1]⁺

Step 3: **342c** (1.40 g, 6.28 mmol), tert-butyl 1-methylhydrazine-1-carboxylate (1.75 g, 12.00 mmol) and sodium cyanoborohydride (756 mg, 12.00 mmol) were dispersed in trifluoroethanol (20 mL), and the mixture was stirred and reacted for 16 hours. The reaction solution was filtered to remove insoluble materials, the filtrate was concentrated under reduced pressure to dryness, and the resulting residue was purified by silica gel column chromatography using eluent system A to obtain **342d** (1.21 g, yield: 54.6%). MS m/z (ESI): 354 [M+1]⁺

Step 4: **342d** (650 mg, 1.84 mmol) and 4-((3,4-dimethoxybenzyl)amino)-1,3-dihydrofuro[3,4-*c*][1,7]naphthyridine-8-carboxylic acid **342e** (762 mg, 2.00 mmol) were dispersed in dichloromethane (10 mL) and pyridine (2 mL). Phosphorus oxychloride (918 mg, 6.00 mmol) was added at 0°C, and the mixture was stirred and reacted at 0°C for 2 hours. The reaction solution was filtered to remove insoluble materials, the filtrate was concentrated under reduced pressure to dryness, and the resulting residue was purified by silica gel column chromatography using eluent system A to obtain **342f** (180 mg, yield: 13.7%). MS m/z (ESI): 717 [M+1]⁺

Referring to the synthesis method described in Step 8 of **Example 8, Example 342** (21 mg, yield: 53.7%) was obtained from **342f** (60 mg, 0.08 mmol). MS m/z (ESI): 467 [M+1]⁺

¹H NMR (400 MHz, DMSO-d₆) δ8.86 (s, 1H), 7.96 (s, 1H), 7.07 (s, 2H), 5.66 (s, 1H), 5.40 (s, 2H), 5.06 (t, 2H), 4.81 (dd, 2H), 4.40 - 4.03 (m, 2H), 2.33 (s, 3H).

### Example 351

### 4-Amino-7-fluoro-N'-(trideuteriomethyl)-N-(3-trifluoromethyl-7,10-dihydro-8H-imidazo [1,2-a] pyrano [3,4-c] pyridin-7-yl)-1,3-dihydrofuro [3,4-c] quinoline-8-carbohydrazide

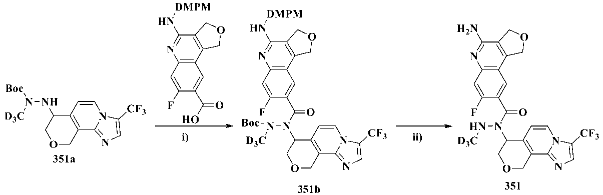

Step 1: tert-Butyl 1-(methyl-d₃)-2-(3-trifluoromethyl-7,10-dihydro-8H-imidazo[1,2-a]pyrano[3,4-c]pyridin-7-yl)hydrazine carboxylate **351a** (150 mg, 385.22 µmol) and 4-[(2,4-dimethoxyphenyl)methylamino]-7-fluoro-1,3-dihydrofuro[3,4-c]quinoline-8-carboxylic acid (184.16 mg, 462.27 µmol) were dissolved in dichloroethane (5 mL), and pyridine (304.71 mg, 3.85 mmol, 310.33 µL) was added. Phosphorus oxychloride (177.20 mg, 1.16 mmol) was slowly added dropwise in an ice bath, and the mixture was stirred at room temperature for 1 hour. Upon completion of the reaction, the reaction solution was quenched with water, extracted with dichloromethane (10 mL × 3), washed with a saturated sodium chloride aqueous solution, dried over anhydrous sodium sulphate, filtered, and concentrated. The residue was purified by silica gel column chromatography using eluent system A to obtain **351b** (94.5 mg, yield: 31.8%). MS m/z (ESI): 770 [M+1]⁺

Step 2: **351b** (94.5 mg, 122.77 µmol) was dissolved in trifluoroacetic acid (5 mL), and the mixture was stirred at 80°C for 1 hour. Upon completion of the reaction as monitored by LCMS, the reaction solution was subjected to rotary evaporation to dryness to obtain a crude product, which was purified by preparative liquid chromatography to obtain **Example 351** (30.2 mg, yield: 47.3%). MS m/z (ESI): 520 [M+1]⁺

¹H NMR (400 MHz, DMSO-d₆) δ 8.42 (s, 1H), 8.10 (s, 1H), 7.64 (s, 1H), 7.21 (d, 2H), 6.53 (s, 2H), 5.35 (s, 2H), 5.03 (s, 5H), 4.23 (s, 2H).

### Example 354

### 4-Amino-N'-(methyl-d3)-N-(3-(trifluoromethyl)-7,10-dihydro-8H-imidazo[1,2-a]pyrano[3,4-c]pyridin-7-yl-7-d)-1,3-dihydrofuro[3,4-c][1,7]naphthyridine-8-carbohydrazide

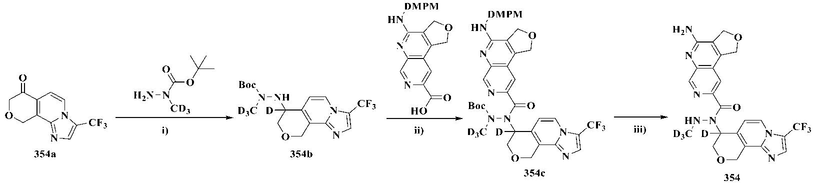

Step 1: 5-(Trifluoromethyl)-12-oxa-3,6-diazatricyclo[7.4.0.02,6]trideca-1(9),2,4,7-tetraen-10-one **354a** (100 mg, 390.35 µmol) and tert-butyl N-amino-N-(trideuteriomethyl)carbamate (87.36 mg, 585.52 µmol) were dissolved in methanol (1 mL), acetic acid (23.44 mg, 390.35 µmol) was added, and the mixture was stirred at room temperature overnight. NaBD₃CN (29.44 mg, 468.42 µmol) was then added, and the mixture was stirred at 60°C for 48 h, during which additional NaBD₃CN could be added as appropriate. Upon completion of the reaction, the reaction solution was subjected to rotary evaporation to dryness, extracted with EA, and washed with a saturated sodium chloride aqueous solution, and the organic phases were combined, dried and purified by column chromatography to obtain **354b** (110 mg, yield: 72.4%). MS m/z (ESI): 391 [M+1]⁺

Step 2: **354b** (127 mg, 325.31 µmol) and 4-[(2,4-dimethoxyphenyl)methylamino]-1,3-dihydrofuro[3,4-c][1,7]naphthyridine-8-carboxylic acid (223.32 mg, 585.56 µmol) were dissolved in DCE (1 mL), pyridine (257.32 mg, 3.25 mmol, 262.07 µL) and POCl3 (149.64 mg, 975.94 µmol, 89.34 µL) were added, and the mixture was stirred at room temperature for 1 h. Upon completion of the reaction, the reaction solution was quenched with water, extracted with DCM, dried and purified by column chromatography to obtain **354c** (186 mg, yield: 75.9%). MS m/z (ESI): 754 [M+1]⁺

Step 3: **354c** (180 mg, 238.80 µmol) was dissolved in TFA (5 mL), and the mixture was stirred at 80°C for 1 h. Upon completion of the reaction as monitored by LCMS, the reaction solution was subjected to rotary evaporation to dryness to obtain a crude product, which was purified by preparative liquid chromatography to obtain Example **354** (41.4 mg, yield: 34.4%). MS m/z (ESI): 504 [M+1]⁺

¹H NMR (400 MHz, DMSO-d₆) δ 8.98 (d, 1H),8.48 (dd, 1H), 8.11 (dd, 4H), 7.18 (dd, 1H), 5.45 (d, 2H), 5.10 (s, 2H), 4.93 (s, 2H), 4.23 (d, 2H).

### Example 354 Preparation of chiral isomers

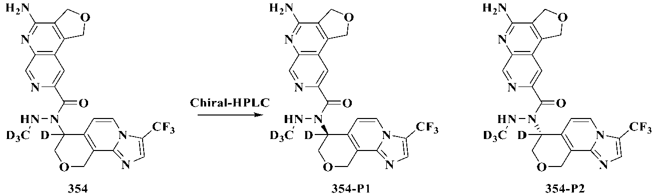

Example **354** (40 mg, 0.079 mmol) was resolved by Chiral-HPLC (condition 3) to obtain **354-P1:** (*R*)-4-amino-N'-(methyl-d3)-N-(3-(trifluoromethyl)-7,10-dihydro-8H-imidazo[1,2-a]pyrano[3,4-c]pyridin-7-yl-7-d)-1,3-dihydrofuro[3,4-c][1,7]naphthyridine-8-carbohydrazide (18.5 mg, yield: 46.3%) and **354-P2:** (*S*)-4-amino-N'-(methyl-d3)-N-(3-(trifluoromethyl)-7,10-dihydro-8H-imidazo[1,2-a]pyrano[3,4-c]pyridin-7-yl-7-d)-1,3-dihydrofuro[3,4-c][1,7]naphthyridine-8-carbohydrazide (18.8 mg, yield: 47.0%), respectively.

**354-P1 (t_{R}: 4.968 min):** ¹H NMR (400 MHz, DMSO-d₆) δ 8.98 (d, 1H),8.48 (dd, 1H), 8.11 (dd, 4H), 7.18 (dd, 1H), 5.45 (d, 2H), 5.10 (s, 2H), 4.93 (s, 2H), 4.23 (d, 2H).

**354-P2 (t_{R}: 4.198 min):** ¹H NMR (400 MHz, DMSO-d₆) δ 8.98 (d, 1H),8.48 (dd, 1H), 8.11 (dd, 4H), 7.18 (dd, 1H), 5.45 (d, 2H), 5.10 (s, 2H), 4.93 (s, 2H), 4.23 (d, 2H).

### Example 357

### 4-Amino-7-methyl-N'-methyl-d₃-N-(3-trifluoromethyl-7,10-dihydro-8H-imidazo[1,2-a]pyrano[3,4-c]pyridin-7-yl)-1,3-dihydrofuro[3,4-c]quinoline-8-carbohydrazide

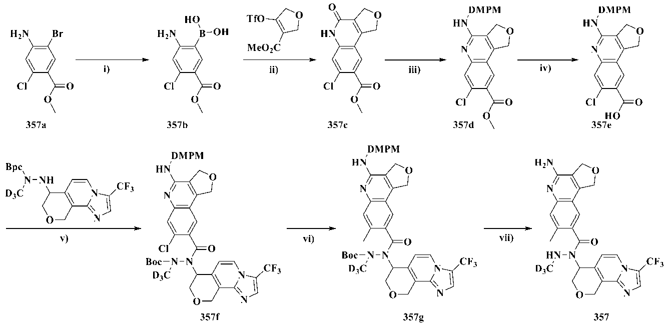

Step 1: Methyl 4-amino-5-bromo-2-methylbenzoate **357a** (11 g, 45.07 mmol), palladium acetate (505.89 mg, 2.25 mmol), n-butyldiphenylphosphine (1.09 g, 4.51 mmol), potassium acetate (8.85 g, 90.13 mmol), B₂pin₂ (22.89 g, 90.13 mmol) and 1'4-dioxane (100 mL) were sequentially added to a single-necked flask, and the mixture was purged with N₂ and reacted at 100°C for 4 h. The reaction system was cooled and quenched. The system was concentrated to dryness, and the residue was purified by normal phase column chromatography using eluent system A to obtain **357b** (6.37 g, yield: 61.6%). MS m/z (ESI): 230 [M+1]⁺

Step 2: **357b** (2.29 g, 10.00 mmol), methyl 4-trifluoromethanesulphonyloxy-2,5-dihydrofuran-3-carboxylate (2.76 g, 10.00 mmol), Pd(dppf)Cl₂ (731.68 mg, 999.97 µmol), Na₂CO₃ (2.12 g, 20.00 mmol), 1'4-dioxane (20 mL) and water (4 mL) were sequentially added to a single-necked flask, and the mixture was purged with N₂ and reacted at 100°C for 16 h. The reaction system was cooled, quenched, concentrated, extracted with EA, washed with a saturated NaCl solution, dried over anhydrous Na₂SO₄, and filtered, the filtrate was concentrated to dryness, and the resulting residue was purified by normal phase column chromatography using eluent system D to obtain **357c** (2.02 g, 7.25 mmol, yield: 72.3%). MS m/z (ESI): 280 [M+1]⁺

Referring sequentially to the synthesis method described in Step 6 to Step 9 of **Example 12, 357f** (105 mg, yield: 2.8%) was obtained from **357c** (2.02 g, 7.25 mmol). MS m/z (ESI): 516 [M+1]⁺

Step 6: **357f** (590 mg, 750.44 µmol), methylboronic acid (135 mg, 2.25 mmol), cataCXiumA-Pd-G3 (55 mg, 75 µmol), Cs₂CO₃ (489 mg, 1.50 mmol), water (3 mL) and 1'4-dioxane (15 mL) were sequentially added to a single-necked flask, and the mixture was purged with N₂ and reacted at 100°C for 12 h. The reaction system was cooled and quenched. The system was concentrated to dryness to obtain **357g** (350 mg, 457.04 µmol, yield: 60.9%). MS m/z (ESI): 766 [M+1]⁺

Referring to the synthesis method described in Step 10 of **Example 12, Example 357** (105 mg, yield: 44.5%) was obtained from **357g** (350 mg, 457 µmol). MS m/z (ESI): 516 [M+1]⁺

¹H NMR (400 MHz, DMSO-d₆) δ 8.48 (m, 1H), 8.18 (m, 2H), 7.38 (m, 2H), 6.64 (s, 1H), 6.50 (s, 1H), 5.51 (s, 1H), 5.32 (s, 3H), 4.96 (m, 5H), 4.26 (m, 1H), 2.38 (s, 2H).

### Example 357 Preparation of chiral isomers

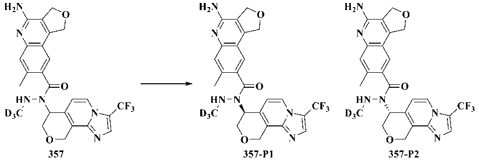

**Example 357** (105 mg, 0.027 mmol) was separated by chiral preparative HPLC (condition 1) to obtain rel-(S)-4-amino-7-methyl-N'-methyl-d₃-N-(3-trifluoromethyl-7,10-dihydro-8H-imidazo[1,2-a]pyrano[3,4-c]pyridin-7-yl)-1,3-dihydrofuro[3,4-c]quinoline-8-carbohydrazide **357-P1** (45 mg, yield: 42.9%) and rel-(R)-4-amino-7-methyl-N'-methyl-d₃-N-(3-trifluoromethyl-7,10-dihydro-8H-imidazo[1,2-a]pyrano[3,4-c]pyridin-7-yl)-1,3-dihydrofuro[3,4-c]quinoline-8-carbohydrazide **357-P2** (46 mg, yield: 43.8%).

**357-P1 (t_{R}: 17.930 min):** ¹H NMR (400 MHz, DMSO-d₆) δ 8.48 (m, 1H), 8.18 (m, 2H), 7.38 (m, 2H), 6.64 (s, 1H), 6.50 (s, 1H), 5.51 (s, 1H), 5.32 (s, 3H), 4.96 (m, 5H), 4.26 (m, 1H), 2.38 (s, 2H).

**357-P1 (t_{R}: 21.680 min):** ¹H NMR (400 MHz, DMSO-d₆) δ 8.48 (m, 1H), 8.18 (m, 2H), 7.38 (m, 2H), 6.64 (s, 1H), 6.50 (s, 1H), 5.51 (s, 1H), 5.32 (s, 3H), 4.96 (m, 5H), 4.26 (m, 1H), 2.38 (s, 2H).

### Example 362

### 4-Amino-3-methyl-N'-(trideuteriomethyl)-N-(3-trifluoromethyl-7,10-dihydro-8H-imidazo[1,2-a]pyrano[3,4-c]pyridin-7-yl)-1,3-dihydrofuro[3,4-c] naphthyridine-8-carbohydrazide

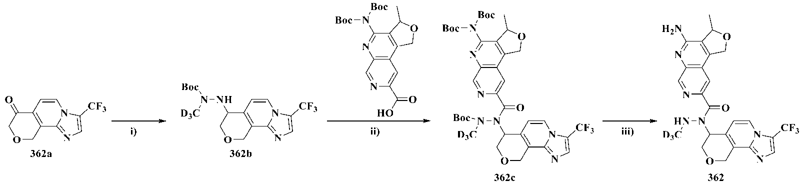

Step 1: 3-Trifluoromethyl-8H-imidazo[1,2-a]pyrano[3,4-c]pyridin-7(10H)-one **362a** (200 mg, 780.70 µmol) and tert-butyl N-amino-N-(trideuteriomethyl)carbamate (128.13 mg, 858.77 µmol) were dissolved in methanol (2 mL). Acetic acid (46.88 mg, 780.70 µmol) and sodium cyanoborohydride (147.18 mg, 2.34 mmol) were added at room temperature under stirring, and the resulting mixture was stirred and reacted at 60°C for 3 h. The solvent was removed under reduced pressure, and the residue was purified and separated by column chromatography using eluents: petroleum ether/ethyl acetate (1:1) to obtain **362b** (180 mg, 462.27 µmol, yield: 59.2%). MS m/z (ESI):390 [M+1]⁺

Step 2: **362b** (118.17 mg, 303.48 µmol) and 4-(tert-butyloxycarbonylamino)-3-methyl-1,3-dihydrofuro[3,4-C][1,7]naphthyridine-8-carboxylic acid (87.34 mg, 252.90 µmol) were dissolved in 1,2-dichloroethane (2.2 mL), pyridine (200.05 mg, 2.53 mmol, 203.73 µL) and phosphorus oxychloride (116.33 mg, 758.71 µmol) were added at 0°C under stirring, and the resulting mixture was stirred and reacted at room temperature for 1 h. LCMS showed completion of the reaction. Upon completion of the reaction, water and a saturated ammonium chloride solution were added to the system. The mixture was extracted three times with DCM (10 mL × 3), washed with saturated sodium chloride, and left to stand for phase separation. The organic phase was collected, dried over anhydrous sodium sulphate, filtered and concentrated under reduced pressure. The crude product was purified by silica gel column chromatography using petroleum ether/ethyl acetate (V:V=1:1) to obtain **362c** (120 mg, 167.43 µmol, yield: 66.2%). MS m/z (ESI): 817 [M+1]⁺

Step 3: **362c** (120 mg, 146.91 µmol) was dissolved in dichloromethane (1.5 mL), trifluoroacetic acid (16.75 mg, 146.91 µmol, 0.3 mL) was added at room temperature under stirring, and the resulting mixture was stirred and reacted at room temperature for 1 h. The solvent was removed under reduced pressure, and the resulting residue was separated by HPLC to obtain **Example 362** (52.2 mg, 101.07 µmol, yield: 68.8%). MS m/z (ESI): 517 [M+1]⁺

**357-P1 (t_{R}: 32.973 min):** ¹H NMR (400 MHz, DMSO-d6) δ 8.88 (s, 1H), 8.40 (d, 1H), 8.09 (s, 1H), 7.95 (s, 1H), 7.21-7.14 (m, 1H), 6.78 (s, 2H), 5.63-5.29 (m, 4H), 4.99 (s, 2H), 4.29-4.15 (m, 2H), 1.47 (d, 3H).

**357-P2 (t_{R}: 18.127 min):** ¹H NMR (400 MHz, DMSO-d6) δ 8.88 (s, 1H), 8.40 (d, 1H), 8.09 (s, 1H), 7.95 (s, 1H), 7.21-7.14 (m, 1H), 6.78 (s, 2H), 5.63-5.29 (m, 4H), 4.99 (s, 2H), 4.29-4.15 (m, 2H), 1.47 (d, 3H).

**357-P3 (t_{R}: 22.473 min):** ¹H NMR (400 MHz, DMSO-d6) δ 8.88 (s, 1H), 8.40 (d, 1H), 8.09 (s, 1H), 7.95 (s, 1H), 7.21-7.14 (m, 1H), 6.78 (s, 2H), 5.63-5.29 (m, 4H), 4.99 (s, 2H), 4.29-4.15 (m, 2H), 1.47 (d, 3H).

**357-P4 (t_{R}: 47.857 min):** ¹H NMR (400 MHz, DMSO-d6) δ 8.88 (s, 1H), 8.40 (d, 1H), 8.09 (s, 1H), 7.95 (s, 1H), 7.21-7.14 (m, 1H), 6.78 (s, 2H), 5.63-5.29 (m, 4H), 4.99 (s, 2H), 4.29-4.15 (m, 2H), 1.47 (d, 3H).

### Example 362 Preparation of chiral isomers

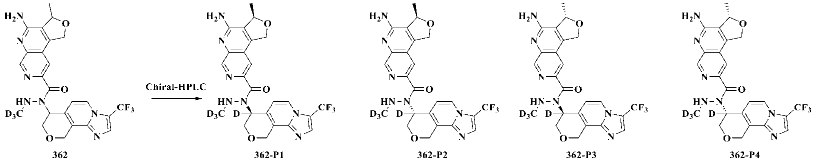

**Example 362** (30 mg, 0.079 mmol) was resolved by Chiral-HPLC to obtain **362-P1:** (*R*)-4-amino-3-methyl-N'-(trideuteriomethyl)-N-((*S*)-3-trifluoromethyl-7,10-dihydro-8H-imidazo[1,2-a]pyrano[3,4-c]pyridin-7-yl)-1,3-dihydrofuro[3,4-c]naphthyridine-8-carbohydrazide (7 mg, yield: 23.3%), **362-P2:** (*R*)-4-amino-3-methyl-N'-(trideuteriomethyl)-N-((*R*)-3-trifluoromethyl-7,10-dihydro-8H-imidazo[1,2-a]pyrano[3,4-c]pyridin-7-yl)-1,3-dihydrofuro[3,4-c]naphthyridine-8-carbohydrazide (7 mg, yield: 23.3%), **362-P3:** (*S*)-4-amino-3-methyl-N'-(trideuteriomethyl)-N-((*S*)-3-trifluoromethyl-7,10-dihydro-8H-imidazo[1,2-a]pyrano[3,4-c]pyridin-7-yl)-1,3-dihydrofuro[3,4-c]naphthyridine-8-carbohydrazide (6.7 mg, yield: 22.3%) and **362-P4:** (*S*)-4-amino-3-methyl-N'-(trideuteriomethyl)-N-((*R*)-3-trifluoromethyl-7,10-dihydro-8H-imidazo[1,2-a]pyrano[3,4-c]pyridin-7-yl)-1,3-dihydrofuro[3,4-c]naphthyridine-8-carbohydrazide (6.3 mg, yield: 21.0%), respectively.

¹H NMR (400 MHz, DMSO-d₆) δ 8.86 (d, 1H), 8.47 (dd, 1H), 8.16 (d, *J =* 11.4 Hz, 1H), 7.95 (d, 1H), 7.24-6.99 (m, 3H), 5.53-5.26 (m, 4H), 5.06-4.90 (m, 2H), 4.28-4.07 (m, 2H), 1.42 (d, 3H).

### Example 371

### 4-Amino-N'-(methyl-d3)-N-(3-(trifluoromethyl)-7,10-dihydro-8H-imidazo[1,2-a]pyrano[3,4-c]pyridin-7-yl)-1,3-dihydrofuro[3,4-c][1,7]naphthyridine-8-carbohydrazide

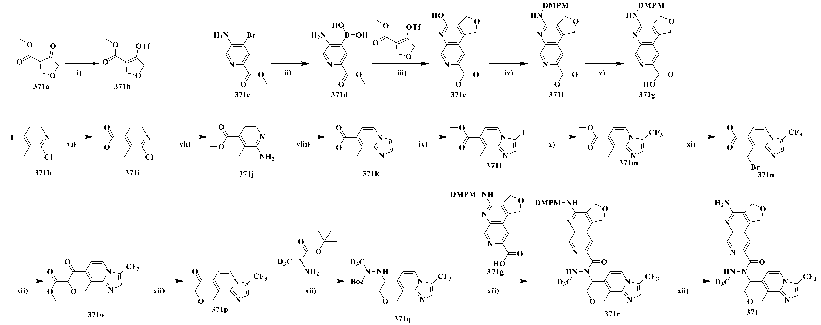

Step 1: Methyl 4-oxotetrahydrofuran-3-carboxylate **371a** (27.98 g, 194.17 mmol) was dissolved in dichloromethane (280 mL), and*N,N*-diisopropylethylamine (27.60 g, 213.59 mmol) and trifluoromethanesulphonic anhydride (54.78 g, 194.17 mmol) were sequentially added in an ice bath. The mixture was stirred and reacted at room temperature for about 16 hours. Water (300 mL) was added to the reaction solution, and the mixture was extracted with ethyl acetate (300 mL × 2). After extraction, the organic phase was separated, dried over anhydrous sodium sulphate, and filtered. The filtrate was concentrated under reduced pressure, and the resulting residue was purified by silica gel column chromatography using eluent system B to obtain **371b** (36 g, yield: 67.0%). MS m/z (ESI): 277 [M+1]⁺

Step 2: Methyl 5-amino-4-bromopyridine carboxylate **371c** (36 g, 155.81 mmol), 4,4,4',4',5,5,5',5'-octamethyl-2,2'-bis(1,3,2-dioxaborolane) (59.35 g, 233.72 mmol), [1,1'-bis(diphenylphosphino)ferrocene]palladium(II) dichloride (22.80 g, 31.16 mmol) and potassium acetate (45.87 g, 467.44 mmol) were added to a reaction flask, followed by toluene (360 mL). The mixture was stirred and reacted at 90°C under nitrogen atmosphere for 16 h. The reaction solution was cooled and filtered by suction to obtain a solid, which was washed with water to obtain **371d** (38.2 g, yield: 88.2%). MS m/z (ESI): 197 [M+1]⁺

Step 3: **371d** (54 g, 194.17 mmol) was dissolved in dioxane (500 mL), H₂O (50 mL) was added, followed by methyl 4-(((trifluoromethyl)sulphonyl)oxy)-2,5-dihydrofuran-3-carboxylate **371b** (53.63 g, 194.17 mmol), Pd(dppf)Cl₂ (7.10 g, 9.71 mmol) and sodium carbonate (61.74 g, 582.50 mmol), and the mixture was stirred and reacted at 90°C under nitrogen atmosphere for 1 hour. The reaction solution was cooled to room temperature and filtered to obtain a solid, which was first slurried with EA, then with water and dried to obtain **371e** (46.2 g, yield: 96.6%). MS m/z (ESI): 247 [M+1]⁺

Step 4: **371e** (70 g, 284.30 mmol) was dissolved in DMSO (700 mL), and 2,4-dimethoxybenzylamine (57.04 g, 341.16 mmol) and DBU (64.92 g, 426.45 mmol, 63.65 mL) were added. BOP (188.61 g, 426.45 mmol) was added portionwise in an ice bath, and the mixture was stirred and reacted at room temperature for 2 hours. The reaction solution was slowly added dropwise to water (1 L), resulting in the precipitation of a large amount of solid. The solid was filtered and slurried with methanol to obtain **371f** (77.6 g, yield: 69.0%). MS m/z (ESI): 396 [M+1]⁺

Step 5: **371f** (82.2 g, 207.89 mmol) was dissolved in a mixed solvent of MeOH (25 mL), THF (25 mL) and H₂O (25 mL), lithium hydroxide monohydrate (43.62 g, 1.04 mol) was added, and the mixture was stirred and reacted at room temperature for 3 h. The organic phase from the reaction solution was concentrated under reduced pressure. The residue was then adjusted to weak acidic pH with 2 N HCl, resulting in the precipitation of a large amount of solid. The solid was filtered by suction to obtain **371g** (73.6 g, yield: 92.8%). MS m/z (ESI): 382 [M+1]⁺

Step 6: 2-Chloro-4-iodo-3-methylpyridine **371h** (150 g, 591.79 mmol), palladium acetate (13.29 g, 59.18 mmol), 1,1'-bis(diphenylphosphino)ferrocene (32.75 g, 59.18 mmol) and sodium bicarbonate (149.14 g, 1.78 mol) were added to a reactor, followed by MeOH (500 mL) and DMF (500 mL), and the mixture was stirred and reacted at 80°C under a 0.4 MPa CO atmosphere for 16 hours. The reaction solution was added to water, the mixture was extracted with EA, and the organic phase was separated, dried and concentrated. The residue was separated by column chromatography to obtain **371i** (137 g, yield: 62.4%). MS m/z (ESI): 186 [M+1]⁺

Step 7: **371i** (73.04 g, 403.00 mmol) was dissolved in 1,4-dioxane (1700 mL), and Pd₂(dba)₃ (33.55 g, 36.64 mmol), Xantphos (42.40 g, 73.27 mmol), benzophenone imine (146 g, 806 mmol) and caesium carbonate (179.05 g, 549.55 mmol) were added. The mixture was heated to 100°C under nitrogen atmosphere, and stirred and reacted for 16 hours. The reaction solution was cooled and filtered by suction through Celite, the filtrate was concentrated, HCl (400 mL, 4 M in dioxane) and MeOH (200 mL) were added, and the mixture was stirred and reacted at room temperature for 16 h. The reaction solution was directly concentrated under reduced pressure, then adjusted to pH=8 with a NaHCO₃ aqueous solution, and extracted with ethyl acetate. The organic phase was separated, dried and concentrated. The residue was separated by column chromatography to obtain **371j** (60 g, yield: 49.3%). MS m/z (ESI): 167 [M+1]⁺

Step 8: **371j** (59 g, 284.03 mmol) was dissolved in EtOH (590 mL), 2-chloroacetaldehyde (83.61 g, 426.05 mmol) and NaHCO₃ (35.79 g, 426.05 mmol) were added, and the mixture was stirred and reacted overnight at 70°C. The reaction solution was cooled to room temperature and concentrated under reduced pressure, and the residue was separated by column chromatography to obtain **371k** (34 g, yield: 62.9%). MS m/z (ESI): 191 [M+1]⁺

Step 9: **371k** (34 g, 178.76 mmol) was dissolved in acetonitrile (500 mL), NIS (42.23 g, 187.70 mmol) was added, and the mixture was stirred and reacted at room temperature for 1 hour. The reaction solution was directly filtered to obtain the solid **371l** (56 g, yield: 99.1%). MS m/z (ESI): 317 [M+1]⁺

Step 10: **371l** (53 g, 167.67 mmol) and methyl 2,2-difluoro-2-(fluorosulphonyl)acetate (96.64 g, 503.01 mmol) were dissolved in DMF (600 mL), and CuI (95.80 g, 503.01 mmol) was added. The mixture was heated to 100°C and stirred and reacted for 16 h. The reaction solution was cooled, diluted with EtOAc (200 mL) and filtered, and the filtrate was extracted with water. The organic phase was washed with a saturated sodium chloride aqueous solution, separated, dried over anhydrous sodium sulphate, and concentrated under reduced pressure. The concentrated residue was separated by column chromatography to obtain **371m** (17 g, yield: 99.1%). MS m/z (ESI): 259 [M+1]⁺

Step 11: **371m** (17 g, 65.84 mmol) was dissolved in carbon tetrachloride (200 mL), and NBS (14.06 g, 79.01 mmol) and azobisisobutyronitrile (2.16 g, 13.17 mmol) were added. The mixture was stirred and reacted at 80°C under nitrogen atmosphere for 2 h. The reaction solution was cooled to room temperature and filtered, the filter cake was washed with a small amount of dichloromethane, and the filtrate was concentrated under reduced pressure and then separated by column chromatography to obtain **371n** (22 g, yield: 99.1%). MS m/z (ESI): 337 [M+1]⁺

Step 12: Methyl 2-hydroxyacetate (16.03 g, 177.99 mmol) was dissolved in DMF (100 mL) and the mixture was cooled to 0°C. NaH (7.12 g, 177.99 mmol, 60% purity) was added portionwise. The reaction solution was stirred at 0°C for 15 min. A solution of **371n** (20 g, 59.33 mmol) in DMF (80 mL) was then added. The mixture was heated to room temperature and stirred for 2 h. The reaction solution was poured into a saturated ammonium chloride solution, and the mixture was extracted with ethyl acetate. The organic phase was separated, washed once with a saturated sodium chloride aqueous solution, and then separated again. The resulting organic phase was dried over anhydrous sodium sulphate and concentrated under reduced pressure. The residue was separated by column chromatography to obtain **371o.** MS m/z (ESI): 315 [M+1]⁺

Step 13: **371o** (14 g, 44.56 mmol) was dissolved in EtOH (150 mL), HCl (12 M, 75 mL) was added, and the mixture was stirred and reacted at 80°C for 2 h. The reaction solution was concentrated under reduced pressure to remove ethanol. The resulting mixture was adjusted to pH 6 with saturated sodium bicarbonate and extracted with ethyl acetate. The organic phase was washed with a saturated sodium chloride aqueous solution, dried over anhydrous sodium sulphate, and concentrated under reduced pressure. The concentrated residue was separated by column chromatography to obtain **371p** (6.2 g, yield: 54.3%). MS m/z (ESI): 257 [M+1]⁺

Step 14: **371p** (100 mg, 390.35 µmol) and tert-butyl N-amino-N-(trideuteriomethyl)carbamate (87.36 mg, 585.52 µmol) were dissolved in methanol (1 mL), acetic acid (23.44 mg, 390.35 µmol) was added, and the mixture was stirred overnight at room temperature. NaBH₃CN (29.44 mg, 468.42 µmol) was then added, and the resulting mixture was stirred and reacted at 60°C for 48 h. Upon completion of the reaction, the reaction solution was concentrated and extracted with ethyl acetate. The organic phase was washed with a saturated sodium chloride aqueous solution, separated, dried and concentrated. The concentrated residue was separated by column chromatography to obtain **371q** (110 mg, yield: 72.4%). MS m/z (ESI): 390 [M+1]⁺

Step 15: **371q** (100 mg, 256.81 µmol) and **371g** (146.92 mg, 385.22 µmol) were dissolved in DCE (896.56 µL), and pyridine (203.14 mg, 2.57 mmol, 206.88 µL) was added. POCl₃ (118.13 mg, 770.44 µmol) was slowly added dropwise in an ice bath, and the mixture was stirred and reacted at room temperature for 1 h. Upon completion of the reaction, the reaction system was quenched with water and extracted with DCM. The organic phase was separated, dried and concentrated, and the concentrated residue was separated by column chromatography to obtain **371r** (190 mg, yield: 98.3%). MS m/z (ESI): 721 [M+1]⁺

Step 16: **371r** (190 mg, 252.41 µmol) was dissolved in TFA (5 mL), and the mixture was stirred and reacted at 80°C for 1 h. The reaction solution was concentrated under reduced pressure to obtain a crude product, which was purified by preparative liquid chromatography to obtain **Example 371** (30 mg, yield: 57.1%). MS m/z (ESI): 503 [M+1]⁺

¹H NMR (400 MHz, DMSO-d₆) δ 8.97 (d, 1H),8.48 (dd, 1H), 8.11 (dd, 3.72H), 7.17 (dd, 1H), 5.46 (t, 3H), 5.13-4.91 (m, 4H), 4.28-4.22 (m, 2H).

### Example 371 Preparation of chiral isomers

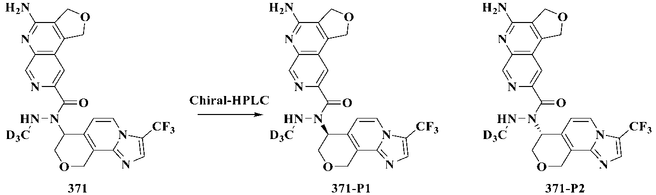

**Example 371** (19 mg, 0.079 mmol) was resolved by Chiral-HPLC (condition 3) to obtain **371-P1:** (*R*)-4-amino-N'-(methyl-d3)-N-(3-(trifluoromethyl)-7,10-dihydro-8H-imidazo[1,2-a]pyrano[3,4-c]pyridin-7-yl)-1,3-dihydrofuro[3,4-c][1,7]naphthyridine-8-carbohydrazide (6 mg, yield: 31.5%) and **371-P2:** (*S*)-4-amino-N'-(methyl-d3)-N-(3-(trifluoromethyl)-7,10-dihydro-8H-imidazo[1,2-a]pyrano[3,4-c]pyridin-7-yl)-1,3-dihydrofuro[3,4-c][1,7]naphthyridine-8-carbohydrazide (11.4 mg, yield: 57.9%), respectively.

**371-P1 (t_{R}: 4.564 min):** ¹H NMR (400 MHz, DMSO-d₆) δ 8.87 (d, 1H), 8.48 (dd, 1H), 8.15 (d, 1H), 7.97 (dd, 1H), 7.15 (t, 3H), 5.63-5.45 (m, 1H), 5.43 (s, 2H), 5.29 (s, 1H), 5.06 (s, 2H), 5.01 (q, 1H), 4.93 (s, 1H), 4.35-4.06 (m, 2H).

**371-P2 (t_{R}: 4.277 min):** ¹H NMR (400 MHz, DMSO-d₆) δ 8.87 (d, 1H), 8.48 (dd, 1H), 8.15 (d, 1H), 7.97 (dd, 1H), 7.15 (t, 3H), 5.63-5.45 (m, 1H), 5.43 (s, 2H), 5.29 (s, 1H), 5.06 (s, 2H), 5.01 (q, 1H), 4.93 (s, 1H), 4.35-4.06 (m, 2H).

### Example 372

### rel-(R)-4-amino-N'-methyl-N-(6-(pentafluoro-6-sulphanyl)-2,3-dihydrobenzofuran-3-yl)-1,3-dihydrofuro[3,4-c][1,7]naphthyridine-8-carbohydrazide

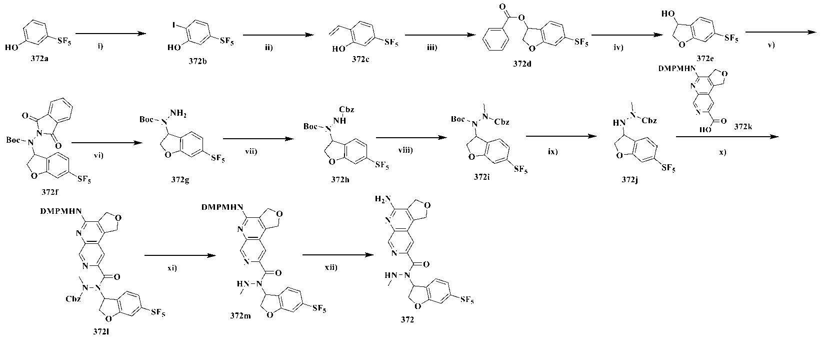

Step 1: 3-(Pentafluorosulphanyl)phenol **372a** (3 g, 13.63 mmol) was dissolved in toluene (30 mL), and the mixture was purged three times with nitrogen and cooled to 0°C. Sodium hydride (981 mg, 24.53 mmol, 60% purity) was added, and the reaction system was reacted at 0°C for 1 hour. Iodine (3.46 g, 13.63 mmol) was then added, and the mixture was reacted at 25°C for 15 hours. The reaction system was quenched with a saturated sodium sulphite aqueous solution, extracted with ethyl acetate (80 mL×3), washed with a saturated sodium chloride aqueous solution (30 mL), dried over anhydrous sodium sulphate, filtered, and concentrated. The residue was purified by silica gel column chromatography using eluent system B to obtain **372b** (4.60 g, yield: 97.6%).

Step 2: **372b** (4.6 g, 13.29 mmol), potassium vinyltrifluoroborate (3.56 g, 26.59 mmol) and potassium carbonate (5.51 g, 39.88 mmol) were dissolved in water (10 mL) and 1'4-dioxane (50 mL), and the mixture was purged three times with nitrogen. 1,1'-Bis(diphenylphosphino)ferrocene palladium(II) dichloride (981 mg, 1.33 mmol) was added, and the reaction system was reacted at 80°C for 3 hours. The reaction system was quenched with water, extracted with ethyl acetate (80 mL × 3), washed with a saturated sodium chloride aqueous solution (30 mL), dried over anhydrous sodium sulphate, filtered, and concentrated. The residue was purified by silica gel column chromatography using eluent system B to obtain **372c** (3 g, yield: 91.7%).

Step 3: **372c** (1.3 g, 5.28 mmol), tetrabutylammonium iodide (390 mg, 1.06 mmol) and benzoic acid (709 mg, 5.81 mmol) were dissolved in 1,2-dichloroethane (15 mL), tert-butyl hydroperoxide (1.43 g, 15.84 mmol, 1.53 mL) was added, and the reaction system was heated to 60°C in an air bath and then reacted for 6 hours. The reaction system was quenched with saturated sodium sulphite, extracted with ethyl acetate (50 mL × 3), washed with a saturated sodium chloride aqueous solution (30 mL), dried over anhydrous sodium sulphate, filtered, and concentrated. The residue was purified by silica gel column chromatography using eluent system B to obtain **372d** (0.6 g, yield: 46.5%).

¹H NMR (400 MHz, CDCl₃) δ 8.07-8.00 (m, 2H), 7.67-7.56 (m, 2H), 7.44 (t, 2H), 7.40-7.30 (m, 2H), 6.51 (dd, 1H), 4.83 (dd, 1H), 4.75 (dd, 1H).

Step 4: **372d** (2.7 g, 7.37 mmol) was dissolved in tetrahydrofuran (10 mL), methanol (10 mL) and water (10 mL), sodium hydroxide (590 mg, 14.74 mmol) was added, and the system was reacted at 25°C for 2 hours. The reaction system was quenched with water, extracted with ethyl acetate (80 mL × 3), washed with a saturated sodium chloride aqueous solution (30 mL), dried over anhydrous sodium sulphate, filtered, and concentrated. The residue was purified by silica gel column chromatography using eluent system B to obtain (6-(pentafluoro-6-sulphanyl)-2,3-dihydrobenzofuran-3-ol **372e** (1.80 g, yield: 93.1%).

¹H NMR (400 MHz, CDCl₃) δ 7.49 (d, 1H), 7.37 (dd, 1H), 7.28 (d, 1H), 5.42 (dd, 1H), 4.65 (dd, J 1H), 4.53 (dd, 1H).

Step 5: **372e** (1.8 g, 6.87 mmol), tert-butyl (1,3-dioxoisoindol-2-yl)carbamate (2.16 g, 8.24 mmol) and triphenylphosphine (2.70 g, 10.30 mmol) were dissolved in tetrahydrofuran (20 mL), and the mixture was purged three times with nitrogen and cooled to 0°C. Diisopropyl azodicarboxylate (2.08 g, 10.30 mmol, 2.03 mL) was added. The mixture was concentrated, and the residue was purified by silica gel column chromatography using eluent system B to obtain **372f** (1.7 g, yield: 48.9%).

¹H NMR (400 MHz, CDCl₃) δ 7.91-7.73 (m, 4H), 7.63 (dd, 1H), 7.33-7.27 (m, 1H), 7.12 (dd, 1H), 6.17 (dd, 1H), 5.04-4.97 (m, 1H), 4.79 (td, 1H), 1.39 (d, 9H).

Step 6: **372f** (1.7 g, 3.36 mmol) was dissolved in dichloromethane (20 mL), the mixture was purged three times with nitrogen, and 85% hydrazine hydrate (336.08 mg, 6.71 mmol, 0.35 mL) was added. The reaction system was stirred at 25°C for 15 hours, filtered and concentrated, and the residue was dissolved in dichloromethane/petroleum ether (v/v=1:3, 5 mL), filtered and concentrated. The operations were repeated twice to obtain **372g** (1.20 g, crude). MS m/z (ESI): 377 [M+1]⁺

Step 7: **372g** (1.2 g, 3.19 mmol) and pyridine (504 mg, 6.38 mmol, 0.51 mL) were dissolved in dichloromethane (15 mL), and the mixture was purged three times with nitrogen. Benzyl chloroformate (816 mg, 4.78 mmol, 0.67 mL) was added dropwise at 0°C, and the reaction system was stirred at 25°C for 2 hours. The reaction system was quenched with water, extracted with ethyl acetate (80 mL × 3), washed with a saturated sodium chloride aqueous solution (30 mL), dried over anhydrous sodium sulphate, filtered, and concentrated. The residue was purified by silica gel column chromatography using eluent system B to obtain **372h** (1.3 g, yield: 80.0%).

Step 8: **372h** (1.3 g, 2.55 mmol) was dissolved in tetrahydrofuran (20 mL), and the mixture was purged three times with nitrogen and cooled to 0°C. NaH (204 mg, 5.09 mmol, 60% purity) was added, and the reaction system was stirred at 0°C for 15 minutes. Iodomethane (542 mg, 3.82 mmol, 0.24 mL) was added, and the reaction system was stirred at 25°C for 2 hours. The reaction system was quenched with water, extracted with ethyl acetate (80 mL × 3), washed with a saturated sodium chloride aqueous solution (30 mL), dried over anhydrous sodium sulphate, filtered, and concentrated. The residue was purified by silica gel column chromatography using eluent system B to obtain **372i** (0.9 g, yield: 67.4%).

Step 9: **372i** (0.9 g, 1.72 mmol) was dissolved in ethyl acetate (5 mL), hydrogen chloride ethyl acetate (5 mL) was added, and the reaction system was stirred at 25°C for 1 hour and concentrated to obtain **372j** (0.7 g, crude).

Step 10: **372k** (108 mg, 0.28 mmol) and benzyl 1-methyl-2-(6-(pentafluoro-6-sulphanyl)-2,3-dihydrobenzofuran-3-yl)hydrazine-1-carboxylate **372j** (0.15 g, 0.35 mmol) were dissolved in dichloromethane (10 mL), and the mixture was purged three times with nitrogen. Phosphorus oxychloride (271 mg, 1.77 mmol, 0.16 mL) and pyridine (140 mg, 1.77 mmol, 0.14 mL) were added, and the reaction system was stirred at 25°C for 15 hours. The reaction system was quenched with water, extracted with ethyl acetate (50 mL × 3), washed with a saturated sodium chloride aqueous solution (30 mL), dried over anhydrous sodium sulphate, filtered, and concentrated. The residue was purified by silica gel column chromatography using eluent system A to obtain **372l** (80 mg, yield: 28.7%). MS m/z (ESI): 788 [M+1]⁺

Step 11: **372l** (60 mg, 76 µmol) was dissolved in tetrahydrofuran (5 mL), palladium on carbon (9 mg, 76 µmol) was added, and the mixture was purged three times with hydrogen. The reaction system was stirred at 25°C under a hydrogen balloon for 1 hour. The system was filtered and concentrated to obtain **372m** (50 mg, crude). MS m/z (ESI): 654 [M+1]⁺

Step 12: **372m** (50 mg, 76 µmol) was dissolved in trifluoroacetic acid (2 mL), and the mixture was purged three times with nitrogen. The reaction system was stirred at 80°C for 15 minutes. The resulting reaction system was concentrated. The residue was purified by silica gel column chromatography using eluent system A to obtain **Example 372** (35 mg, yield: 90.9%). MS m/z (ESI): 504 [M+1]⁺

¹H NMR (400 MHz, DMSO-d₆) δ 8.81 (d, 1H), 8.10-7.65 (m, 2H), 7.53-7.25 (m, 2H), 7.07 (s, 2H), 6.29-5.92 (m, 2H), 5.33 (s, 2H), 4.98 (s, 2H), 4.81-4.49 (m, 2H), 2.27-1.89 (m, 3H).

### Example 372 Preparation of chiral isomers

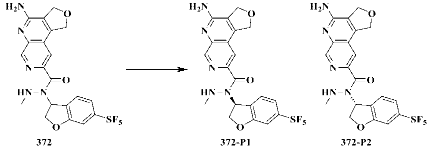

**Example 372** (35 mg, 0.07 mmol) was separated by chiral preparative HPLC (condition 2) to obtain rel-(R)-4-amino-N'-methyl-N-(6-(pentafluoro-6-sulphanyl)-2,3-dihydrobenzofuran-3-yl)-1,3-dihydrofuro[3,4-c][1,7]naphthyridine-8-carbohydrazide **372-P1** (14 mg, yield: 40.6%) and rel-(R)-4-amino-N'-methyl-N-(6-(pentafluoro-6-sulphanyl)-2,3-dihydrobenzofuran-3-yl)-1,3-dihydrofuro[3,4-c][1,7]naphthyridine-8-carbohydrazide **372-P2** (13 mg, yield: 36.0%).

**372-P1 (t_{R}: 1.169 min):** ¹H NMR (400 MHz, DMSO-d₆) δ 8.87 (d, 1H), 7.87 (d, 2H), 7.50-7.31 (m, 2H), 7.15 (s, 2H), 6.38-5.96 (m, 2H), 5.40 (s, 2H), 5.05 (t, 2H), 4.85-4.52 (m, 2H), 2.14 (d, 3H).

**372-P2 (t_{R}: 1.856 min):** ¹H NMR (400 MHz, DMSO-d₆) δ 8.87 (d, 1H), 7.87 (d, 2H), 7.50-7.33 (m, 2H), 7.14 (s, 2H), 6.37-5.98 (m, 2H), 5.40 (s, 2H), 5.05 (s, 2H), 4.87-4.55 (m, 2H), 2.14 (d, 3H).

### Example 374

### 4-Amino-N'-methyl-N-(2-pentafluorosulphanyl-5,8-dihydro-6H-pyrano[3,4-b]pyridin-5-yl)-1,3-dihydrofuran[3,4-c][1,7]naphthyridine-8-hydrazide

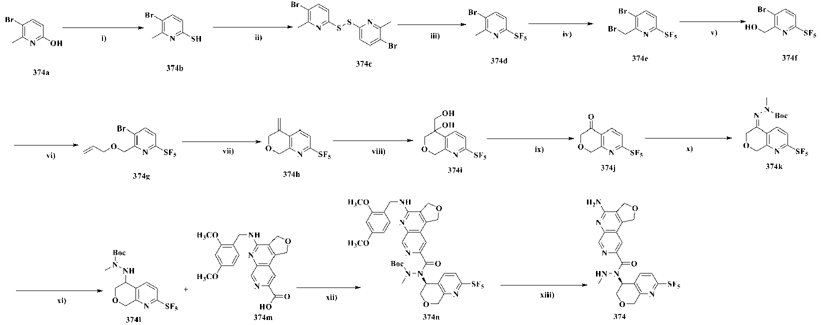

Step 1: 5-Bromo-6-methyl-2(1H)-pyridone **374a** (35 g, 186 mmol) and Lawson's reagent (37.6 g, 93 mmol) were dissolved in toluene (350 mL), and the reaction system was reacted at 110°C for 16 hours. The resulting reaction system was concentrated. The system was filtered to obtain **374b** (30 g, yield: 78.7%). MS m/z (ESI): 205 [M+1]⁺

Step 2: **374b** (60 g, 294 mmol), ferric chloride (23.9 g, 147 mmol) and sodium iodide (22 g, 147 mmol) were dissolved in acetonitrile (600 mL), and the reaction system was reacted at 20°C under open atmosphere for 16 hours. The reaction solution was first quenched with saturated sodium thiosulphate and filtered, water (800 mL) and ethyl acetate (2000 mL) were then added to the mixed solution, and the organic phase was washed with a saturated sodium chloride aqueous solution (200 mL × 3), then dried and subjected to rotary evaporation to dryness. The residue was purified by reversed-phase column chromatography using eluent system A to obtain **374c** (42 g, yield: 48.3%). MS m/z (ESI): 591 [M+1]⁺

Step 3: **374c** (18 g, 44.3 mmol), tetraethyl ammonium chloride (14.7 g, 88.6 mmol) and silver difluoride (129 g, 886 mmol) were dissolved in dichloromethane (200 mL), and the mixture was reacted at 20°C in a sealed system in a sealed tube for 16 hours. The system was filtered and concentrated. The residue was purified by reversed-phase column chromatography using eluent system A to obtain **374d** (21.4 g, yield: 80.8%). MS m/z (ESI): 299 [M+1]⁺

Step 4: **374d** (10.5 g, 35.2 mmol), N-bromosuccinimide (6.27 g, 35.2 mmol) and azobisisobutyronitrile (1.15 g, 7 mmol) were dissolved in carbon tetrachloride (200 mL), and the reaction system was reacted at 80°C for 16 hours, concentrated, filtered, and then quenched with ammonium chloride. Water (50 mL) and ethyl acetate (500 mL) were then added to the mixed solution, and the organic phase was washed with a saturated sodium chloride aqueous solution (20 mL × 3), then dried and subjected to rotary evaporation to dryness. The residue was purified by reversed-phase column chromatography using eluent system A to obtain **374e** (4.7 g, yield: 35.3%). MS m/z (ESI): 378 [M+1]⁺

Step 5: **374e** (4.27 g, 11.3 mmol) and potassium carbonate (4.69 g, 40 mmol) were dissolved in dioxane (40 mL) and water (40 mL), and the reaction system was reacted at 100°C for 16 hours. The resulting reaction system was concentrated. The residue was purified by reversed-phase column chromatography using eluent system A to obtain **374f** (1.8 g, yield: 50.6%). MS m/z (ESI): 315 [M+1]⁺

Step 6: **374f** (15 g, 47.7 mmol), tetrabutylammonium bisulphate (3.24 g, 9.5 mmol) and potassium hydroxide (5.35 g, 95.5 mmol) were dissolved in allyl bromide (30 mL), and the mixture was reacted at 20°C in a solvent-free system for 16 hours. The reaction solution was first quenched with water, and water (100 mL) and ethyl acetate (500 mL) were then added to the mixed solution. The organic phase was washed with a saturated sodium chloride aqueous solution (50 mL × 3), then dried and subjected to rotary evaporation to dryness. The residue was purified by reversed-phase column chromatography using eluent system A to obtain **374g** (13.5 g, yield: 79.7%). MS m/z (ESI): 355 [M+1]⁺

Step 7: **374g** (22 g, 62.1 mmol), tri-o-tolylphosphine (11.3 g, 37.2 mmol), palladium acetate (2.78 g, 12.4 mmol) and caesium carbonate (24.2 g, 74.5 mmol) were dissolved in N,N-dimethylformamide (440 mL), and the reaction system was reacted at 90°C for 2 hours. The reaction solution was first quenched with water, and water (200 mL) and ethyl acetate (1000 mL) were then added to the mixed solution. The organic phase was washed with a saturated sodium chloride aqueous solution (100 mL × 3), then dried and subjected to rotary evaporation to dryness. The residue was purified by reversed-phase column chromatography using eluent system A to obtain **374h** (11.1 g, yield: 65.2%). MS m/z (ESI): 274 [M+1]⁺

Step 8: **374h** (4 g, 14.6 mmol), N-methylmorpholine oxide (5.15 g, 43.9 mmol) and potassium osmate dihydrate (228 mg, 0.73 mmol) were dissolved in acetone (40 mL) and water (8 mL), and the reaction system was reacted at 20°C for 16 hours. The reaction solution was first quenched with water and filtered, water (50 mL) and ethyl acetate (300 mL) were then added to the mixed solution, and the organic phase was washed with a saturated sodium chloride aqueous solution (20 mL × 3), then dried and subjected to rotary evaporation to dryness. The residue was purified by reversed-phase column chromatography using eluent system A to obtain **374i** (3.5 g, yield: 77.8%). MS m/z (ESI): 308 [M+1]⁺

Step 9: **374i** (3.5 g, 11.3 mmol) and sodium periodate (7.36 g, 34.1mmol) were dissolved in tetrahydrofuran (40 mL) and water (4 mL), and the reaction system was reacted at 20°C for 4 hours. The system was filtered and concentrated. The residue was purified by reversed-phase column chromatography using eluent system A to obtain **374j** (2.86 g, yield: 91.7%). MS m/z (ESI): 276 [M+1]⁺

Step 10: **374j** (260 mg, 0.945 mmol), N-methyl-Boc hydrazide (276 mg, 1.89 mmol) and acetic acid (57 mg, 0.945 mmol) were dissolved in methanol (6 mL), and the reaction system was reacted at 20°C for 16 hours. The reaction solution was first quenched with water, and water (10 mL) and ethyl acetate (100 mL) were then added to the mixed solution. The organic phase was washed with a saturated sodium chloride aqueous solution (10 mL × 3), then dried and subjected to rotary evaporation to dryness. The residue was purified by reversed-phase column chromatography using eluent system A to obtain **374k** (310 mg, yield: 81.2%). MS m/z (ESI): 404 [M+1]⁺

Step 11: **374k** (310 mg, 0.77 mmol) and sodium borohydride (87 mg, 2.3 mmol) were dissolved in ethanol (6 mL), and the reaction system was reacted at 20°C for 16 hours. The reaction solution was first quenched with ammonium chloride, and water (10 mL) and ethyl acetate (100 mL) were then added to the mixed solution. The organic phase was washed with a saturated sodium chloride aqueous solution (10 mL × 3), then dried and subjected to rotary evaporation to dryness. The residue was purified by reversed-phase column chromatography using eluent system A to obtain **374l** (220 mg, yield: 70.4%). MS m/z (ESI): 406 [M+1]⁺

Step 12: **374l** (45 mg, 0.11 mmol), 4-((2,4-dimethoxyphenyl)amino)-1,3-dihydrofuran[3,4-c] [1,7]naphthyridine-8-carboxylic acid **374m** (50 mg, 0.13 mmol), pyridine (70 mg, 0.88 mmol) and phosphorus oxychloride (100 mg, 0.66 mmol) were dissolved in N,N-dimethylacetamide (3 mL), and the reaction system was reacted at 20°C for 16 hours. The reaction solution was first quenched with ammonium chloride, and water (10 mL) and ethyl acetate (100 mL) were then added to the mixed solution. The organic phase was washed with a saturated sodium chloride aqueous solution (10 mL × 3), then dried and subjected to rotary evaporation to dryness. The residue was purified by reversed-phase column chromatography using eluent system A to obtain **374n** (29 mg, yield: 34.3%). MS m/z (ESI): 769 [M+1]⁺

Step 13: **374n** (29 mg, 0.034 mmol) was dissolved in trifluoroacetic acid (4 mL), and the mixture was purged three times with nitrogen. The reaction system was reacted at 90°C for 0.5 hours. The resulting reaction system was concentrated, and the residue was purified by silica gel column chromatography using eluent system A to obtain **Example 374** (11.2 mg, yield: 63.5%). MS m/z (ESI): 519 [M+1]⁺

¹HNMR (400 MHz, DMSO-d₆) δ 8.85 (s, 1H), 8.11 (m, 1H), 7.99 - 7.90 (m, 2H), 7.15 (s, 2H), 5.64 (m, 1H), 5.42 - 5.37 (m, 2H), 5.05 (m, 2H), 4.88 - 4.79 (m, 1H), 4.78 - 4.65 (m, 1H), 4.25 (m, 1H), 4.09 (m, 1H), 2.47 (s, 2H), 2.19 (s, 1H).

### Example 375

### 4-Amino-N',1-dimethyl-N-(6-(pentafluoro-6-sulphanyl)-2,3-dihydrobenzofuran-3-yl)-1H-pyrazolo[4,3-c][1,7]naphthyridine-8-carbohydrazide

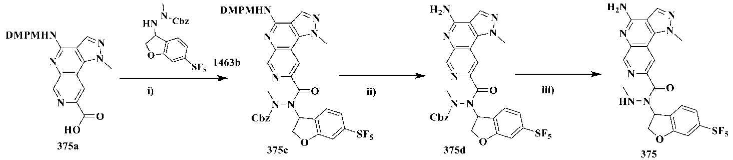

Referring to the synthesis method described in Step 10 to Step 12 of **Example 372, Example 375** (5 mg, yield: 3.9%) was obtained from 4-(3,4-dimethoxybenzyl)amino)-1-methyl-1H-pyrazolo[4,3-c][1,7]naphthyridine-8-carboxylic acid **375a** (100 mg, 0.25 mmol) and benzyl 1-methyl-2-(6-(pentafluoro-6-sulphanyl)-2,3-dihydrobenzofuran-3-yl)hydrazide-1-carboxylate **375b** (108 mg, 0.25 mmol). MS m/z (ESI): 516 [M+1]⁺

¹H NMR (400 MHz, DMSO-d6) δ 8.80 (d, 1H), 8.45 (s, 1H), 8.30 (s, 1H), 7.76 (s, 1H), 7.50 (s, 2H), 7.40 (dd, 1H), 7.30 (s, 1H), 6.21 (d, 2H), 5.31 (s, 1H), 4.73 (d, 1H), 4.40 (s, 3H), 2.12 (d, 3H).

### Example 375 Preparation of chiral isomers

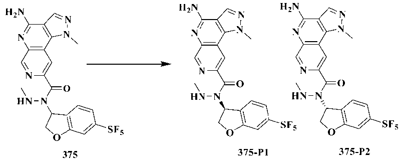

**Example 375** (130 mg, 0.25 mmol) was separated by chiral preparative HPLC (condition 2) to obtain rel-(R)-4-amino-N',1-dimethyl-N-(6-(pentafluoro-6-sulphanyl)-2,3-dihydrobenzofuran-3-yl)-1H-pyrazolo[4,3-c][1,7]naphthyridine-8-carbohydrazide **375-P1** (33 mg, yield: 25.4%) and rel-(R)-4-amino-N',1-dimethyl-N-(6-(pentafluoro-6-sulphanyl)-2,3-dihydrobenzofuran-3-yl)-1H-pyrazolo[4,3-c][1,7]naphthyridine-8-carbohydrazide **375-P2** (32 mg, yield: 24.6%).

**375-P1 (t_{R}: 0.656 min):** ¹H NMR (400 MHz, DMSO-d6) δ8.90 (s, 1H), 8.52 (s, 1H), 8.37 (s, 1H), 7.83 (s, 1H), 7.57 (s, 2H), 7.49-7.44 (m, 1H), 7.32 (d, 1H), 6.29 (d, 2H), 4.92-4.60 (m, 2H), 4.47 (s, 3H), 2.20 (d, 3H).

**375-P2 (t_{R}: 0.911 min):** ¹H NMR (400 MHz, DMSO-d₆) δ 8.90 (s, 1H), 8.52 (s, 1H), 8.37 (s, 1H), 7.83 (s, 1H), 7.57 (s, 2H), 7.46 (d, 1H), 7.37 (s, 1H), 6.29 (d, 2H), 4.94- 4.61 (m, 2H), 4.47 (s, 3H), 2.33 (s, 3H).

### Example 385

### 4-Amino-N'-methyl-N-(6-(pentafluoro-6-sulphanyl)-2,3-dihydrobenzofuran-3-yl)imidazo [1,5-a] pyrido [3,4-e] pyrazine-8-carbohydrazide

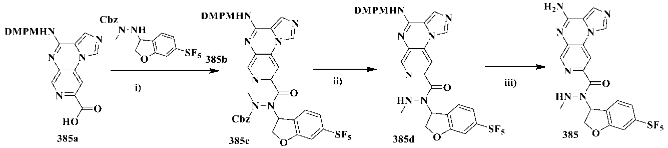

Referring to the synthesis method described in Step 10 to Step 12 of **Example 372, Example 385** (15 mg, yield: 7.5%) was obtained from 4-(3,4-dimethoxybenzyl)amino)imidazo[1,5-a]pyrido[3,4-e]pyrazine-8-carboxylic acid **385a** (150 mg, 0.40 mmol) and benzyl 1-methyl-2-(6-(pentafluoro-6-sulphanyl)-2,3-dihydrobenzofuran-3-yl)hydrazide-1-carboxylate **385b** (166 mg, 0.40 mmol). MS m/z (ESI): 502 [M+1]⁺

¹H NMR (400 MHz, DMSO-d6) δ 9.28 (d, 1H), 8.59 (d, 2H), 7.93 (s, 1H), 7.73 (s, 2H), 7.54-7.22 (m, 2H), 6.08 (d, 2H), 5.35 (s, 1H), 4.78 (d, 1H), 4.67-4.51 (m, 1H), 2.13 (dd, 3H).

### Example 385 Preparation of chiral isomers

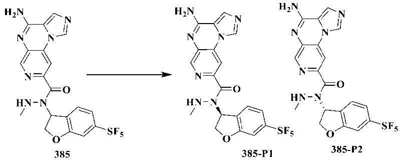

**Example 385** (110 mg, 0.22 mmol) was separated by chiral preparative HPLC (condition 2) to obtain rel-(R)-4-amino-N'-methyl-N-(6-(pentafluoro-6-sulphanyl)-2,3-dihydrobenzofuran-3-yl)imidazo[1,5-a]pyrido[3,4-e]pyrazine-8-carbohydrazide **385-P1** (43 mg, yield: 39.1%) and rel-(R)-4-amino-N'-methyl-N-(6-(pentafluoro-6-sulphanyl)-2,3-dihydrobenzofuran-3-yl)imidazo[1,5-a]pyrido[3,4-e]pyrazine-8-carbohydrazide **385-P2** (42 mg, yield: 38.2%).

**385-P1 (t_{R}: 1.101 min):** ¹H NMR (400 MHz, DMSO-d6) δ 9.35 (d, 1H), 8.65 (d, 2H), 8.00 (s, 1H), 7.80 (s, 2H), 7.52-7.32 (m, 2H), 6.22 (s, 1H), 6.10 (s, 1H), 5.42 (d, , 1H), 4.91-4.57 (m, 2H), 2.20 (d, 3H).

**385-P2 (t_{R}: 1.591 min):** ¹H NMR (400 MHz, DMSO-d6) δ 9.35 (d, 1H), 8.66 (d, 2H), 8.00 (s, 1H), 7.80 (s, 2H), 7.57-7.32 (m, 2H), 6.16 (d, 2H), 5.42 (s, 1H), 4.89-4.57 (m, 2H), 2.36-2.03 (m, 3H).

### Example 387

### 4-Amino-N'-methyl-N-(2-pentafluorosulphanyl-5,8-dihydro-6H-pyrano[3,4-b]pyridin-5-yl)imidazo[1,5-a]pyridone[3,4-e]pyrazoline-8-hydrazide

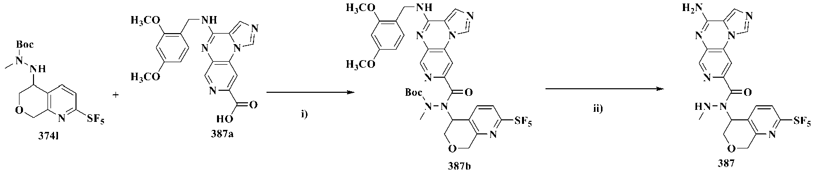

Step 1: **374l** (50 mg, 0.123 mmol), 4-((2,4-dimethoxyphenyl)amino)imidazo[1,5-a]pyridone[3,4-e]pyrazoline-8-carboxylic acid **387a** (47 mg, 0.123 mmol), pyridine (78 mg, 0.98 mmol) and phosphorus oxychloride (113 mg, 0.74 mmol) were dissolved in N,N-dimethylacetamide (3 mL), and the reaction system was reacted at 20°C for 16 hours. The reaction solution was first quenched with ammonium chloride, and water (10 mL) and ethyl acetate (100 mL) were then added to the mixed solution. The organic phase was washed with a saturated sodium chloride aqueous solution (10 mL × 3), then dried and subjected to rotary evaporation to dryness. The residue was purified by reversed-phase column chromatography using eluent system A to obtain **387b** (50 mg, yield: 53.0%). MS m/z (ESI): 767 [M+1]⁺

Step 2: **387b** (50 mg, 0.065 mmol) was dissolved in trifluoroacetic acid (4 mL), and the mixture was purged three times with nitrogen. The reaction system was reacted at 90°C for 0.5 hours. The resulting reaction system was concentrated, and the residue was purified by silica gel column chromatography using eluent system A to obtain **Example 387** (22 mg, yield: 65.4%). MS m/z (ESI): 517 [M+1]⁺

¹H NMR (400 MHz, DMSO-d₆) δ 9.33 (d, 1H), 8.66-8.54 (m, 2H), 8.11 (m, 1H), 8.01 - 7.92 (m, 2H), 7.79 (m, 2H), 5.63 (t, 1H), 5.41 (m, 1H), 4.90 - 4.80 (m, 1H), 4.79 - 4.66 (m, 1H), 4.26 (m, 1H), 4.11 (m, 1H), 2.49 (s, 2H), 2.26 (m, 1H).

### Example 391

### 4-Amino-1-methyl-N'-(methyl-d3)-N-(3-trifluoromethyl-7,10-dihydro-8H-imidazo[1,2-a]pyrano[3,4-c]pyridin-7-yl)-1H-pyrazolo[4,3-c][1,7]naphthyridine-8-carbohydrazide

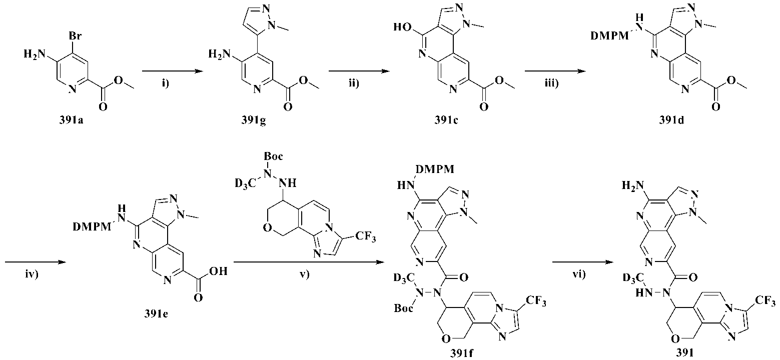

Step 1: Methyl 5-amino-4-bromopyridine-2-carboxylate **391a** (5 g, 17.31 mmol), 1-methyl-5-(4,4,5,5-tetramethyl-1,3,2-dioxaborolan-2-yl)-1H-pyrazole (4.32 g, 20.78 mmol), Pd(dppf)Cl₂ (2.53 g, 3.46 mmol) and potassium phosphate (11.02 g, 51.94 mmol) were dissolved in dioxane/water (100 mL, 5: 1), and the mixture was purged three times with nitrogen, heated to 100°C and reacted for 2 h. LCMS showed completion of the reaction. The reaction solution was cooled to room temperature, the phases were directly separated, and the organic phase was collected and subjected to rotary evaporation to dryness to obtain a crude product. The crude product was purified by silica gel column chromatography using dichloromethane/methanol (V : V=10 : 1) to obtain a product as a light brown solid. The brown solid product was further purified by reversed-phase column chromatography and then lyophilized to obtain **391b** (3.5 g, yield: 85%). MS m/z (ESI): 233 [M+1]⁺

Step 2: **391b** (3.5 g, 15.07 mmol) and triphosgene (13.42g, 45.21 mmol) were dissolved in a THF solution (50 mL), triethylamine (6.3 mL, 45.31 mmol) was added dropwise to the reaction solution at ambient temperature, and the resulting reaction solution was heated to 80°C and stirred for 16 h. LCMS showed an obvious product peak. The reaction solution was cooled to ambient temperature and filtered, the filter residue was slurried with cold water and further filtered. The filter residue was oven-dried to obtain **391c** (1 g, yield: 25.7%). MS m/z (ESI): 259 [M+1]⁺

Step 3: **391c** (1.0 g, 3.87 mmol) was dissolved in dimethyl sulphoxide (10 mL), (2,4-dimethoxyphenyl)methanamine (0.647 g, 3.87 mmol), BOP(1.71 g, 3.87 mmol) and DIEA (1.0 g, 7.74 mmol) were added, and the mixture was stirred and reacted at room temperature for 1 h. LCMS showed completion of the reaction. Water (10 mL) was added to the reaction solution, resulting in the precipitation of solids, which were filtered to obtain a crude product. The crude product was dissolved in ethyl acetate (10 mL), and the mixture was slurried and filtered to obtain **391d** (0.75 g, yield: 47.5%).

Step 4: **391d** (0.75 g, 1.84 mmol) was dissolved in tetrahydrofuran (5 mL), water (1 mL) was added, followed by lithium hydroxide monohydrate (0.75 g, 17.87 mmol), and the mixture was stirred and reacted at room temperature for 16 h. LCMS showed completion of the reaction. The reaction solution was adjusted to neutral with 1 N HCl and subjected to rotary evaporation to dryness to obtain a crude product. The crude product was purified by preparative liquid chromatography to obtain **391e** (80 mg, yield: 11%).

Step 5: **391e** (50 mg, 0.127 mmol) was dissolved in 1,2-dichloroethane (5 mL), tert-butyl N-(trideuteriomethyl)-N-[5-(trifluoromethyl)-12-oxa-3,6-diazatricyclo[7.4.0.02,6]trideca-1(9),2,4,7-tetraen-10-yl]amino]carbamate (49.5 mg, 0.127 mmol) and pyridine (0.1 g, 1.27 mmol) were added, and the mixture was purged three times with nitrogen. Under nitrogen atmosphere, phosphorus oxychloride (58.5 mg, 0.381 mmol) was slowly added, and the mixture was reacted at room temperature for 16 h. LCMS showed completion of the reaction. The reaction solution was subjected to rotary evaporation to dryness to obtain **391f** (0.1 g, crude), which was used directly in the next step.

Step 6: **391f** (0.1 g, 0.130 mmol) was dissolved in trifluoroacetic acid (5 mL), and the mixture was heated to 80°C and reacted for 0.5 h. LCMS showed completion of the reaction. The reaction solution was subjected to rotary evaporation to dryness to remove the solvent to obtain a crude product. The crude product was purified by preparative liquid chromatography to obtain **Example 391** (12 mg, yield: 17.8%). MS m/z (ESI): 515 [M+1]⁺

¹H NMR (400 MHz, DMSO-d₆) δ 8.91(s, 1H), 8.57(s ,1H), 8.42(s, 2H), 8.10(s, 1H), 7.49(s, 2H), 7.23(d, 1H), 5.64(s, 1H), 4.99(s, 2H), 4.49(s, 3H), 4.35-4.17 (m,2H).

### Example 391 Preparation of chiral isomers

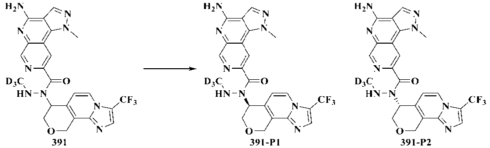

**Example 391** (14 mg, 0.027 mmol) was separated by chiral preparative HPLC (condition 2) to obtain rel-(S)-4-amino-1-methyl-N'-(methyl-d3)-N-(3-trifluoromethyl-7,10-dihydro-8H-imidazo[1,2-a]pyrano[3,4-c]pyridin-7-yl)-1H-pyrazolo[4,3-c][1,7]naphthyridine-8-carbohydrazide **391-P1** (6 mg, yield: 42.9%) and rel-(R)-4-amino-1-methyl-N'-(methyl-d3)-N-(3-trifluoromethyl-7,10-dihydro-8H-imidazo[1,2-a]pyrano[3,4-c]pyridin-7-yl)-1H-pyrazolo[4,3-c][1,7] naphthyridine-8-carbohydrazide **391-P2** (6 mg, yield: 42.9%).

**391-P1 (t_{R}: 5.738 min):** ¹H NMR (400 MHz, DMSO-d₆) δ 8.90 (s, 1H), 8.57 (s ,1H), 8.51 (s ,1H), 8.49 (d, 1H), 8.16 (d, 1H), 7.89 (br s, 2H), 7.21 (dd, 1H), 5.56 (d, 1H), 5.36 (s, 1H), 5.03 (q, 1H), 4.93 (s, 1H), 4.49 (s, 3H), 4.35-4.17 (m, 2H).

**391-P2 (t_{R}: 4.953 min):** ¹H NMR (400 MHz, DMSO-d₆) δ 8.85 (s, 1H), 8.57 (d ,1H), 8.44 (dd, 1H), 8.37 (d ,1H), 8.16 (d, 1H), 7.57 (s, 2H), 7.20 (dd, 1H), 5.56 (d, 1H), 5.36 (s, 1H), 4.99 (q, 1H), 4.94 (s, 1H), 4.47 (s, 3H), 4.35-4.07 (m, 2H).

### Example 392

### 4-Amino-N'-(trideuteriomethyl)-N-(3-trifluoromethyl-7,10-dihydro-8H-imidazo [1,2-a]pyrano [3,4-c]pyridin-7-yl)-1,3-dihydrofuro [3,4-c] quinoline-8-carbohydrazide

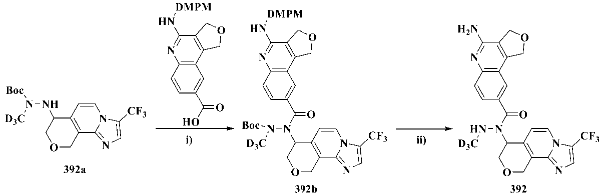

Step 1: tert-Butyl 1-(methyl-d₃)-2-(3-trifluoromethyl-7,10-dihydro-8H-imidazo[1,2-a]pyrano[3,4-c]pyridin-7-yl)hydrazine carboxylate **392a** (150 mg, 385.22 µmol) and 4-[(2,4-dimethoxyphenyl)methylamino]-7-fluoro-1,3-dihydrofuro[3,4-c]quinoline-8-carboxylic acid (175.84 mg, 462.27 µmol) were dissolved in dichloroethane (5 mL), and pyridine (304.71 mg, 3.85 mmol, 310.33 µL) was added. Phosphorus oxychloride (177.20 mg, 1.16 mmol) was slowly added dropwise in an ice bath, and the mixture was stirred at room temperature for 1 hour. Upon completion of the reaction, the reaction solution was quenched with water, extracted with dichloromethane (10 mL × 3), washed with a saturated sodium chloride aqueous solution, dried over anhydrous sodium sulphate, filtered, and concentrated. The residue was purified by silica gel column chromatography using eluent system A to obtain **392b** (130 mg, yield: 44.9%). MS m/z (ESI): 752 [M+1]⁺

Step 2: **392b** (130 mg, 172.93 µmol) was dissolved in trifluoroacetic acid (5 mL), and the mixture was stirred at 80°C for 1 hour. Upon completion of the reaction as monitored by LCMS, the reaction solution was subjected to rotary evaporation to dryness to obtain a crude product, which was purified by preparative liquid chromatography to obtain **Example 392** (28.1 mg, yield: 32.4%). MS m/z (ESI): 502 [M+1]⁺

¹H NMR (400 MHz, DMSO-d₆) δ 8.38 (d, 1H), 8.35 - 8.15 (m, 1H), 8.11 (s, 1H), 7.97 (d, 1H), 7.93 (s, 1H), 7.77 (d, 1H), 7.21 (d, 1H), 5.47 (s, 2H), 5.39 - 5.18 (m, 1H), 5.12 (s, 2H), 5.00 (d, 2H), 4.26 (s, 2H).

### Example 393

### 4-Amino-7-fluoro-3-methyl-N'-(trideuteriomethyl)-N-(3-trifluoromethyl-7,10-dihydro-8H-imidazo[1,2-a]pyrano[3,4-c]pyridin-7-yl)-1,3-dihydrofuro[3,4-c]quinoline-8-carbohydrazide

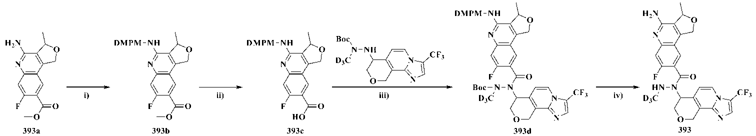

Step 1: Methyl 4-amino-7-fluoro-3-methyl-1,3-dihydrofuro[3,4-c]quinoline-8-carboxylate **393a** (300 mg, 1.09 mmol) was dissolved in toluene (10 mL), 2,4-dimethoxybenzaldehyde (180 mg, 1.09 mmol) was added, followed by a catalytic amount of acetic acid, and the mixture was heated to 125°C, stirred for 16 h, and cooled to room temperature. Sodium cyanoborohydride (136 mg, 2.17 mmol) was slowly added, and the resulting mixture was reacted at room temperature for 1 h. The reaction solution was subjected to rotary evaporation to dryness, and the residue was purified by column chromatography using eluent A to obtain **393b** (64 mg, yield: 13.8%). MS m/z (ESI): 427 [M+1]⁺

Step 2: **393b** (64 mg, 0.15 mmol) was dissolved in tetrahydrofuran (5 mL), water (1 mL) and lithium hydroxide hydrate (0.18 g, 0.45 mmol) were added, and the mixture was stirred and reacted at room temperature for 16 h. The reaction solution was adjusted to neutral pH with 1 N HCl solution, and subjected to rotary evaporation to dryness to remove the solvent to obtain **393c** (62 mg, crude), which was used directly in the next step. MS m/z (ESI): 413 [M+1]⁺

Step 3: **393c** (62 mg, 0.150 mmol) was dissolved in 1,2-dichloroethane (5 mL), tert-butyl N-(trideuteriomethyl)-N-[5-(trifluoromethyl)-12-oxa-3,6-diazatricyclo[7.4.0.02,6]trideca-1(9),2,4,7-tetraen-10-yl]amino]amino]carboxylate (87.8 mg, 0.225 mmol), tetramethylchloroformamidinium hexafluorophosphate (50 mg, 0.180 mmol) and N-methylimidazole (60 mg, 0.750 mmol) were added, and the mixture was reacted at room temperature for 1 hour. The reaction solution was subjected to rotary evaporation to dryness to remove the solvent, and the residue was purified by reversed-phase chromatography to obtain **393d** (28 mg, yield: 23.8%). MS m/z (ESI): 784 [M+1]⁺

Step 4: **393d** (28 mg, 0.036 mmol) was dissolved in trifluoroacetic acid (5 mL), and the mixture was heated to 80°C and reacted for 0.5 h. LCMS showed completion of the reaction. The reaction solution was subjected to rotary evaporation to dryness to remove the solvent to obtain a crude product. The crude product was purified by preparative liquid chromatography to obtain **Example 393** (14 mg, yield: 73.4%). MS m/z (ESI): 534 [M+1]⁺

¹H NMR (400 MHz, DMSO-d₆) δ 9.04 (s, 0.4H), 8.62 - 7.97 (m, 3.5H), 7.70 (t, 0.8H), 7.55 - 7.44 (m, 0.3H), 7.32 (d, 1H), 7.22 (s, 0.5H), 6.79 (dd, 0.5H), 5.85 (s, 0.5H), 5.53 - 5.30 (m, 1.5H), 5.25 - 5.06 (m, 1H), 4.81 (m, , 1H), 4.62 (d, 1H), 4.41 (d, 0.7H), 3.99 (d, 0.5H), 3.93 (s, 0.4H), 3.86 (d, 1.5H), 3.77 (s, 0.2H), 3.67 (d, 0.9H), 1.47 - 1.34 (m, 3H).

The examples in the table below were prepared with reference to **Example 371:**

| **Example** | **Structure** | **MS m/z (ESI)** | **Example** | **Structure** | **MS m/z (ESI)** |
|---|---|---|---|---|---|
| | | **[M+1]⁺** | | | **[M+1]⁺** |
| **394-P1** | 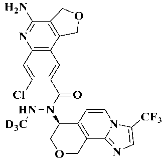 | 536 | **424-P1** | 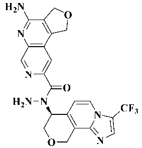 | 486 |
| **395-P1** | 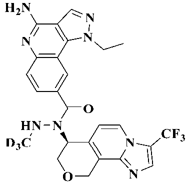 | 528 | **425** | 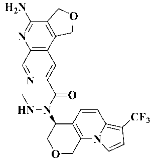 | 499 |
| **396** | 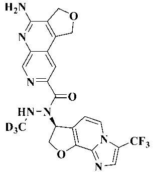 | 489 | **426** | 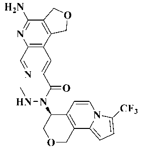 | 499 |
| **397** | 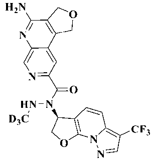 | 489 | **427** | 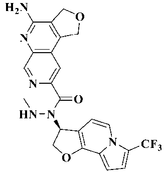 | 485 |
| **398** | 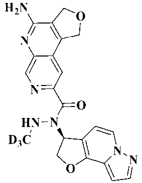 | 421 | **428** | 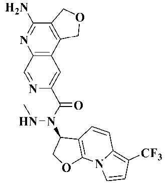 | 485 |
| **399** | 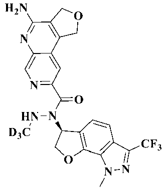 | 503 | **429** | 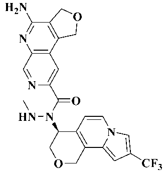 | 499 |
| **400** | 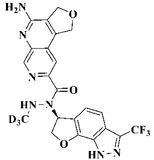 | 489 | **430** | 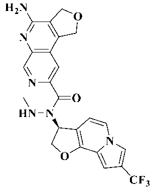 | 485 |
| **401** | 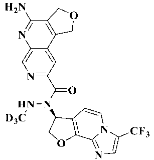 | 489 | **431** | 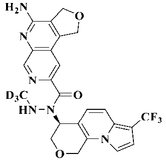 | 502 |
| **402** | 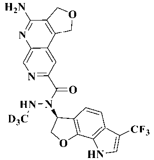 | 488 | **432** | 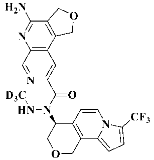 | 502 |
| **403** | 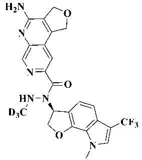 | 502 | **433** | 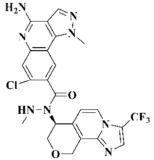 | 545 |
| **404** | 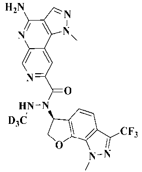 | 515 | **434** | 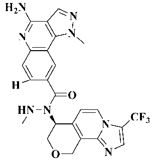 | 511 |
| **405** | 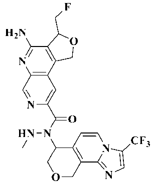 | 532 | **435-P1** | 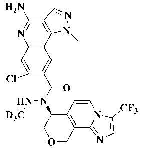 | 548 |
| **405-P1** | 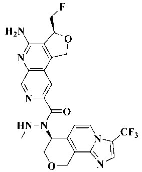 | 532 | **436** | 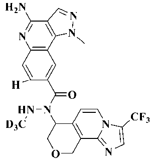 | 514 |
| **405-P2** | 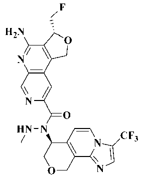 | 532 | **436-P1** | 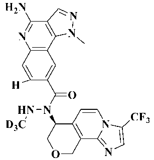 | 514 |
| **406** | 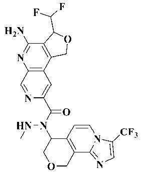 | 550 | **436-P2** | 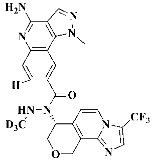 | 514 |
| **406-P1** | 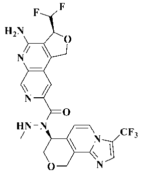 | 550 | **437** | 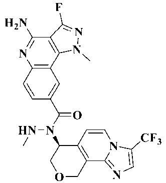 | 529 |
| **406-P2** | 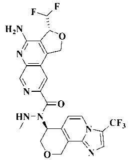 | 550 | **438** | 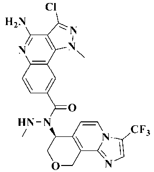 | 545 |
| **407** | 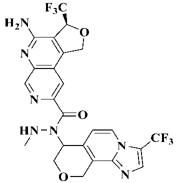 | 568 | **439** | 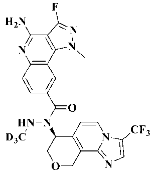 | 532 |
| **407-P1** | 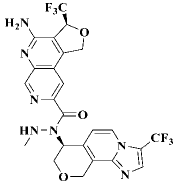 | 568 | **440-P1** | 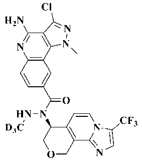 | 548 |
| **407-P2** | 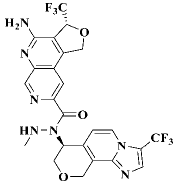 | 568 | **441** | 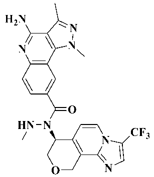 | 525 |
| **408** | 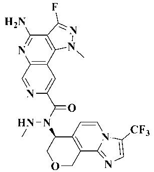 | 530 | **442** | 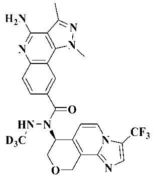 | 528 |
| **409** | 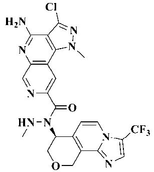 | 546 | **443** | 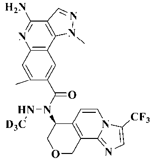 | 528 |
| **410** | 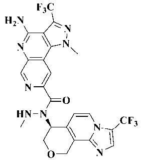 | 580 | **444-P1** | 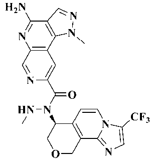 | 512 |
| **411** | 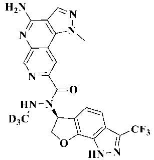 | 501 | **445** | 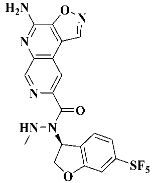 | 503 |
| **412** | 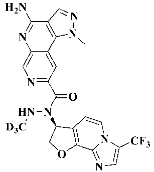 | 501 | **446** | 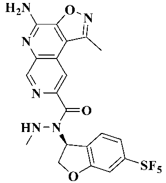 | 517 |
| **413** | 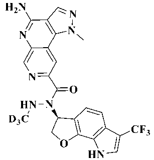 | 500 | **447** | 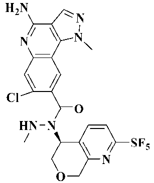 | 564 |
| **414** | 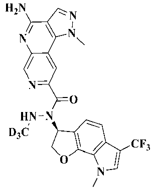 | 514 | **448-P1** | 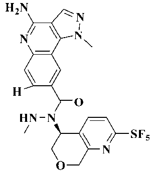 | 530 |
| **415** | 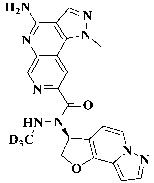 | 433 | **449-P1** | 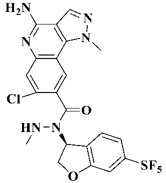 | 549 |
| **416** | 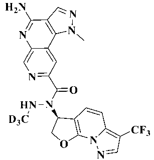 | 501 | **450-P1** | 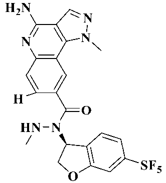 | 515 |
| **417** | 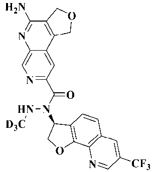 | 500 | **451-P1** | 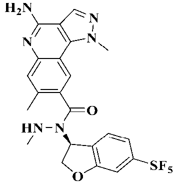 | 529 |
| **418** | 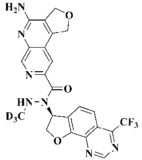 | 501 | **452** | 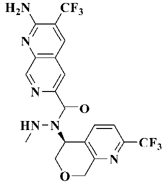 | 487 |
| **419** | 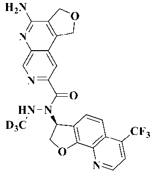 | 500 | **453-P1** | 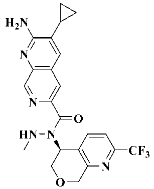 | 459 |
| **420** | 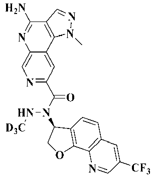 | 512 | **454** | 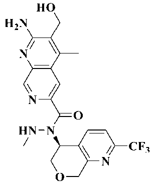 | 463 |
| **421** | 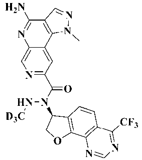 | 513 | **455** | 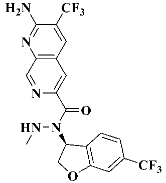 | 472 |
| **422** | 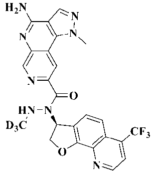 | 512 | **456** | 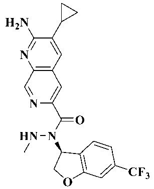 | 444 |
| **423** | 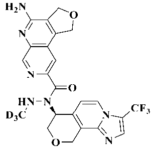 | 503 | **457** | 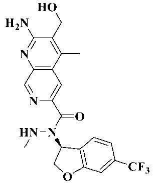 | 448 |

Alternatively, the following examples were prepared using the following method:

### Example 394

### 4-Amino-7-chloro-N'-(methyl-d3)-N-(3-(trifluoromethyl)-7,10-dihydro-8H-imidazo [1,2-a]pyrano [3,4-c]pyridin-7-yl)-1,3-dihydrofuro [3,4-c] quinoline-8-carbohydrazide

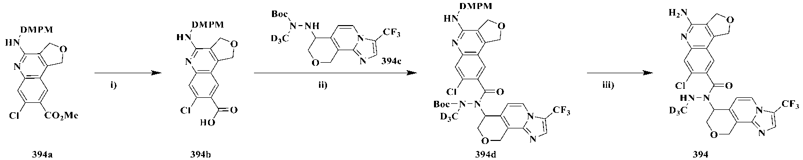

Referring to the method described in Step 2 to Step 4 of **Example 393, Example 394** (67 mg, yield: 26.8%) was obtained from methyl 7-chloro-4-((3,4-dimethoxybenzyl)amino)-1,3-dihydrofuro[3,4-c]quinoline-8-carboxylate **394a** (200 mg, 466 µmol). MS m/z (ESI): 536 [M+1]⁺

¹H NMR (400 MHz, DMSO-d₆) δ 8.50 (t, 1H), 8.21 - 8.10 (m, 1H), 7.94 - 7.44 (m, 3H), 7.22 (dd, 1H), 6.99 (s, 2H), 5.34 (t, 2H), 5.12 - 4.88 (m, 4H), 4.60 (d, 1H), 4.34 - 4.08 (m, 2H).

### Example 395

### 4-Amino-1-ethyl-N'-(methyl-d3)-N-(3-(trifluoromethyl)-7,10-dihydro-8H-imidazo[1,2-a]pyrano[3,4-c]pyridin-7-yl)-1H-pyrrolo[3,4-c]quinoline-8-carbohydrazide

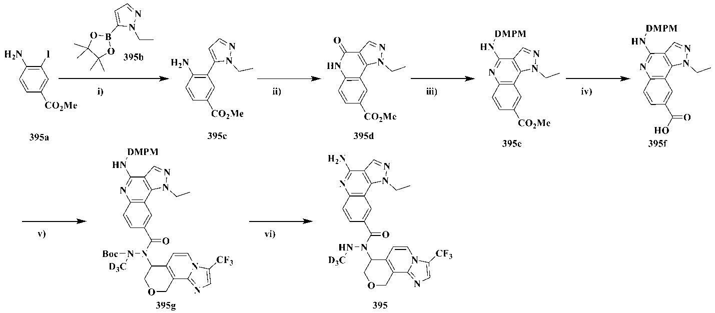

Referring to the method described in Step 1 to Step 6 of **Example 391, Example 395** (70 mg, yield: 10.8%) was obtained from methyl 4-amino-3-iodobenzoate **395a** (2 g, 7.22 mmol). MS m/z (ESI): 528 [M+1]⁺

¹H NMR (400 MHz, DMSO-d₆) δ 13.41 (s, 1H), 8.95 - 8.29 (m, 4H), 8.17 (s, 1H), 7.98 (d, 1H), 7.79 (s, 1H), 7.28 (s, 1H), 5.36 (d, 2H), 5.16 - 4.79 (m, 4H), 4.48 - 4.16 (m, 2H), 1.50 (t, 3H).

### Example 424

### 4-Amino-N-(3-(trifluoromethyl)-7,10-dihydro-8H-imidazo[1,2-a]pyrano[3,4-c]pyridin-7-yl)-1,3-dihydrofuro[3,4-c][1,7]naphthyridine-8-carbohydrazide

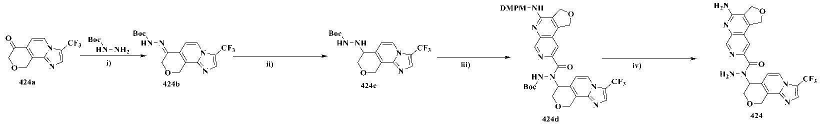

Step 1: A reaction solution of 3-(trifluoromethyl)-8H-imidazo[1,2-a]pyrano[3,4-c]pyridin-7(10H)-one **424a** (500 mg, 1.95 mmol) and acetic acid (117 mg, 1.95 mmol) in methanol (10 mL) and tetrahydrofuran (5 mL) was stirred at room temperature for 16 hours. The reaction solution was concentrated. The organic phase was concentrated, and the residue was purified by silica gel column chromatography to obtain **424b** (600 mg, yield: 91.3%). MS m/z (ESI): 371 [M+1]⁺

Step 2: A reaction solution of **424b** (500 mg, 1.35 mmol) and palladium hydroxide on carbon (500 mg, containing 20% palladium, containing 55% water) in tetrahydrofuran (20 mL) was stirred at room temperature under hydrogen atmosphere for 16 hours. Upon completion of the reaction, the reaction solution was filtered to remove solids. The filtrate was concentrated, and the residue was purified by silica gel column chromatography to obtain **424c** (430 mg, yield: 85.5%). MS m/z (ESI): 373 [M+1]⁺

Referring to the synthesis method described in Step 5 to Step 6 of **Example 391, Example 424** (20 mg, yield: 18.7%) was obtained from **424c** (80 mg, 0.22 mmol). MS m/z (ESI): 486 [M+1]⁺

¹H NMR (400 MHz, CD₃OD) *δ* 9.05 (d, 1H), 8.41 (m, 1H), 8.10 (m, 2H), 7.28 (m, 1H), 5.88 (s, 1H), 5.40 (m, 2H), 5.10 (m, 4H), 4.31 (m, 2H)

### Example 424 Preparation of chiral isomers

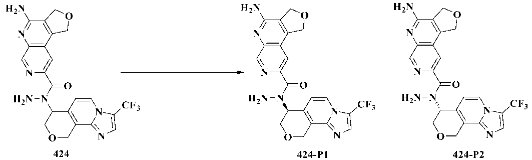

**Example 424** (260 mg, 0.54 mmol) was separated by chiral preparative HPLC (condition 2) to obtain: (S)-4-amino-N-(3-(trifluoromethyl)-7,10-dihydro-8H-imidazo[1,2-a]pyrano[3,4-c]pyridin-7-yl)-1,3-dihydrofuro[3,4-c][1,7]naphthyridine-8-carbohydrazide **424-P1** (80 mg, yield: 90.8%) and (R)-4-amino-N-(3-(trifluoromethyl)-7,10-dihydro-8H-imidazo[1,2-a]pyrano[3,4-c]pyridin-7-yl)-1,3-dihydrofuro[3,4-c][1,7]naphthyridine-8-carbohydrazide **424-P2** (80 mg, yield: 80.8%).

**424-P1 (t_{R}: 1.732 min):** ¹H NMR (400 MHz, CD₃OD) δ 9.05 (d, 1H), 8.41 (m, 1H), 8.10 (m, 2H), 7.28 (m, 1H), 5.88 (s, 1H), 5.40 (m, 2H), 5.10 (m, 4H), 4.31 (m, 2H).

**424-P2 (t_{R}: 2.891 min):** ¹H NMR (400 MHz, CD₃OD) δ 9.05 (d, 1H), 8.41 (m, 1H), 8.10 (m, 2H), 7.28 (m, 1H), 5.88 (s, 1H), 5.40 (m, 2H), 5.10 (m, 4H), 4.31 (m, 2H).

### Example 435

### 4-Amino-7-chloro-1-methyl-N'-(methyl-d3)-N-(3-(trifluoromethyl)-7,10-dihydro-8H-imidazo[1,2-a]pyrano[3,4-c]pyridin-7-yl)-1H-pyrazolo[4,3-c] quinoline-8-carbohydrazide

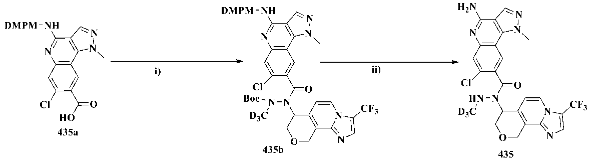

Referring to the synthesis method described in Step 5 to Step 6 of **Example 391, Example 435** (30 mg, yield: 11.7%) was obtained from 7-chloro-4-((3,4-dimethoxybenzyl)amino)-1-methyl-1H-pyrazolo[4,3-c]quinoline-8-carboxylic acid **435a** (200 mg, 0.47 mmol). MS m/z (ESI): 548 [M+1]⁺

¹H NMR (400 MHz, CD₃OD) δ 8.45 (m, 3H), 8.07 (d, 1H), 7.80 (s, 1H), 7.38 (d, 1H), 5.72 (s, 1H), 5.25 (d, 1H), 5.01 (d, 1H), 4.53 (m, 4H), 4.29 (m, 1H)

### Example 436

### 4-Amino-1-methyl-N'-(trideuteriomethyl)-N-(3-(trifluoromethyl-7,10-dihydro-8H-imidazo[1,2-a]pyrano[3,4-c]pyridin-7-yl)-1H-pyrazolo[4,3-c]quinoline-8-carbohydrazide

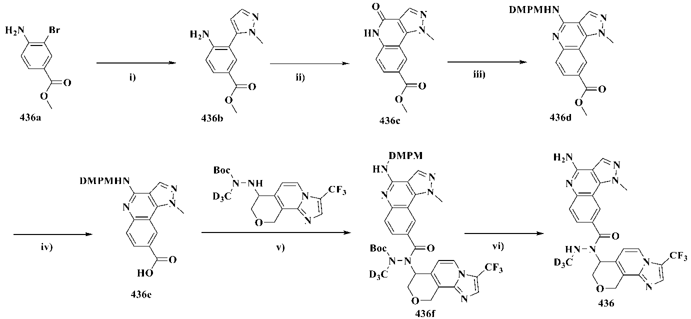

Referring to the method described in Step 1 to Step 3 of **Example 391, 436d** (9.68 g, yield: 68.0%) was obtained from methyl 4-amino-3-bromobenzoate **436a** (8.00 g, 34.96 mmol). MS m/z (ESI):407 [M+1]⁺

Step 4: **436d** (9.68 g, 23.84 mmol) and lithium hydroxide (1.92 g, 80.00 mmol) were dispersed in methanol (80 mL) and water (16 mL). The reaction solution was stirred and reacted at 25°C for 16 hours. The reaction solution was adjusted to neutral pH with hydrochloric acid, resulting in the precipitation of solids, which was filtered to obtain **436e** (7.62 g, yield: 81.5%). MS m/z (ESI): 393 [M+1]⁺

Referring to the method described in Step 3 to Step 4 of **Example 393, Example 436** (80 mg, yield: 3.8%) was obtained from **436e** (1.6 g, 4.08 mmol). MS m/z (ESI): 514 [M+1]⁺

¹H NMR (400 MHz, DMSO-d₆) δ 8.44 (dd, 3H), 8.15 (d, 1H), 7.76 (d, 3H), 7.24 (s, 1H), 5.10 (d, 3H), 4.36 (d, 5H).

### Example 436 Preparation of chiral isomers

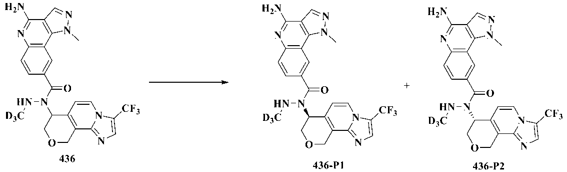

**Example 436** (70 mg, 0.14 mmol) was resolved by Chiral-HPLC (condition 2) to obtain **436-P1:** (*S*)-4-amino-1-methyl-*N'*-(trideuteriomethyl)-*N*-(3-(trifluoromethyl-7,10-dihydro-8*H*-imidazo[1,2-*a*]pyrano[3,4-*c*]pyridin-7-yl)-1*H-*pyrazolo[4,3-c]quinoline-8-carbohydrazide (22 mg, yield: 31.4%) and **436-P2:** (*R*)-4-amino-1-methyl-*N'*-(trideuteriomethyl)-*N*-(3-(trifluoromethyl-7,10-dihydro-8*H-*imidazo[1,2-*a*]pyrano[3,4-*c*]pyridin-7-yl)-1*H*-pyrazolo[4,3-*c*]quinoline-8-carbohydrazide (23 mg, yield: 32.9%), respectively.

**436-P1 (t_{R}: 2.850 min):** ¹H NMR (400 MHz, DMSO-d₆) δ 8.44 (dd, 3H), 8.15 (d, 1H), 7.76 (d, 3H), 7.24 (s, 1H), 5.10 (d, 3H), 4.36 (d, 5H).

**436-P2 (t_{R}: 3.204 min):** ¹H NMR (400 MHz, DMSO-d₆) δ 8.44 (dd, 3H), 8.15 (d, 1H), 7.76 (d, 3H), 7.24 (s, 1H), 5.10 (d, 3H), 4.36 (d, 5H).

### Example 440

### 4-Amino-3-chloro-1-methyl-N'-(methyl-d3)-N-(3-(trifluoromethyl)-7,10-dihydro-8H-imidazo[1,2-a]pyrano[3,4-c]pyridin-7-yl)-1H-pyrazole[4,3-c] quinoline-8-carbohydrazide

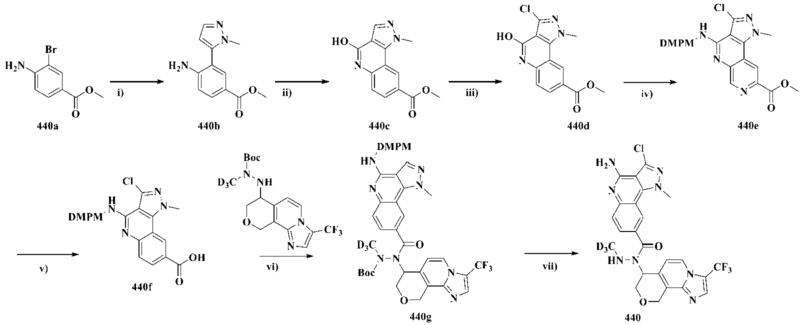

Referring to the method described in Step 1 to Step 2 of **Example 391, 440c** (4.1 g, yield: 73.1%) was obtained from methyl 4-amino-3-bromobenzoate **440a** (5 g, 21.82 mmol). MS m/z (ESI): 258 [M+1]⁺

Step 3: **440c** (900 mg, 3.50 mmol) was dissolved in glacial acetic acid (50 mL), N-chlorosuccinimide (1.40 g, 10.5 mmol) was added to the reaction solution at ambient temperature, and the reaction solution was heated at 80°C and stirred for 16 hours. The reaction solution was cooled to ambient temperature, concentrated under reduced pressure, adjusted to alkaline pH with a saturated sodium bicarbonate solution, and filtered, and the filter residue was washed with clean water (10 ml) and ethyl acetate (10 ml) and oven-dried to obtain **440d** (500 mg, yield: 49.1%). MS m/z (ESI): 292 [M+1]⁺

Referring to the method described in Step 3 to Step 6 of **Example 391, Example 440** (120 mg, yield: 12.8%) was obtained from **440d** (500 mg, 1.71 mmol). MS m/z (ESI): 548 [M+1]⁺

1H NMR (400 MHz, DMSO-d₆) δ 8.54 (s, 1H), 8.47 (s, 1H), 8.39 (d, 1H), 8.11 (d, 1H), 8.07 (d, 1H), 7.28 (d, 1H), 5.39 (s, 1H), 5.01 (q, 2H), 4.46 (s, 3H), 4.30 (tt, 2H)

### Example 444

### 4-Amino-N',1-dimethyl-N-(3-(trifluoromethyl)-7,10-dihydro-8H-imidazo[1,2-a]pyrano[3,4-c]pyridin-7-yl)-1H-pyrazolo[3,4-c][1,7]naphthyridine-8-carbohydrazide

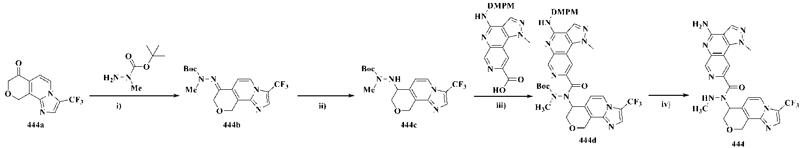

Step 1: 3-(Trifluoromethyl)-8*H*-imidazo[1,2-a]pyrano[3,4-c]pyridin-7(10*R*)-one **444a** (1.50 g, 5.86 mmol) and tert-butyl 1-methylhydrazide-1-carboxylate (1.28 g, 8.78 mmol) were dissolved in methanol (20 mL), acetic acid (703.24 mg, 11.71 mmol) was added, and the mixture was stirred overnight at room temperature. Upon completion of the reaction, the reaction solution was subjected to rotary evaporation to dryness to obtain **444b** (2.82 g). MS m/z (ESI): 285 [M+1]⁺

Step 2: **444b** (2.82 g, 5.86 mmol) was dissolved in methanol (40 mL), acetic acid (23.44 mg, 390.35 µmol), 10% palladium on carbon (623 mg) and palladium hydroxide (822 mg) were added, and the mixture was purged three times with hydrogen and stirred overnight at room temperature under hydrogen atmosphere (15 psi). Upon completion of the reaction, the reaction solution was filtered through Celite and washed with methanol. The filtrate was subjected to rotary evaporation to dryness and column chromatography to obtain **444c** (2.10 g, yield: 92.8%). MS m/z (ESI): 287 [M+1]⁺

Step 3: **444c** (500 mg, 1.27 mmol) and 4-[(2,4-dimethoxyphenyl)methylamino]-1-methyl-1H-pyrazolo[3,4-c][1,7] naphthyridine-8-carboxylic acid (589.28 mg, 1.53 mmol) were dissolved in DCE (20 mL), pyridine (804.28 mg, 10.17 mmol, 819.11 µL) and POCl3 (1.17 g, 7.63 mmol, 712.99 µL) were added, and the mixture was stirred at room temperature for 16 h. Upon completion of the reaction, the reaction system was quenched with a sodium bicarbonate solution, extracted with DCM, dried, and subjected to rotary evaporation to dryness and column chromatography to obtain **444d** (489 mg, yield: 50.5%). MS m/z (ESI): 762 [M+1]⁺

Step 4: **444d** (480 mg, 630.13 µmol) was dissolved in TFA (10 mL), and the mixture was stirred at 80°C for 2 h. Upon completion of the reaction as monitored by LCMS, the reaction solution was subjected to rotary evaporation to dryness to obtain a crude product, which was purified by preparative liquid chromatography to obtain **444** (188.8 mg, yield: 58.6%). MS m/z (ESI):512 [M+1]⁺

¹H NMR (400 MHz, DMSO-d₆) δ 8.96 (s, 1H), 8.41 - 8.18 (m, 2 H), 7.98 (br s, 1H), 7.79 (d, 1 H), 7.62 - 7.44 (m, 1 H), 7.44 - 7.08 (m, 1 H), 5.16 (br d, 1 H), 4.41 (s, 3H), 4.54 - 4.32 (m, 1 H), 4.02 - 3.78 (m, 1 H), 3.67 - 3.36 (m, 2H), 3.31 - 3.27 (m, 3H).

### Example 448

### 4-Amino-N',1-dimethyl-N-(2-pentafluorosulphanyl)-5,8-dihydro-6H-pyrano [3,4-b]pyridin-5-yl)-1H-pyrazoline[4,3-c] quinoline-8-carbohydrazide

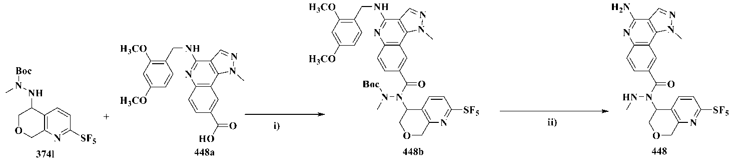

Step 1: tert-Butyl 1-methyl-2-(2-pentafluorosulphanyl)-5,8-dihydro-6H-pyrano[3,4-b]pyridin-5-yl)hydrazide-1-carbonate **374l** (50 mg, 0.123 mmol), 4-((2,4-dimethoxyphenyl)amino)pyrazole[1,5-a]pyrazoline[3,4-e]pyrazoline-8-carboxylic acid **448a** (48 mg, 0.123 mmol), pyridine (78 mg, 0.98 mmol) and phosphorus oxychloride (113 mg, 0.74 mmol) were dissolved in N,N-dimethylacetamide (3 mL), and the reaction system was reacted at 20°C for 16 hours. The reaction solution was first quenched with ammonium chloride, and water (10 mL) and ethyl acetate (100 mL) were then added to the mixed solution. The organic phase was washed with a saturated sodium chloride aqueous solution (10 mL × 3), then dried and subjected to rotary evaporation to dryness. The residue was purified by reversed-phase column chromatography using eluent system A to obtain **448b** (61 mg, yield: 63%). MS m/z (ESI): 780 [M+1]⁺

Step 2: **448b** (60 mg, 0.077 mmol) was dissolved in trifluoroacetic acid (4 mL), and the mixture was purged three times with nitrogen. The reaction system was reacted at 90°C for 0.5 hours. The resulting reaction system was concentrated, and the residue was purified by silica gel column chromatography using eluent system A to obtain **Example 448** (30 mg, yield: 73%). MS m/z (ESI): 530 [M+1]⁺

¹HNMR (400 MHz, DMSO-d₆) δ 8.51 (s, 1H), 8.43 (s, 1H), 8.22 (m, 1H), 7.89 (m, 2H), 7.69 (m, 1H), 5.46 - 5.24 (m, 2H), 4.82 (m, 2H), 4.44 (s, 3H), 4.30 (m, 1H), 4.21 (s, 1H), 3.17 (s, 3H).

### Example 449

### 4-Amino-7-chloro-N',1-dimethyl-N-(6-(pentafluorosulphanyl)-2,3-dihydrobenzofuran-3-yl)-1H-pyrazolo[4,3-c]quinoline-8-carbohydrazide

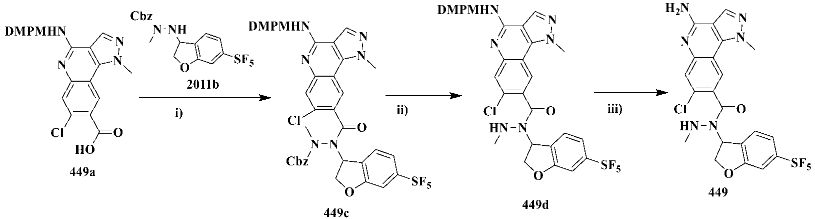

Referring to the synthesis method described in Step 10 to Step 12 of **Example 372, Example 449** (9 mg, yield: 7.0%) was obtained from 7-chloro-4-((3,4-dimethoxybenzyl)amino)-1-methyl-1*H*-pyrazolo[4,3-c]quinoline-8-carboxylic acid **449a** (100 mg, 0.25 mmol) and benzyl 1-methyl-2-(6-(pentafluorosulphanyl)-2,3-dihydrobenzofuran-3-yl)hydrazide-1-carboxylate **449b** (120 mg, 0.28 mmol). MS m/z (ESI): 549 [M+1]⁺

¹H NMR (400 MHz, DMSO-*d6*) δ 8.30 (d, 2H), 8.09 - 7.53 (m, 4H), 7.46 (s, 1H), 7.35 (s, 1H), 6.00 (s, 1H), 5.34 (d, 1H), 4.78 (d, 2H), 4.43 (s, 3H), 2.30 (d, 3H).

### Example 450

### 4-Amino-N',1-dimethyl-N-(6-(pentafluoro-6-sulphanyl)-2,3-dihydrobenzofuran-3-yl)-1H-pyrazolo[4,3-c]quinoline-8-carbohydrazide

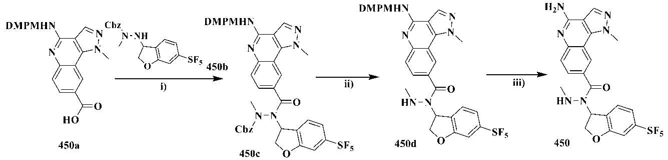

Referring to the synthesis method described in Step 10 to Step 12 of **Example 372, Example 450** (8 mg, yield: 6.2%) was obtained from 4-(3,4-dimethoxybenzyl)amino)-1-methyl-1H-pyrazolo[4,3-c]quinoline-8-carboxylic acid **450a** (100 mg, 0.25 mmol) and benzyl 1-methyl-2-(6-(pentafluoro-6-sulphanyl)-2,3-dihydrobenzofuran-3-yl)hydrazide-1-carboxylate **450b** (120 mg, 0.28 mmol). MS m/z (ESI): 515 [M+1]⁺

¹H NMR (400 MHz, DMSO-d6) δ 13.27 (s, 1H), 8.51 (s, 2H), 7.90 (s, 1H), 7.83 - 7.61 (m, 2H), 7.44 (dd, 1H), 7.37 (d, 1H), 5.84 (s, 1H), 5.38 (s, 1H), 4.80 (s, 2H), 4.48 (s, 3H), 2.37 (d, 3H).

### Example 451

### 4-Amino-N',1,7-trimethyl-N-(6-(pentafluorosulphanyl)-2,3-dihydrobenzofuran-3-yl)-1H-pyrazolo[4,3-c]quinoline-8carbohydrazide

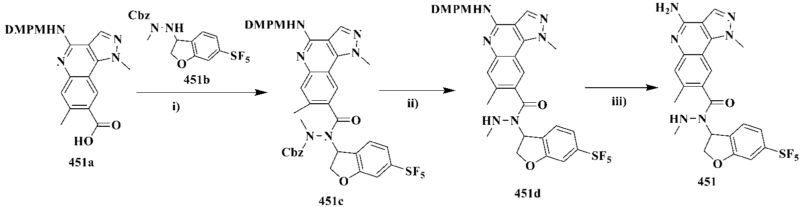

Referring to the synthesis method described in Step 10 to Step 12 of **Example 372, Example 451** (8 mg, yield: 6.1%) was obtained from 4-((3,4-dimethoxybenzyl)amino)-1,7-dimethyl-1*H*-pyrazolo[4,3-c]quinoline-8-carboxylic acid **451a** (100 mg, 0.25 mmol) and benzyl 1-methyl-2-(6-(pentafluorosulphanyl)-2,3-dihydrobenzofuran-3-yl)hydrazide-1-carboxylate **451b** (120 mg, 0.28 mmol). MS m/z (ESI): 529 [M+1]⁺

¹H NMR (400 MHz, DMSO-*d6*) δ 8.51 (s, 1H), 8.17 (s, 1H), 7.59 (s, 2H), 7.44 (d, 1H), 7.30 (s, 2H), 5.87 - 5.42 (m, 3H), 5.24 (s, 2H), 4.85 (s, 3H), 2.47 (s, 3H), 2.34 (s, 3H).

### Example 453

### 2-Amino-3-cyclopropyl-N'-methyl-N-(2-(trifluoromethyl)-5,8-dihydro-6H-pyrano[3,4-b]pyridin-5-yl)-1,7-naphthyridine-6-carbohydrazide

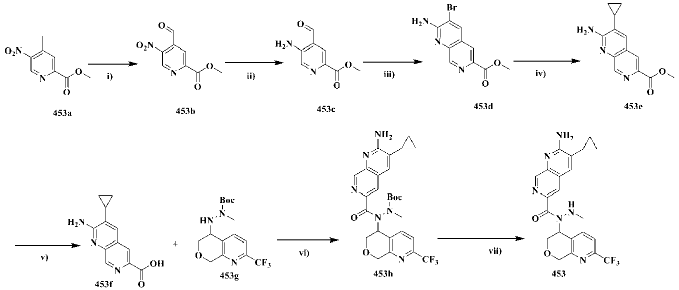

Step 1: Methyl 4-formyl-5-nitropyridine-2-carboxylate **453a** (2.30 g, 11.73 mmol) and SeO₂ (5.20 g, 46.90 mmol) were dissolved in dioxane (23 mL), and the reaction system was reacted at 110°C for 16 hours. The resulting reaction system was concentrated. The residue was purified by reversed-phase column chromatography using eluent system A to obtain **453b** (1.67 g, yield: 67%). MS m/z (ESI): 211 [M+1]⁺

Step 2: **453b** (4.00 g, 19.03 mmol) and Fe (10.63 g, 190.35 mmol) were dissolved in acetic acid (100 mL), and the reaction system was reacted at 50°C under nitrogen atmosphere for 1 hour. The reaction solution was cooled and filtered, and the filtrate was concentrated to dryness. Ethyl acetate was added at 0°C, and the resulting mixture was adjusted to pH 8 with a saturated sodium bicarbonate solution and extracted. The organic phases were combined, dried over anhydrous sodium sulphate, filtered and concentrated to obtain a crude product, which was slurried with THF to obtain **453c** (1.5 g, yield: 43%). MS m/z (ESI):181 [M+1]⁺

Step 3: **453c** (1.5 g, 8.33 mmol), NaH (500 mg, 12.49 mmol) and diethyl 1-bromo-1-cyanomethyl phosphate (4.26 g, 16.65 mmol) were dissolved in N,N-dimethylformamide (45 mL), and the mixture was reacted at 0°C for 2 hours. The system was filtered and concentrated. The residue was purified by reversed-phase column chromatography using eluent system A to obtain **453d** (2.3 g, yield: 97%). MS m/z (ESI): 283 [M+1]⁺

Step 4: **453d** (1.60 g, 5.67 mmol), cyclopropylboronic acid (975 mg, 11.34 mmol), 1,1'-bis(diphenylphosphino)ferrocene palladium(II) dichloride (415 mg, 0.56 mmol) and K₂CO₃ (2.35 g, 17.02 mmol) were dissolved in ethylene glycol dimethyl ether (20 mL), and the reaction system was reacted at 85°C for 3 hours, concentrated, filtered, and then quenched with ammonium chloride. Water (50 mL) and ethyl acetate (500 mL) were then added to the mixed solution, and the organic phase was washed with a saturated sodium chloride aqueous solution (20 mL × 3), then dried and subjected to rotary evaporation to dryness to obtain **453e** (1.9 g, crude), which was used directly in the next step without purification. MS m/z (ESI): 244 [M+1]⁺

Step 5: **453e** (400 mg, 1.64 mmol) and LiOH (138 mg, 3.29 mmol) were dissolved in THF (4 mL), MeOH (4 mL) and H₂O (4 mL), and the reaction system was reacted at 20°C for 1 hour, then adjusted to pH 3-4 with 1 M hydrochloric acid, and filtered to obtain **453f** (60 mg, yield: 15%). MS m/z (ESI): 230 [M+1]⁺

Step 6: **453f** (60 mg, 0.26 mmol), tert-butyl 1-methyl-2-(2-(trifluoromethyl)-5,8-dihydro-6H-pyrano[3,4-b]pyridin-5-yl)hydrazide-1-carbonate **453g** (136 mg, 0.39 mmol), pyridine (166 mg, 2.09 mmol) and phosphorus oxychloride (400 mg, 2.62 mmol) were dissolved in N,N-dimethylacetamide (3 mL), and the mixture was reacted at 20°C for 16 hours. A saturated sodium bicarbonate solution (10 mL) was added to the reaction solution, resulting in the precipitation of a small amount of solids. The solids were filtered, collected and washed with water to obtain **453h** (100 mg, crude), which was used directly in the next step without purification. MS m/z (ESI): 559 [M+1]⁺

Step 7: **453h** (100 mg, 0.18 mmol) was dissolved in dichloromethane (2 mL) and trifluoroacetic acid (0.4 mL), and the reaction system was reacted at 20°C for 1 hour. The resulting reaction system was concentrated, and the residue was purified by high-performance liquid chromatography using eluent system A to obtain **Example 453** (27.4 mg, yield: 33%). MS m/z (ESI): 459 [M+1]⁺

¹HNMR (400 MHz, DMSO-d₆) δ 8.81 (s, 1H), 8.14 (s, 1H), 8.01 (m, 1H), 7.91 (s, 1H), 7.84 (m, 1H), 7.55 (s, 2H), 5.63 (s, 1H), 4.83 (m, 1H), 4.79 - 4.67 (m, 1H), 4.25 (m, 1H), 4.14 - 4.01 (m, 1H), 2.47 (s, 1H), 2.18 (s, 1H), 2.04 - 1.86 (m, 2H), 1.09 - 1.02 (m, 2H), 0.76 (m, 2H).

### Biological assay and evaluation

The present invention will be further described and explained below in conjunction with test examples, but these examples are not meant to limit the scope of the present invention.

### Test Example 1: Determination of the ability of the compounds of the present invention to improve the thermal stability (melting temperature) of the PRMT5/MEP50-MTA protein complex.

1. Experimental objective: The objective of this test example is to determine the ability of the compound to improve the thermal stability of the PRMT5/MEP50-MTA protein complex, resulting in an increase in the protein melting temperature.
2. Experimental instruments: Quantitative PCR instrument (Quantstudio6 Flex) was purchased from Life Technologies; pipettes were purchased from Eppendorf or Rainin.
3. Experimental reagents and consumables: PRMT5/MEP50 protein was prepared by Viva Biotech; HEPES was purchased from Thermo Fisher Scientific (Cat. No. 15630080); DTT was purchased from Sigma (Cat. No. 43816-10ml); sodium chloride was purchased from Invitrogen (Cat. No. AM9760G); Protein Thermal Shift^{™} Dye Kit was purchased from Thermo Fisher Scientific (Cat. No. 4461146); methylthioadenosine (MTA) was purchased from Sigma (Cat. No. D5011).
4. Experimental methods: In this experiment, the thermal shift method was used to measure the change in the melting temperature (Tm) of the PRMT5/MEP50-MTA protein complex before and after compound binding, thereby characterising the ability of the compound to improve the thermal stability of the PRMT5/MEP50-MTA protein complex. A solution containing 50 mM HEPES, 10 mM DTT, 2 µM MTA, SYPRO Orange and 250 mM NaCl was prepared as the experimental buffer. Human PRMT5/MEP50 protein was added to a final concentration of 2 µM, and the mixture was incubated at room temperature for 30 minutes. The above reaction mixture was aliquoted into 8-strip PCR tubes at 19.5 µL per tube. Then 0.5 µL of test compound or DMSO was added to each tube, providing a total reaction volume of 20 µL and a final compound concentration of 8 µM. 2.5% DMSO was set as the vehicle control. After incubation at room temperature for 10 minutes, the PCR tubes were placed in a PCR instrument, and the melt curve function was selected to measure the melting temperature of the PRMT5/MEP50-MTA protein complex in different treatment groups (heated from 25°C to 95°C, 0.03°C/s).
5. Experimental data processing method: The experimental data files from the PCR instrument were imported into the thermal shift software to obtain the melting temperature (Tm) for each treatment group. The Tm of the DMSO vehicle control group was subtracted to yield the change in melting temperature (ΔTm) as shown in the table below:

| **Example** | **ΔTm (°C)** | **Example** | **ΔTm (°C)** |
|---|---|---|---|
| **37** | 11.33 | **322** | 10.72 |
| **37-P1** | 10.51 | **324** | 11.05 |
| **39** | 11.73 | **354** | 10.77 |
| **39-P1** | 12.45 | **354-P1** | 11.61 |
| **48** | 11.73 | **357** | 12.18 |
| **62** | 12.46 | **357-P1** | 12.45 |
| **62-P1** | 12.46 | **362** | 11.19 |
| **68** | 10.37 | **362-P1** | 12.03 |
| **81** | 10.36 | **371** | 10.63 |
| **83** | 11.76 | **371-P1** | 11.61 |
| **144** | 10.22 | **351** | 12.59 |
| **146** | 10.21 | **372** | 11.20 |
| **234** | 10.91 | **372-P1** | 11.61 |
| **240** | 10.76 | **374** | 12.32 |
| **276** | 10.36 | **375** | 12.73 |
| **277** | 12.87 | **375-P1** | 13.02 |
| **278** | 12.73 | **385** | 11.62 |
| **278-P1** | 12.59 | **385-P1** | 13.99 |
| **279** | 12.17 | **385-P2** | 12.31 |
| **280** | 10.92 | **387** | 12.60 |
| **281** | 12.70 | **391** | 12.73 |
| **282** | 12.30 | **391-P1** | 13.02 |
| **285** | 11.61 | **392** | 10.36 |
| **286** | 10.49 | **394** | 13.16 |
| **286-P1** | 11.05 | **435** | 13.70 |
| **298** | 11.42 | **436** | 11.47 |
| **301** | 11.59 | **436-P1** | 11.89 |
| **306** | 12.73 | **444** | 12.73 |
| **307** | 12.73 | **448** | 12.31 |
| **317** | 11.61 | **450** | 12.03 |
| **321** | 10.89 | **451** | 13.71 |

6 Experimental conclusion: The compounds of the present invention exhibit good binding ability to the PRMT5/MEP50-MTA protein complex.

### Test Example 2: Determination of the inhibitory effect of the compounds of the present invention on the proliferation activity of HCT116 and MTAP Knockout HCT116 cells.

1. Experimental objective: to determine the inhibitory effect of the compound on the proliferation activity of HCT116 wild-type and HCT116 MTAP knockout cells.
2. Experimental instruments: Centrifuge (Eppendorf 5810R); microplate reader (BioTek Synergy H1 or PerkinElmer Envision); pipette (Eppendorf or Rainin).
3. Experimental reagents: HCT116 and MTAP Knockout HCT116 cells were purchased from Nanjing Cobioer; Cell Titer-Glo was purchased from Promega (Cat. No. G7573); McCoy' 5A was purchased from Gibco (Cat. No. 12330031); FBS was purchased from Gibco (Cat. No. 10091148); PBS was purchased from Gibco (Cat. No. 10010023); trypsin was purchased from Gibco (Cat. No. 25200056); cell culture plates were purchased from Corning (Cat. No. 3610).
4. Experimental methods: HCT116 and MTAP Knockout HCT116 cells were cultured in McCoy'5A medium containing 10% FBS until an appropriate cell density was reached. The cells were then collected and adjusted to a suitable concentration using complete medium. The cell suspension was seeded into a 96-well plate at 90 µL per well and allowed to adhere overnight in a 37°C, 5% CO₂ incubator. Compound solutions of various concentrations were prepared using DMSO and culture medium, with a vehicle control set. The compound solutions were added to the 96-well plate at 10 µL per well, and the plate was incubated in a 37°C, 5% CO₂ incubator for an additional 72-240 hours. After incubation, CellTiter-Glo solution was added, and the mixture was mixed uniformly by shaking, followed by incubation in the dark for 10 to 30 minutes. Luminescence was measured using a Synergy H1 or Envision microplate reader.
5. Experimental data processing method: The inhibition rate was calculated using the luminescence signal values, and the concentrations along with the inhibition rates were subjected to non-linear regression curve fitting using Graphpad Prism software to obtain the IC₅₀ values.

| **Example** | **MTAP Knockout** | | **Selectivity** |
|---|---|---|---|
| | **HCT116** | **HCT116** | |
| | **IC₅₀ nM** | **IC₅₀ nM** | |
| **37** | 9.2 | 1518 | 166 |
| **39-P1** | 13 | 1387 | 108 |
| **62** | 7.2 | 808 | 113 |
| **83** | 4.8 | 790 | 164 |
| **277** | 12 | 207 | 17 |
| **282** | 17 | 2449 | 145 |
| **285** | 10 | 1148 | 120 |
| **286-P1** | 23 | 2679 | 118 |
| **301** | 14 | 1357 | 94 |
| **307** | 15 | 1059 | 73 |
| **317** | 3.9 | 508 | 130 |
| **324** | 15 | 2377 | 155 |
| **354-P1** | 23 | 2474 | 107 |
| **357** | 7.6 | 760 | 100 |
| **362-P1** | 6.7 | 450 | 68 |
| **371-P1** | 8.5 | 1035 | 121 |
| **372-P1** | 12 | 819 | 69 |
| **374** | 13 | 303 | 23 |
| **375-P1** | 7.9 | 133 | 17 |
| **391** | 13 | 358 | 28 |
| **391-P1** | 8.8 | 121 | 14 |
| **435** | 7.1 | 31 | 44 |
| **436** | 8.9 | 1644 | 185 |
| **448** | 7.8 | 344 | 44 |
| **451** | 4.2 | 89 | 21 |

6. Experimental conclusion: The compounds of the present invention exhibit good proliferation inhibitory activity against HCT116 MTAP knockout cells, and good selectivity against HCT116 wild-type cells.

### Test Example 3: Determination of the pharmacokinetics of the compounds of the present invention in mice (plasma) after oral administration.

1. Study objective: Balb/c mice were used as test animals to study the pharmacokinetic behaviour of the compounds in mice (plasma) after oral administration.
2. Test scheme
2.1 Test drugs: The example compounds of the present invention, made in house.
2.2 Test animals: Balb/c mice, male, purchased from Shanghai Jiesijie Experimental Animal Co., Ltd., with animal production license number (SCXK (Shanghai) 2013-0006, No.311620400001794).
2.3 Drug formulation: Formulation of drug for oral administration: 0.5% CMC-Na (1% Tween 80).
5 g of hydroxyethyl cellulose (HEC, CMC-Na, viscosity: 800-1200 Cps) was weighed and dissolved in 1000 mL of purified water, and 10 g of Tween 80 was added. These materials were uniformly mixed to form a clear solution.
4 mg of the example compound was weighed and dissolved in the above solution. The mixture was shaken well, homogenized using a cell disruptor for 1 min, and ultrasonicated for 10 minutes to obtain a suspension with a concentration of 3 mg/mL.

The example compound was weighed, and 5% DMSO was added based on the total administration volume; the mixture was vortexed and ultrasonicated for 2 min to ensure complete dissolution; then, 10% Solutol HS15 was added, and the mixture was vortexed and ultrasonicated for 2 min to achieve complete dissolution; finally, 85% PBS was added, and the mixture was vortexed and ultrasonicated for 5 min; the resulting mixture was filtered through a 0.22 um filter membrane to obtain a colourless, transparent, and clear solution with a concentration of 0.2 mg/mL.

2.4 Administration: 3 Balb/c mice, male, were fasted overnight and then respectively administered PO at a dose of 30 mg/kg and a volume of 10 mL/kg.

2.5 Sample collection: 0.04 mL of blood was collected from the orbit at 0.083 h, 0.25 h, 0.5 h, 1 h, 2 h, 4 h, 6 h, 8 h, and 24 h after administration and placed in EDTA-2K test tubes, and the test tubes were centrifuged at 6,000 rpm for 6 min at 4°C to separate the plasma, which was stored at -20°C. The animals were fed 4 h after administration.

3. Determination results: The final determination results were obtained using the LCMS/MS method, which showed that the example compounds exhibited high oral exposure.

### Test Example 4: Determination of the pharmacokinetics of the compounds of the present invention in rats (plasma) after oral administration.

1. Study objective: SD rats were used as test animals to study the pharmacokinetic behaviour of the following example compounds in rats (plasma) after oral administration at a dose of 5 mg/kg.
2. Test scheme
2.1 Test drugs: Vehicle formula: 0.5% CMC-Na (1% Tween 80); the examples of the present invention, prepared in house.
2.2 Test animals: 3 SD rats per group, male, purchased from Shanghai Bikai Keyi Biotechnology Co., Ltd., with animal license number (SCXK (Shanghai) 2018-0006, No.20180006037467).
2.3 Administration: 3 SD rats per group, male, were fasted overnight and then respectively administered PO at a dose of 5 mg/kg and a volume of 10 mL/kg.
2.4 Sample collection: 0.2 mL of blood was collected from the jugular vein of rats before administration and at 0, 0.25, 0.5, 1, 2, 4, 6, 8 and 24 h after administration, and placed in EDTA-K₂test tubes, and the test tubes were centrifuged at 6,000 rpm at 4°C for 6 min to separate the plasma, which was stored at -80°C. The animals were fed 4 h after administration.
2.5 Sample treatment
1) 160 uL of acetonitrile was added to 40 uL of plasma sample for precipitation, and after mixing, the mixture was centrifuged at 3,500 × g for 5-20 minutes.
2) 100 uL of the treated supernatant solution was taken for LC/MS/MS analysis to determine the concentration of the test compounds.

2.6 Liquid phase analysis
• Liquid phase conditions: Shimadzu LC-20AD pump;
• Mass spectrometry conditions: AB Sciex API 4000 mass spectrometer;
• Chromatographic column: phenomenex Gemiu 5 um C18 50 × 4.6 mm;
• Mobile phase: solution A: 0.1% formic acid in water, solution B: methanol;
• flow rate: 1.0 mL/min; Elution time: 0-4.0 min, with eluents as follows:

| Time/min | Solution A | Solution B |
|---|---|---|
| 0.01 | 90% | 10% |
| 0.5 | 90% | 10% |
| 0.8 | 5% | 95% |
| 2.4 | 5% | 95% |
| 2.5 | 90% | 10% |
| 4.0 | Stop | |

3. Experimental results and analysis: The main pharmacokinetic parameters were calculated using WinNonlin 8.2. The results of pharmacokinetic experiments in rats are as shown in the table below:

| **Example No.** | Pharmacokinetic experiments in rats (p.o. 5 mg/kg) | | | | |
|---|---|---|---|---|---|
| | tₘₐₓ (h) | Cₘₐₓ (ng/mL) | AUC _{0-∞}(ng/mL*h) | t_{1/2} (h) | MRT_{0-∞} (h) |
| **62-P1** | 0.5 | 441 | 1358 | 4.1 | 4.0 |
| **279** | 0.5 | 233 | 729 | 1.0 | 2.1 |
| **285** | 0.8 | 999 | 4983 | 4.2 | 4.8 |
| **286-P1** | 0.5 | 480 | 1283 | 2.1 | 2.5 |
| **317** | 1.0 | 240 | 739 | 1.9 | 2.8 |
| **357** | 0.7 | 360 | 1079 | 1.2 | 2.2 |
| **362-P1** | 0.7 | 2753 | 8184 | 2.7 | 2.8 |
| **371** | 0.5 | 1053 | 3728 | 4.4 | 4.4 |
| **371-P1** | 0.8 | 1603 | 4797 | 1.6 | 2.5 |
| **351** | 0.5 | 2333 | 9656 | 2.3 | 3.3 |
| **372-P1** | 2.7 | 378 | 2568 | 2.7 | 4.8 |
| **374** | 0.5 | 652 | 1180 | 1.9 | 1.9 |
| **375-P1** | 1.3 | 287 | 2131 | 3.3 | 5.0 |
| **391** | 1.5 | 1693 | 11895 | 4.4 | 5.8 |
| **424** | 2.7 | 226 | 2385 | 6.1 | 8.9 |
| **436** | 3.3 | 143 | 2032 | 10.8 | 14.9 |

4 Experimental conclusion: After oral administration at a dose of 5 mg/kg, the example compounds of the present invention exhibit favourable metabolic properties, with good exposure (AUC) and maximum plasma concentration (Cₘₐₓ).

### Test Example 5: hERG potassium channel inhibitory activity assay

1. Study objective: to determine the inhibitory effect of the compound on the hERG potassium channel activity.
2. Experimental instruments and reagents:
2.1 Reagents

| Reagent name | Manufacturer | Cat. No. | Specification (mL) |
|---|---|---|---|
| Hams'F-12 nutrient mixture, GlutaMAXTM supplement | ThermoFisher | 31765-092 | 10 x 500 |
| Fetal bovine serum (FBS) | ThermoFisher | 10099-141 | 500 |
| GeneticinTM selective antibiotic (G418 sulphate) (50 mg/mL) | ThermoFisher | 10131027 | 100 |
| Hygromycin B (50 mg/mL) | ThermoFisher | 10687010 | 20 |
| TrypLE recombinant trypsin | ThermoFisher | 12605036 | 100 |

2.2. Instruments and consumables

| Name | Model | Manufacturer |
|---|---|---|
| pH meter | PB-21 | Sartorius |
| Osmometer | VAPRO 5520 | WESCOR |
| Amplifier | EPC10 | HEKA Elektronik |
| Motorized micromanipulator | MP-225 | SUTTER |
| Inverted microscope | ECLIPSE-Ti | Nikon |
| Electrode puller | PC-10 | NARISHIGE |
| Glass microelectrode | GC150tF-10 | Harvard Apparatus |
| 35 mm culture dish | 430165 | Corning |

3. Experimental methods
3.1 Cell culture: The CHO cells stably expressing the hERG potassium channel (CHO-hERG) used in the experiment were obtained from Sophion Bioscience (Ballerup, Denmark), and passaged and cryopreserved at WuXi AppTec Co., Ltd. in Shanghai. The CHO-hERG cells were cultured and passaged in Ham's F-12 medium containing 1 × GlutaMAX, 10% fetal bovine serum, 100 µg/mL G418 and 100 µg/mL hygromycin B at 37°C with 5% CO₂.
3.2 Preparation of intracellular and extracellular solutions: The extracellular solution was prepared once a month, and aliquoted and stored in 1 L storage bottles. The intracellular solution was prepared once every three months, and aliquoted and cryopreserved at -20°C. The intracellular solution was thawed in a 37°C water bath before the experiment and then placed in an ice bath for later use.
3.3 Preparation of compounds: The compounds were dissolved in 100% DMSO (Sigma-Aldrich, D2650) to prepare 10 mM or 30 mM stock solutions. Before the experiment, the stock solutions of the test compounds were diluted with DMSO to prepare solutions 1000-fold or 333-fold higher than the desired test concentrations, and then further diluted 1000-fold or 333-fold with the extracellular solution to achieve the desired concentrations.
3.4 Cell preparation: The CHO-hERG cells used in the experiment were cultured for at least two days. When the cell density reached more than 75%, the cells were digested with TrypLE, then resuspended in the extracellular solution, and centrifuged. The supernatant was discarded, and then the cells were further resuspended in 2 mL of extracellular solution for later use.

4. Experimental data processing: In data processing, the peak tail current was baseline-corrected when evaluating the hERG blocking effect. The effect of each compound at various concentrations was expressed as the inhibition rate of the tail current. IC₅₀ values were obtained by fitting the data to the Hill equation:$I / Icontrol = Bottom + \left(Top-Bottom\right) / \left(1+{10}^{∧}\left(\left({LogIC}_{50}-X\right)*HillSlope\right)\right)$
Top: maximum effect; Bottom: minimum effect; Hillslope: slope; X: concentration of the test compound.
IC₅₀: half-maximal inhibitory concentration of the test compound on hERG. If the inhibition rate at the lowest concentration exceeded 50%, or the inhibition rate at the highest concentration failed to reach 50%, the corresponding IC₅₀ of the compound would be lower than the lowest concentration or greater than the highest concentration, respectively.

5. Experimental conclusion: The advantageous compounds of the present invention exhibit inhibitory activity (IC₅₀) against the cardiac hERG potassium channel of greater than 5 µM, or even greater than 15 µM, and in particular, the inhibitory activity (IC₅₀) of the compounds in **Examples 276, 286-P1, 321, 362-P1, 371, 374, 391 and 391-P1** is greater than 30 µM, thereby avoiding cardiotoxic side effects at high doses.

### Test Example 6: In vivo pharmacodynamic study of the compounds of the present invention in subcutaneous graft tumour model of human lung cancer cell line LU99 in nude mice

1. Study objective: To evaluate the in vivo efficacy of the compounds in a subcutaneous graft tumour model of human lung cancer cell line LU99 in nude mice.
2. Experimental instruments and reagents
   2.1 Instruments: Refrigerator (BCD-268TN, Haier); biosafety cabinet (BSC-1300II A2, Medical Equipment Factory, Shanghai Boxun Industrial Co., Ltd.); ultraclean workbench (CJ-2F, Suzhou Fengshi Experimental Animal Equipment Co., Ltd.); electric pipette-aid (Easypet 3, Eppendorf); thermostatic water bath (HWS-12, Shanghai Yiheng Scientific Instruments Co., Ltd.); CO₂ incubator (Thermo-311, Thermo); centrifuge (Centrifuge 5720R, Eppendorf); automated cell counter (Countess II, Life Technologies); vernier calliper (CD-6"AX, Mitutoyo, Japan); cell culture flask (T25/T75/T225, Corning); electronic balance (CPA2202S, Sartorius); electronic balance (BSA2202S-CW, Sartorius); ultrasonic cleaner (115F0032, Shanghai Kudos); water purification system (Pacific TII, Thermo); magnetic stirrer (08-2G, Chijiu).
   2.2 Reagents: RPMI-1640 medium (22400-089, Gibco); foetal bovine serum (FBS) (A5669701, Gibco); phosphate buffer solution (PBS) (10010-023, Gibco); Matrigel (356234, Corning); sodium carboxymethylcellulose (30036365, Sinopharm Chemical Reagent Co., Ltd.); Tween-80 (30189828, Sinopharm Chemical Reagent Co., Ltd.).
3. Experimental operations and data processing
   3.1 Animals: BALB/c nude mice, 6-8 weeks old, female, purchased from Beijing Vital River Laboratory Animal Technology Co., Ltd.
   3.2 Cell culture and cell suspension preparation
      a) A vial of LU99 cells was taken from a cell bank and thawed using RPMI-1640 medium (RPMI-1640 + 10% FBS), and the thawed cells were placed in a cell culture flask and cultured in a CO₂ incubator (incubator temperature at 37°C, and CO₂ concentration at 5%).
      b) The cells were passaged every three days, and after passage, the cells were further cultured in the CO₂ incubator. This process was repeated until the cell number met the requirements for *in vivo* efficacy.
      c) The cells in the exponential growth phase were collected, counted using an automated cell counter, and resuspended in PBS based on the counting results. The resuspended cells were mixed with Matrigel at a 1 : 1 ratio to achieve a concentration of 5 × 10⁷ cells/mL, and then placed in an ice box for later use.
   3.3 Cell inoculation
      a) Before inoculation, the nude mice were marked with disposable universal ear tags for mice and rats.
      b) At the time of inoculation, the cell suspension was mixed uniformly, 0.1-1 mL of the cell suspension was drawn into a 1 mL syringe with air bubbles expelled, and the syringe was then placed on an ice pack for later use.
      c) The nude mouse was restrained with the left hand, and the inoculation site (the right dorsal side near the right shoulder) was disinfected with a 75% alcohol cotton swab; inoculation was performed 30 seconds later.
      d) The experimental nude mice were inoculated sequentially (0.1 mL of cell suspension per mouse).
   3.4 Tumour volume measurement, grouping and administration in tumour-bearing mice
      a) Based on tumour growth, on days 9-12 post-inoculation, tumour volumes were measured and tumour sizes were calculated. Tumour volume calculation: $tumour volume \left({mm}^{3}\right) = length \left(mm\right) \times width \left(mm\right) \times width \left(mm\right) / 2 .$
      b) Based on the body weight and tumour size of the tumour-bearing mice, the mice were randomly assigned to groups.
      c) Based on the grouping results, administration of the test drug was started (mode of administration: oral administration; administration volume: 10 mL/kg; administration frequency: once/day or twice/day; administration cycle: 28 days; vehicle: 0.5% CMC-Na (1% Tween 80)).
      d) After the start of administration, tumour volume and body weight were measured twice a week.
      e) The animals were euthanized at the end of the experiment.
      f) The data was processed with software such as Excel. Calculation of tumour growth inhibition (TGI) rate (%) for the compound: When there is no tumour regression, TGI (%) = [1 - (average tumour volume at the end of administration in a treatment group - average tumour volume at the start of administration in the treatment group) / (average tumour volume at the end of treatment in the solvent control group - average tumour volume at the start of treatment in the solvent control group)] × 100%. When there is tumour regression, TGI (%) = [1 - (average tumour volume at the end of administration in a treatment group - average tumour volume at the start of administration in the treatment group) / average tumour volume at the start of administration in the treatment group] × 100%.
4. Experimental conclusion: The advantageous compounds of the present invention exhibit a tumour growth inhibition (TGI) rate of greater than 80%, even greater than 90%, at low doses, demonstrating significant tumour growth inhibitory effects. The compounds exhibit a tumour growth inhibition (TGI) rate of greater than 100%, even greater than 150%, at high doses, demonstrating significant tumour regression effects.

## Claims

1. A compound represented by general formula (I), or a stereoisomer or a pharmaceutically acceptable salt thereof: 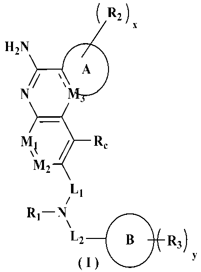
M₁ is selected from -N- or -CRₐ-;
M₂ is selected from -N- or -CR_{b}-;
M₃ is selected from N or C;
ring A is selected from C₃₋₈ cycloalkyl, 3- to 8-membered heterocyclyl, C₆₋₁₀ aryl or 5- to 10-membered heteroaryl; preferably C₃₋₆ cycloalkyl, 3- to 6-membered heterocyclyl, phenyl or 5- to 6-membered heteroaryl;
ring B is selected from C₃₋₁₄ cycloalkyl, 3- to 14-membered heterocyclyl, C₆₋₁₄ aryl or 5- to 14-membered heteroaryl; preferably C₃₋₁₂ cycloalkyl, 3- to 12-membered heterocyclyl, C₆₋₁₂ aryl or 5- to 12-membered heteroaryl; more preferably C₃₋₆ cycloalkyl, 3- to 6-membered heterocyclyl, C₆₋₁₀ fused cycloalkyl, 6- to 10-membered fused heterocyclyl, C₆₋₁₀ aryl or 5- to 10-membered heteroaryl;
L₁ is selected from a bond, -(CRₐₐR_{bb})ₘ₂-, -(CRₐₐR_{bb})ₘ₂C(O)-, - (CRₐₐR_{bb})ₘ₂C(S)-, -(CRₐₐR_{bb})ₘ₂C(NR_{cc})-, -(CRₐₐR_{bb})ₘ₂NR_{cc}C(O)-, - (CRₐₐR_{bb})ₘ₂S(O)ₘ₁-, -(CRₐₐR_{bb})ₘ₂NR_{cc}-, -(CRₐₐR_{bb})ₘ₂P(O)₂-, - (CRₐₐR_{bb})ₘ₂P(O)(OR_{cc})-, C₃₋₁₂ cycloalkylene, 3- to 12-membered heterocyclylene, C₆₋₁₂ arylene or 5- to 12-membered heteroarylene, wherein the C₃₋₁₂ cycloalkylene, 3- to 12-membered heterocyclylene, C₆₋₁₂ arylene and 5- to 12-membered heteroarylene are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl; preferably a bond, - (CRₐₐR_{bb})ₘ₂-, -(CRₐₐR_{bb})ₘ₂C(O)-, -(CRₐₐR_{bb})ₘ₂NR_{cc}C(O), -(CRₐₐR_{bb})ₘ₂S(O)ₘ₁- or - (CRₐₐR_{bb})ₘ₂NR_{cc}-; more preferably a bond, -CRₐₐR_{bb}-, -C(O)-, -NR_{cc}C(O), -S(O)ₘ₁- or -NR_{cc}-; further preferably -CRₐₐRbb-, -C(O)-, -S(O)ₘ₁- or -NR_{cc}-;
L₂ is selected from a bond, -(CRₐₐR_{bb})ₘ₂-, -(CRₐₐR_{bb})ₘ₂C(O)-, - (CRₐₐR_{bb})ₘ₂NR_{cc}C(0), -(CRₐₐR_{bb})ₘ₂S(O)ₘ₁- or -(CRₐₐR_{bb})ₘ₂NR_{cc}-; preferably a bond, -CRₐₐRbb-, -C(O)-, -S(O)ₘ₁- or NR_{cc}; more preferably -CRₐₐR_{bb}-, -C(O)-, - S(O)ₘ₁- or NR_{cc};
R₁ is selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, -(CR_{cc}R_{dd})ₙ₁-C₃₋₁₂ cycloalkyl, - (CR_{cc}R_{dd})ₙ₁-3- to 12-membered heterocyclyl, -(CR_{cc}R_{dd})ₙ₁-C₆₋₁₂ aryl, -(CR_{cc}R_{dd})ₙ₁-5- to 12-membered heteroaryl, -SF₅, -ORₑ, -NRₑR_{f}, -C(O)Rₑ, -C(O)ORₑ, - C(O)NRₑR_{f}, -N=S(O)RₑR_{f}, -S(O)Rₑ(=NR_{f}) or -P(O)RₑR_{f}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl and =CR_{gg}Rₕₕ; preferably hydrogen, deuterium, fluorine, chlorine, bromine, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ haloalkoxy, C₁₋₃ hydroxyalkyl, -(CR_{cc}R_{dd})ₙ₁-C₃₋₈ cycloalkyl, -(CR_{cc}R_{dd})ₙ₁-3- to 8-membered heterocyclyl, -(CR_{cc}R_{dd})ₙ₁-C₆₋₁₀ aryl, -(CR_{cc}R_{dd})ₙ₁-5- to 10-membered heteroaryl, -ORₑ, -NRₑR_{f}, -C(O)Rₑ, -C(O)NRₑR_{f} or -P(O)RₑR_{f}, wherein the amino, C₁₋₃ alkyl, C₂₋₆ alkenyl, C₂₋₆ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3-to 8-membered heterocyclyl, C₆₋₁₀ aryl and 5- to 10-membered heteroaryl are optionally substituted with one or more substituents selected from deuterium, halogen, amino, hydroxyl, cyano, nitro, C₁₋₃ alkyl, C₂₋₄ alkenyl, C₂₋₄ alkynyl, oxo, thio, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl;
alternatively, R₁ and Rₐ, R_{b} or R_{c} are connected to form C₃₋₁₂ cycloalkyl, 3- to 12-membered heterocyclyl, C₆₋₁₂ aryl and 5- to 12-membered heteroaryl, which are optionally further substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl; preferably form C₃₋₈ cycloalkyl, 3- to 8-membered heterocyclyl, C₆₋₁₀ aryl and 5- to 10-membered heteroaryl, which are optionally further substituted with one or more substituents selected from deuterium, fluorine, chlorine, bromine, amino, hydroxyl, cyano, nitro, C₁₋₃ alkyl, C₂₋₄ alkenyl, C₂₋₄ alkynyl, oxo, thio, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl;
R₂ is selected from hydrogen, deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl, -(CH₂)ₙ₂ORₑₑ, -(CH₂)ₙ₂NRₑₑR_{ff}, -(CH₂)ₙ₂C(O)Rₑₑ, -(CH₂)ₙ₂C(O)NRₑₑR_{ff}, -(CH₂)ₙ₂P(O)RₑₑR_{ff} or =CRₑₑR_{ff}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl; preferably hydrogen, deuterium, fluorine, chlorine, bromine, amino, hydroxyl, cyano, nitro, C₁₋₃ alkyl, C₂₋₄ alkenyl, C₂₋₄ alkynyl, oxo, thio, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl or 5- to 10-membered heteroaryl, wherein the amino, C₁₋₃ alkyl, C₂₋₆ alkenyl, C₂₋₆ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl are optionally substituted with one or more substituents selected from deuterium, halogen, amino, hydroxyl, cyano, nitro, C₁₋₃ alkyl, C₂₋₄ alkenyl, C₂₋₄ alkynyl, oxo, thio, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl;
R₃ is selected from hydrogen, deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, -Y₁-C₃₋₁₂ cycloalkyl, -Y₁-3- to 12-membered heterocyclyl, -Y₁-C₆₋₁₂ aryl, -Y₁-5- to 12-membered heteroaryl, -SF₅, -OR_{g}, -NR_{g}Rₕ, -C(O)R_{g}, -C(O)OR_{g}, -C(O)NR_{g}Rₕ, - N=S(O)R_{g}Rₕ, -S(O)R_{g}(=NRₕ), -P(O)R_{g}Rₕ, -C(=NRᵢ)NR_{g}Rₕ or =R_{g}Rₕ, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, substituted or unsubstituted C₃₋₁₂ cycloalkyl, substituted or unsubstituted 3- to 12-membered heterocyclyl, substituted or unsubstituted C₆₋₁₂ aryl, substituted or unsubstituted 5- to 12-membered heteroaryl and =CRₑR_{f}; preferably hydrogen, deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl, -SF₅, -OR_{g}, -NR_{g}Rₕ, -C(O)R_{g}, -C(O)OR_{g}, - C(O)NR_{g}Rₕ, -N=S(O)R_{g}Rₕ, -S(O)R_{g}(=NRₕ), -P(O)R_{g}Rₕ or =R_{g}Rₕ, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, substituted or unsubstituted C₃₋₁₂ cycloalkyl, substituted or unsubstituted 3- to 12-membered heterocyclyl, substituted or unsubstituted C₆₋₁₂ aryl, substituted or unsubstituted 5- to 12-membered heteroaryl and =CRₑR_{f}; more preferably hydrogen, deuterium, fluorine, chlorine, bromine, amino, hydroxyl, cyano, nitro, C₁₋₃ alkyl, C₂₋₄ alkenyl, C₂₋₄ alkynyl, oxo, thio, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl or 5- to 10-membered heteroaryl, wherein the amino, C₁₋₃ alkyl, C₂₋₆ alkenyl, C₂₋₆ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl are optionally substituted with one or more substituents selected from deuterium, halogen, amino, hydroxyl, cyano, nitro, C₁₋₃ alkyl, C₂₋₄ alkenyl, C₂₋₄ alkynyl, oxo, thio, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl, 5-to 10-membered heteroaryl and =CRₑR_{f};
alternatively, any two R₃, together with adjacent atoms to which they are attached, form C₃₋₁₂ cycloalkyl, 3- to 12-membered heterocyclyl, C₆₋₁₂ aryl and 5- to 12-membered heteroaryl, which are optionally further substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl; preferably form C₃₋₈ cycloalkyl, 3- to 8-membered heterocyclyl, C₆₋₁₀ aryl and 5- to 10-membered heteroaryl, which are optionally further substituted with one or more substituents selected from deuterium, fluorine, chlorine, bromine, amino, hydroxyl, cyano, nitro, C₁₋₃ alkyl, C₂₋₄ alkenyl, C₂₋₄ alkynyl, oxo, thio, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl;
Rₐ, R_{b}, R_{c}, Rₑ and R_{f} are each independently selected from hydrogen, deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl, -(CH₂)ₙ₂ORₑₑ, - (CH₂)ₙ₂NRₑₑR_{ff}, -(CH₂)ₙ₂C(O)Rₑₑ, -(CH₂)ₙ₂C(O)ORₑₑ, -(CH₂)ₙ₂C(O)NRₑₑR_{ff}, - (CH₂)ₙ₂N=S(O)RₑₑR_{ff}, -(CH₂)ₙ₂S(O)Rₑₑ(=NR_{ff}) or -(CH₂)ₙ₂P(O)RₑₑR_{ff}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋12 cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl and =CR_{gg}Rₕₕ; preferably hydrogen, deuterium, fluorine, chlorine, bromine, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, -C(O)-C₁₋₃ alkyl, C₃₋₈ cycloalkyl, 3- to 8-membered heterocyclyl, C₆₋₁₀ aryl or 5- to 10-membered heteroaryl, wherein the amino, C₁₋₃ alkyl, C₂₋₆ alkenyl, C₂₋₆ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl are optionally substituted with one or more substituents selected from deuterium, halogen, amino, hydroxyl, cyano, nitro, C₁₋₃ alkyl, C₂₋₄ alkenyl, C₂₋₄ alkynyl, oxo, thio, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl;
alternatively, Rₐ and R_{b} are connected to form C₃₋₁₂ cycloalkyl, 3- to 12-membered heterocyclyl, C₆₋₁₂ aryl and 5- to 12-membered heteroaryl, which are optionally further substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
Y₁ is selected from a bond, -O-, -S-, -C(O), -NRⱼ-, -C(O)NRⱼ-, -NRⱼC(O)-, - S(O)₂NRⱼ-, -NRⱼS(O)₂-, C₁₋₆ alkylene, -O-C₁₋₆ alkylene-, -C₁₋₆ alkylene-O-, -NRⱼ-C₁₋₆ alkylene-, -C₁₋₆ alkylene-NRⱼ-, C₂₋₆ alkenylene or C₂₋₆ alkynylene, wherein the C₁₋₆ alkylene, C₂₋₆ alkenylene and C₂₋₆ alkynylene are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
R_{g}, Rₕ, Rᵢ and Rⱼ are each independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, -C(O)-C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl; preferably hydrogen, deuterium, fluorine, chlorine, bromine, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, -C(O)-C₁₋₃ alkyl, C₃₋₈ cycloalkyl, 3-to 8-membered heterocyclyl, C₆₋₁₀ aryl or 5- to 10-membered heteroaryl, wherein the amino, C₁₋₃ alkyl, C₂₋₆ alkenyl, C₂₋₆ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl are optionally substituted with one or more substituents selected from deuterium, halogen, amino, hydroxyl, cyano, nitro, C₁₋₃ alkyl, C₂₋₄ alkenyl, C₂₋₄ alkynyl, oxo, thio, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl;
alternatively, R_{g} and Rₕ are connected to form C₃₋₁₂ cycloalkyl, 3- to 12-membered heterocyclyl, C₆₋₁₂ aryl and 5- to 12-membered heteroaryl, which are optionally further substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
Rₐₐ, R_{bb}, R_{cc} and R_{dd} are each independently selected from hydrogen, deuterium, halogen, amino, hydroxyl, cyano, nitro, C₁₋₆ alkyl, C₂₋₆ alkenyl, C₂₋₆ alkynyl, C₁₆ deuteroalkyl, C₁₋₆ haloalkyl, C₁₋₆ alkoxy, halogenated C₁₋₆ alkoxy, C₁₋₆ hydroxyalkyl, cyano-substituted C₁₆ alkyl, -C(O)-C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl; preferably hydrogen, deuterium, fluorine, chlorine, bromine, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, -C(O)-C₁₋₃ alkyl, C₃₋₈ cycloalkyl, 3-to 8-membered heterocyclyl, C₆₋₁₀ aryl or 5- to 10-membered heteroaryl, wherein the amino, C₁₋₃ alkyl, C₂₋₆ alkenyl, C₂₋₆ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl are optionally substituted with one or more substituents selected from deuterium, halogen, amino, hydroxyl, cyano, nitro, C₁₋₃ alkyl, C₂₋₄ alkenyl, C₂₋₄ alkynyl, oxo, thio, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl;
Rₑₑ and R_{ff} are each independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, -C(O)-C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl; preferably hydrogen, deuterium, fluorine, chlorine, bromine, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, -C(O)-C₁₋₃ alkyl, C₃₋₈ cycloalkyl, 3-to 8-membered heterocyclyl, C₆₋₁₀ aryl or 5- to 10-membered heteroaryl, wherein the amino, C₁₋₃ alkyl, C₂₋₆ alkenyl, C₂₋₆ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl are optionally substituted with one or more substituents selected from deuterium, halogen, amino, hydroxyl, cyano, nitro, C₁₋₃ alkyl, C₂₋₄ alkenyl, C₂₋₄ alkynyl, oxo, thio, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl;
R_{gg} and Rₕₕ are each independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, -C(O)-C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl; preferably hydrogen, deuterium, fluorine, chlorine, bromine, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, -C(O)-C₁₋₃ alkyl, C₃₋₈ cycloalkyl, 3-to 8-membered heterocyclyl, C₆₋₁₀ aryl or 5- to 10-membered heteroaryl, wherein the amino, C₁₋₃ alkyl, C₂₋₆ alkenyl, C₂₋₆ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl are optionally substituted with one or more substituents selected from deuterium, halogen, amino, hydroxyl, cyano, nitro, C₁₋₃ alkyl, C₂₋₄ alkenyl, C₂₋₄ alkynyl, oxo, thio, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl;
x is selected from 0, 1, 2, 3, 4, 5 or 6;
y is selected from 0, 1, 2, 3, 4, 5 or 6;
m1 is selected from 0, 1 or 2;
m2 is selected from 0, 1 or 2;
n1 is selected from 0, 1, 2, 3 or 4; and
n2 is selected from 0, 1, 2, 3 or 4.

2. The compound, or the stereoisomer or the pharmaceutically acceptable salt thereof according to claim 1, **characterised in that** the 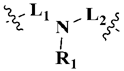 is selected from

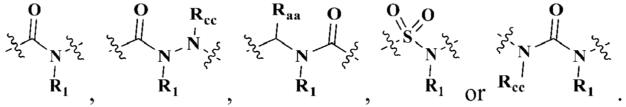

3. The compound, or the stereoisomer or the pharmaceutically acceptable salt thereof according to claim 1, **characterised in that** the general formula is further as represented by general formula (II): 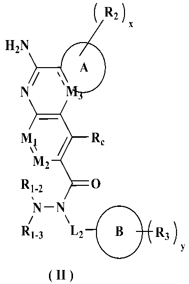
R₁₋₂ is selected from hydrogen, deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl, -(CH₂)ₙ₂ORₑₑ, -(CH₂)ₙ₂NRₑₑR_{ff}, -(CH₂)ₙ₂C(O)Rₑₑ, -(CH₂)ₙ₂C(O)ORₑₑ, - (CH₂)ₙ₂C(O)NRₑₑR_{ff}, -(CH₂)ₙ₂N=S(O)RₑₑR_{ff}, -(CH₂)ₙ₂S(O)Rₑₑ(=NR_{ff}) or - (CH₂)ₙ₂P(O)RₑₑR_{ff}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl and =CR_{gg}Rₕₕ; preferably C₁₋₃ alkyl, C₁₋₃ deuteroalkyl, C₁₋₃ haloalkyl, C₁₋₃ alkoxy, halogenated C₁₋₃ alkoxy, C₁₋₃ hydroxyalkyl or cyano-substituted C₁₋₃ alkyl, wherein the C₁₋₃ alkyl, C₁₋₃ deuteroalkyl, C₁₋₃ haloalkyl, C₁₋₃ alkoxy, halogenated C₁₋₃ alkoxy, C₁₋₃ hydroxyalkyl and cyano-substituted C₁₋₃ alkyl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
R₁₋₃ is selected from hydrogen, deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl, -(CH₂)ₙ₂ORₑₑ, -(CH₂)ₙ₂NRₑₑR_{ff}, -(CH₂)ₙ₂C(O)Rₑₑ, -(CH₂)ₙ₂C(O)ORₑₑ, - (CH₂)ₙ₂C(O)NRₑₑR_{ff}, -(CH₂)ₙ₂N=S(O)RₑₑR_{ff}, -(CH₂)ₙ₂S(O)Rₑₑ(=NR_{ff}) or - (CH₂)ₙ₂P(O)RₑₑR_{ff}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl and =CR_{gg}Rₕₕ; preferably hydrogen, deuterium, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl or cyano-substituted C₁₋₃ alkyl, wherein the C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl and cyano-substituted C₁₋₃ alkyl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
alternatively, R₁₋₂ and R₁₋₃ are connected to form 3- to 10-membered heterocyclyl, wherein the 3- to 10-membered heterocyclyl is optionally substituted with one or more substitutents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl and =CR_{gg}Rₕₕ;
Rₑₑ and R_{ff} are each independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, -C(O)-C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
R_{gg} and Rₕₕ are each independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, -C(O)-C₁₋₆ alkyl, C₃₋₁₂ cycloalkyl, 3- to 12-membered heterocyclyl, C₆₋₁₂ aryl or 5- to 12-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
n2 is selected from 0, 1, 2, 3 or 4;
n3 is selected from 0, 1, 2, 3 or 4;
ring A, ring B, M₁, M₂, M₃, L₂, R₂, R₃, R_{c}, x and y are as defined in claim 1.

4. A compound represented by general formula (III) or (III-1), or a stereoisomer or a pharmaceutically acceptable salt thereof: 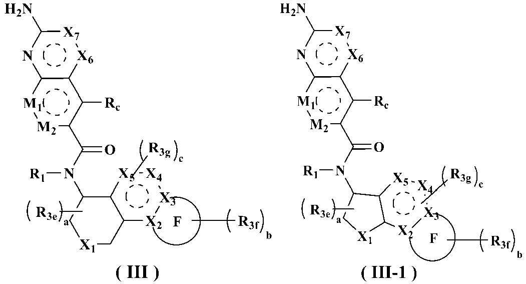
X₁ is selected from O or CH₂;
X₂ is selected from C or N;
X₃ is selected from C or N;
X₄ is selected from CH or N;
X₅ is selected from CH or N;
X₆ is selected from CR₄ₐ, NR₄ₐ or N;
X₇ is selected from CR_{4b}, NR_{4b} or N;
alternatively, R₄ₐ and R_{4b} are connected to form ring A;
ring A is selected from C₃₋₈ cycloalkyl, 3- to 8-membered heterocyclyl, C₆₋₁₀ aryl or 5- to 10-membered heteroaryl, which is optionally substituted with 0, 1, 2, 3, 4, 5 or 6 R_{2c};
ring F is selected from C₃₋₁₀ cycloalkyl, 3- to 10-membered heterocyclyl, C₆₋₁₀ aryl or 5- to 10-membered heteroaryl;
R_{2c} is selected from hydrogen, deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl, -(CH₂)ₙ₂ORₑₑ, -(CH₂)ₙ₂NRₑₑR_{ff}, -(CH₂)ₙ₂C(O)Rₑₑ, -(CH₂)ₙ₂C(O)NRₑₑR_{ff}, -(CH₂)ₙ₂P(O)RₑₑR_{ff} or =CRₑₑR_{ff}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
R₃ₑ and R_{3g} are each independently selected from hydrogen, deuterium, halogen, amino, hydroxyl, cyano, nitro, C₁₋₃ alkyl, C₂₋₄ alkenyl, C₂₋₄ alkynyl, oxo, thio, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 10-membered heterocyclyl, C₆₋₁₀ aryl, 5- to 10-membered heteroaryl, -SF₅, -ORₐ₁, -NRₐ₁Rₐ₂, -C(O)Rₐ₁, - C(O)NRₐ₁Rₐ₂, -N=S(O)Rₐ₁Rₐ₂, -S(O)Rₐ₁(=NRₐ₂), -P(O)Rₐ₁Rₐ₂ or =Rₐ₁Rₐ₂, wherein the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₁₀ cycloalkyl, 3- to 10-membered heterocyclyl, C₆₋₁₀ aryl and 5- to 10-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, substituted or unsubstituted C₃₋₁₂ cycloalkyl, substituted or unsubstituted 3- to 12-membered heterocyclyl, substituted or unsubstituted C₆₋₁₂ aryl, substituted or unsubstituted 5- to 12-membered heteroaryl and =CRₐ₃Rₐ₄;
alternatively, two R₃ₑ are connected to form C₃₋₁₀ cycloalkyl, 3- to 10-membered heterocyclyl, C₆₋₁₀ aryl or 5- to 10-membered heteroaryl, wherein the C₃₋₁₀ cycloalkyl, 3- to 10-membered heterocyclyl, C₆₋₁₀ aryl and 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -SF₅, =N-ORₐ₅, =CRₐ₅CRₐ₆, -S(O)Rₐ₅(=NRₐ₆) and -N=S(O)Rₐ₅Rₐ₆;
R_{3f} is selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, -(CR_{b1}R_{b2})ₙ₆-C₃₋₈ cycloalkyl, -(CR_{b1}R_{b2})ₙ₆-3- to 8-membered heterocyclyl, -(CR_{b1}R_{b2})ₙ₆-C₆₋₁₀ aryl, -(CR_{b1}R_{b2})ₙ₆-5- to 10-membered heteroaryl, -SF₅, -OR_{b1}, -NRₚ₁Rₚ₂, -C(O)R_{b1}, - C(O)NR_{b1}R_{b2}, =N-OR_{b1}, =CR_{b1}CR_{b2}, -S(O)R_{b1}(=NR_{b2}) and -N=S(O)R_{b1}R_{b2}, wherein the hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl and =CR_{b3}CR_{b4}; preferably hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl, 5- to 10-membered heteroaryl, -SF₅, -OR_{b1}, -NR_{b1}R_{b2}, -C(O)R_{b1}, - C(O)NR_{b1}R_{b2}, =N-OR_{b1}, =CR_{b1}CR_{b2}, -S(O)R_{b1}(=NR_{b2}) and -N=S(O)R_{b1}R_{b2};
alternatively, two R_{3f} are connected to form C₃₋₁₀ cycloalkyl, 3- to 10-membered heterocyclyl, C₆₋₁₀ aryl or 5- to 10-membered heteroaryl, wherein the C₃₋₁₀ cycloalkyl, 3- to 10-membered heterocyclyl, C₆₋₁₀ aryl and 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R₄ₐ is selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl or 5- to 14-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R_{4b} is selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, 5- to 14-membered heteroaryl, -OR_{c1}, -C(O)R_{c1}, -C(O)OR_{c1}, -NR_{c1}R_{c2}, -P(O)R_{c1}R_{c2}, - NR_{c3}C(O)R_{c1}, -C(O)NR_{c1}R_{c2}, -S(O)₂R_{c1}, -S(O)₂NR_{c1}R_{c2}, -NR_{c3}S(O)₂R_{c1}, - S(O)R_{c1}(=NR_{c3}) or -N=S(O)R_{c1}R_{c2}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
Rₐ₁, Rₐ₂, Rₐ₃, Rₐ₄, Rₐ₅ and Rₐ₆ are each independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl or 5- to 14-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R_{b1}, R_{b2}, R_{b3} and R_{b4} are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl or 5- to 14-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
R_{c1}, R_{c2} and R_{c3} are each independently selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl or 5- to 14-membered heteroaryl, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuteroalkyl, C₁₋₆ haloalkyl, C₁₋₆ alkoxy, C₁₋₆ deuteroalkoxy, C₁₋₆ haloalkoxy, C₁₋₆ hydroxyalkyl, C₃₋₈ cycloalkyl, 3- to 8-membered heterocyclyl, C₆₋₁₀ aryl and 5- to 10-membered heteroaryl;
a is selected from 0, 1, 2, 3 or 4;
b is selected from 0, 1, 2, 3 or 4;
c is selected from 0, 1, 2, 3 or 4; and
n6 is selected from 0, 1, 2, 3 or 4;
M₁, M₂, R₁, R_{c}, Rₑₑ, R_{ff} and n2 are as defined in claim 1.

5. The compound, or the stereoisomer or the pharmaceutically acceptable salt thereof according to claim 4, **characterised in that** the general formula is further as represented by general formula (IV) or (IV-1): 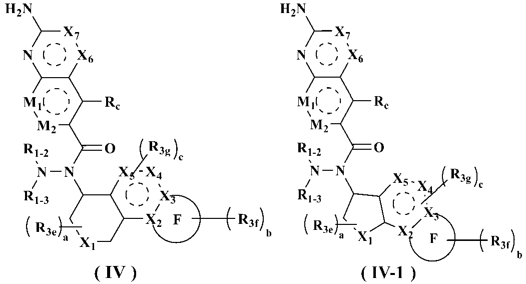
R₁₋₂ is selected from hydrogen, deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C6-12 aryl, 5- to 12-membered heteroaryl, -(CH₂)ₙ₂ORₑₑ, -(CH₂)ₙ₂NRₑₑR_{ff}, -(CH₂)ₙ₂C(O)Rₑₑ, -(CH₂)ₙ₂C(O)ORₑₑ, - (CH₂)ₙ₂C(O)NRₑₑR_{ff}, -(CH₂)ₙ₂N=S(O)RₑₑR_{ff}, -(CH₂)ₙ₂S(O)Rₑₑ(=NR_{ff}) or - (CH₂)ₙ₂P(O)RₑₑR_{ff}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl and =CR_{gg}Rₕₕ;
R₁₋₃ is selected from hydrogen, deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl, -(CH₂)ₙ₂ORₑₑ, -(CH₂)ₙ₂NRₑₑR_{ff}, -(CH₂)ₙ₂C(O)Rₑₑ, -(CH₂)ₙ₂C(O)ORₑₑ, - (CH₂)ₙ₂C(O)NRₑₑR_{ff}, -(CH₂)ₙ₂N=S(O)RₑₑR_{ff}, -(CH₂)ₙ₂S(O)Rₑₑ(=NR_{ff}) or - (CH₂)ₙ₂P(O)RₑₑR_{ff}, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl and =CR_{gg}Rₕₕ;
alternatively, R₁₋₂ and R₁₋₃ are connected to form 3- to 10-membered heterocyclyl, wherein the 3- to 10-membered heterocyclyl is optionally substituted with one or more substitutents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl and =CR_{gg}Rₕₕ.

6. The compound, or the stereoisomer or the pharmaceutically acceptable salt thereof according to any one of claims 1-5, **characterised in that** the general formula is further as represented by general formula (V) or (V-1): 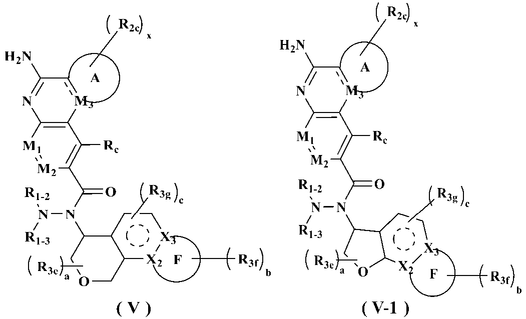
ring A, ring F, X₂, X₃, R_{2c}, R₃ₑ, R_{3g}, R_{3f}, a, b and c are as defined in claim 4;
R₁₋₂ and R₁₋₃ are as defined in claim 3 or 5;
M₁, M₂, M₃, R_{c} and x are as defined in claim 1.

7. The compound, or the stereoisomer or the pharmaceutically acceptable salt thereof according to any one of claims 1-6, **characterised in that** ring A is selected from 3- to 12-membered heterocyclyl or 5- to 12-membered heteroaryl; preferably 5-membered heterocyclyl, 6-membered heterocyclyl, 5-membered heteroaryl or 6-membered heteroaryl; more preferably

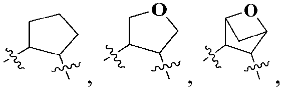

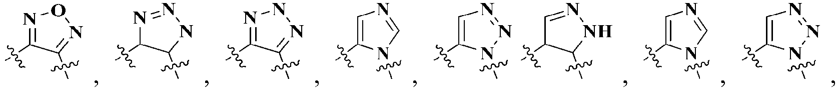

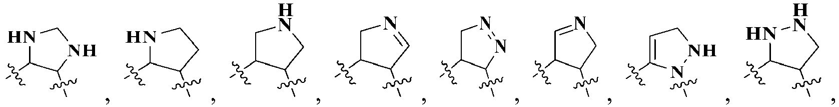

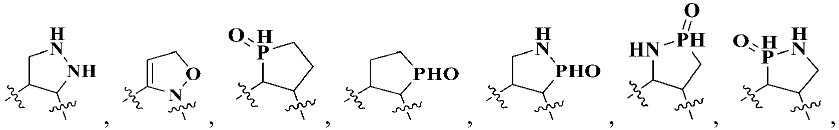

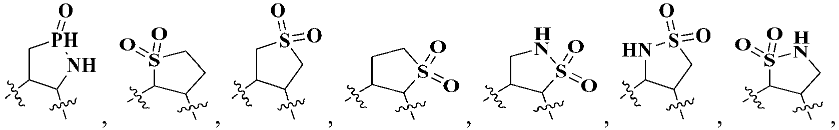

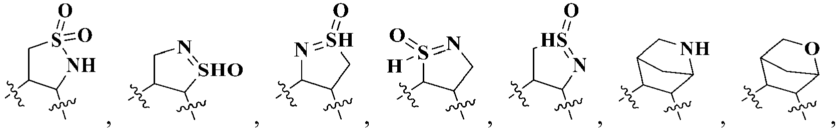

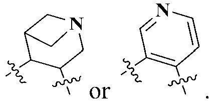

8. The compound, or the stereoisomer or the pharmaceutically acceptable salt thereof according to any one of claims 1-7, **characterised in that** the general formula is further as represented by general formula (VI):

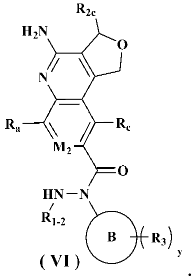

9. The compound, or the stereoisomer or the pharmaceutically acceptable salt thereof according to any one of claims 1-8, **characterised in that** ring B is selected from 3- to 6-membered heterocyclyl fused phenyl or 3- to 6-membered heterocyclyl fused 5- to 6-membered heteroaryl; preferably 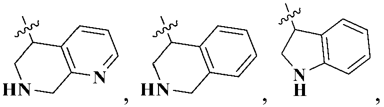 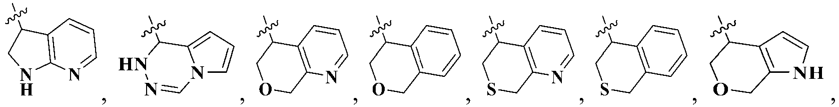 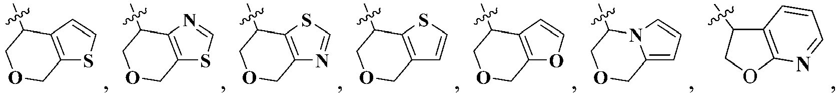 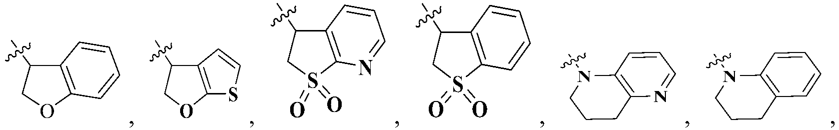 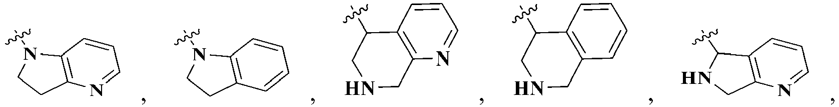 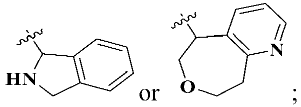 alternatively, ring B is 6- to 14-membered tricyclic heterocyclyl; preferably 6-to 14-membered tricyclic spiro heterocyclyl or 6- to 14-membered tricyclic fused heterocyclyl; more preferably 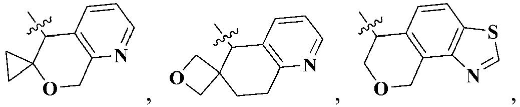 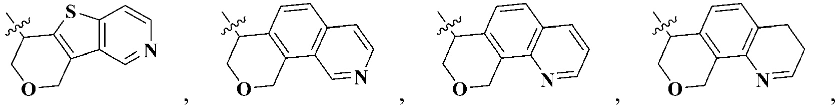 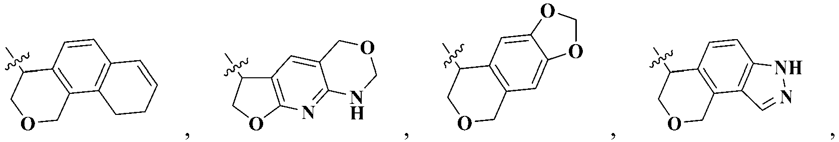 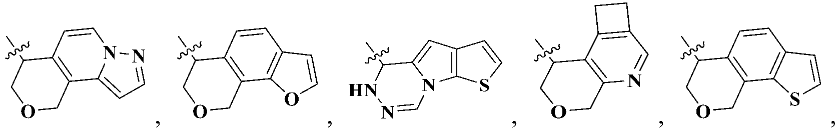 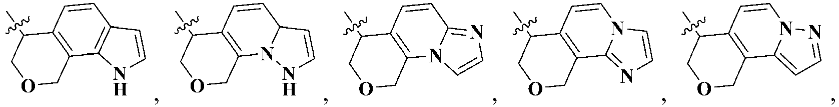 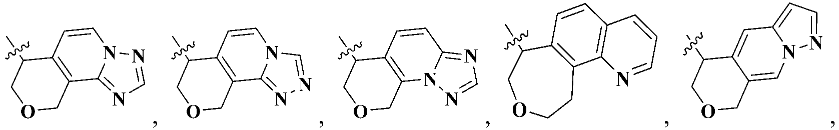 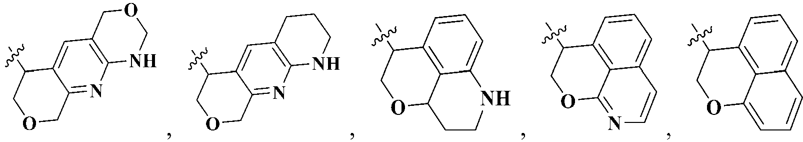 or

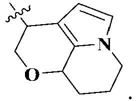

10. The compound, or the stereoisomer or the pharmaceutically acceptable salt thereof according to any one of claims 1-9, **characterised in that** the general formula is further as represented by general formula (VI) or (VI-A): 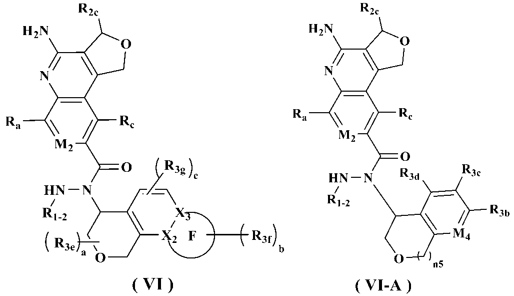
M₄ is N or R₃ₐ;
X₂ is C or N;
X₃ is C or N;
R₃ₐ, R_{3c} and R_{3d} are each independently selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl,C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl and -SF₅, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy,C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, substituted or unsubstituted C₃₋₁₂ cycloalkyl, substituted or unsubstituted 3- to 12-membered heterocyclyl, substituted or unsubstituted C₆₋₁₂ aryl, substituted or unsubstituted 5- to 12-membered heteroaryl and =CRₑR_{f};
R_{3b} is selected from hydrogen, deuterium, halogen, amino, hydroxyl, cyano, nitro, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl, 5- to 12-membered heteroaryl and -SF₅, wherein the amino, C₁₋₆ alkyl, C₂₋₆ alkenyl, C₂₋₆ alkynyl, C₁₋₆ deuteroalkyl, C₁₋₆ haloalkyl, C₁₋₆ alkoxy, halogenated C₁₋₆ alkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, substituted or unsubstituted C₃₋₁₂ cycloalkyl, substituted or unsubstituted 3- to 12-membered heterocyclyl, substituted or unsubstituted C₆₋₁₂ aryl, substituted or unsubstituted 5-to 12-membered heteroaryl and =CRₑR_{f};
n5 is selected from 0, 1 or 2.

11. The compound, or the stereoisomer or the pharmaceutically acceptable salt thereof according to any one of claims 1-10, **characterised in that** each R₂ or R_{2c} is independently selected from hydrogen, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, cyano-substituted C₁₋₃ alkyl, C₃₋₈ cycloalkyl or -C(O)NRₑₑR_{ff}, wherein the amino, C₁₋₃ alkyl, C₂₋₄ alkenyl, C₂₋₄ alkynyl, C₁₋₃ deuteroalkyl, C₁₋₃ haloalkyl, cyano-substituted C₁₋₃ alkyl and C₃₋₈ cycloalkyl are optionally substituted with one or more substituents selected from deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, cyano-substituted C₁₋₃ alkyl and C₃₋₈ cycloalkyl; preferably hydrogen, deuterium, fluorine, chlorine, methyl, deuterated methyl, difluoromethyl, trifluoromethyl, 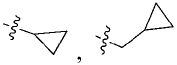 or 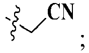
R_{c} is selected from hydrogen, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl or cyano-substituted C₁₋₃ alkyl; preferably hydrogen, deuterium, fluorine, chlorine, methyl, deuterated methyl, difluoromethyl or trifluoromethyl;
Rₐ is selected from hydrogen, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl or cyano-substituted C₁₋₃ alkyl; preferably hydrogen, deuterium, fluorine, chlorine, methyl, deuterated methyl, difluoromethyl or trifluoromethyl;
R_{b} is selected from hydrogen, deuterium, halogen, cyano, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₈ cycloalkyl, 3- to 8-membered heterocyclyl, -(CH₂)ₙ₂ORₑₑ, -(CH₂)ₙ₂NRₑₑR_{ff}, -(CH₂)ₙ₂C(O)Rₑₑ, - (CH₂)ₙ₂C(O)ORₑₑ, -(CH₂)ₙ₂C(O)NRₑₑR_{ff}, -(CH₂)ₙ₂N=S(O)RₑₑR_{ff}, - (CH₂)ₙ₂S(O)Rₑₑ(=NR_{ff}) or -(CH₂)ₙ₂P(O)RₑₑR_{ff}, wherein the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₈ cycloalkyl and 3- to 8-membered heterocyclyl are optionally substituted with one or more substituents selected from deuterium, halogen, amino, hydroxyl, cyano, nitro, C₁₋₃ alkyl, C₂₋₄ alkenyl, C₂₋₄ alkynyl, oxo, thio, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl and =CR_{gg}Rₕₕ; preferably hydrogen, deuterium, halogen, cyano, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₈ cycloalkyl, 3- to 8-membered heterocyclyl, -(CH₂)ₙ₂ORₑₑ, -(CH₂)ₙ₂NRₑₑR_{ff}, - (CH₂)ₙ₂C(O)Rₑₑ, -(CH₂)ₙ₂C(O)NRₑₑR_{ff} or -(CH₂)ₙ₂P(O)RₑₑR_{ff}, wherein the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₈ cycloalkyl and 3- to 8-membered heterocyclyl are optionally substituted with one or more substituents selected from deuterium, halogen, amino, hydroxyl, cyano, nitro, C₁₋₃ alkyl, C₂₋₄ alkenyl, C₂₋₄ alkynyl, oxo, thio, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl or 3- to 8-membered heterocyclyl;
Rₑₑ and R_{ff} are each independently selected from hydrogen, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, -C(O)-C₁₋₃ alkyl, C₃₋₈ cycloalkyl or 3- to 8-membered heterocyclyl, wherein the amino, C₁₋₃ alkyl, C₂₋₆ alkenyl, C₂₋₆ alkynyl, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl and 3- to 8-membered heterocyclyl are optionally substituted with one or more substituents selected from deuterium, halogen, amino, hydroxyl, cyano, nitro, C₁₋₃ alkyl, C₂₋₄ alkenyl, C₂₋₄ alkynyl, oxo, thio, C₁₋₃ deuteroalkyl, C₁₋₃ haloalkyl, C₁₋₃ alkoxy, C₁₋₃ haloalkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl; n2 is selected from 0, 1 or 2; preferably hydrogen, deuterium, fluorine, chlorine, cyano, methyl, ethyl, ethynyl, propynyl, deuterated methyl, deuterated ethyl, difluoromethyl, trifluoromethyl, trifluoroethyl, methoxy, ethoxy, deuterated methoxy, difluoromethoxy, trifluoromethoxy,

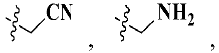

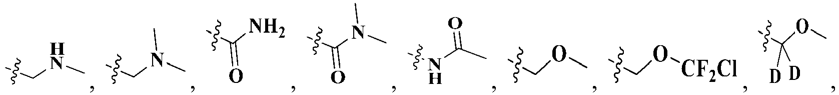

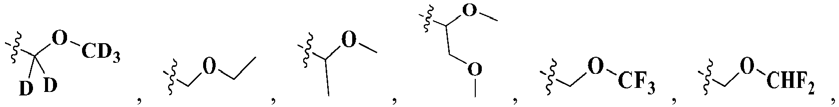

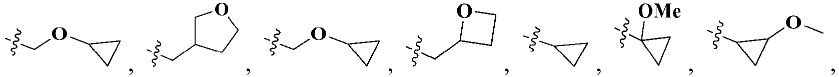

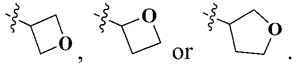

12. The compound, or the stereoisomer or the pharmaceutically acceptable salt thereof according to any one of claims 1-11, **characterised in that** R₃ and R_{3f} are each independently selected from hydrogen, deuterium, halogen, amino, hydroxyl, cyano, nitro, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₈ cycloalkyl, 3- to 8-membered heterocyclyl, C₆₋₁₀ aryl, 5- to 10-membered heteroaryl or -SF₅, wherein the amino, C₁₋₃ alkyl, C₂₋₄ alkenyl, C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl;
R₃ₑ and R_{3g} are each independently selected from hydrogen, deuterium, halogen, amino, hydroxyl, cyano, nitro, C₁₋₃ alkyl, C₂₋₄ alkenyl,C₂₋₄ alkynyl, C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₈ cycloalkyl, 3- to 8-membered heterocyclyl, C₆₋₁₀ aryl or 5- to 10-membered heteroaryl, wherein the amino, C₁₋₃ alkyl,C₂₋₄ alkenyl,C₂₋₄ alkynyl,C₁₋₃ deuteroalkyl, C₁₋₃ haloalkyl, C₁₋₃ alkoxy, halogenated C₁₋₃ alkoxy, C₁₋₃ hydroxyalkyl, cyano-substituted C₁₋₃ alkyl, C₃₋₈ cycloalkyl, 3- to 8-membered heterocyclyl, C₆₋₁₀ aryl and 5- to 10-membered heteroaryl are optionally substituted with one or more substituents selected from deuterium, halogen, amino, hydroxyl, cyano, nitro, C₁₋₆ alkyl, C₂₋₆ alkenyl, C₂₋₆ alkynyl, oxo, thio, C₁₋₆ deuteroalkyl, C₁₋₆ haloalkyl, C₁₋₆ alkoxy,C₁₋₆ haloalkoxy, C₁₋₆ hydroxyalkyl, cyano-substituted C₁₋₆ alkyl, C₃₋₁₂ cycloalkyl, 3- to 12-membered heterocyclyl, C₆₋₁₂ aryl and 5- to 12-membered heteroaryl.

13. The compound, or the stereoisomer or the pharmaceutically acceptable salt thereof according to any one of claims 1-12, **characterised in that** the compound has the following structure: 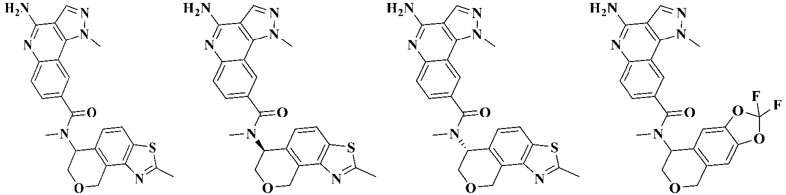 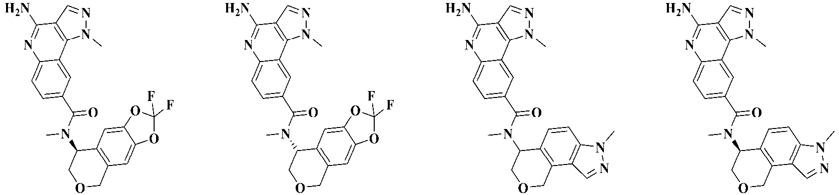 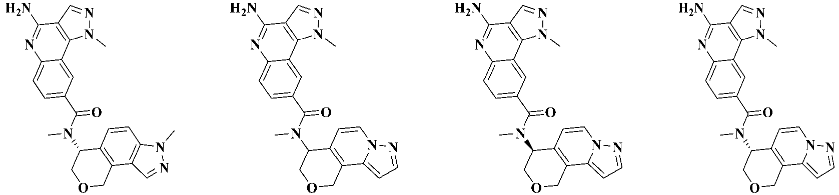 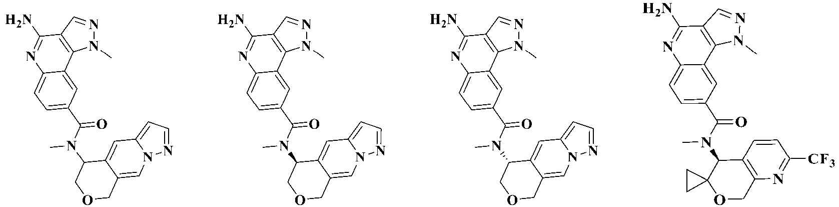 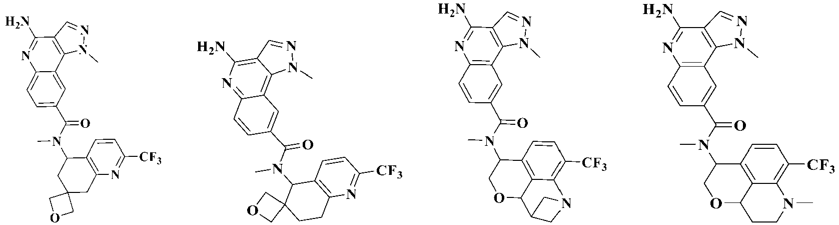 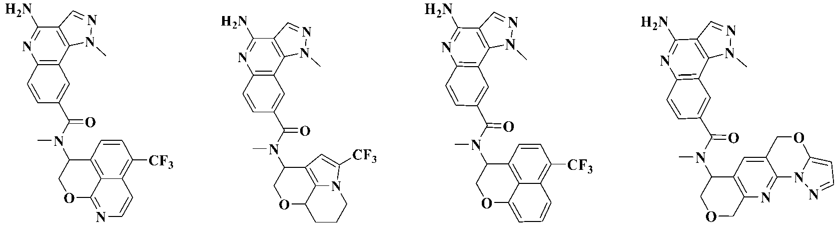 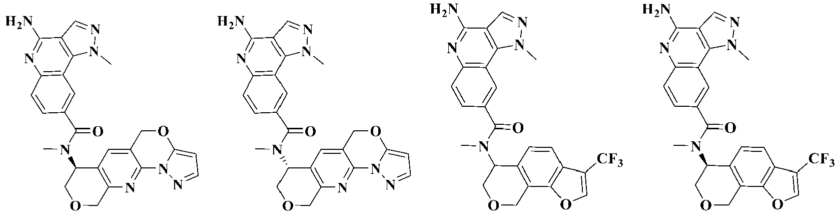 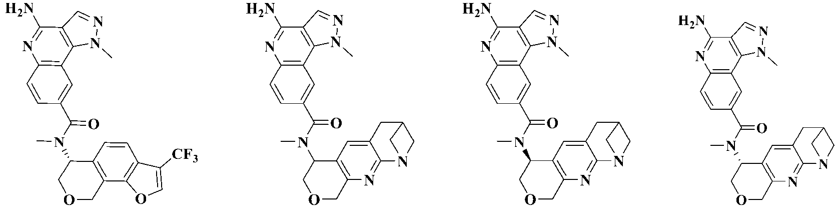 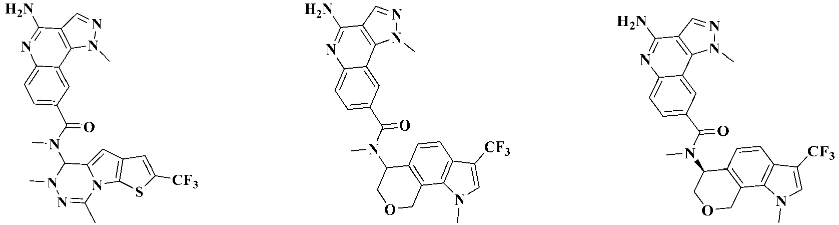 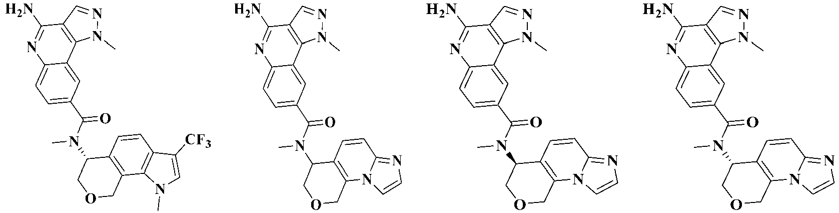 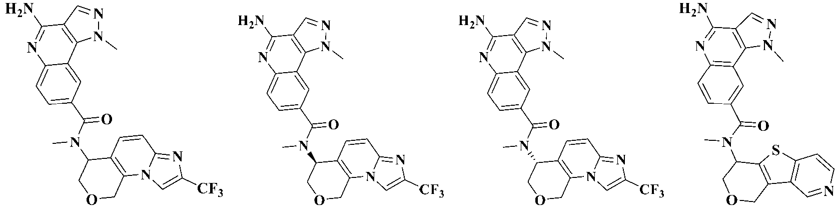 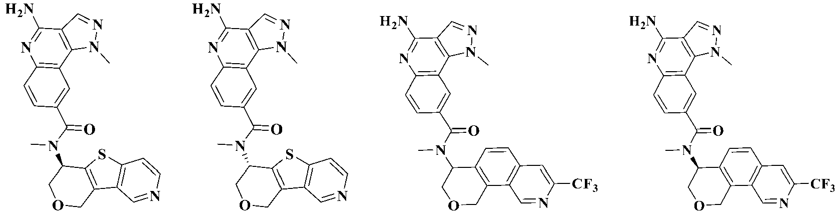 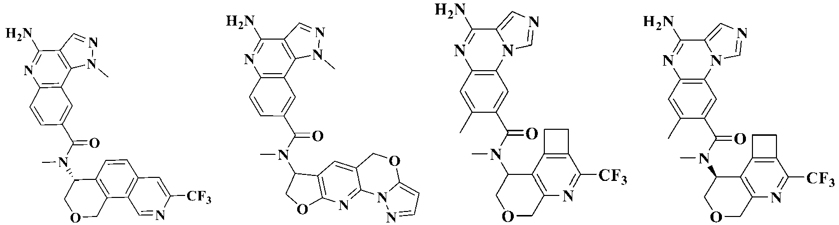 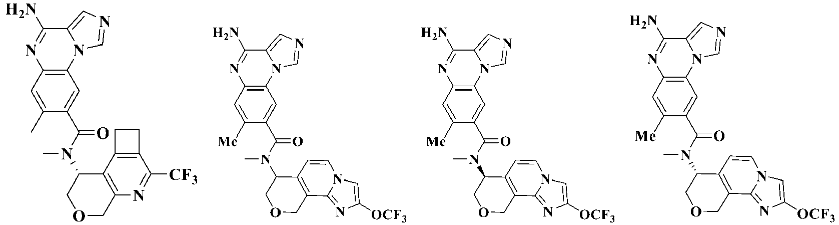 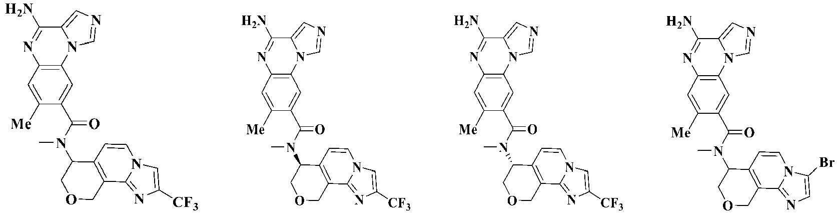 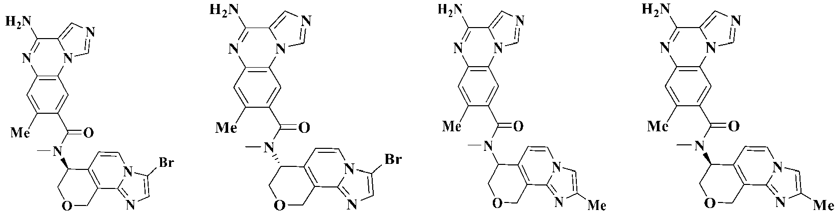 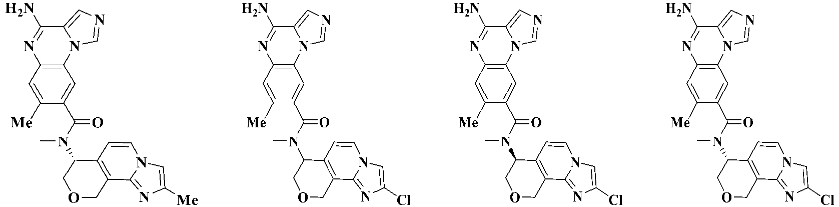 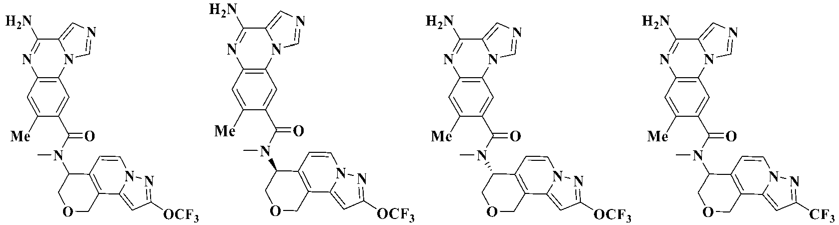 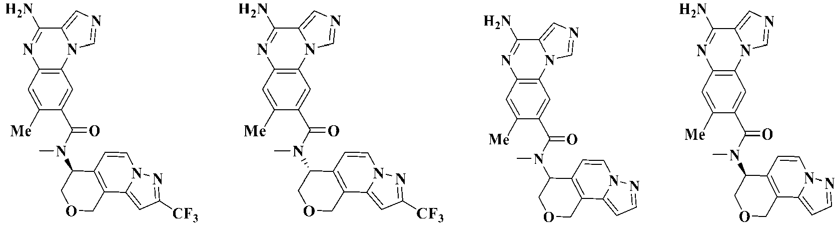 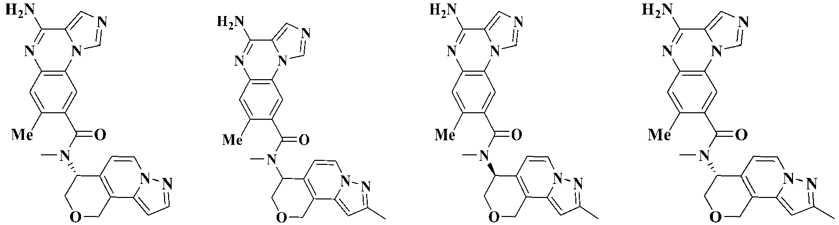 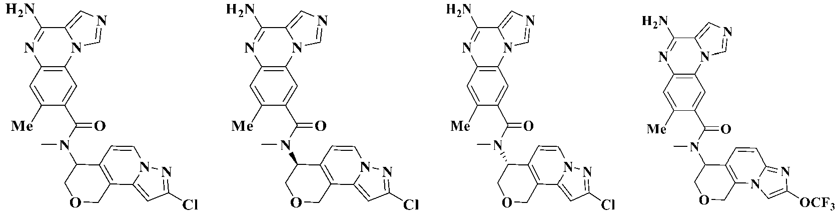 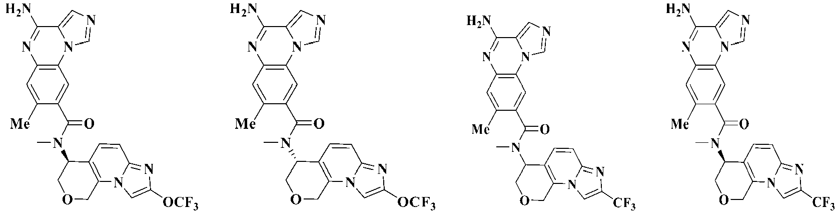 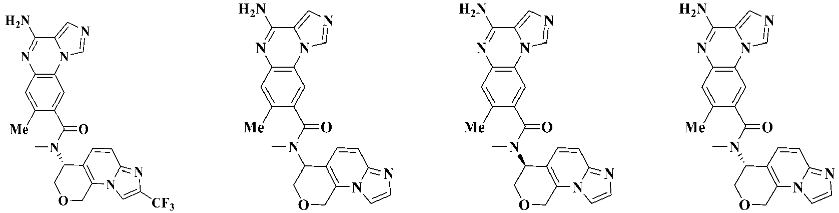 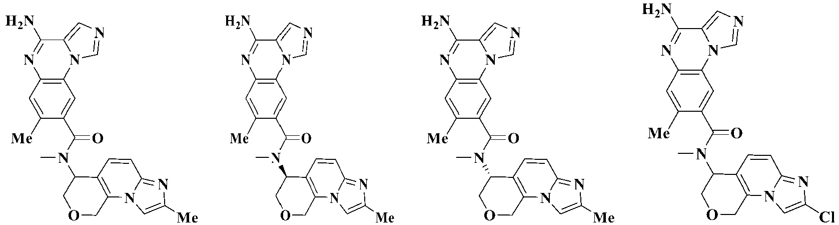 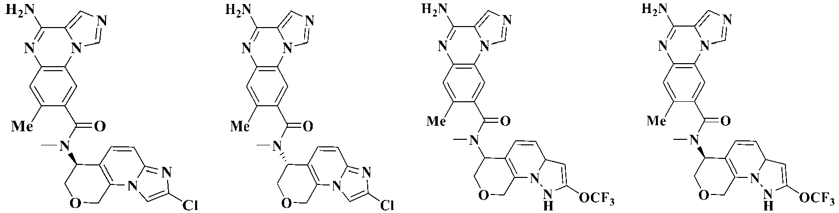 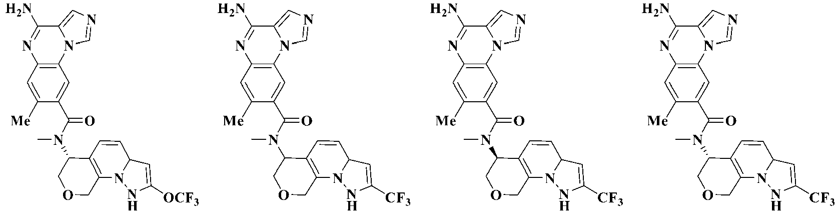 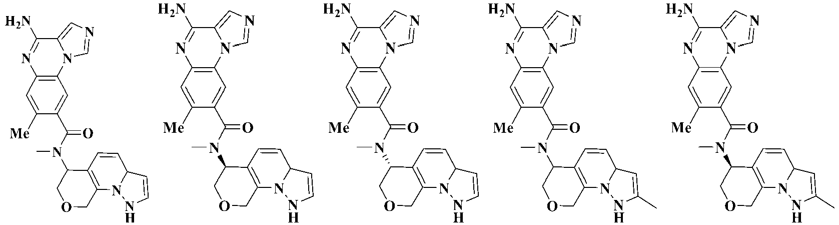 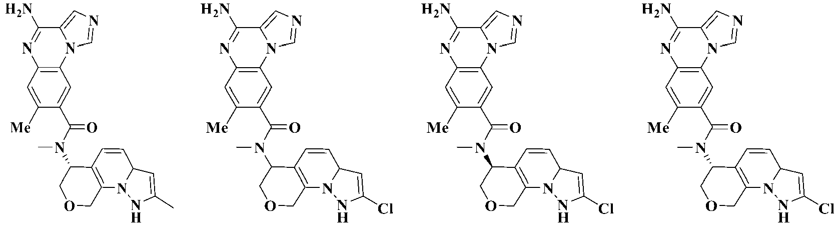 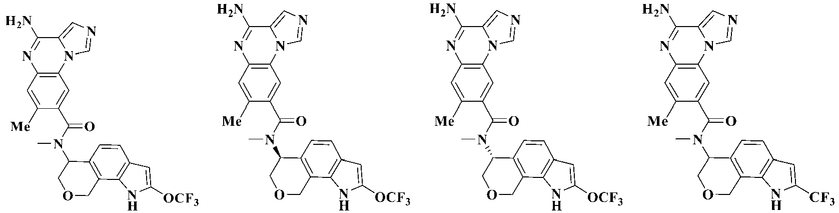 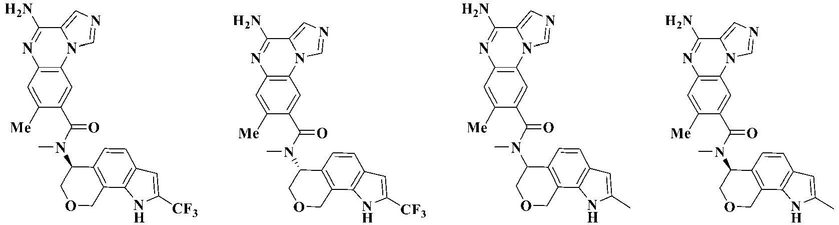 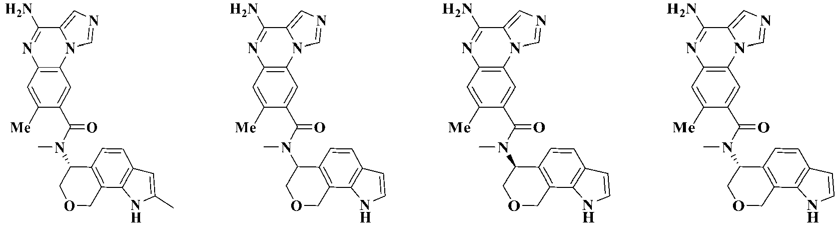 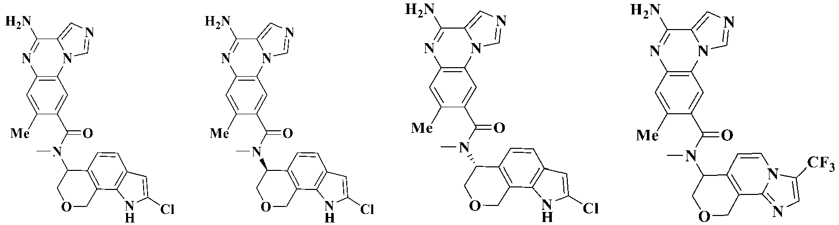 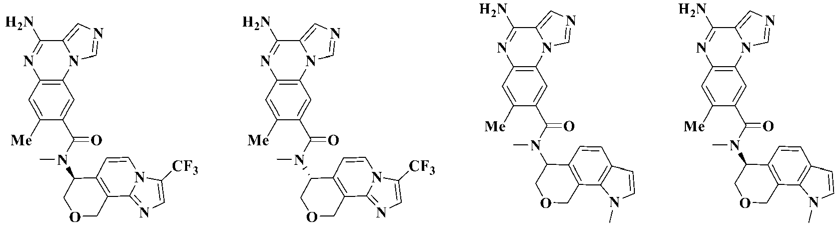 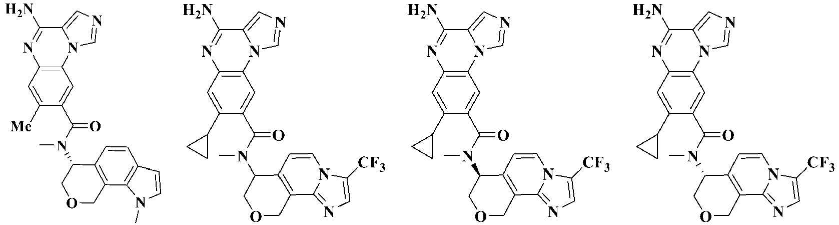 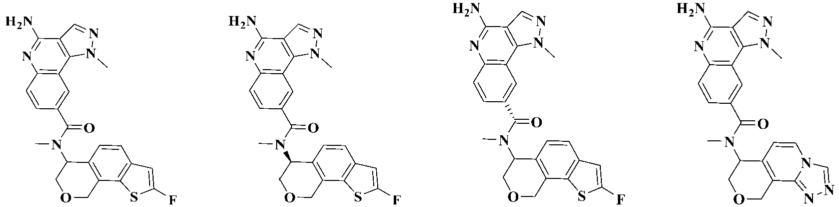 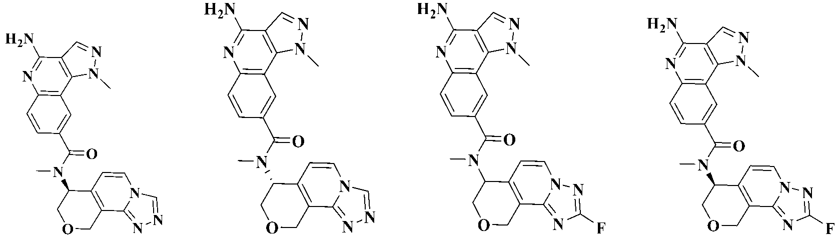 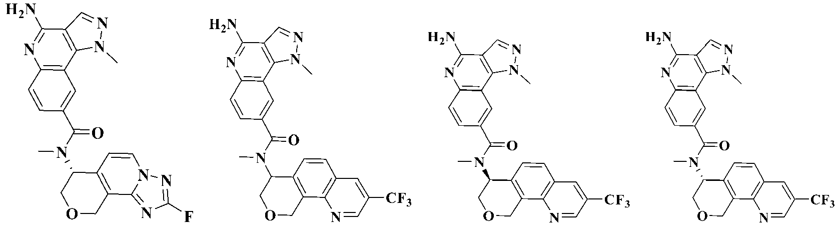 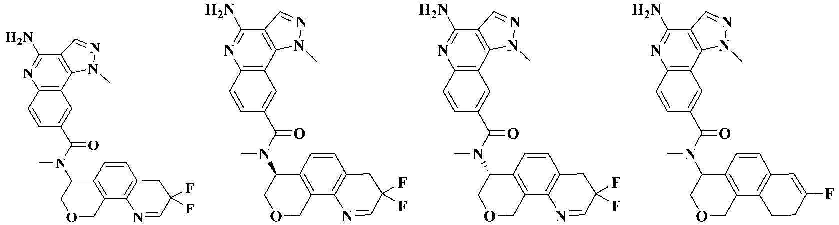 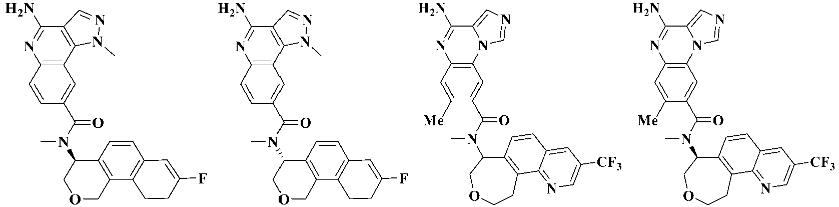 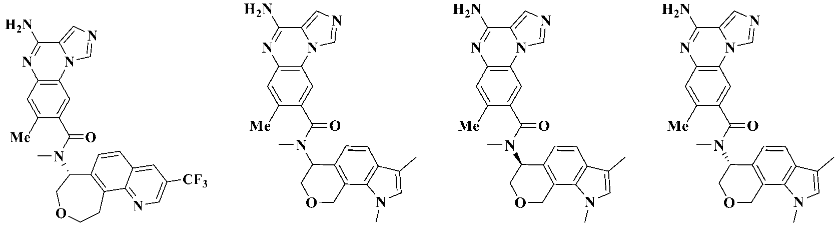 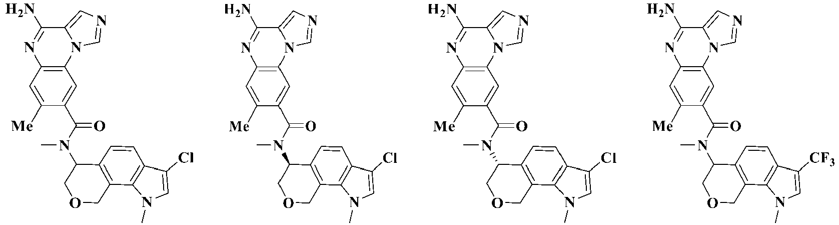 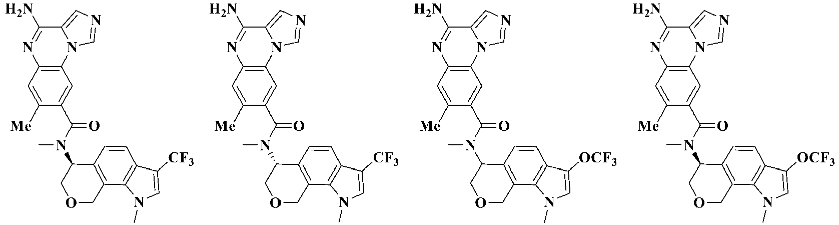 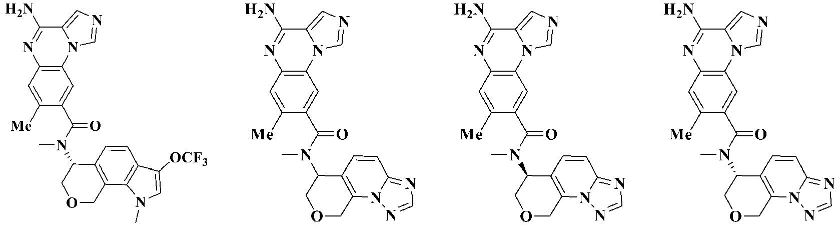 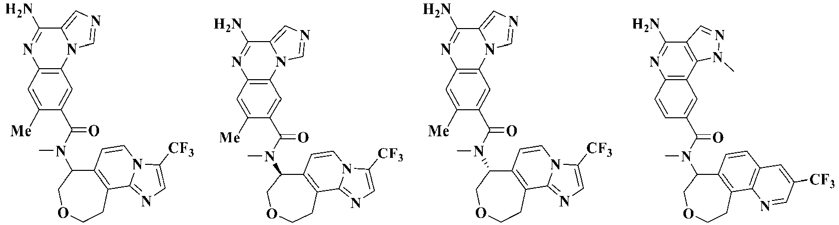 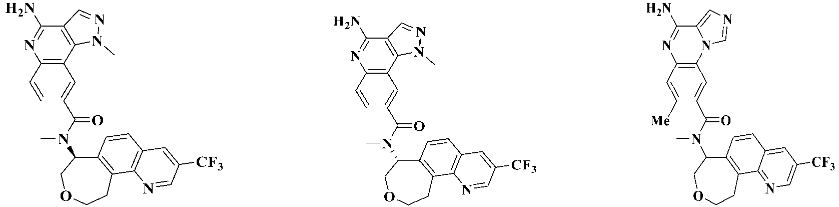 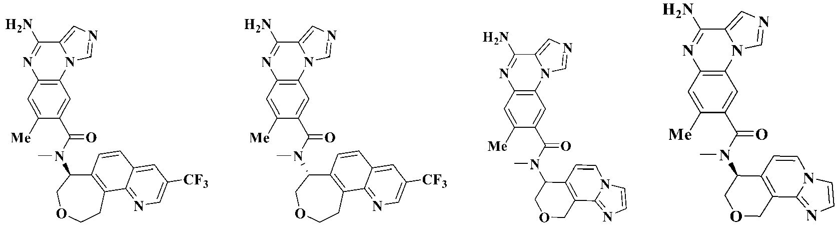 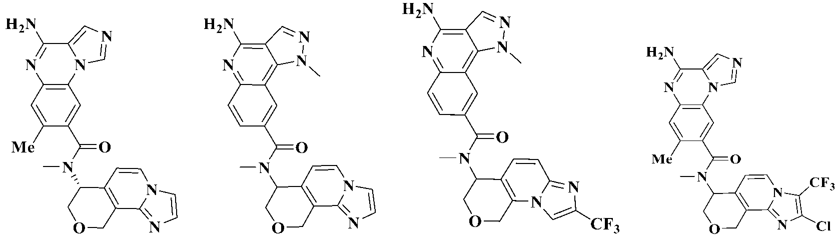 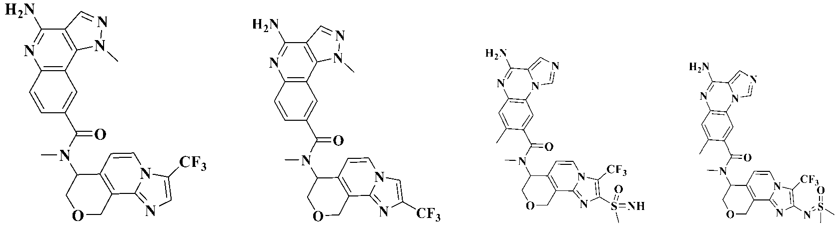 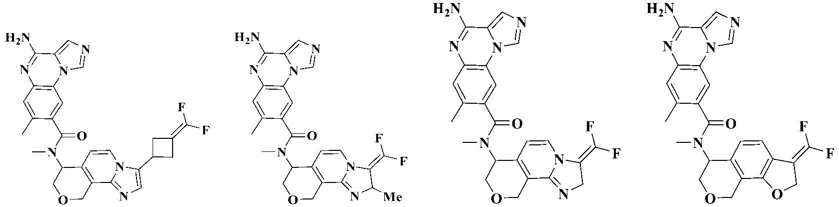 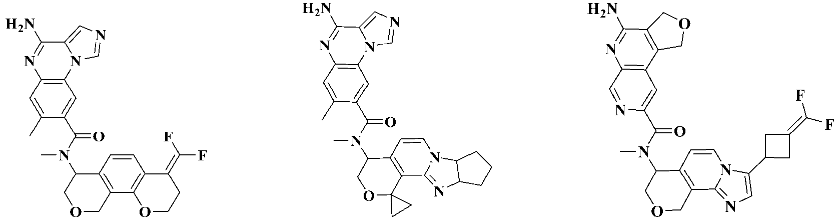 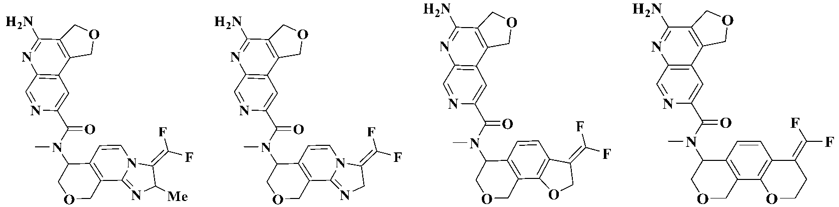 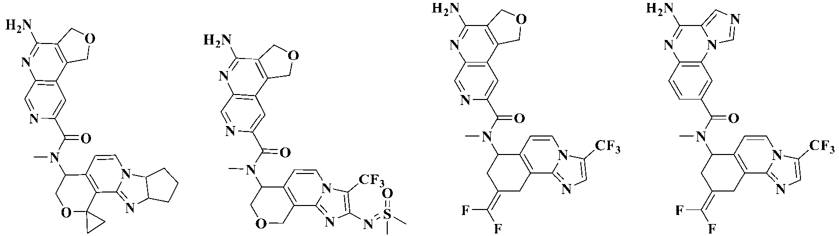 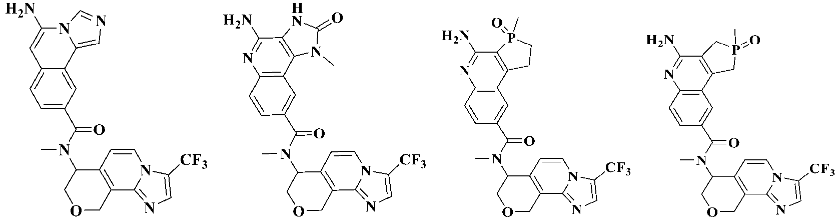 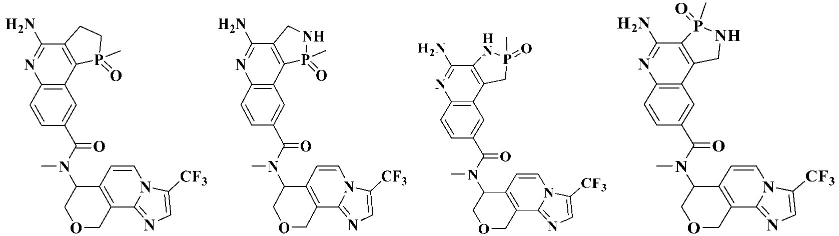 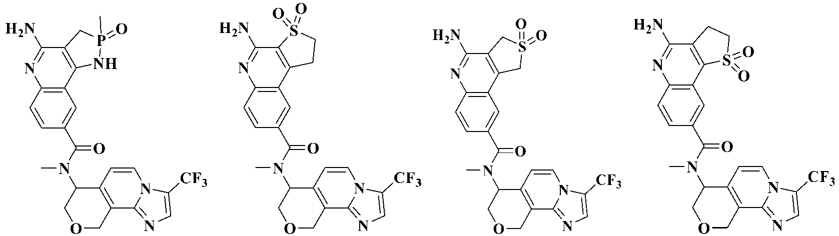 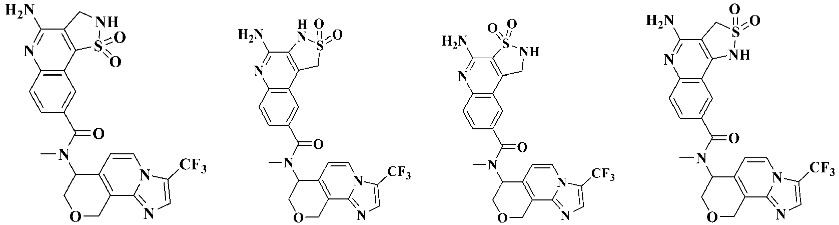 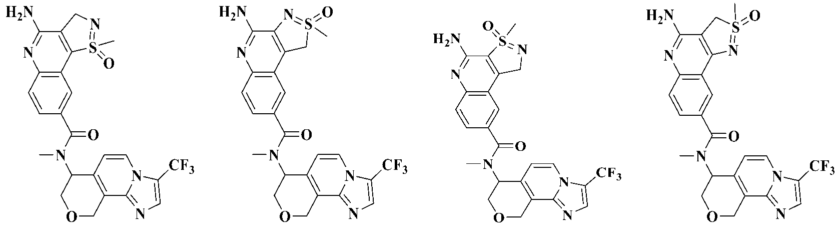 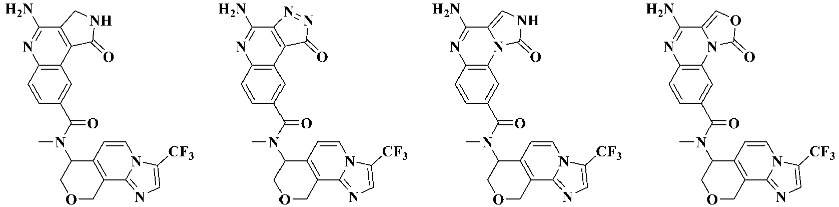 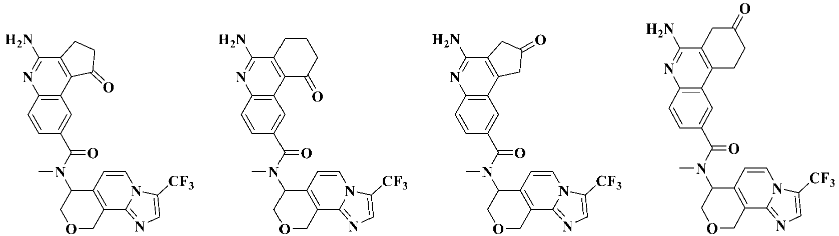 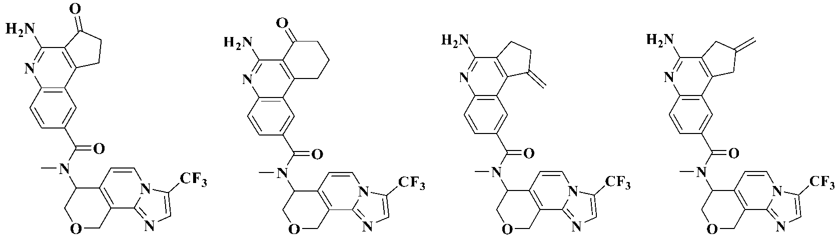 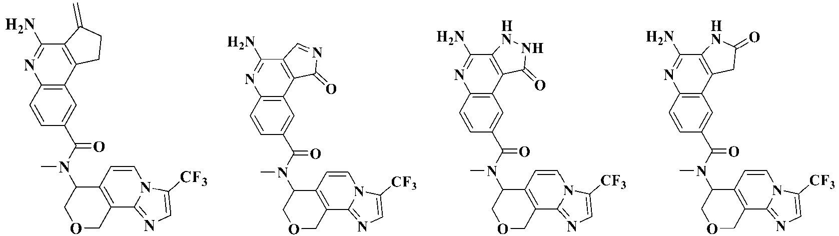 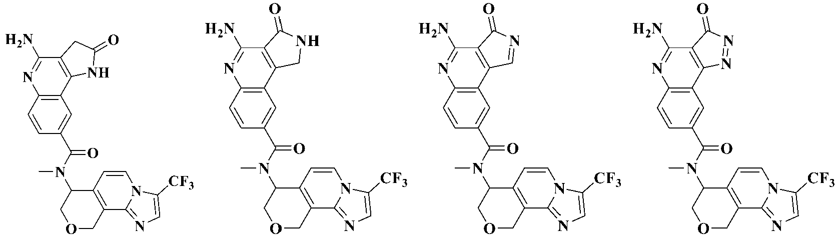 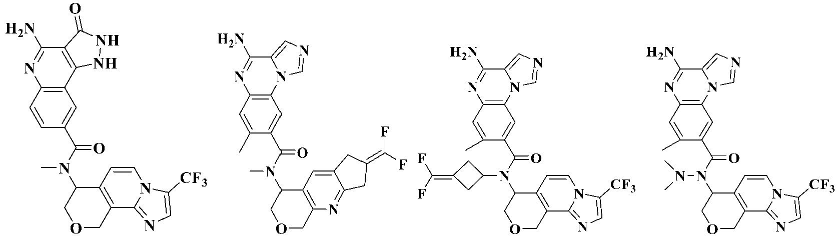 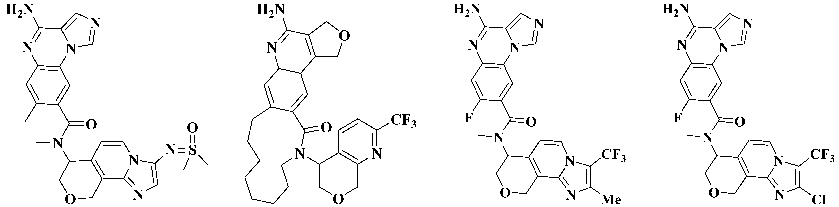 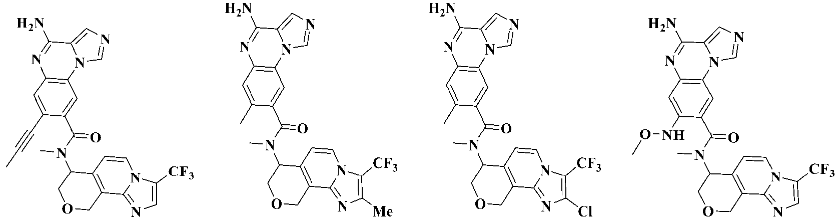 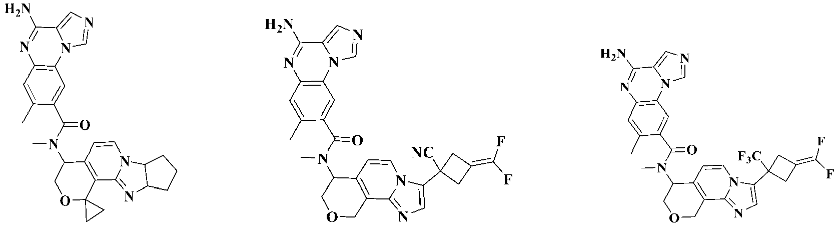 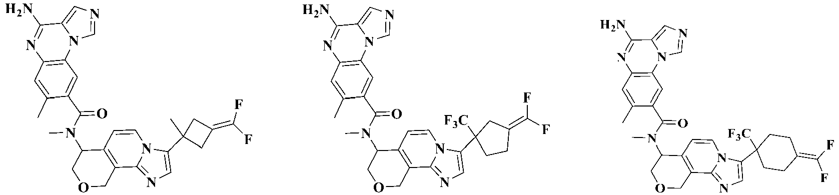 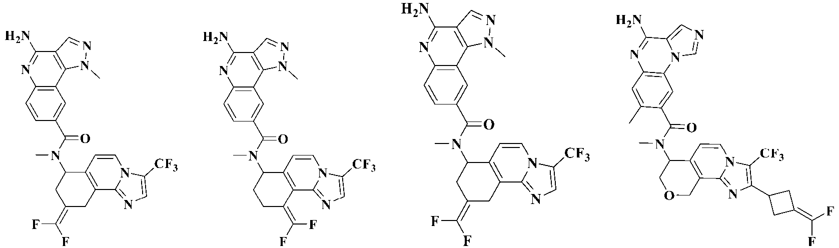 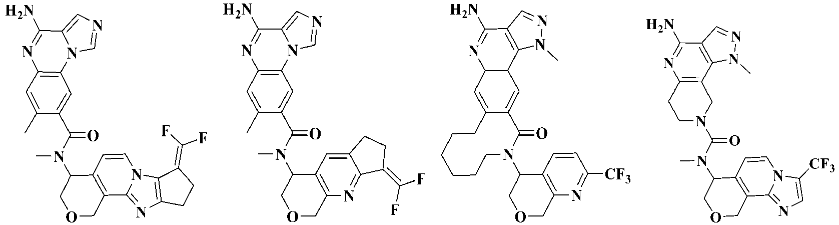 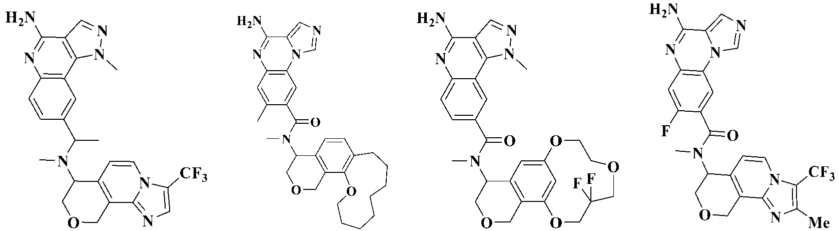 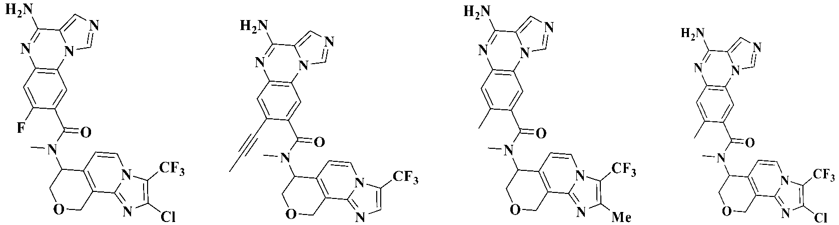 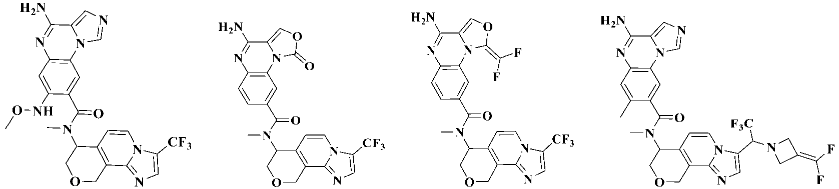 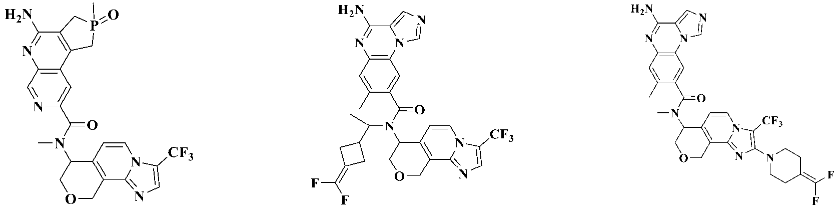 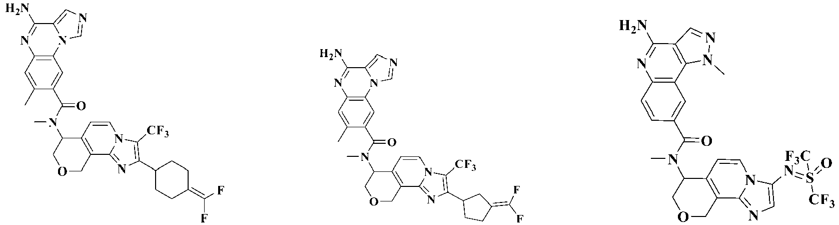 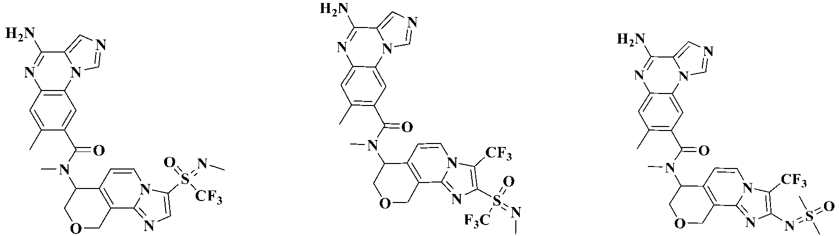 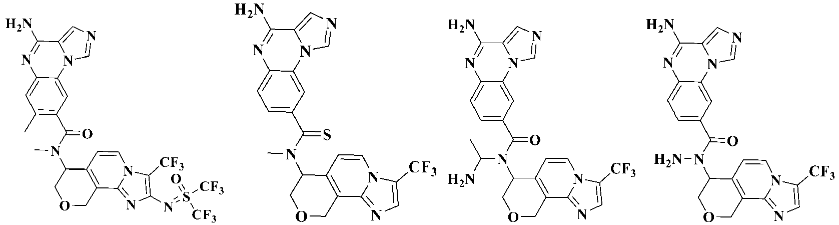 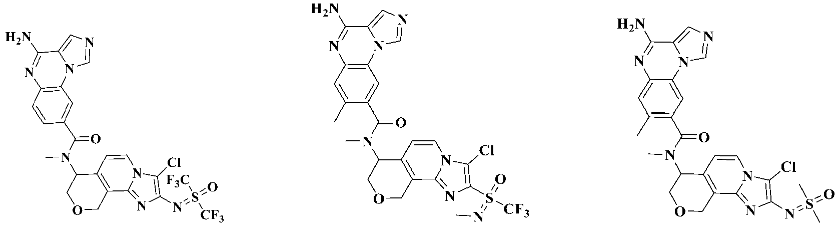 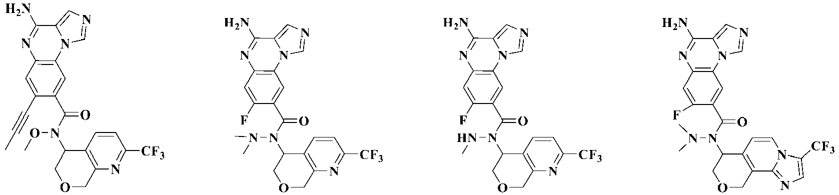 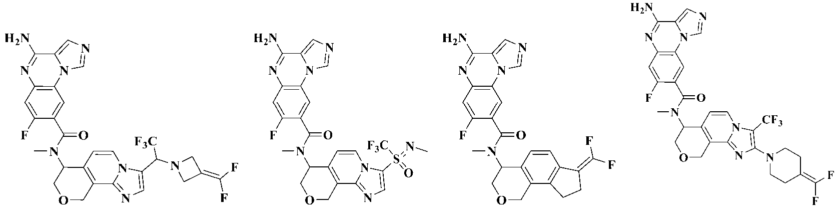 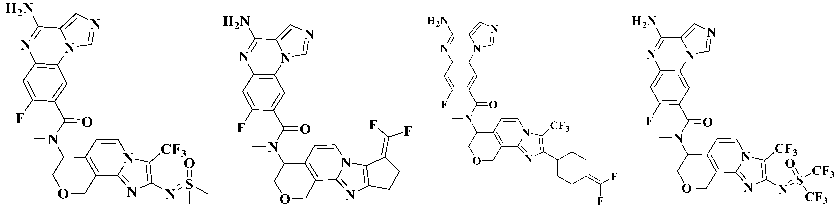 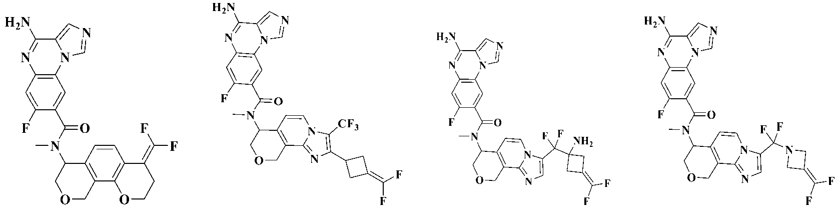 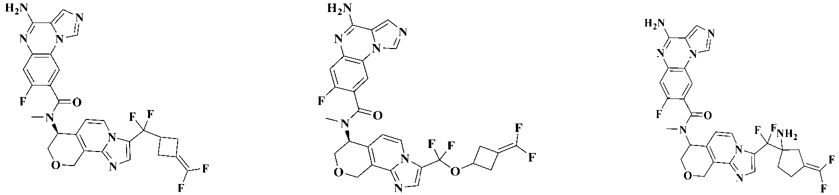 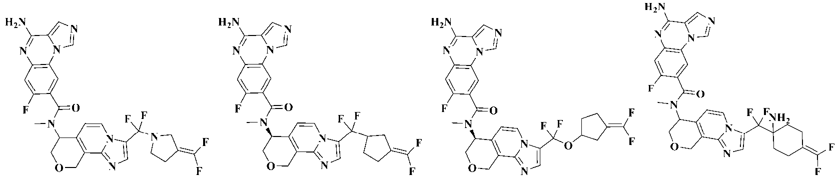 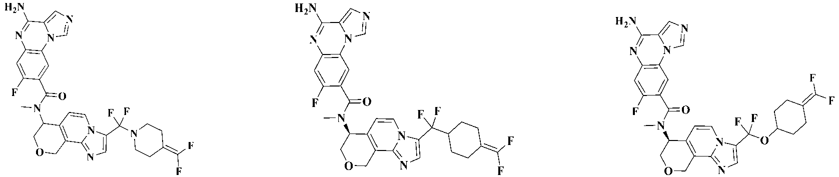 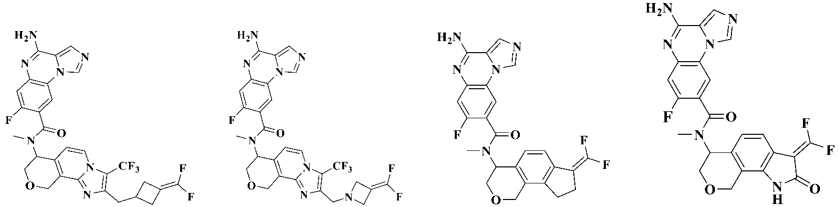 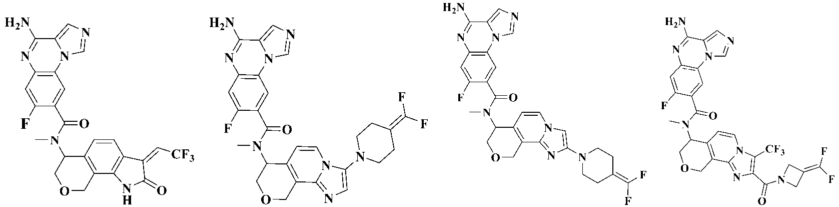 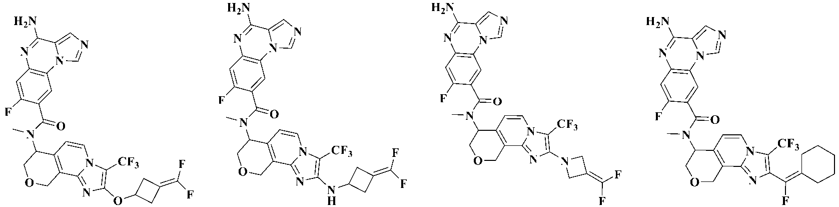 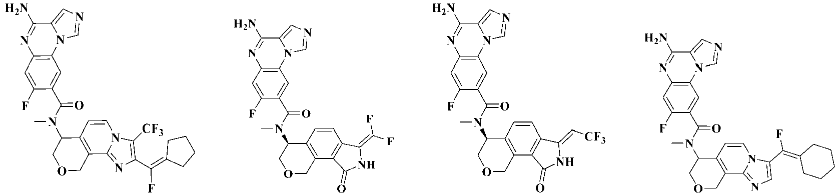 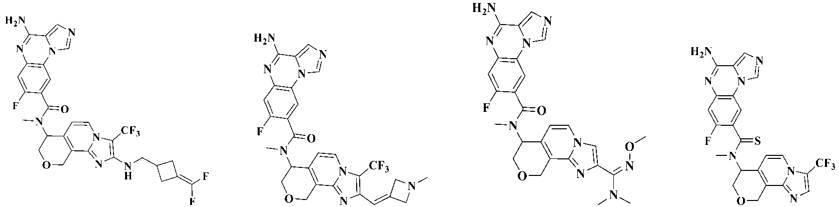 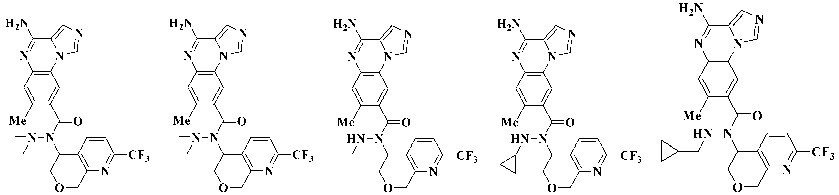 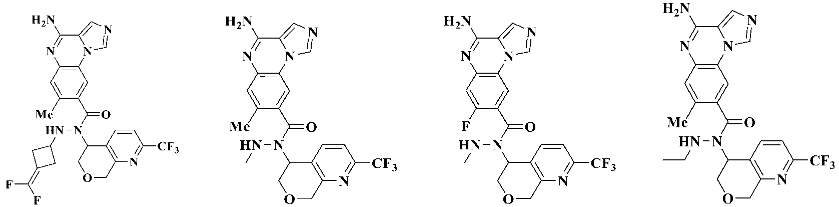 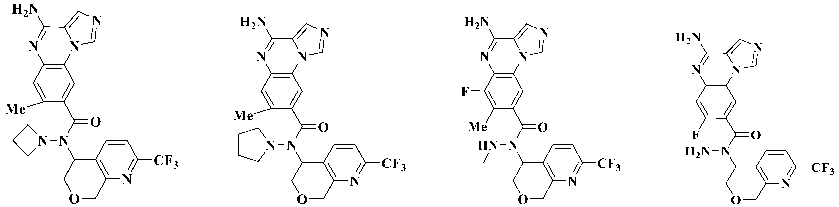 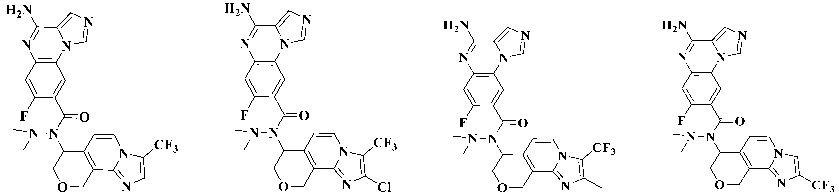 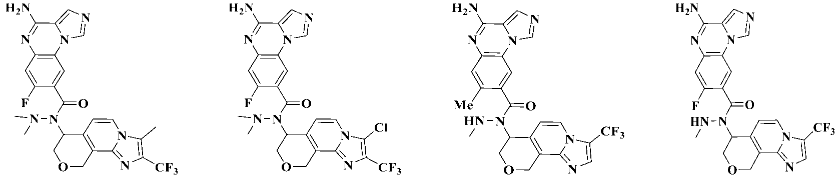 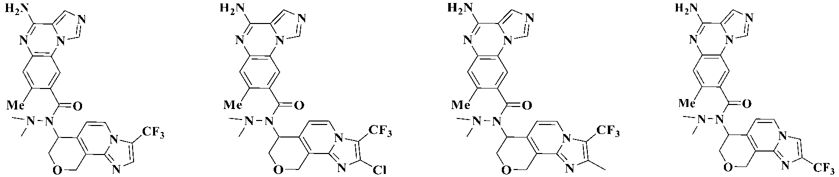 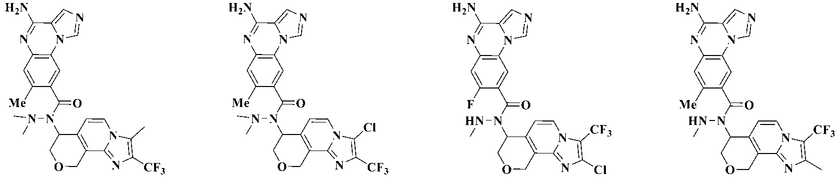 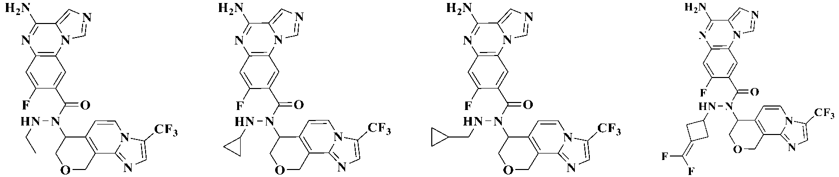 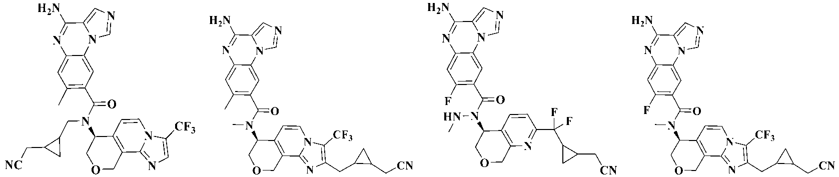 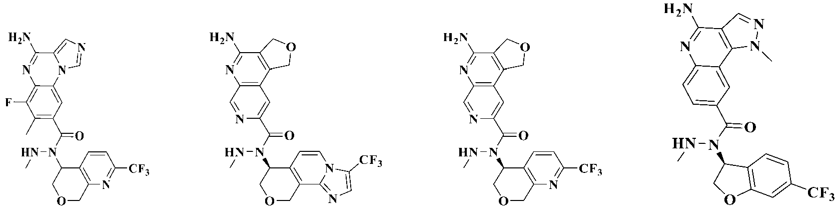 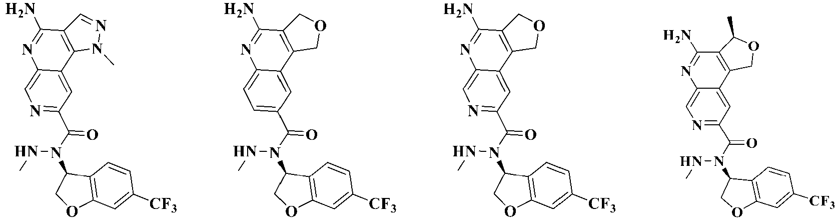 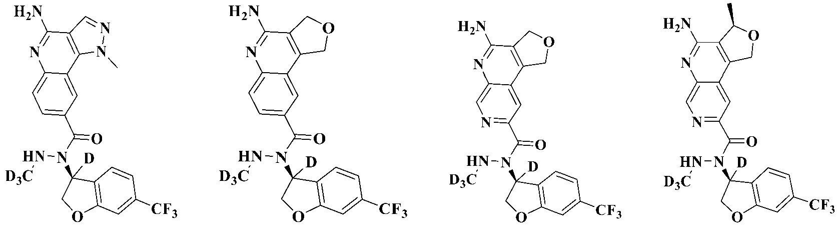 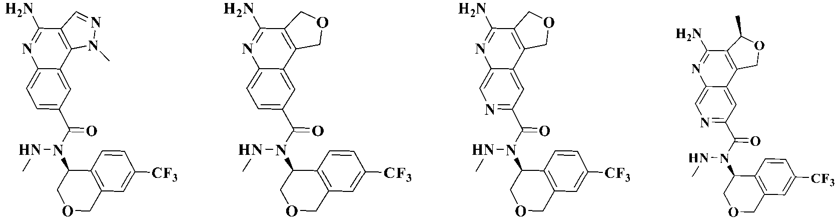 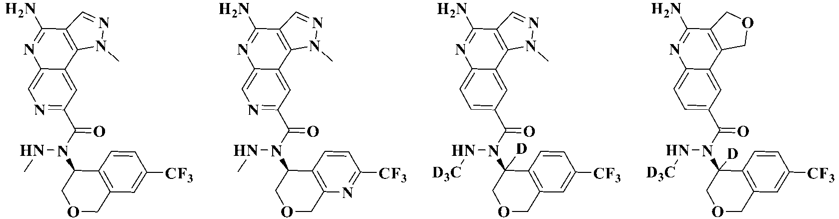 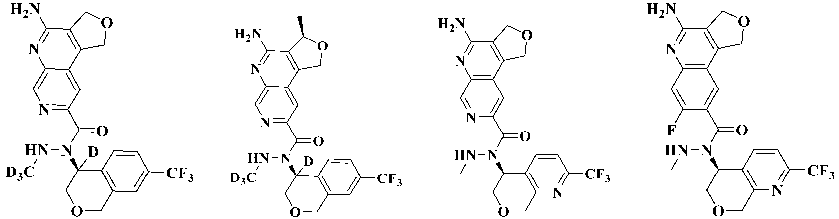 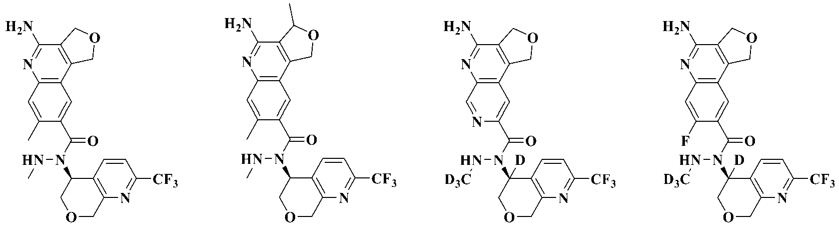 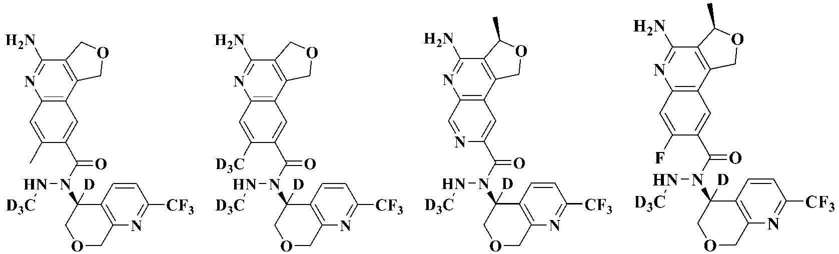 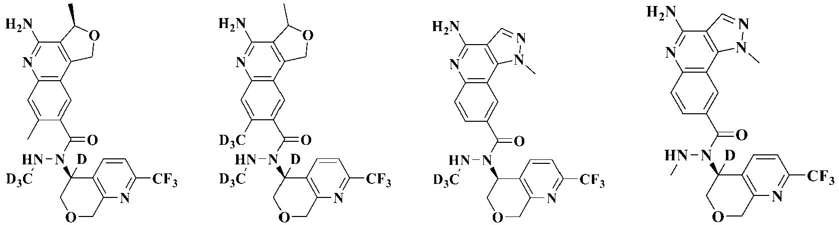 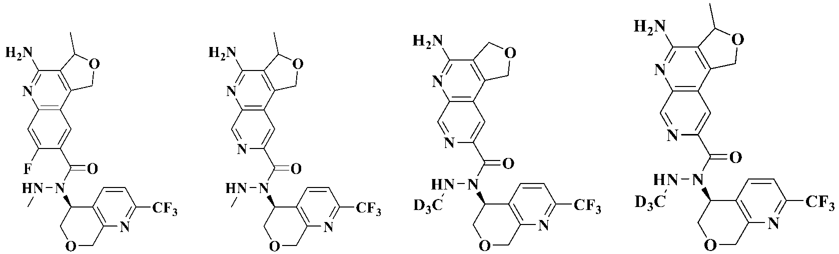 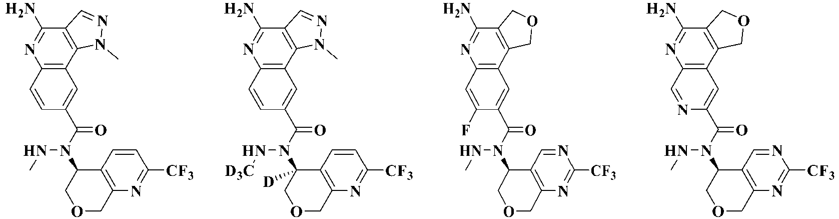 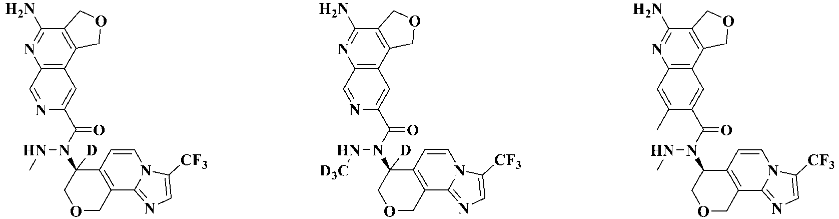 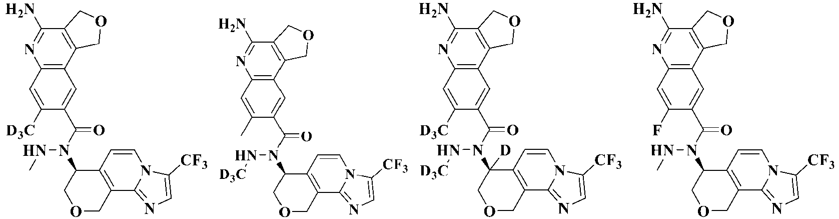 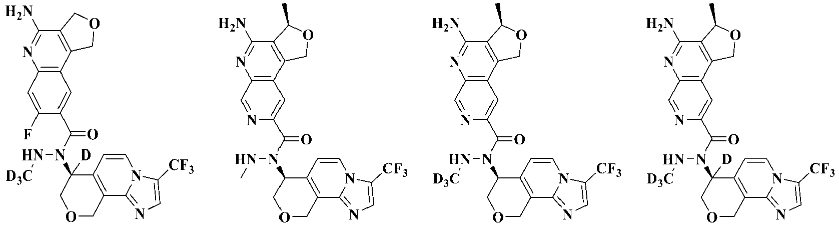 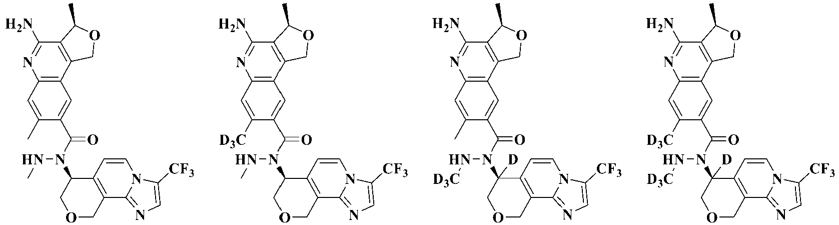 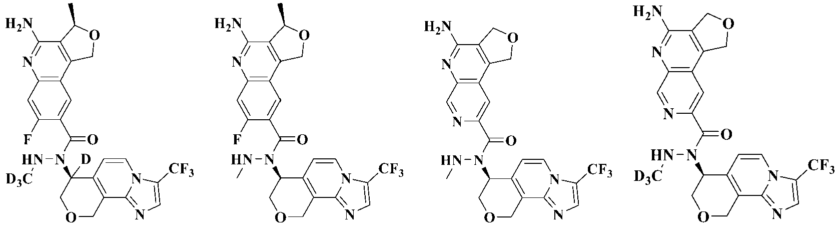 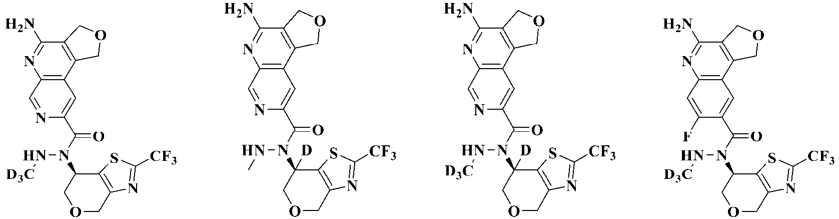 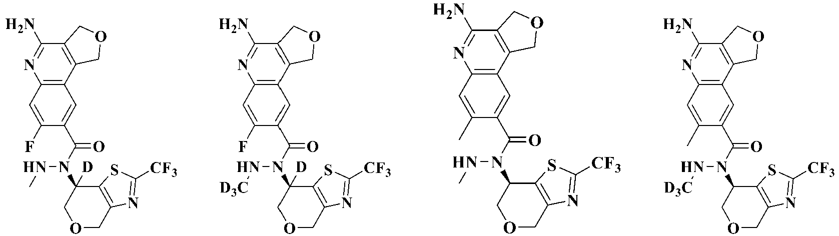 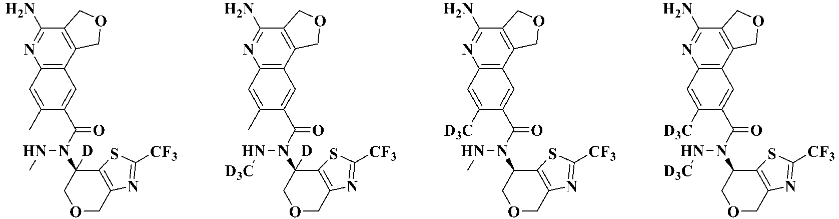 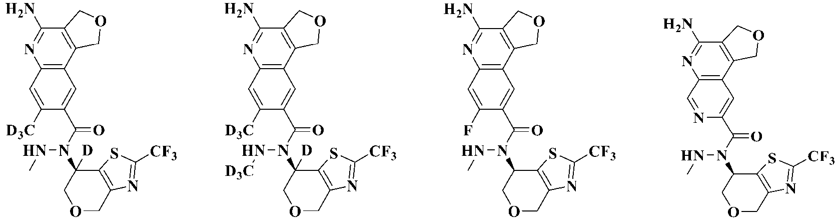 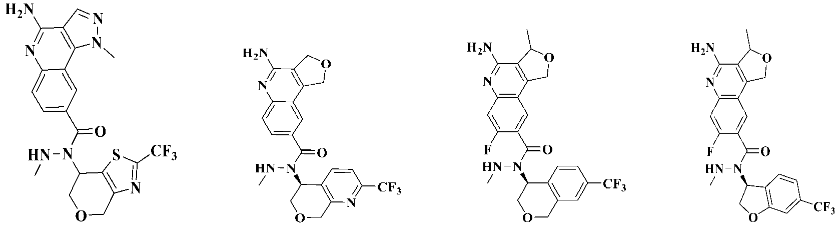 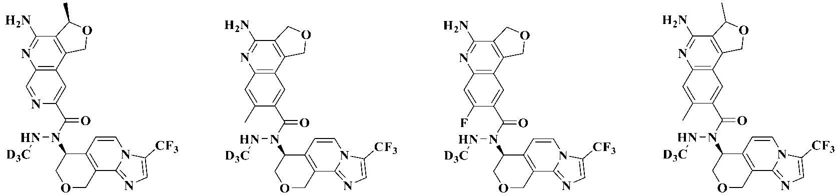 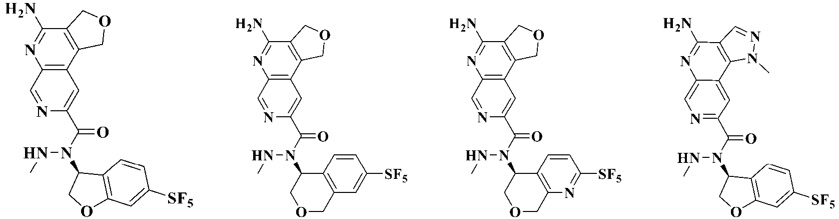 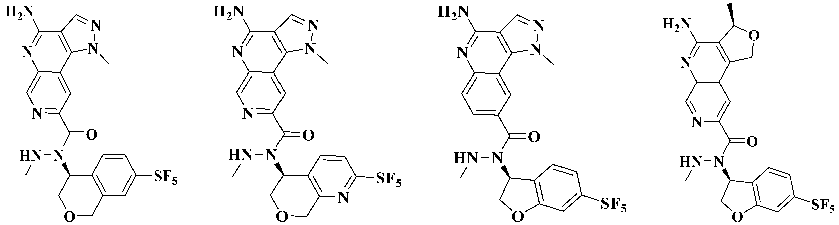 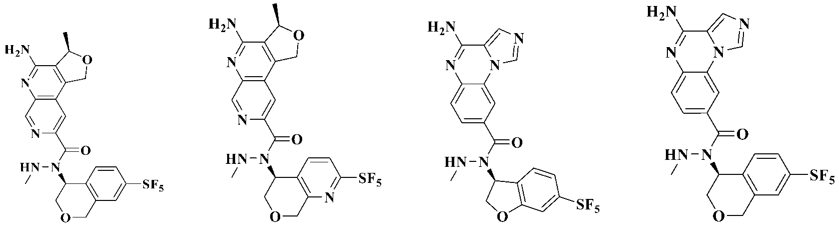 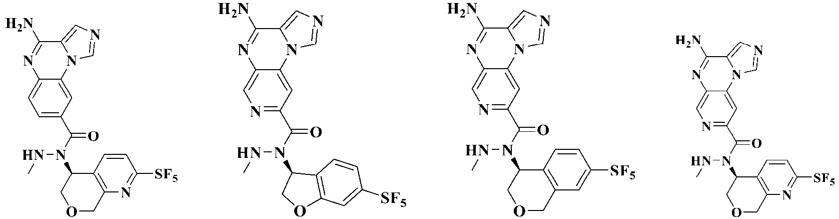 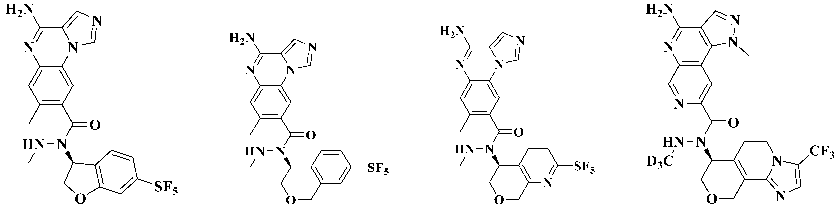 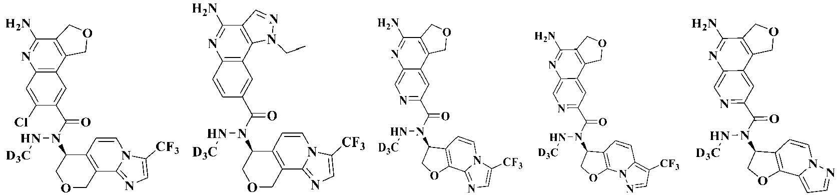 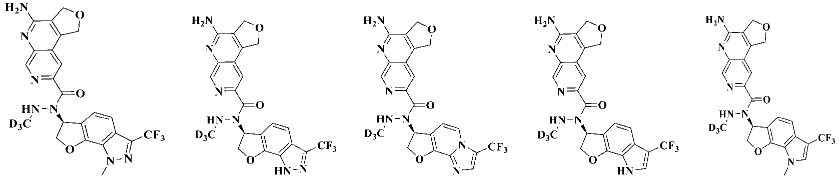 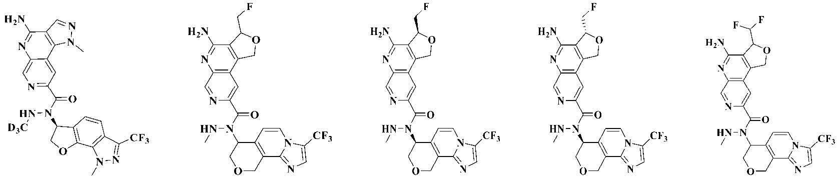 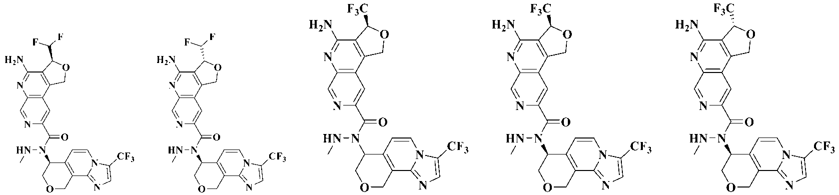 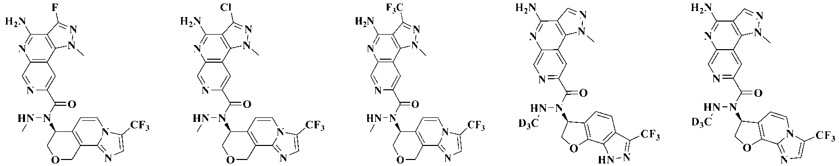 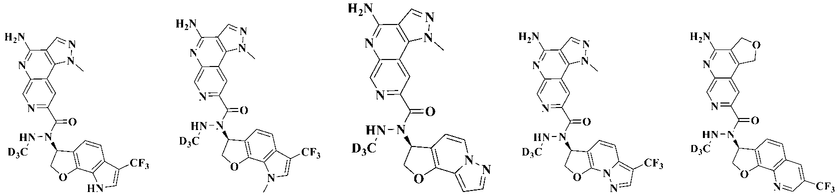 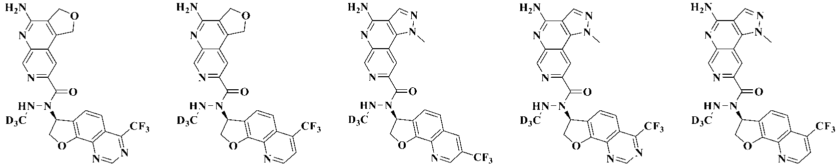 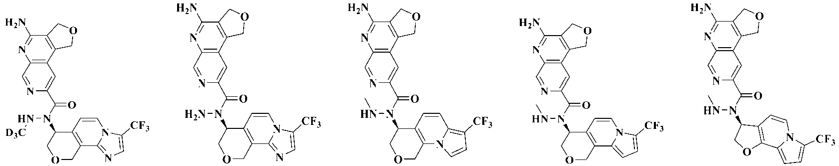 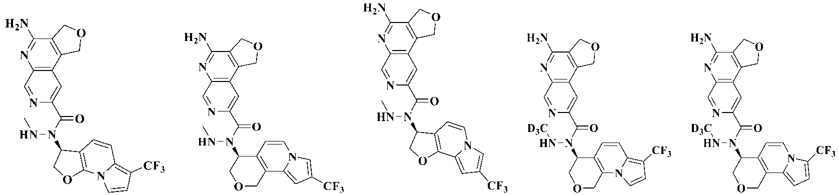 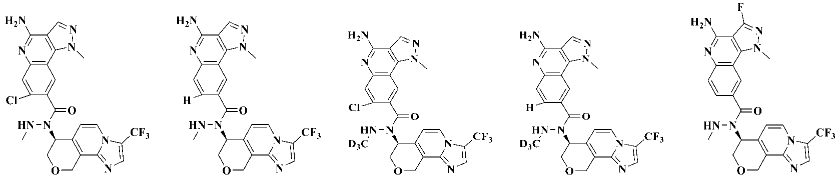 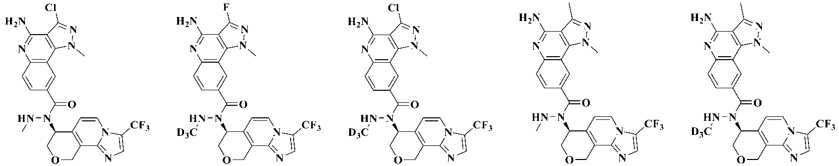 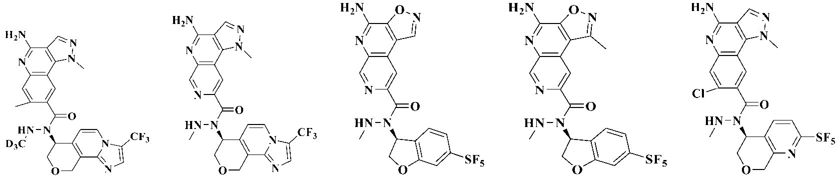 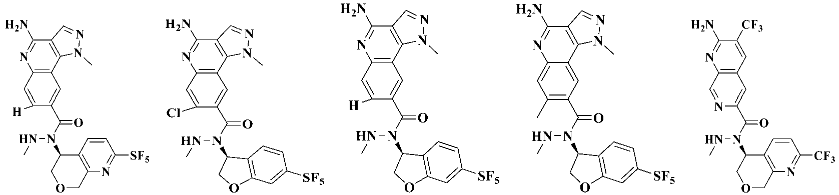 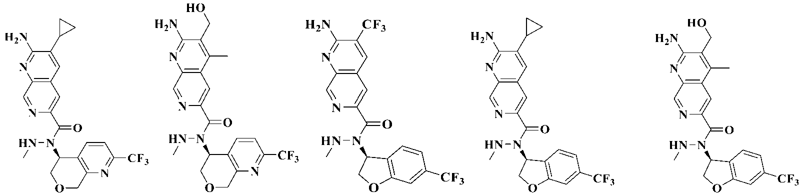 or

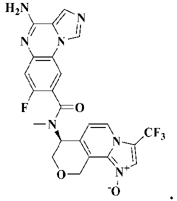

14. A compound represented by general formula (VII), or a stereoisomer or a pharmaceutically acceptable salt thereof: 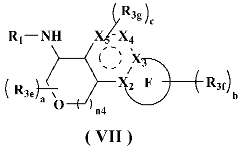 preferably, a compound represented by general formula (VII-1), or a stereoisomer or a pharmaceutically acceptable salt thereof: 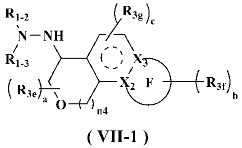
n4 is selected from 0 or 1;
X₂, X₃, R₁₋₂, R₁₋₃, R₃ₑ, R_{3f}, R_{3g}, a, b, c and n4 are as defined in claim 3 or 4.

15. The compound, or the stereoisomer or the pharmaceutically acceptable salt thereof according to claim 13, **characterised in that** the compound has the following structure:

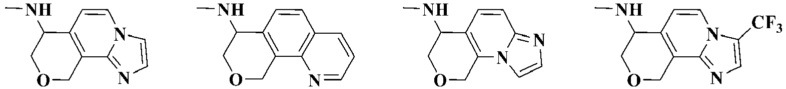

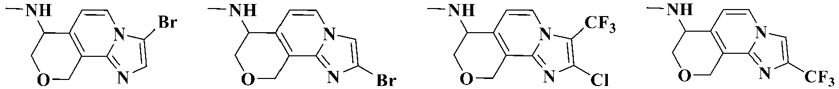

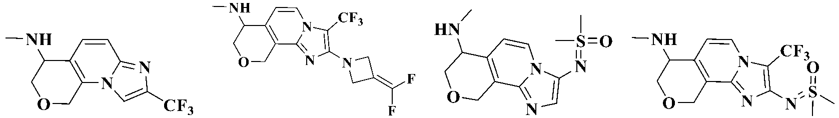

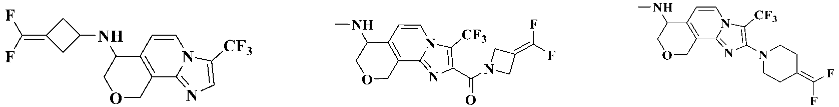

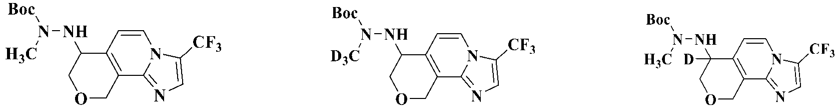

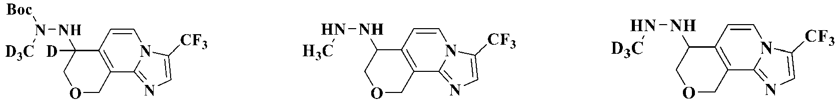

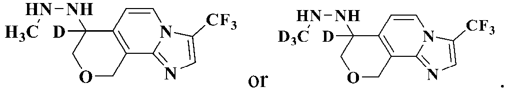

16. A method for preparing a compound represented by general formula (III), or a stereoisomer or a pharmaceutically acceptable salt thereof, **characterised in that** 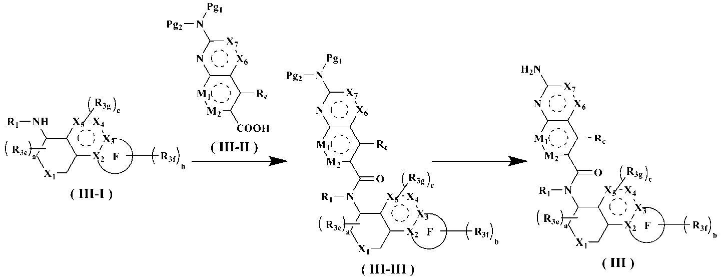
a compound represented by general formula (III-I) and a compound represented by general formula (III-II) are reacted in the presence of a condensing agent and a base to obtain a compound represented by general formula (III-III), which is further deprotected to obtain the compound represented by general formula (III);
preferably, the method is a method for preparing a compound represented by general formula (IV), or a stereoisomer or a pharmaceutically acceptable salt thereof: 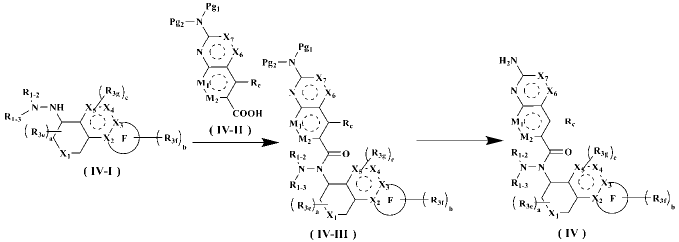
a compound represented by general formula (IV-I) and a compound represented by general formula (IV-II) are reacted in the presence of a condensing agent and a base to obtain a compound represented by general formula (IV-III), which is further deprotected to obtain the compound represented by general formula (IV);
more preferably, the method is a method for preparing a compound represented by general formula (V), or a stereoisomer or a pharmaceutically acceptable salt thereof: 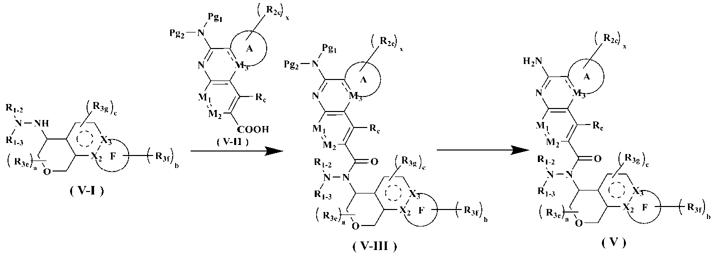
a compound represented by general formula (V-I) and a compound represented by general formula (V-II) are reacted in the presence of a condensing agent and a base to obtain a compound represented by general formula (V-III), which is further deprotected to obtain the compound represented by general formula (V);
Pg₁ is selected from hydrogen, allyloxycarbonyl, trifluoroacetyl, tert-butylsulphinyl, 2,4-dimethoxybenzyl, 3,5-dimethoxybenzyl, nitrobenzenesulphonyl, trityl, fluorenylmethoxycarbonyl, 9-fluorenylmethoxycarbonyl, benzyl, p-toluenesulphonyl, p-methoxybenzyl, formate, acetyl, benzyloxycarbonyl, phthaloyl, tert-butyloxycarbonyl, benzyl or p-methoxyphenyl;
Pg₂ is selected from hydrogen, allyloxycarbonyl, trifluoroacetyl, tert-butylsulphinyl, 2,4-dimethoxybenzyl, 3,5-dimethoxybenzyl, nitrobenzenesulphonyl, trityl, fluorenylmethoxycarbonyl, 9-fluorenylmethoxycarbonyl, benzyl, p-toluenesulphonyl, p-methoxybenzyl, formate, acetyl, benzyloxycarbonyl, phthaloyl, tert-butyloxycarbonyl, benzyl or p-methoxyphenyl;
M₁, M₂, M₃, Rₐ, R_{b} and R_{c} are as defined in claim 1;
X₂, X₃, R₁₋₂, R₁₋₃, R_{2c}, R₃ₑ, R_{3f}, R_{3g}, a, b, c, x and n4 are as defined in claim 3 or 4.

17. A pharmaceutical composition, **characterised by** comprising a therapeutically effective dose of the compound, or the stereoisomer or the pharmaceutically acceptable salt thereof according to any one of claims 1-13, and one or more pharmaceutically acceptable carriers, diluents, or excipients.

18. Use of the compound, or the stereoisomer or the pharmaceutically acceptable salt thereof according to any one of claims 1-13, or the pharmaceutical composition according to claim 17 in the preparation of a PRMT5 inhibitor drug.

19. Use of the compound, or the stereoisomer or the pharmaceutically acceptable salt thereof according to any one of claims 1-13, or the pharmaceutical composition according to claim 17 in the preparation of a drug for the treatment of cancer.

20. The use according to claim 19, **characterised in that** the cancer is selected from lung cancer, hepatocellular carcinoma, breast cancer, skin cancer, bladder cancer, liver cancer, pancreatic cancer, head and neck cancer, glioma, glioblastoma, oesophageal cancer, pancreatic cancer, mesothelioma, melanoma, astrocytoma, undifferentiated pleomorphic sarcoma, leukaemia, gastric adenocarcinoma, myxofibrosarcoma, cholangiosarcoma, brain cancer, gastric cancer, renal cancer, endometrial cancer, ovarian tumour, prostate cancer, diffuse large B-cell lymphoma, non-Hodgkin lymphoma, urinary tract cancer, soft tissue cancer, pleural cancer, large intestine cancer, colorectal cancer, biliary tract cancer or cholangiocarcinoma; the lung cancer is selected from non-small cell lung cancer, lung squamous cell carcinoma or lung adenocarcinoma; the oesophageal cancer is selected from oesophageal squamous cell carcinoma or oesophageal adenocarcinoma.
